# EUROPEAN PATENT APPLICATION

(11) **EP 4 438 594 A1**
(43) Date of publication of application: **02.10.2024**
(21) Application number: 22887172.9
(22) Date of filing: 28.10.2022
(51) Int. Cl.: C07D 205/12, A61K 31/397, A61K 31/4025, A61K 31/4035, A61K 31/416, A61K 31/4178, A61K 31/4184, A61K 31/429, A61K 31/437, A61K 31/4375, A61K 31/4427, A61K 31/4436, A61K 31/4725, A61K 31/4985, A61K 31/502, A61K 31/506, A61K 31/5355, A61K 31/5377, A61K 31/541, A61K 31/55, A61K 31/551

(54) **NOVEL SPIRO COMPOUND**

(30) Priority: 29.10.2021 JP 2021177933
(71) Applicant: Mitsubishi Tanabe Pharma Corporation, Osaka-shi Osaka 541-8505 (JP)
(72) Inventor: HAYASHI Norimitsu, Osaka-shi, Osaka 541-8505 (JP); IKUBO Masaya, Osaka-shi, Osaka 541-8505 (JP); NAKAO Akira, Osaka-shi, Osaka 541-8505 (JP); OGATA Shingo, Osaka-shi, Osaka 541-8505 (JP); NAGAOKA Minami, Osaka-shi, Osaka 541-8505 (JP); FUKUNAGA Kenji, Osaka-shi, Osaka 541-8505 (JP); KANNO Rentarou, Osaka-shi, Osaka 541-8505 (JP); YAMADA Takahiro, Osaka-shi, Osaka 541-8505 (JP)
(74) Representative: Ter Meer Steinmeister & Partner
(86) International application number: PCT/JP2022/040370
(87) International publication number: WO 2023/074847

(57) **Abstract**

The present invention provides a novel spiro compound or a pharmacologically acceptable salt thereof that has a TRH-DE inhibitory activity and is useful for preventing or treating various diseases associated with TRH and/or symptoms associated therewith, a method for producing the same, a use thereof, and a pharmaceutical composition containing the compound or the pharmacologically acceptable salt thereof as an active ingredient.

There is provided a compound represented by the following Formula [I]: 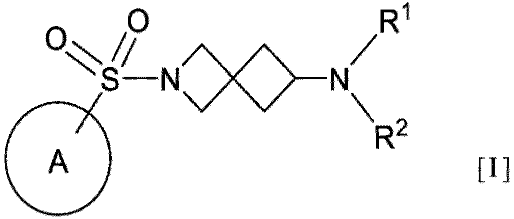 wherein,
a ring A represents an aryl group which may be substituted, a heteroaryl group which may be substituted, or an aliphatic heterocyclyl group which may be substituted, and
R¹ and R² represent, indipendently, an alkyl group which may be substituted, a cycloalkyl group which may be substituted, an alkenyl group which may be substituted, a cycloalkenyl group which may be substituted, an alkynyl group which may be substituted, an aryl group which may be substituted, a heteroaryl group which may be substituted, an aliphatic heterocyclyl group which may be substituted and may have a double bond in a part of a ring, an amino group which may be substituted, an alkoxy group which may be substituted, or an alkylthio group which may be substituted, or a group in which R¹ and R² together form a ring,
or a pharmacologically acceptable salt thereof.

## Description

### Technical Field

The present invention relates to a novel spiro compound that has an inhibitory action to a thyrotropin-releasing hormone (hereinafter, referred to as "TRH")-degrading ectoenzyme (hereinafter, referred to as "TRH-DE", or "PAP-II" or "PP-II", which is also known as pyroglutamyl aminopeptidase II), which is one of TRH-releasing hormones, and is useful for prevention and/or treatment of various diseases and/or symptoms associated therewith that can be ameliorated by increasing a central TRH concentration.

### Background Art

TRH is a peptide consisting of three amino acid residues of pGlu-His-Pro-NH2 isolated as a hypothalamic hormone. TRH develops an action by binding to a TRH receptor present in a cell membrane of a target cell while undergoing quantitative control at each stage of synthesis/secretion/degradation. In peripheral tissues, TRH mainly promotes synthesis and secretion of a thyroid-stimulating hormone (hereinafter, referred to as "TSH") and prolactin, which are anterior pituitary hormones, and contributes to an increase in amounts of respiration/energy production and mammary gland development. On the other hand, TRH also has a role in the central tissue, and is known to exhibit various physiological activities such as acting as a central activator by regulating the production of neurotransmitters in the nervous system. Therefore, quantitative control of TRH has attracted attention as a method for ameliorating various diseases associated with TRH and/or symptoms associated therewith.

TRH-DE, which is a TRH-degrading enzyme, is a zinc-metalloprotease that hydrolyzes a pyroglutamyl-histidyl peptide bond of TRH, and is a membrane binding enzyme composed of a large extracellular domain including a catalytic site, a cell transmembrane site, and a small intracellular domain. TRH-DE is highly expressed in the brain and therefore plays a major role in the degradation of TRH in neuronal synaptic clefts. On the other hand, examples of the enzyme involved in the degradation of TRH include pyroglutamyl-peptidase I (EC 3.4.19.3, hereinafter also referred to as "PP I") and prolyl oligopeptidase (EC 3.4.21.26, hereinafter also referred to as "POP") in addition to TRH-DE. PP I and POP are also expressed in various peripheral tissues (Non Patent Literatures 1 and 2), and target a plurality of substrates other than TRH, but TRH-DE can selectively control the degradation of TRH (Non Patent Literature 3). In evaluation of a TRH degradation activity using a rat brain homogenate, it has been shown that a PP I specific inhibitor does not have a TRH degradation inhibitory action, but a TRH-DE specific inhibitor has a TRH degradation inhibitory action (Non Patent Literature 4).

Hitherto, as a compound having a TRH-DE inhibitory activity, for example, a nucleic acid having a sequence partially or completely identical to that of RNA of TRH-DE (Patent Literature 1), TRH-like peptide derivatives (Patent Literatures 2 and 3), and the like are known.

### PRIOR ART DOCUMENTS

### Patent Literatures

Patent Literature 1: WO 2007/113784 A
Patent Literature 2: WO 01/60843 A
Patent Literature 3: WO 2006/038206 A

### Non Patent Literatures

Non Patent Literature 1: European Journal of Biochemistry (Eur. J. Biochem.), 1999; 265: p.415-422
Non Patent Literature 2: Peptides, 2003; 24: p.1367-1372
Non Patent Literature 3: Neuropharmacology, 2007; 52: p.1472-1481
Non Patent Literature 4: Biological and Pharmaceutical Bulletin (Biol. Pharm. Bull.) 2004; 27(8): p.1197-1201

### Summary of Invention

Hitherto, it has not been reported that a spiro compound such as a compound of the present invention has a TRH-DE inhibitory action.

The present invention relates to a novel spiro compound having a TRH-DE inhibitory action or a pharmacologically acceptable salt thereof. The compound of the present invention is useful for prevention and/or treatment of various diseases and/or symptoms associated therewith that can be ameliorated by increasing a central TRH concentration.

Specifically, the present invention relates a compound represented by the following Formula [I] (hereinafter, also simply referred to as a compound [I]): 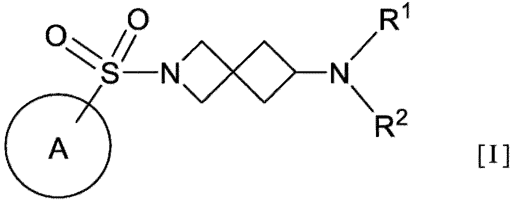 wherein,
a ring A represents an aryl group which may be substituted, a heteroaryl group which may be substituted, or an aliphatic heterocyclyl group which may be substituted, and
R¹ and R² represent, indipendently, an alkyl group which may be substituted, a cycloalkyl group which may be substituted, an alkenyl group which may be substituted, a cycloalkenyl group which may be substituted, an alkynyl group which may be substituted, an aryl group which may be substituted, a heteroaryl group which may be substituted, an aliphatic heterocyclyl group which may be substituted and may have a double bond in a part of a ring, an amino group which may be substituted, an alkoxy group which may be substituted, or an alkylthio group which may be substituted, or a group in which R¹ and R² together form a ring, or a pharmacologically acceptable salt thereof.

In the present invention, the compound of Formula [I] is preferably a compound of Formula [II], Formula [III] or Formula [IV] shown below.

Specifically, the present invention also relates a compound represented by the following Formula [II] (hereinafter, also simply referred to as a compound [II]): 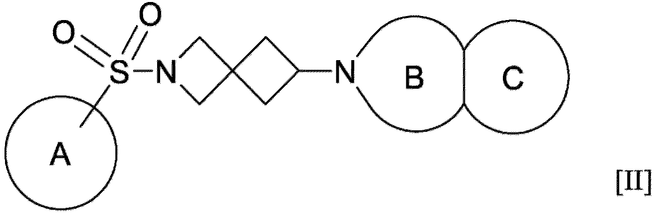 wherein,
a ring A represents an aryl group which may be substituted, a heteroaryl group which may be substituted, or an aliphatic heterocyclyl group which may be substituted,
a ring B represents an aliphatic heterocyclyl group which may be substituted and may have a double bond in a part of a ring, and
a ring C represents an aryl group which may be substituted and may be partially hydrogenated, a heteroaryl group which may be substituted and may be partially hydrogenated, a cycloalkyl group which may be substituted, or an aliphatic heterocyclyl group which may be substituted,
   or a pharmacologically acceptable salt thereof.

The present invention also relates a compoundrepresented by the following Formula [III] (hereinafter, also simply referred to as a compound [III]): 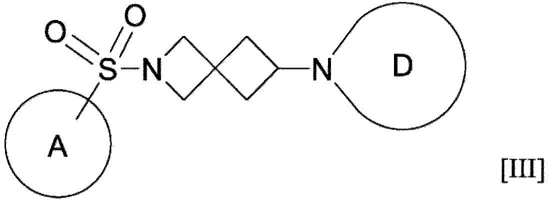 wherein,
a ring A represents an aryl group which may be substituted, a heteroaryl group which may be substituted, or an aliphatic heterocyclyl group which may be substituted, and
a ring D represents an aliphatic heterocyclyl group which may be substituted and may have a double bond in a part of a ring,
   or a pharmacologically acceptable salt thereof.

The present invention also relates a compound represented by the following Formula [IV] (hereinafter, also simply referred to as a compound [IV]): 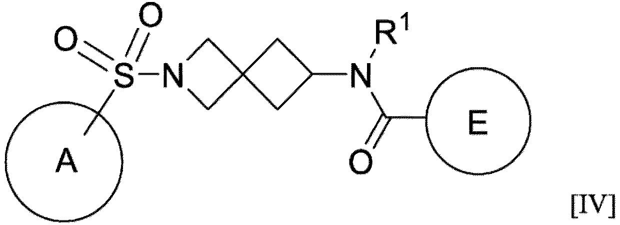 wherein,
a ring A represents an aryl group which may be substituted, a heteroaryl group which may be substituted, or an aliphatic heterocyclyl group which may be substituted,
R¹ represents alkyl group which may be substituted, a cycloalkyl group which may be substituted, an alkenyl group which may be substituted, a cycloalkenyl group which may be substituted, an alkynyl group which may be substituted, an aryl group which may be substituted, a heteroaryl group which may be substituted, an aliphatic heterocyclyl group which may be substituted, an amino group which may be substituted, an alkoxy group which may be substituted, or an alkylthio group which may be substituted, and
a ring E represents an aryl group which may be substituted, a heteroaryl group which may be substituted, or an aliphatic heterocyclyl group which may be substituted and may have a double bond in a part of a ring,
   or a pharmacologically acceptable salt thereof.

In addition, the present invention relates to a method for preventing and/or treating various diseases and/or symptoms associated therewith that can be ameliorated by increasing a TRH concentration, the method including administering an effective amount of a compound represented by Formula [I], [II], [III] or [IV], or a pharmacologically acceptable salt thereof to a patient. In addition, the present invention relates to a pharmaceutical composition containing the compound [I], [II], [III] or [IV] or the pharmacologically acceptable salt thereof as an active ingredient, and a use of the compound [I], [II], [III] or [IV] for preparing the pharmaceutical composition. In addition, the present invention relates to the compound [I], [II], [III] or [IV] or the pharmacologically acceptable salt thereof, or a pharmaceutical composition containing the compound [I], [II], [III] or [IV] or the pharmacologically acceptable salt thereof as an active ingredient, for use in prevention and/or treatment of various diseases and/or symptoms associated therewith that can be ameliorated by increasing a central TRH concentration. In addition, the present invention relates to a method for producing the compound [I], [II], [III] or [IV], or the pharmacologically acceptable salt thereof.

The compound [I], [II], [III] or [IV] of the present invention or the pharmacologically acceptable salt thereof has an excellent inhibitory activity on TRH-DE, and is thus useful for prevention and/or treatment for various diseases and/or symptoms associated therewith that can be ameliorated by increasing a central TRH concentration, such as spinocerebellar degeneration, pain, a sleep disorder, or other psychoneurotic diseases, or for improvement of prognosis of these diseases.

### Description of Embodiments

The definitions of the respective groups in the present specification can be freely combined unless otherwise specified.

In the present invention, alkyl refers to a linear or branched saturated hydrocarbon group having 1 to 6 carbon atoms (C₁₋₆). Particularly, a group having 1 to 4 carbon atoms (C₁₋₄) is preferable. Specific examples thereof include methyl, ethyl, n-propyl, i-propyl, n-butyl, t-butyl, i-amyl, n-pentyl, n-hexyl, and n-propyl. Particularly, methyl ethyl and n-propyl are preferable.

Cycloalkyl refers to a monocyclic saturated hydrocarbon group having 3 to 8 carbon atoms (C₃₋₈) or adamantyl. In addition, cycloalkyl also includes cycloalkyl in which two carbon atoms constituting a ring are crosslinked by an alkylene group to form a bicyclo ring. Specific examples thereof include cyclopropyl, cyclobutyl, cyclopentyl, cyclohexyl, cycloheptyl, and adamantyl. Particularly, a monocyclic group having 3 to 7 carbon atoms (C₃₋₇) is preferable.

Alkoxy refers to a monovalent group in which the alkyl is bonded to oxygen. Examples thereof include a linear or branched alkyl-O- having 1 to 6 carbon atoms (C₁₋₆), and alkyl-O- having 1 to 4 carbon atoms (C₁₋₄) is preferable. Specific examples thereof include methoxy, ethoxy, n-propoxy, i-propoxy, n-butoxy, and t-butoxy.

Cycloalkoxy refers to a monovalent group in which the cycloalkyl is bonded to oxygen. Examples thereof include a monocyclic saturated hydrocarbon group having 3 to 8 carbon atoms (C₃₋₈) and adamantyl-O-, and a monocyclic group -O- having 3 to 7 carbon atoms (C₃₋₇) is preferable. Specific examples thereof include cyclopropyloxy, cyclobutyloxy, and cyclopentyloxy.

Alkylene refers to a linear or branched saturated hydrocarbon divalent group having 1 to 6 carbon atoms (C₁₋₆), and is preferably a group having 1 to 4 carbon atoms (C₁₋₄). Specific examples thereof include methylene, ethylene, trimethylene, and tetramethylene. Both ends of the alkylene may be substituted on the same carbon, may be substituted on two adjacent carbons, or may be substituted on two non-adjacent carbons.

Halogen or halo refers to fluorine, chlorine, bromine, iodine, or the like. Fluorine, chlorine, and bromine are preferable, and fluorine is particularly preferable.

Haloalkyl refers to the above-described alkyl substituted with 1 to 3 halogen atoms, and difluoromethyl, trifluoromethyl, or the like is preferable.

Alkoxyalkyl refers to the above-described alkyl substituted with 1 or 2 alkoxy, and methoxymethyl, methoxyethyl, methoxypropyl, or the like is preferable.

Alkanoyl refers to a monovalent group in which the above-described alkyl is bonded to carbonyl, and examples thereof include a linear or branched alkyl-CO-having 1 to 6 carbon atoms (C₁₋₆). Specific examples thereof include acetyl, propionyl, pivaloyl, butanoyl, pentanoyl, hexanoyl, and heptanoyl.

Alkenyl refers to a hydrocarbon group having one or more double bonds in any position of the aforementioned alkyl, and is preferably a group having 2 to 4 carbon atoms (C₂₋₄). Specific examples thereof include vinyl, allyl, propenyl, isopropenyl, butenyl, isobutenyl, prenyl, butadienyl, pentenyl, isopentenyl, pentadienyl, hexenyl, isohexenyl, and hexadienyl. Particularly, vinyl, allyl, propenyl, isopropenyl, butenyl, isobutenyl, butadienyl, and the like are preferable.

Cycloalkenyl refers to a monocyclic hydrocarbon group having one or more double bonds in any position of the cycloalkyl. Cycloalkenyl also includes those in which the two carbon atoms constituting the ring are bridged by an alkylene group to form a bicyclo ring. Particularly, a monocyclic group having 3 to 7 (C₃₋₇) carbons are preferable. Specific examples thereof include cyclopropenyl, cyclobutenyl, cyclopentenyl, cyclohexenyl, cycloheptenyl, and cyclooctenyl.

Alkynyl refers to a hydrocarbon group having one or more triple bonds in any position of the alkyl, and is preferably a group having 2 to 4 carbon atoms (C₂₋₄). Specific examples thereof include ethynyl, propynyl, butynyl, pentynyl, and hexynyl. Ethynyl, propynyl, butynyl, and the like are preferable.

Aryl refers to a 6- to 10-membered aromatic hydrocarbon cyclic group, and is preferably a monocyclic or bicyclic aryl. Specific examples thereof include phenyl and naphthyl, and phenyl is particularly preferable.

Partially hydrogenated aryl refers to aryl obtained by partially hydrogenating the above-described aryl, and specific examples thereof include dihydrophenyl, cyclohexenyl, indanyl, and tetrahydronaphthyl.

Accordingly, aryl which may be partially hydrogenated includes phenyl, naphthyl, tetrahydronaphthyl, cyclohexenyl, and the like.

Heteroaryl refers to a 5- to 10-membered aromatic heterocyclyl group having 1 to 4 heteroatoms independently selected from the group consisting of oxygen, nitrogen, and sulfur, and a monocyclic or bicyclic heteroaryl is preferable. The heteroaryl is more preferably a 5- to 10-membered monocyclic heteroaryl having 1 to 3 heteroatoms independently selected from the group consisting of oxygen, nitrogen, and sulfur. Specific examples thereof include pyrrolyl, furanyl, thienyl, imidazolyl, pyrazolyl, oxazolyl, isoxazolyl, thiazolyl, isothiazolyl, triazolyl, tetrazolyl, pyridyl, pyrazinyl, pyrimidinyl, pyridazinyl, thiazinyl, triazinyl, indolyl, isoindolyl, indazolyl, benzoimidazolyl, benzothiazolyl, benzofuranyl, quinolyl, isoquinolyl, imidazopyridyl, and benzopyranyl.

Among the specific heteroaryls, pyrrolyl, furanyl, thienyl, imidazolyl, pyrazolyl, oxazolyl, isoxazolyl, thiazolyl, isothiazolyl, triazolyl, tetrazolyl, pyridyl, pyrazinyl, pyrimidinyl, pyridazinyl, thiazinyl, triazinyl, and the like are preferable, and pyrrolyl, thienyl, imidazolyl, pyrazolyl, oxazolyl, isoxazolyl, thiazolyl, isothiazolyl, triazolyl, pyridyl, pyrazinyl, pyrimidinyl, and the like are particularly preferable.

In addition, among the specific heteroaryls, pyrrolyl, imidazolyl, pyrazolyl, oxazolyl, isoxazolyl, thiazolyl, isothiazolyl, triazolyl, tetrazolyl, pyridyl, pyrazinyl, pyrimidinyl, pyridazinyl, thiazinyl, triazinyl, and the like are preferable.

Partially hydrogenated heteroaryl refers to heteroaryl obtained by partially hydrogenating the above-described heteroaryl. Specific examples thereof include dihydropyridyl, tetrahydropyridyl, and tetrahydropyridazinyl.

Accordingly, examples of heteroaryl which may be partially hydrogenated include pyrrolyl, furanyl, thienyl, imidazolyl, pyrazolyl, oxazolyl, isoxazolyl, thiazolyl, isothiazolyl, triazolyl, tetrazolyl, pyridyl, pyrazinyl, pyrimidinyl, pyridazinyl, thiazinyl, triazinyl, indolyl, isoindolyl, indazolyl, benzoimidazolyl, benzothiazolyl, benzofuranyl, quinolyl, isoquinolyl, imidazopyridyl, benzopyranyl, dihydropyridyl, tetrahydropyridyl, and tetrahydropyridazinyl, and pyrrolyl, furanyl, thienyl, imidazolyl, pyrazolyl, oxazolyl, isoxazolyl, thiazolyl, isothiazolyl, triazolyl, tetrazolyl, pyridyl, pyrazinyl, pyrimidinyl, pyridazinyl, thiazinyl, triazinyl, dihydropyridyl, tetrahydropyridyl, tetrahydropyridazinyl, and the like are included.

For example, in General Formula [I], [II], [III], or [IV] as heteroaryl in the heteroaryl group which may be substituted represented by the ring A, thienyl, pyrazolyl, pyridyl, and the like are preferable, and thienyl and pyridyl are particularly preferable. In addition, in General Formula [II], as the heteroaryl which may be partially hydrogenated in the heteroaryl group which may be substituted and may be partially hydrogenated represented by the ring C, pyrrolyl, thienyl, imidazolyl, pyrazolyl, oxazolyl, isoxazolyl, thiazolyl, isothiazolyl, triazolyl, pyridyl, pyrazinyl, pyrimidinyl, dihydropyridyl, tetrahydropyridyl, tetrahydropyridazinyl, and the like are preferable. In other aspect, in General Formula [IV], as the heteroaryl in the heteroaryl group which may be substituted represented by the ring E, pyridyl, azaindolyl, imidazopyridyl, benzoimidazolyl, and the like are preferable.

An aliphatic heterocyclic ring refers to a 4- to 9-membered monocyclic or bicyclic aliphatic heterocyclyl group having 1 to 3 heteroatoms independently selected from the group consisting of oxygen, nitrogen, and sulfur. A bicyclic aliphatic heterocyclyl group includes an aliphatic heterocyclyl group in which cycloalkyl or an aliphatic heterocyclic ring is fused or bonded by a spiro atom to one aliphatic heterocyclic ring. Specific examples thereof include azetidinyl, oxetanyl, oxolanyl, azolidinyl, thiolanyl, oxazolidinyl, diazolidinyl, thiazolidinyl, oxanyl, azinanyl, dioxanyl, oxazinanyl, diazinanyl, thiazinanyl, thianyl, oxepanyl, azepanyl, thiepanyl, oxazepanyl, diazepanyl, and thiazepanyl. In addition, examples thereof include a bicyclic aliphatic heterocyclyl group in which these rings and cycloalkyl or an aliphatic heterocyclic ring are fused or bonded by a spiro atom. Preferred examples thereof include a bicyclic aliphatic heterocyclyl group in which two identical or different aliphatic heterocyclic rings selected from azetidinyl, oxetanyl, oxolanyl, azolidinyl, thiolanyl, oxazolidinyl, diazolidinyl, thiazolidinyl, oxanyl, azinanyl, dioxanyl, oxazinanyl, diazinanyl, thiazinanyl, thianyl, oxepanyl, azepanyl, thiepanyl, oxazepanyl, diazepanyl, and thiazepanyl are fused or bonded by a spiro atom.

In addition, other preferred examples thereof include a 4- to 9-membered monocyclic or bicyclic aliphatic heterocyclyl group which may have at least one nitrogen atom and may further have one heteroatom selected from the group consisting of oxygen, nitrogen, and sulfur. Specific examples thereof include azetidinyl, azolidinyl, oxazolidinyl, diazolidinyl, thiazolidinyl, azinanyl, oxazinanyl, diazinanyl, thiazinanyl, azepanyl, oxazepanyl, diazepanyl, and thiazepanyl. Azolidinyl, oxazolidinyl, diazolidinyl, thiazolidinyl, azinanyl, oxazinanyl, diazinanyl, thiazinanyl, azepanyl, oxazepanyl, diazepanyl, thiazepanyl, and the like are preferable, and azolidinyl, diazolidinyl, azinanyl, oxazinanyl, diazinanyl, azepanyl, oxazepanyl, diazepanyl, thiazepanyl, and the like are preferable. In addition, examples thereof include a bicyclic aliphatic heterocyclyl group in which these rings and cycloalkyl or an aliphatic heterocyclic ring are fused or bonded by a spiro atom. Particularly preferred examples thereof include a bicyclic aliphatic heterocyclyl group in which two identical or different aliphatic heterocyclic rings selected from azolidinyl, diazolidinyl, azinanyl, oxazinanyl, diazinanyl, azepanyl, oxazepanyl, diazepanyl, and thiazepanyl are fused or bonded by a spiro atom.

Examples of an aliphatic heterocyclic ring which may have a double bond in a part of the ring include a ring group having one double bond in the aliphatic heterocyclic ring. Examples thereof include a group in which one of carbon bonds constituting a ring of azinanyl, diazinanyl, azepanyl, or the like is a double bond.

For example, in General Formula [II], as an aliphatic heterocyclic moiety of the aliphatic heterocyclyl group which may be substituted and may have a double bond in a part of a ring represented by the ring B, azolidinyl, diazolidinyl, azinanyl, oxazinanyl, diazinanyl, azepanyl, oxazepanyl, diazepanyl, thiazepanyl, and the like are preferable. In addition, in General Formula [II], as an aliphatic heterocyclic moiety of the aliphatic heterocyclyl group which may be substituted represented by the ring C, azolidinyl, oxazinanyl, and the like are preferable. Further, in General Formula [II], in a substituent of the group represented by the ring C, as an aliphatic heterocyclic moiety of the aliphatic heterocyclyl group which may be substituted in the selected group, azolidinyl, azinanyl, oxazinanyl, diazinanyl, and the like are preferable. For example, in General Formula [III], as an aliphatic heterocyclic moiety of the aliphatic heterocyclyl group which may be substituted and may have a double bond in a part of a ring represented by the ring D, azolidinyl, diazolidinyl, azinanyl, oxazinanyl, diazinanyl, thiazinanil, azepanyl, oxazepanyl, diazepanyl, thiazepanil and the like are preferable.

In one aspect of the present invention, in General Formula [I], [II], [III] or [IV], each of substituents of the aryl group which may be substituted, the heteroaryl group which may be substituted, or the aliphatic heterocyclyl group which may be substituted represented by the ring A includes one to three groups independently selected from the group consisting of a halogen atom, a cyano group, an alkyl group, an alkoxyalkyl group, a haloalkyl group, an alkoxy group, a haloalkoxy group, an alkanoyl group, an alkoxycarbonyl group, an amino group which may be substituted with one to two alkyl groups, an alkylthio group, a sulfonyl group which may be substituted with an alkyl group, an aminocarbonyl group which may be substituted with one to two alkyl groups, a nitro group, an aryl group, and a cycloalkyl group. In another aspect of the present invention, in General Formula [I], [II], [III] or [IV], each of substituents of the phenyl group which may be substituted or the monocyclic heteroaryl group which may be substituted represented by the ring A includes one to two groups independently selected from the group consisting of a halogen atom, an alkyl group, a haloalkyl group, an alkoxy group, and a nitro group.

R¹ and R² include, indipendently, an alkyl group which may be substituted, a cycloalkyl group which may be substituted, an alkenyl group which may be substituted, a cycloalkenyl group which may be substituted, an alkynyl group which may be substituted, an aryl group which may be substituted, a heteroaryl group which may be substituted, an aliphatic heterocyclyl group which may be substituted and may have a double bond in a part of a ring, an amino group which may be substituted, an alkoxy group which may be substituted or an alkylthio group which may be substituted, or a group in which R¹ and R² together form a ring. In General Formula [IV], R¹ is preferably an alkyl group which may be substituted, an alkenyl group which may be substituted, and the like, more preferably an alkyl group and the like, and particularly preferably a propyl group and the like.

The group in which R¹ and R² together form a ring can be any group as long as R¹, R², and a nitrogen atom to which they are bonded together form a ring structure, and includes not only a monocyclic group, but also a group with multiple rings attached. The group in which R¹ and R² together form a ring may be substituted. Examples of such a ring include a heteroaryl group which may be substituted and may be partially hydrogenated, or an aliphatic heterocyclyl group which may be substituted and may have a double bond in a part of a ring. Examples of the ring structure contained in the group with multiple rings attached include a fused ring, a bridged ring, or a spiro ring.

In General Formula [I], each of substituents of the alkyl group which may be substituted, the cycloalkyl group which may be substituted, the alkenyl group which may be substituted, the cycloalkenyl group which may be substituted, the alkynyl group which may be substituted, the aryl group which may be substituted, the heteroaryl group which may be substituted, the aliphatic heterocyclyl group which may be substituted and may have a double bond in a part of a ring, the amino group which may be substituted, the alkoxy group which may be substituted, the alkylthio group which may be substituted, or the group in which R¹ and R² together form a ring, represented by R¹ and R², may be any substituent.

In one aspect, each of substituents of the alkyl group which may be substituted, the alkenyl group which may be substituted, the alkynyl group which may be substituted, the alkoxy group which may be substituted or the alkylthio group which may be substituted, represented by R¹ and R², may be one to eight groups independently selected from the group consisting of a cycloalkyl group which may be substituted; a cycloalkenyl group which may be substituted; an aryl group which may be substituted; a heteroaryl group which may be substituted; an aliphatic heterocyclyl group which may be substituted and may have a double bond in a part of a ring; a halogen atom; a hydroxyl group; an oxo group; a cyano group; an alkoxy group which may be substituted; a cycloalkoxy group which may be substituted; an amino group which may be substituted; an alkylthio group which may be substituted; an alkanoyl group which may be substituted; and an alkoxycarbonyl group which may be substituted.

In one aspect, each of substituents of the cycloalkyl group which may be substituted, the cycloalkenyl group which may be substituted, the aryl group which may be substituted, the heteroaryl group which may be substituted, the aliphatic heterocyclyl group which may be substituted and may have a double bond in a part of a ring, the amino group which may be substituted, or the group in which R¹ and R² together form a ring, represented by R¹ and R², may be one to eight groups independently selected from the group consisting of an alkyl group which may be substituted; a cycloalkyl group which may be substituted; an alkenyl group which may be substituted; a cycloalkenyl group which may be substituted; an alkynyl group which may be substituted; an aryl group which may be substituted; a heteroaryl group which may be substituted; an aliphatic heterocyclyl group which may be substituted and may have a double bond in a part of a ring; a halogen atom; a hydroxyl group; an oxo group; a cyano group; an alkoxy group which may be substituted; a cycloalkoxy group which may be substituted; an amino group which may be substituted; an alkylthio group which may be substituted; an alkanoyl group which may be substituted; and an alkoxycarbonyl group which may be substituted.
(1) One aspect of the present invention includes a compound represented by the following Formula [I] or a pharmacologically acceptable salt thereof: 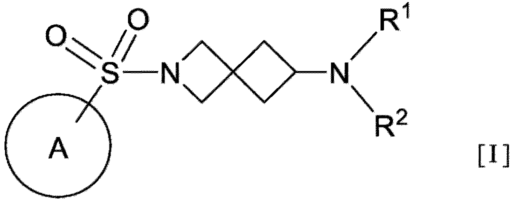 wherein,
   a ring A represents an aryl group which may be substituted, a heteroaryl group which may be substituted, or an aliphatic heterocyclyl group which may be substituted, and
   R¹ and R² represent, indipendently, an alkyl group which may be substituted, a cycloalkyl group which may be substituted, an alkenyl group which may be substituted, a cycloalkenyl group which may be substituted, an alkynyl group which may be substituted, an aryl group which may be substituted, a heteroaryl group which may be substituted, an aliphatic heterocyclyl group which may be substituted and may have a double bond in a part of a ring, an amino group which may be substituted, an alkoxy group which may be substituted, or an alkylthio group which may be substituted, or a group in which R¹ and R² together form a ring.
(2) Another aspect of the present invention includes the compound or the pharmacologically acceptable salt thereof according to the aspect (1), wherein the compound of Formula [I] is a compound represented by the following Formula [II]: 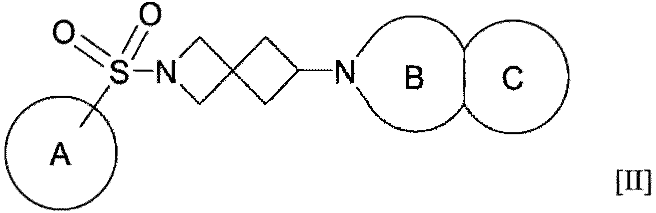 wherein,
   a ring A represents an aryl group which may be substituted, a heteroaryl group which may be substituted, or an aliphatic heterocyclyl group which may be substituted,
   a ring B represents an aliphatic heterocyclyl group which may be substituted and may have a double bond in a part of a ring, and
   a ring C represents an aryl group which may be substituted and may be partially hydrogenated, a heteroaryl group which may be substituted and may be partially hydrogenated, a cycloalkyl group which may be substituted, or an aliphatic heterocyclyl group which may be substituted.
(3) Another aspect of the present invention includes the compound or the pharmacologically acceptable salt thereof according to the aspect (2), wherein in the group represented by Formula [II],
   each of substituents of the aryl group which may be substituted, the heteroaryl group which may be substituted, or the aliphatic heterocyclyl group which may be substituted represented by the ring A is one to three groups independently selected from the group consisting of a halogen atom, a cyano group, an alkyl group, an alkoxyalkyl group, a haloalkyl group, an alkoxy group, a haloalkoxy group, an alkanoyl group, an alkoxycarbonyl group, an amino group which may be substituted with one to two alkyl groups, an alkylthio group, a sulfonyl group which may be substituted with an alkyl group, an aminocarbonyl group which may be substituted with one to two alkyl groups, a nitro group, and aryl group,
   an aryl moiety of the aryl group which may be substituted represented by the ring A is a 6- to 10-membered monocyclic or bicyclic aryl,
   a heteroaryl moiety of the heteroaryl group which may be substituted represented by the ring A is a 5- to 10-membered monocyclic or bicyclic heteroaryl having 1 to 4 heteroatoms independently selected from the group consisting of oxygen, nitrogen, and sulfur,
   an aliphatic heterocyclic moiety of the aliphatic heterocyclyl group which may be substituted represented by the ring A is a 5- to 10-membered monocyclic or bicyclic aliphatic heterocyclic ring having 1 to 3 heteroatoms independently selected from the group consisting of oxygen, nitrogen, and sulfur,
   each of substituents of the aliphatic heterocyclyl group which may be substituted and may have a double bond in a part of a ring represented by the ring B; the aryl group which may be substituted and may be partially hydrogenated represented by the ring C; the heteroaryl group which may be substituted and may be partially hydrogenated represented by the ring C; the cycloalkyl group which may be substituted represented by the ring C; and the aliphatic heterocyclyl group which may be substituted represented by the ring C is one to eight groups independently selected from the group consisting of a halogen atom; a hydroxyl group; an oxo group; a cyano group; an alkyl group; a haloalkyl group; an alkoxyalkyl group; an alkoxy group; a cycloalkoxy group which may be substituted with a cyano group; a haloalkoxy group; an alkylene group which may be substituted with one to two groups independently selected from the group consisting of a halogen atom; an amino group which may be substituted with one to two groups independently selected from the group consisting of an alkyl group which may be substituted with a cycloalkyl group or an aryl group, an aryl group which may be substituted with a halogen atom, and a cycloalkyl group; an alkylthio group; an aryl group; a cycloalkyl group which may be substituted with one to two groups independently selected from the group consisting of a halogen atom and a haloalkyl group; an aliphatic heterocyclyl group which may be substituted with one to four groups independently selected from the group consisting of a halogen atom, a cyano group, an alkyl group, a haloalkyl group, an alkylene group, and an alkoxy group; an alkanoyl group which may be substituted with one to two groups independently selected from the group consisting of a cycloalkyl group, an aryl group, and an alkoxy group; an aryloxy group; an aliphatic heterocyclyl oxy group; and an alkoxycarbonyl group which may be substituted with an aryl group,
   an aliphatic heterocyclic moiety of the aliphatic heterocyclyl group which may be substituted and may have a double bond in a part of a ring represented by the ring B is a 5- to 7-membered monocyclic aliphatic heterocyclic ring having 1 to 3 heteroatoms independently selected from the group consisting of oxygen, nitrogen, and sulfur,
   an aryl moiety of the aryl group which may be substituted and may be partially hydrogenated represented by the ring C is a 6- to 10-membered monocyclic or bicyclic aryl,
   a heteroaryl moiety of the heteroaryl group which may be substituted and may be partially hydrogenated represented by the ring C is a 5- to 10-membered monocyclic or bicyclic heteroaryl having 1 to 4 heteroatoms independently selected from the group consisting of oxygen, nitrogen, and sulfur,
   an aliphatic heterocyclic moiety of the aliphatic heterocyclyl group which may be substituted represented by the ring C is a 4- to 9-membered monocyclic or bicyclic aliphatic heterocyclic ring having 1 to 3 heteroatoms independently selected from the group consisting of oxygen, nitrogen, and sulfur,
   in each of the substituents of the groups represented by the ring B or the ring C, the aryl group in the selected group is a 6- to 10-membered monocyclic or bicyclic aryl, and
   in each of the substituents of the groups represented by the ring B or the ring C, the aliphatic heterocyclic moiety of the aliphatic heterocyclyl group which may be substituted in the selected group is a 4- to 9-membered monocyclic or bicyclic aliphatic heterocyclic ring having 1 to 3 heteroatoms independently selected from the group consisting of oxygen, nitrogen, and sulfur.
(4) Still another aspect of the present invention includes the compound or the pharmacologically acceptable salt thereof according to the aspect (2) or (3), wherein in the group represented by Formula [II],
   the ring A is an aryl group which may be substituted or a heteroaryl group which may be substituted,
   an aryl moiety of the aryl group which may be substituted represented by the ring A is a 6- to 10-membered monocyclic or bicyclic aryl,
   a heteroaryl moiety of the heteroaryl group which may be substituted represented by the ring A is a 5- to 10-membered monocyclic or bicyclic heteroaryl having 1 to 4 heteroatoms independently selected from the group consisting of oxygen, nitrogen, and sulfur,
   an aliphatic heterocyclic moiety of the aliphatic heterocyclyl group which may be substituted and may have a double bond in a part of a ring represented by the ring B is a 5- to 7-membered monocyclic aliphatic heterocyclic ring having 1 to 3 heteroatoms independently selected from the group consisting of oxygen, nitrogen, and sulfur,
   the ring C is an aryl group which may be substituted and may be partially hydrogenated, a heteroaryl group which may be substituted and may be partially hydrogenated, a cycloalkyl group which may be substituted, or an aliphatic heterocyclyl group which may be substituted,
   an aryl moiety of the aryl group which may be substituted and may be partially hydrogenated represented by the ring C is a 6- to 10-membered monocyclic or bicyclic aryl,
   a heteroaryl moiety of the heteroaryl group which may be substituted and may be partially hydrogenated represented by the ring C is a 5- to 10-membered monocyclic or bicyclic heteroaryl having 1 to 4 heteroatoms independently selected from the group consisting of oxygen, nitrogen, and sulfur, and
   an aliphatic heterocyclic moiety of the aliphatic heterocyclyl group which may be substituted represented by the ring C is a 4- to 9-membered monocyclic or bicyclic aliphatic heterocyclic ring having 1 to 3 heteroatoms independently selected from the group consisting of oxygen, nitrogen, and sulfur.
(5) In addition, still another aspect includes the compound or the pharmacologically acceptable salt thereof according to any one of the aspects (2) to (4), wherein in the group represented by Formula [II],
   the ring A is a phenyl group which may be substituted or a monocyclic heteroaryl group which may be substituted,
   each of substituents of the phenyl group which may be substituted or the monocyclic heteroaryl group which may be substituted represented by the ring A is one to two groups independently selected from the group consisting of a halogen atom, an alkyl group, a haloalkyl group, an alkoxy group, and a nitro group,
   a heteroaryl moiety of the monocyclic heteroaryl group which may be substituted represented by the ring A is a 5- or 6-membered monocyclic heteroaryl having 1 to 4 heteroatoms independently selected from the group consisting of oxygen, nitrogen, and sulfur,
   a substituent of the aliphatic heterocyclyl group which may be substituted and may have a double bond in a part of a ring represented by the ring B is one to four groups independently selected from the group consisting of a halogen atom; a hydroxyl group; an oxo group; an alkyl group; a haloalkyl group; an alkoxyalkyl group; an alkoxy group; an alkylene group which may be substituted with one to two groups independently selected from a halogen atom; a cycloalkyl group; an alkanoyl group which may be substituted with one to two groups independently selected from the group consisting of a cycloalkyl group, an aryl group, and an alkoxy group; and an alkoxycarbonyl group which may be substituted with an aryl group,
   in each of the substituents of the groups represented by the ring B, the aryl group in the selected group is phenyl,
   the ring C is a phenyl group which may be substituted, a monocyclic heteroaryl group which may be substituted and may be partially hydrogenated, a monocyclic cycloalkyl group which may be substituted, or a monocyclic aliphatic heterocyclyl group which may be substituted,
   each of substituents of the phenyl group which may be substituted; the monocyclic heteroaryl group which may be substituted and may be partially hydrogenated; the monocyclic cycloalkyl group which may be substituted; or the monocyclic aliphatic heterocyclyl group which may be substituted represented by the ring C is one to four groups independently selected from the group consisting of a halogen atom; a hydroxyl group; an oxo group; a cyano group; an alkyl group; a cycloalkoxy group which may be substituted with a cyano group; a haloalkyl group; an alkoxy group; a haloalkoxy group; an alkylene group; an amino group which may be substituted with one to two groups independently selected from the group consisting of an alkyl group which may be substituted with a cycloalkyl group or an aryl group, an aryl group which may be substituted with a halogen atom, and a cycloalkyl group; an alkylthio group; an aryl group; a cycloalkyl group which may be substituted with one to two groups independently selected from the group consisting of a halogen atom and a haloalkyl group; an aliphatic heterocyclyl group which may be substituted with one to four groups independently selected from the group consisting of a halogen atom, a cyano group, an alkyl group, a haloalkyl group, an alkylene group, and an alkoxy group; an aryloxy group; an aliphatic heterocyclyl oxy group; and an alkanoyl group,
   a heteroaryl moiety of the monocyclic heteroaryl group which may be substituted and may be partially hydrogenated represented by the ring C is a 5- or 6-membered monocyclic heteroaryl having 1 to 4 heteroatoms independently selected from the group consisting of oxygen, nitrogen, and sulfur,
   an aliphatic heterocyclic moiety of the monocyclic aliphatic heterocyclyl group which may be substituted represented by the ring C is a 4- to 7-membered monocyclic aliphatic heterocyclic ring having 1 to 3 heteroatoms independently selected from the group consisting of oxygen, nitrogen, and sulfur,
   in each of the substituents of the groups represented by the ring C, the aryl group in the selected group is phenyl, and
   in each of the substituents of the groups represented by the ring C, an aliphatic heterocyclic moiety of the aliphatic heterocyclyl group which may be substituted in the selected group is a 4- to 9-membered monocyclic or bicyclic aliphatic heterocyclic ring having 1 to 3 heteroatoms independently selected from the group consisting of oxygen, nitrogen, and sulfur.
(6) In addition, still another aspect of the present invention includes the compound or the pharmacologically acceptable salt thereof according to any one of the aspects (2) to (5), wherein in the group represented by Formula [II],
   the ring A is a phenyl group which may be substituted or a monocyclic heteroaryl group which may be substituted,
   each of substituents of the phenyl group which may be substituted or the monocyclic heteroaryl group which may be substituted represented by the ring A is one to two groups independently selected from the group consisting of a halogen atom, an alkyl group, a haloalkyl group, an alkoxy group, and a nitro group,
   a heteroaryl moiety of the monocyclic heteroaryl group which may be substituted represented by the ring A is pyrrolyl, furanyl, thienyl, imidazolyl, pyrazolyl, oxazolyl, isoxazolyl, thiazolyl, isothiazolyl, triazolyl, tetrazolyl, pyridyl, pyrazinyl, pyrimidinyl, pyridazinyl, thiazinyl, or triazinyl,
   a substituent of the aliphatic heterocyclyl group which may be substituted and may have a double bond in a part of a ring represented by the ring B is one to four groups independently selected from the group consisting of a halogen atom; a hydroxyl group; an oxo group; an alkyl group; a haloalkyl group; an alkoxyalkyl group; an alkoxy group; an alkylene group which may be substituted with one to two groups independently selected from a halogen atom; a cycloalkyl group; an alkanoyl group which may be substituted with one to two groups independently selected from the group consisting of a cycloalkyl group, an aryl group, and an alkoxy group; and an alkoxycarbonyl group which may be substituted with an aryl group,
   an aliphatic heterocyclic moiety of the aliphatic heterocyclyl group which may be substituted and may have a double bond in a part of a ring represented by the ring B is azolidinyl, diazolidinyl, azinanyl, oxazinanyl, diazinanyl, thiazinanyl, azepanyl, oxazepanyl, diazepanyl, or thiazepanyl,
   in each of the substituents of the groups represented by the ring B, the aryl group in the selected group is phenyl,
   the ring C is a phenyl group which may be substituted, a monocyclic heteroaryl group which may be substituted and may be partially hydrogenated, a monocyclic cycloalkyl group which may be substituted, or a monocyclic aliphatic heterocyclyl group which may be substituted,
   each of substituents of the phenyl group which may be substituted; the monocyclic heteroaryl group which may be substituted and may be partially hydrogenated; the monocyclic cycloalkyl group which may be substituted; or the monocyclic aliphatic heterocyclyl group which may be substituted represented by the ring C is one to four groups independently selected from the group consisting of a halogen atom; a hydroxyl group; an oxo group; a cyano group; an alkyl group; a haloalkyl group; an alkoxy group; a cycloalkoxy group which may be substituted with a cyano group; a haloalkoxy group; an alkylene group; an amino group which may be substituted with one to two groups independently selected from the group consisting of an alkyl group which may be substituted with a cycloalkyl group or an aryl group, an aryl group which may be substituted with a halogen atom, and a cycloalkyl group; an alkylthio group; an aryl group; a cycloalkyl group which may be substituted with one to two groups independently selected from the group consisting of a halogen atom and a haloalkyl group; an aliphatic heterocyclyl group which may be substituted with one to four groups independently selected from the group consisting of a halogen atom, a cyano group, an alkyl group, a haloalkyl group, an alkylene group, and an alkoxy group; an aryloxy group; an aliphatic heterocyclyl oxy group; and an alkanoyl group,
   a heteroaryl moiety of the monocyclic heteroaryl group which may be substituted and may be partially hydrogenated represented by the ring C is pyrrolyl, furanyl, thienyl, imidazolyl, pyrazolyl, oxazolyl, isoxazolyl, thiazolyl, isothiazolyl, triazolyl, tetrazolyl, pyridyl, pyrazinyl, pyrimidinyl, pyridazinyl, thiazinyl, or triazinyl,
   an aliphatic heterocyclic moiety of the monocyclic aliphatic heterocyclyl group which may be substituted represented by the ring C is azetidinyl, oxetanyl, oxolanyl, azolidinyl, thiolanyl, oxazolidinyl, diazolidinyl, thiazolidinyl, oxanyl, azinanyl, dioxanyl, oxazinanyl, diazinanyl, thiazinanyl, thianyl, oxepanyl, azepanyl, thiepanyl, oxazepanyl, diazepanyl, or thiazepanyl,
   in each of the substituents of the groups represented by the ring C, the aryl group in the selected group is phenyl, and
   in each of the substituents of the groups represented by the ring C, an aliphatic heterocyclic moiety of the aliphatic heterocyclyl group which may be substituted in the selected group is azetidinyl, oxetanyl, oxolanyl, azolidinyl, thiolanyl, oxazolidinyl, diazolidinyl, thiazolidinyl, oxanyl, azinanyl, dioxanyl, oxazinanyl, diazinanyl, thiazinanyl, thianyl, oxepanyl, azepanyl, thiepanyl, oxazepanyl, diazepanyl, thiazepanyl, or a ring in which two aliphatic heterocyclic rings are fused or bonded by a spiro atom.
(7) In addition, still another aspect of the present invention includes the compound or the pharmacologically acceptable salt thereof according to any one of the aspects (2) to (5), wherein in the group represented by Formula [II],
   the ring A is a phenyl group which may be substituted or a monocyclic heteroaryl group which may be substituted,
   each of substituents of the phenyl group which may be substituted or the monocyclic heteroaryl group which may be substituted represented by the ring A is one to two groups independently selected from the group consisting of a halogen atom, an alkyl group, a haloalkyl group, an alkoxy group, and a nitro group,
   a heteroaryl moiety of the monocyclic heteroaryl group which may be substituted represented by the ring A is thienyl or pyridyl,
   a substituent of the aliphatic heterocyclyl group which may be substituted and may have a double bond in a part of a ring represented by the ring B is one to four groups independently selected from the group consisting of a halogen atom; a hydroxyl group; an oxo group; an alkyl group; a haloalkyl group; an alkoxyalkyl group; an alkoxy group; an alkylene group which may be substituted with one to two groups independently selected from a halogen atom; a cycloalkyl group; an alkanoyl group which may be substituted with one to two groups independently selected from the group consisting of a cycloalkyl group, an aryl group, and an alkoxy group; and an alkoxycarbonyl group which may be substituted with an aryl group,
   an aliphatic heterocyclic moiety of the aliphatic heterocyclyl group which may be substituted and may have a double bond in a part of a ring represented by the ring B is azolidinyl, diazolidinyl, azinanyl, oxazinanyl, diazinanyl, azepanyl, oxazepanyl, diazepanyl, or thiazepanyl,
   in each of the substituents of the groups represented by the ring B, the aryl group in the selected group is phenyl,
   the ring C is a phenyl group which may be substituted, a monocyclic heteroaryl group which may be substituted and may be partially hydrogenated, a monocyclic cycloalkyl group which may be substituted, or a monocyclic aliphatic heterocyclyl group which may be substituted,
   each of substituents of the phenyl group which may be substituted, the monocyclic heteroaryl group which may be substituted and may be partially hydrogenated, the monocyclic cycloalkyl group which may be substituted, or the monocyclic aliphatic heterocyclyl group which may be substituted represented by the ring C is one to four groups independently selected from the group consisting of a halogen atom; a hydroxyl group; an oxo group; a cyano group; an alkyl group; a haloalkyl group; an alkoxy group; a cycloalkoxy group which may be substituted with a cyano group; a haloalkoxy group; an alkylene group; an amino group which may be substituted with one to two groups independently selected from the group consisting of an alkyl group which may be substituted with a cycloalkyl group or an aryl group, an aryl group which may be substituted with a halogen atom, and a cycloalkyl group; an alkylthio group; an aryl group; a cycloalkyl group which may be substituted with one to two groups independently selected from the group consisting of a halogen atom and a haloalkyl group; an aliphatic heterocyclyl group which may be substituted with one to four groups independently selected from the group consisting of a halogen atom, a cyano group, an alkyl group, a haloalkyl group, an alkylene group, and an alkoxy group; an aryloxy group; an aliphatic heterocyclyl oxy group; and an alkanoyl group,
   a heteroaryl moiety of the monocyclic heteroaryl group which may be substituted and may be partially hydrogenated represented by the ring C is pyrrolyl, thienyl, imidazolyl, pyrazolyl, oxazolyl, isoxazolyl, thiazolyl, isothiazolyl, triazolyl, pyridyl, pyrazinyl, or pyrimidinyl,
   an aliphatic heterocyclic moiety of the monocyclic aliphatic heterocyclyl group which may be substituted represented by the ring C is azolidinyl or oxazinanyl,
   in each of the substituents of the groups represented by the ring C, the aryl group in the selected group is phenyl, and
   in each of the substituents of the groups represented by the ring C, an aliphatic heterocyclic moiety of the aliphatic heterocyclyl group which may be substituted in the selected group is azolidinyl, azinanyl, oxazinanyl, diazinanyl, azepanyl, oxazepanyl, or a ring in which two aliphatic heterocyclic rings are fused or bonded by a spiro atom.
(8) One preferred aspect of the present invention includes the compound or the pharmacologically acceptable salt thereof according to any one of the aspects (2) to (7), wherein in the group represented by Formula [II],
   the ring B is a 7-membered monocyclic aliphatic heterocyclyl group which may be substituted and may have a double bond in a part of a ring, and
   the ring C is a phenyl group which may be substituted, a 6-membered monocyclic heteroaryl group which may be substituted and may be partially hydrogenated, or a 6-membered monocyclic aliphatic heterocyclyl group which may be substituted.
(9) In addition, still another aspect of the present invention includes the compound or the pharmacologically acceptable salt thereof according to the aspect (8), wherein in the group represented by Formula [II],
   the ring A is a phenyl group which may be substituted or a monocyclic heteroaryl group which may be substituted,
   each of substituents of the phenyl group which may be substituted or the monocyclic heteroaryl group which may be substituted represented by the ring A is one to two groups independently selected from the group consisting of a halogen atom, an alkyl group, a haloalkyl group, an alkoxy group, and a nitro group,
   a heteroaryl moiety of the monocyclic heteroaryl group which may be substituted represented by the ring A is a 5- or 6-membered monocyclic heteroaryl having 1 to 4 heteroatoms independently selected from the group consisting of oxygen, nitrogen, and sulfur,
   the ring B is a 7-membered monocyclic aliphatic heterocyclyl group which may be substituted and may have a double bond in a part of a ring,
   a substituent of the 7-membered monocyclic aliphatic heterocyclyl group which may be substituted and may have a double bond in a part of a ring represented by the ring B is one to four groups independently selected from the group consisting of a halogen atom; an oxo group; an alkyl group; an alkoxyalkyl group; an alkylene group; a cycloalkyl group; an alkanoyl group which may be substituted with one to two groups independently selected from the group consisting of a cycloalkyl group, a phenyl group, and an alkoxy group; and an alkoxycarbonyl group which may be substituted with an aryl group,
   an aliphatic heterocyclic moiety of the 7-membered monocyclic aliphatic heterocyclyl group which may be substituted and may have a double bond in a part of a ring represented by the ring B is a 7-membered monocyclic aliphatic heterocyclic ring having 1 to 3 heteroatoms independently selected from the group consisting of oxygen, nitrogen, and sulfur,
   the ring C is a phenyl group which may be substituted, a 6-membered monocyclic heteroaryl group which may be substituted and may be partially hydrogenated, or a 6-membered monocyclic aliphatic heterocyclyl group which may be substituted,
   each of substituents of the phenyl group which may be substituted, the 6-membered monocyclic heteroaryl group which may be substituted and may be partially hydrogenated, or the 6-membered monocyclic aliphatic heterocyclyl group which may be substituted represented by the ring C is one to two groups independently selected from the group consisting of a halogen atom; an oxo group; a cyano group; an alkyl group; a haloalkyl group; an alkoxy group; a cycloalkoxy group which may be substituted with a cyano group; a haloalkoxy group; an amino group which may be substituted with one to two groups independently selected from the group consisting of an alkyl group which may be substituted with a cycloalkyl group or an aryl group, an aryl group which may be substituted with a halogen atom, and a cycloalkyl group; a cycloalkyl group; an aliphatic heterocyclyl group which may be substituted with one to four groups independently selected from the group consisting of a halogen atom, a cyano group, an alkyl group, a haloalkyl group, an alkylene group, and an alkoxy group; an aryloxy group; an aliphatic heterocyclyl oxy group; and an alkanoyl group,
   in each of the substituents of the groups represented by the ring C, the aryl group in the selected group is phenyl,
   in each of the substituents of the groups represented by the ring C, an aliphatic heterocyclic moiety of the aliphatic heterocyclyl group which may be substituted in the selected group is a 5- to 7-membered monocyclic aliphatic heterocyclic ring having 1 to 3 heteroatoms independently selected from the group consisting of oxygen, nitrogen, and sulfur,
   a heteroaryl moiety of the 6-membered monocyclic heteroaryl group which may be substituted and may be partially hydrogenated represented by the ring C is a 6-membered monocyclic heteroaryl having 1 to 3 heteroatoms independently selected from the group consisting of oxygen, nitrogen, and sulfur, and
   an aliphatic heterocyclic moiety of the 6-membered monocyclic aliphatic heterocyclyl group which may be substituted represented by the ring C is a 6-membered monocyclic aliphatic heterocyclic ring having 1 to 3 heteroatoms independently selected from the group consisting of oxygen, nitrogen, and sulfur.
(10) In addition, still another aspect of the present invention includes the compound or the pharmacologically acceptable salt thereof according to the aspect (8) or (9), wherein in the group represented by Formula [II],
   a heteroaryl moiety of the heterocyclyl group which may be substituted represented by the ring A is thienyl or pyridyl,
   an aliphatic heterocyclic moiety of the 7-membered monocyclic aliphatic heterocyclyl group which may be substituted and may have a double bond in a part of a ring represented by the ring B is azepanyl, oxazepanyl, diazepanyl, or thiazepanyl,
   an aryl moiety of the aryl group in the substituent of the group represented by the ring B is phenyl,
   a heteroaryl moiety of the 6-membered monocyclic heteroaryl group which may be substituted and may be partially hydrogenated represented by the ring C is pyridyl, pyrazinyl, or pyrimidinyl,
   an aliphatic heterocyclic moiety of the 6-membered monocyclic aliphatic heterocyclyl group which may be substituted represented by the ring C is oxazinanyl,
   in each of the substituents of the groups represented by the ring C, the aryl group in the selected group is phenyl, and
   in each of the substituents of the groups represented by the ring C, an aliphatic heterocyclic moiety of the aliphatic heterocyclyl group which may be substituted in the selected group is azolidinyl, azinanyl, oxazinanyl, diazinanyl, azepanyl, or oxazepanyl.
(11) In addition, one preferred aspect of the present invention includes the compound or the pharmacologically acceptable salt thereof according to any one of the aspects (2) to (7), wherein in the group represented by Formula [II],
   the ring B is a 7-membered monocyclic aliphatic heterocyclyl group which may be substituted and may have a double bond in a part of a ring, and
   the ring C is a 5-membered monocyclic heteroaryl group which may be substituted or a 5-membered monocyclic aliphatic heterocyclyl group which may be substituted.
(12) In addition, still another aspect of the present invention includes the compound or the pharmacologically acceptable salt thereof according to the aspect (11), wherein in the group represented by Formula [II],
   the ring A is a phenyl group which may be substituted,
   a substituent of the phenyl group which may be substituted represented by the ring A is a halogen atom,
   the ring B is a 7-membered monocyclic aliphatic heterocyclyl group which may be substituted and may have a double bond in a part of a ring,
   a substituent of the 7-membered monocyclic aliphatic heterocyclyl group which may be substituted and may have a double bond in a part of a ring represented by the ring B is one to four groups independently selected from the group consisting of an oxo group; an alkyl group; and an alkylene group which may be substituted with one to two groups independently selected from a halogen atom,
   an aliphatic heterocyclic moiety of the 7-membered monocyclic aliphatic heterocyclyl group which may be substituted and may have a double bond in a part of a ring represented by the ring B is a 7-membered monocyclic aliphatic heterocyclic ring having 1 to 3 heteroatoms independently selected from the group consisting of oxygen, nitrogen, and sulfur,
   the ring C is a 5-membered monocyclic heteroaryl group which may be substituted or a 5-membered monocyclic aliphatic heterocyclyl group which may be substituted,
   each of substituents of the 5-membered monocyclic heteroaryl group which may be substituted or the 5-membered monocyclic aliphatic heterocyclyl group which may be substituted represented by the ring C is one to two groups independently selected from the group consisting of a halogen atom; an alkyl group; a haloalkyl group; an alkylene group; a cycloalkyl group which may be substituted with one to two groups independently selected from the group consisting of a halogen atom and a haloalkyl group; and an aryl group,
   in each of the substituents of the groups represented by the ring C, the aryl group in the selected group is phenyl,
   a heteroaryl moiety of the 5-membered monocyclic heteroaryl group which may be substituted represented by the ring C is a 5-membered monocyclic heteroaryl having 1 to 4 heteroatoms independently selected from the group consisting of oxygen, nitrogen, and sulfur, and
   an aliphatic heterocyclic moiety of the 5-membered monocyclic aliphatic heterocyclyl group which may be substituted represented by the ring C is a 5-membered monocyclic aliphatic heterocyclic ring having 1 to 3 heteroatoms independently selected from the group consisting of oxygen, nitrogen, and sulfur.
(13) In addition, still another aspect of the present invention includes the compound or the pharmacologically acceptable salt thereof according to the aspect (11) or (12), wherein in the group represented by Formula [II],
   an aliphatic heterocyclic moiety of the 7-membered monocyclic aliphatic heterocyclyl group which may be substituted and may have a double bond in a part of a ring represented by the ring B is azepanyl, oxazepanyl, or diazepanyl,
   a heteroaryl moiety of the 5-membered monocyclic heteroaryl group which may be substituted represented by the ring C is imidazolyl, pyrazolyl, oxazolyl, isoxazolyl, thiazolyl, isothiazolyl, or triazolyl,
   an aliphatic heterocyclic moiety of the 5-membered monocyclic aliphatic heterocyclyl group which may be substituted represented by the ring C is azolidinyl, and
   in each of the substituents of the groups represented by the ring C, the aryl group in the selected group is phenyl.
(14) In addition, another preferred aspect of the present invention includes the compound or the pharmacologically acceptable salt thereof according to any one of the aspects (2) to (7), wherein in the group represented by Formula [II],
   the ring B is a 6-membered monocyclic aliphatic heterocyclyl group which may be substituted and may have a double bond in a part of a ring, and
   the ring C is a phenyl group which may be substituted or a 6-membered monocyclic heteroaryl group which may be substituted.
(15) In addition, another aspect of the present invention includes the compound or the pharmacologically acceptable salt thereof according to the aspect (14), wherein in the group represented by Formula [II],
   the ring A is a phenyl group which may be substituted,
   a substituent of the phenyl group which may be substituted represented by the ring A is a halogen atom,
   the ring B is a 6-membered monocyclic aliphatic heterocyclyl group which may be substituted and may have a double bond in a part of a ring,
   a substituent of the 6-membered monocyclic aliphatic heterocyclyl group which may be substituted and may have a double bond in a part of a ring represented by the ring B is one to three groups independently selected from the group consisting of an oxo group; an alkyl group; and a haloalkyl group,
   an aliphatic heterocyclic moiety of the 6-membered monocyclic aliphatic heterocyclyl group which may be substituted and may have a double bond in a part of a ring represented by the ring B is a 6-membered monocyclic aliphatic heterocyclic ring having 1 to 3 heteroatoms independently selected from the group consisting of oxygen, nitrogen, and sulfur,
   the ring C is a phenyl group which may be substituted or a 6-membered monocyclic heteroaryl group which may be substituted,
   each of substituents of the phenyl group which may be substituted or the 6-membered monocyclic heteroaryl group which may be substituted represented by the ring C is a group independently selected from the group consisting of a halogen atom and a haloalkyl group, and
   a heteroaryl moiety of the 6-membered monocyclic heteroaryl group which may be substituted represented by the ring C is a 6-membered monocyclic heteroaryl having 1 to 3 heteroatoms independently selected from the group consisting of oxygen, nitrogen, and sulfur.
(16) In addition, still another aspect of the present invention includes the compound or the pharmacologically acceptable salt thereof according to the aspect (14) or (15), wherein in the group represented by Formula [II],
   an aliphatic heterocyclic moiety of the 6-membered monocyclic aliphatic heterocyclyl group which may be substituted and may have a double bond in a part of a ring represented by the ring B is azinanyl, oxazinanyl, or diazinanyl, and
   a heteroaryl moiety of the 6-membered monocyclic heteroaryl group which may be substituted represented by the ring C is pyridyl.
(17) In addition, another preferred aspect of the present invention includes the compound or the pharmacologically acceptable salt thereof according to any one of the aspects (2) to (7), wherein in the group represented by Formula [II],
   the ring B is a 6-membered monocyclic aliphatic heterocyclyl group which may be substituted and may have a double bond in a part of a ring, and
   the ring C is a 5-membered monocyclic heteroaryl group which may be substituted.
(18) In addition, still another aspect of the present invention includes the compound or the pharmacologically acceptable salt thereof according to the aspect (17), wherein in the group represented by Formula [II],
   the ring A is a phenyl group which may be substituted,
   a substituent of the phenyl group which may be substituted represented by the ring A is a halogen atom,
   the ring B is a 6-membered monocyclic aliphatic heterocyclyl group which may be substituted and may have a double bond in a part of a ring,
   a substituent of the 6-membered monocyclic aliphatic heterocyclyl group which may be substituted and may have a double bond in a part of a ring represented by the ring B is one to two groups independently selected from the group consisting of an oxo group and an alkyl group,
   an aliphatic heterocyclic moiety of the 6-membered monocyclic aliphatic heterocyclyl group which may be substituted and may have a double bond in a part of a ring represented by the ring B is a 6-membered monocyclic aliphatic heterocyclic ring having 1 to 3 heteroatoms independently selected from the group consisting of oxygen, nitrogen, and sulfur,
   the ring C is a 5-membered monocyclic heteroaryl group which may be substituted,
   a substituent of the 5-membered monocyclic heteroaryl group which may be substituted represented by the ring C is one to two groups independently selected from the group consisting of a halogen atom; an alkyl group; a haloalkyl group; and a cycloalkyl group which may be substituted with one to two groups independently selected from the group consisting of a halogen atom and a haloalkyl group, and
   a heteroaryl moiety of the 5-membered monocyclic heteroaryl group which may be substituted represented by the ring C is a 5-membered monocyclic heteroaryl having 1 to 4 heteroatoms independently selected from the group consisting of oxygen, nitrogen, and sulfur.
(19) In addition, still another aspect of the present invention includes the compound or the pharmacologically acceptable salt thereof according to the aspect (17) or (18), wherein in the group represented by Formula [II],
   an aliphatic heterocyclic moiety of the 6-membered monocyclic aliphatic heterocyclyl group which may be substituted and may have a double bond in a part of a ring represented by the ring B is diazinanyl, and
   a heteroaryl moiety of the 5-membered monocyclic heteroaryl group which may be substituted represented by the ring C is pyrrolyl, imidazolyl, pyrazolyl, or triazolyl.
(20) In addition, another preferred aspect of the present invention includes the compound or the pharmacologically acceptable salt thereof according to any one of the aspects (2) to (7), wherein in the group represented by Formula [II],
   the ring B is a 5-membered monocyclic aliphatic heterocyclyl group which may be substituted and may have a double bond in a part of a ring, and
   the ring C is a phenyl group which may be substituted or a 6-membered monocyclic heteroaryl group which may be substituted.
(21) In addition, still another aspect of the present invention includes the compound or the pharmacologically acceptable salt thereof according to the aspect (20), wherein in the group represented by Formula [II],
   the ring A is a phenyl group which may be substituted,
   a substituent of the phenyl group which may be substituted represented by the ring A is one to two groups independently selected from the group consisting of a halogen atom, an alkyl group, and an alkoxy group,
   the ring B is a 5-membered monocyclic aliphatic heterocyclyl group which may be substituted and may have a double bond in a part of a ring,
   a substituent of the 5-membered monocyclic aliphatic heterocyclyl group which may be substituted and may have a double bond in a part of a ring represented by the ring B is one to three groups independently selected from the group consisting of a hydroxyl group; an oxo group; an alkyl group; and an alkylene group,
   an aliphatic heterocyclic moiety of the 5-membered monocyclic aliphatic heterocyclyl group which may be substituted and may have a double bond in a part of a ring represented by the ring B is a 5-membered monocyclic aliphatic heterocyclic ring having 1 to 3 heteroatoms independently selected from the group consisting of oxygen, nitrogen, and sulfur,
   the ring C is a phenyl group which may be substituted or a 6-membered monocyclic heteroaryl group which may be substituted,
   each of substituents of the phenyl group which may be substituted or the 6-membered monocyclic heteroaryl group which may be substituted represented by the ring C is a group independently selected from the group consisting of a halogen atom; an alkyl group; a haloalkyl group; and an alkoxy group, and
   a heteroaryl moiety of the 6-membered monocyclic heteroaryl group which may be substituted represented by the ring C is a 6-membered monocyclic heteroaryl having 1 to 3 heteroatoms independently selected from the group consisting of oxygen, nitrogen, and sulfur.
(22) In addition, still another aspect of the present invention includes the compound or the pharmacologically acceptable salt thereof according to the aspect (20) or (21), wherein in the group represented by Formula [II],
   an aliphatic heterocyclic moiety of the 5-membered monocyclic aliphatic heterocyclyl group which may be substituted and may have a double bond in a part of a ring represented by the ring B is azolidinyl or diazolidinyl, and
   a heteroaryl moiety of the 6-membered monocyclic heteroaryl group which may be substituted represented by the ring C is pyridyl.
(23) In addition, another preferred aspect of the present invention includes the compound or the pharmacologically acceptable salt thereof according to any one of the aspects (2) to (7), wherein in the group represented by Formula [II],
   the ring B is a 5-membered monocyclic aliphatic heterocyclyl group which may be substituted and may have a double bond in a part of a ring, and
   the ring C is a 5-membered monocyclic heteroaryl group which may be substituted.
(24) In addition, another aspect of the present invention include the compound or the pharmacologically acceptable salt thereof according to the aspect (23), wherein in the group represented by Formula [II],
   the ring A is a phenyl group which may be substituted,
   a substituent of the phenyl group which may be substituted represented by the ring A is a halogen atom,
   the ring B is a 5-membered monocyclic aliphatic heterocyclyl group which may be substituted and may have a double bond in a part of a ring,
   a substituent of the 5-membered monocyclic aliphatic heterocyclyl group which may be substituted and may have a double bond in a part of a ring represented by the ring B is one to two groups independently selected from the group consisting of an oxo group and an alkyl group,
   an aliphatic heterocyclic moiety of the 5-membered monocyclic aliphatic heterocyclyl group which may be substituted and may have a double bond in a part of a ring represented by the ring B is a 5-membered monocyclic aliphatic heterocyclic ring having 1 to 3 heteroatoms independently selected from the group consisting of oxygen, nitrogen, and sulfur,
   the ring C is a 5-membered monocyclic heteroaryl group which may be substituted,
   a substituent of the 5-membered monocyclic heteroaryl group which may be substituted represented by the ring C is an alkyl group, and
   a heteroaryl moiety of the 5-membered monocyclic heteroaryl group which may be substituted represented by the ring C is a 5-membered monocyclic heteroaryl having 1 to 4 heteroatoms independently selected from the group consisting of oxygen, nitrogen, and sulfur.
(25) In addition, still another aspect of the present invention includes the compound or the pharmacologically acceptable salt thereof according to the aspect (23) or (24), wherein in the group represented by Formula [II],
   an aliphatic heterocyclic moiety of the 5-membered monocyclic aliphatic heterocyclyl group which may be substituted and may have a double bond in a part of a ring represented by the ring B is azolidinyl, and
   a heteroaryl moiety of the 5-membered monocyclic heteroaryl group which may be substituted represented by the ring C is thiazolyl.
(26) In addition, still another aspect of the present invention includes the compound or the pharmacologically acceptable salt thereof according to any one of the aspects (2) to (25), wherein in the group represented by Formula [II],
   the ring A is a phenyl group which may be substituted with a halogen atom.
(27) In addition, another preferred aspect of the present invention includes the compound or the pharmacologically acceptable salt thereof according to the aspect (2), wherein in the group represented by Formula [II],
   the ring A is a phenyl group which may be substituted or a monocyclic heteroaryl group which may be substituted,
   each of substituents of the phenyl group which may be substituted or the monocyclic heteroaryl group which may be substituted represented by the ring A is one to two groups independently selected from the group consisting of a halogen atom, an alkyl group, a haloalkyl group, an alkoxy group, and a nitro group,
   a heteroaryl moiety of the heterocyclyl group which may be substituted represented by the ring A is a 5- or 6-membered monocyclic heteroaryl having 1 to 4 heteroatoms independently selected from the group consisting of oxygen, nitrogen, and sulfur,
   the ring B is a 7-membered monocyclic aliphatic heterocyclyl group which may be substituted and may have a double bond in a part of a ring,
   a substituent of the 7-membered monocyclic aliphatic heterocyclyl group which may be substituted and may have a double bond in a part of a ring represented by the ring B is one to four groups independently selected from the group consisting of a halogen atom; a hydroxyl group; an oxo group; an alkyl group; a haloalkyl group; an alkoxyalkyl group; an alkoxy group; an alkylene group which may be substituted with one to two groups independently selected from a halogen atom; a cycloalkyl group; an alkanoyl group which may be substituted with one to two groups independently selected from the group consisting of a cycloalkyl group, an aryl group, and an alkoxy group; and an alkoxycarbonyl group which may be substituted with an aryl group,
   an aliphatic heterocyclic moiety of the 7-membered monocyclic aliphatic heterocyclyl group which may be substituted and may have a double bond in a part of a ring represented by the ring B is a 7-membered monocyclic aliphatic heterocyclic ring having 1 to 3 heteroatoms independently selected from the group consisting of oxygen, nitrogen, and sulfur,
   in each of the substituents of the groups represented by the ring B, an aryl moiety of the aryl group is phenyl,
   the ring C is a phenyl group which may be substituted, a 6-membered monocyclic heteroaryl group which may be substituted and may be partially hydrogenated, or a 6-membered monocyclic aliphatic heterocyclyl group which may be substituted,
   each of substituents of the phenyl group which may be substituted, the 6-membered monocyclic heteroaryl group which may be substituted and may be partially hydrogenated, or the 6-membered monocyclic aliphatic heterocyclyl group which may be substituted represented by the ring C is one to four groups independently selected from the group consisting of a halogen atom; a hydroxyl group; an oxo group; a cyano group; an alkyl group; a haloalkyl group; an alkoxy group; a cycloalkoxy group which may be substituted with a cyano group; a haloalkoxy group; an alkylene group; an amino group which may be substituted with one to two groups independently selected from the group consisting of an alkyl group which may be substituted with a cycloalkyl group or an aryl group, an aryl group which may be substituted with a halogen atom, and a cycloalkyl group; an alkylthio group; an aryl group; a cycloalkyl group which may be substituted with one to two groups independently selected from the group consisting of a halogen atom and a haloalkyl group; an aliphatic heterocyclyl group which may be substituted with one to four groups independently selected from the group consisting of a halogen atom, a cyano group, an alkyl group, a haloalkyl group, an alkylene group, and an alkoxy group; an aryloxy group; an aliphatic heterocyclyl oxy group; and an alkanoyl group,
   a heteroaryl moiety of the 6-membered monocyclic heteroaryl group which may be substituted and may be partially hydrogenated represented by the ring C is a 6-membered monocyclic heteroaryl having 1 to 3 heteroatoms independently selected from the group consisting of oxygen, nitrogen, and sulfur,
   an aliphatic heterocyclic moiety of the 6-membered monocyclic aliphatic heterocyclyl group which may be substituted represented by the ring C is a 6-membered monocyclic aliphatic heterocyclic ring having 1 to 3 heteroatoms independently selected from the group consisting of oxygen, nitrogen, and sulfur,
   in each of the substituents of the groups represented by the ring C, an aryl moiety of the aryl group is phenyl, and
   in each of the substituents of the groups represented by the ring C, an aliphatic heterocyclic moiety of the aliphatic heterocyclyl group is a 4- to 9-membered monocyclic or bicyclic aliphatic heterocyclic ring having 1 to 3 heteroatoms independently selected from the group consisting of oxygen, nitrogen, and sulfur.
(28) In addition, another preferred aspect of the present invention includes the compound or the pharmacologically acceptable salt thereof according to the aspect (2), wherein in the group represented by Formula [II],
   the ring A is a phenyl group which may be substituted or a monocyclic heteroaryl group which may be substituted,
   each of substituents of the phenyl group which may be substituted or the monocyclic heteroaryl group which may be substituted represented by the ring A is one to two groups independently selected from the group consisting of a halogen atom, an alkyl group, a haloalkyl group, an alkoxy group, and a nitro group,
   a heteroaryl moiety of the heterocyclyl group which may be substituted represented by the ring A is a 5- or 6-membered monocyclic heteroaryl having 1 to 4 heteroatoms independently selected from the group consisting of oxygen, nitrogen, and sulfur,
   the ring B is a 7-membered monocyclic aliphatic heterocyclyl group which may be substituted and may have a double bond in a part of a ring,
   a substituent of the 7-membered monocyclic aliphatic heterocyclyl group which may be substituted and may have a double bond in a part of a ring represented by the ring B is one to four groups independently selected from the group consisting of a halogen atom; a hydroxyl group; an oxo group; an alkyl group; a haloalkyl group; an alkoxyalkyl group; an alkoxy group; an alkylene group which may be substituted with one to two groups independently selected from a halogen atom; a cycloalkyl group; an alkanoyl group which may be substituted with one to two groups independently selected from the group consisting of a cycloalkyl group, an aryl group, and an alkoxy group; and an alkoxycarbonyl group,
   an aliphatic heterocyclic moiety of the 7-membered monocyclic aliphatic heterocyclyl group which may be substituted and may have a double bond in a part of a ring represented by the ring B is a 7-membered monocyclic aliphatic heterocyclic ring having 1 to 3 heteroatoms independently selected from the group consisting of oxygen, nitrogen, and sulfur,
   in each of the substituents of the groups represented by the ring B, an aryl moiety of the aryl group is phenyl,
   the ring C is a 5-membered monocyclic heteroaryl group which may be substituted or a 5-membered monocyclic aliphatic heterocyclyl group which may be substituted,
   each of substituents of the 5-membered monocyclic heteroaryl group which may be substituted or the 5-membered monocyclic aliphatic heterocyclyl group which may be substituted represented by the ring C is one to four groups independently selected from the group consisting of a halogen atom; a hydroxyl group; an oxo group; a cyano group; an alkyl group; a haloalkyl group; an alkoxy group; a cycloalkoxy group which may be substituted with a cyano group; a haloalkoxy group; an alkylene group; an amino group which may be substituted with one to two groups independently selected from the group consisting of an alkyl group which may be substituted with a cycloalkyl group or an aryl group, an aryl group which may be substituted with a halogen atom, and a cycloalkyl group; an alkylthio group; an aryl group; a cycloalkyl group which may be substituted with one to two groups independently selected from the group consisting of a halogen atom and a haloalkyl group; an aliphatic heterocyclyl group which may be substituted with one to four groups independently selected from the group consisting of a halogen atom, a cyano group, an alkyl group, a haloalkyl group, an alkylene group, and an alkoxy group; and an alkanoyl group,
   a heteroaryl moiety of the 5-membered monocyclic heteroaryl group which may be substituted represented by the ring C is a 5-membered monocyclic heteroaryl having 1 to 4 heteroatoms independently selected from the group consisting of oxygen, nitrogen, and sulfur,
   an aliphatic heterocyclic moiety of the 5-membered monocyclic aliphatic heterocyclyl group which may be substituted represented by the ring C is a 6-membered monocyclic aliphatic heterocyclic ring having 1 to 3 heteroatoms independently selected from the group consisting of oxygen, nitrogen, and sulfur,
   in each of the substituents of the groups represented by the ring C, the aryl group which may be substituted in the selected group is phenyl, and
   in each of the substituents of the groups represented by the ring C, an aliphatic heterocyclic moiety of the aliphatic heterocyclyl group which may be substituted in the selected group is a 4- to 9-membered monocyclic or bicyclic aliphatic heterocyclic ring having 1 to 3 heteroatoms independently selected from the group consisting of oxygen, nitrogen, and sulfur.
(29) In addition, another preferred aspect of the present invention includes the compound or the pharmacologically acceptable salt thereof according to the aspect (2), wherein in the group represented by Formula [II],
   the ring A is a phenyl group which may be substituted or a monocyclic heteroaryl group which may be substituted,
   each of substituents of the phenyl group which may be substituted or the monocyclic heteroaryl group which may be substituted represented by the ring A is one to two groups independently selected from the group consisting of a halogen atom, an alkyl group, a haloalkyl group, an alkoxy group, and a nitro group,
   a heteroaryl moiety of the heterocyclyl group which may be substituted represented by the ring A is a 5- or 6-membered monocyclic heteroaryl having 1 to 4 heteroatoms independently selected from the group consisting of oxygen, nitrogen, and sulfur,
   the ring B is a 6-membered monocyclic aliphatic heterocyclyl group which may be substituted and may have a double bond in a part of a ring,
   a substituent of the 6-membered monocyclic aliphatic heterocyclyl group which may be substituted and may have a double bond in a part of a ring represented by the ring B is one to four groups independently selected from the group consisting of a halogen atom; a hydroxyl group; an oxo group; an alkyl group; a haloalkyl group; an alkoxyalkyl group; an alkoxy group; an alkylene group which may be substituted with one to two groups independently selected from a halogen atom; a cycloalkyl group; an alkanoyl group which may be substituted with one to two groups independently selected from the group consisting of a cycloalkyl group, an aryl group, and an alkoxy group; and an alkoxycarbonyl group,
   an aliphatic heterocyclic moiety of the 6-membered monocyclic aliphatic heterocyclyl group which may be substituted and may have a double bond in a part of a ring represented by the ring B is a 6-membered monocyclic aliphatic heterocyclic ring having 1 to 3 heteroatoms independently selected from the group consisting of oxygen, nitrogen, and sulfur,
   in each of the substituents of the groups represented by the ring B, the aryl group in the selected group is phenyl,
   the ring C is a phenyl group which may be substituted or a 6-membered monocyclic heteroaryl group which may be substituted,
   each of substituents of the phenyl group which may be substituted or the 6-membered monocyclic heteroaryl group which may be substituted represented by the ring C is one to four groups independently selected from the group consisting of a halogen atom; a hydroxyl group; an oxo group; a cyano group; an alkyl group; a haloalkyl group; an alkoxy group; a cycloalkoxy group which may be substituted with a cyano group; a haloalkoxy group; an alkylene group; an amino group which may be substituted with one to two groups independently selected from the group consisting of an alkyl group which may be substituted with a cycloalkyl group or an aryl group, an aryl group which may be substituted with a halogen atom, and a cycloalkyl group; an alkylthio group; an aryl group; a cycloalkyl group which may be substituted with one to two groups independently selected from the group consisting of a halogen atom and a haloalkyl group; an aliphatic heterocyclyl group which may be substituted with one to four groups independently selected from the group consisting of a halogen atom, a cyano group, an alkyl group, a haloalkyl group, an alkylene group, and an alkoxy group; and an alkanoyl group,
   a heteroaryl moiety of the 6-membered monocyclic heteroaryl group which may be substituted represented by the ring C is a 6-membered monocyclic heteroaryl having 1 to 3 heteroatoms independently selected from the group consisting of oxygen, nitrogen, and sulfur,
   in each of the substituents of the groups represented by the ring C, the aryl group in the selected group is phenyl, and
   in each of the substituents of the groups represented by the ring C, an aliphatic heterocyclic moiety of the aliphatic heterocyclyl group which may be substituted in the selected group is a 4- to 9-membered monocyclic or bicyclic aliphatic heterocyclic ring having 1 to 3 heteroatoms independently selected from the group consisting of oxygen, nitrogen, and sulfur.
(30) In addition, another preferred aspect of the present invention includes the compound or the pharmacologically acceptable salt thereof according to the aspect (2), wherein in the group represented by Formula [II],
   the ring A is a phenyl group which may be substituted or a monocyclic heteroaryl group which may be substituted,
   each of substituents of the phenyl group which may be substituted or the monocyclic heteroaryl group which may be substituted represented by the ring A is one to two groups independently selected from the group consisting of a halogen atom, an alkyl group, a haloalkyl group, an alkoxy group, and a nitro group,
   a heteroaryl moiety of the heterocyclyl group which may be substituted represented by the ring A is a 5- or 6-membered monocyclic heteroaryl having 1 to 4 heteroatoms independently selected from the group consisting of oxygen, nitrogen, and sulfur,
   the ring B is a 6-membered monocyclic aliphatic heterocyclyl group which may be substituted and may have a double bond in a part of a ring,
   a substituent of the 6-membered monocyclic aliphatic heterocyclyl group which may be substituted and may have a double bond in a part of a ring represented by the ring B is one to four groups independently selected from the group consisting of a halogen atom; a hydroxyl group; an oxo group; an alkyl group; a haloalkyl group; an alkoxyalkyl group; an alkoxy group; an alkylene group which may be substituted with one to two groups independently selected from a halogen atom; a cycloalkyl group; an alkanoyl group which may be substituted with one to two groups independently selected from the group consisting of a cycloalkyl group, an aryl group, and an alkoxy group; and an alkoxycarbonyl group,
   an aliphatic heterocyclic moiety of the 6-membered monocyclic aliphatic heterocyclyl group which may be substituted and may have a double bond in a part of a ring represented by the ring B is a 6-membered monocyclic aliphatic heterocyclic ring having 1 to 3 heteroatoms independently selected from the group consisting of oxygen, nitrogen, and sulfur,
   in each of the substituents of the groups represented by the ring B, the aryl group in the selected group is phenyl,
   the ring C is a 5-membered monocyclic heteroaryl group which may be substituted,
   a substituent of the 5-membered monocyclic heteroaryl group which may be substituted represented by the ring C is one to four groups independently selected from the group consisting of a halogen atom; a hydroxyl group; an oxo group; a cyano group; an alkyl group; a haloalkyl group; an alkoxy group; a cycloalkoxy group which may be substituted with a cyano group; a haloalkoxy group; an alkylene group; an amino group which may be substituted with one to two groups independently selected from the group consisting of an alkyl group which may be substituted with a cycloalkyl group or an aryl group, an aryl group which may be substituted with a halogen atom, and a cycloalkyl group; an alkylthio group; an aryl group; a cycloalkyl group which may be substituted with one to two groups independently selected from the group consisting of a halogen atom and a haloalkyl group; an aliphatic heterocyclyl group which may be substituted with one to four groups independently selected from the group consisting of a halogen atom, a cyano group, an alkyl group, a haloalkyl group, an alkylene group, and an alkoxy group; and an alkanoyl group,
   a heteroaryl moiety of the 5-membered monocyclic heteroaryl group which may be substituted represented by the ring C is a 5-membered monocyclic heteroaryl having 1 to 4 heteroatoms independently selected from the group consisting of oxygen, nitrogen, and sulfur,
   in the substituent of the group represented by the ring C, the aryl group in the selected group is phenyl, and
   in the substituent of the group represented by the ring C, an aliphatic heterocyclic moiety of the aliphatic heterocyclyl group in the selected group is a 4- to 9-membered monocyclic or bicyclic aliphatic heterocyclic ring having 1 to 3 heteroatoms independently selected from the group consisting of oxygen, nitrogen, and sulfur.
(31) In addition, another preferred aspect of the present invention includes the compound or the pharmacologically acceptable salt thereof according to the aspect (2), wherein in the group represented by Formula [II],
   the ring A is a phenyl group which may be substituted or a monocyclic heteroaryl group which may be substituted,
   each of substituents of the phenyl group which may be substituted or the monocyclic heteroaryl group which may be substituted represented by the ring A is one to two groups independently selected from the group consisting of a halogen atom, an alkyl group, a haloalkyl group, an alkoxy group, and a nitro group,
   a heteroaryl moiety of the heterocyclyl group which may be substituted represented by the ring A is a 5- or 6-membered monocyclic heteroaryl having 1 to 4 heteroatoms independently selected from the group consisting of oxygen, nitrogen, and sulfur,
   the ring B is a 5-membered monocyclic aliphatic heterocyclyl group which may be substituted and may have a double bond in a part of a ring,
   a substituent of the 5-membered monocyclic aliphatic heterocyclyl group which may be substituted and may have a double bond in a part of a ring represented by the ring B is one to four groups independently selected from the group consisting of a halogen atom; a hydroxyl group; an oxo group; an alkyl group; a haloalkyl group; an alkoxyalkyl group; an alkoxy group; an alkylene group which may be substituted with one to two groups independently selected from a halogen atom; a cycloalkyl group; an alkanoyl group which may be substituted with one to two groups independently selected from the group consisting of a cycloalkyl group, an aryl group, and an alkoxy group; and an alkoxycarbonyl group,
   an aliphatic heterocyclic moiety of the 5-membered monocyclic aliphatic heterocyclyl group which may be substituted and may have a double bond in a part of a ring represented by the ring B is a 5-membered monocyclic aliphatic heterocyclic ring having 1 to 3 heteroatoms independently selected from the group consisting of oxygen, nitrogen, and sulfur,
   in each of the substituents of the groups represented by the ring B, the aryl group in the selected group is phenyl,
   the ring C is a phenyl group which may be substituted or a 6-membered monocyclic heteroaryl group which may be substituted,
   each of substituents of the phenyl group which may be substituted or the 6-membered monocyclic heteroaryl group which may be substituted represented by the ring C is one to four groups independently selected from the group consisting of a halogen atom; a hydroxyl group; an oxo group; a cyano group; an alkyl group; a haloalkyl group; an alkoxy group; a cycloalkoxy group which may be substituted with a cyano group; a haloalkoxy group; an alkylene group; an amino group which may be substituted with one to two groups independently selected from the group consisting of an alkyl group which may be substituted with a cycloalkyl group or an aryl group, an aryl group which may be substituted with a halogen atom, and a cycloalkyl group; an alkylthio group; an aryl group; a cycloalkyl group which may be substituted with one to two groups independently selected from the group consisting of a halogen atom and a haloalkyl group; an aliphatic heterocyclyl group which may be substituted with one to four groups independently selected from the group consisting of a halogen atom, a cyano group, an alkyl group, a haloalkyl group, an alkylene group, and an alkoxy group; and an alkanoyl group,
   a heteroaryl moiety of the 6-membered monocyclic heteroaryl group which may be substituted represented by the ring C is a 6-membered monocyclic heteroaryl having 1 to 3 heteroatoms independently selected from the group consisting of oxygen, nitrogen, and sulfur,
   in each of the substituents of the groups represented by the ring C, the aryl group in the selected group is phenyl, and
   in each of the substituents of the groups represented by the ring C, an aliphatic heterocyclic moiety of the aliphatic heterocyclyl group which may be substituted in the selected group is a 4- to 9-membered monocyclic or bicyclic aliphatic heterocyclic ring having 1 to 3 heteroatoms independently selected from the group consisting of oxygen, nitrogen, and sulfur.
(32) In addition, another preferred aspect of the present invention includes the compound or the pharmacologically acceptable salt thereof according to the aspect (2), wherein in the group represented by Formula [II],
   the ring A is a phenyl group which may be substituted or a monocyclic heteroaryl group which may be substituted,
   each of substituents of the phenyl group which may be substituted or the monocyclic heteroaryl group which may be substituted represented by the ring A is one to two groups independently selected from the group consisting of a halogen atom, an alkyl group, a haloalkyl group, an alkoxy group, and a nitro group,
   a heteroaryl moiety of the heterocyclyl group which may be substituted represented by the ring A is a 5- or 6-membered monocyclic heteroaryl having 1 to 4 heteroatoms independently selected from the group consisting of oxygen, nitrogen, and sulfur,
   the ring B is a 5-membered monocyclic aliphatic heterocyclyl group which may be substituted and may have a double bond in a part of a ring,
   a substituent of the 5-membered monocyclic aliphatic heterocyclyl group which may be substituted and may have a double bond in a part of a ring represented by the ring B is one to four groups independently selected from the group consisting of a halogen atom; a hydroxyl group; an oxo group; an alkyl group; a haloalkyl group; an alkoxyalkyl group; an alkoxy group; an alkylene group which may be substituted with one to two groups independently selected from a halogen atom; a cycloalkyl group; an alkanoyl group which may be substituted with one to two groups independently selected from the group consisting of a cycloalkyl group, an aryl group, and an alkoxy group; and an alkoxycarbonyl group,
   an aliphatic heterocyclic moiety of the 5-membered monocyclic aliphatic heterocyclyl group which may be substituted and may have a double bond in a part of a ring represented by the ring B is a 5-membered monocyclic aliphatic heterocyclic ring having 1 to 3 heteroatoms independently selected from the group consisting of oxygen, nitrogen, and sulfur,
   in each of the substituents of the groups represented by the ring B, the aryl group in the selected group is phenyl,
   the ring C is a 5-membered monocyclic heteroaryl which may be substituted,
   a substituent of the 5-membered monocyclic heteroaryl group which may be substituted represented by the ring C is one to four groups independently selected from the group consisting of a halogen atom; a hydroxyl group; an oxo group; a cyano group; an alkyl group; a haloalkyl group; an alkoxy group; a cycloalkoxy group which may be substituted with a cyano group; a haloalkoxy group; an alkylene group; an amino group which may be substituted with one to two groups independently selected from the group consisting of an alkyl group which may be substituted with a cycloalkyl group or an aryl group, an aryl group which may be substituted with a halogen atom, and a cycloalkyl group; an alkylthio group; an aryl group; a cycloalkyl group which may be substituted with one to two groups independently selected from the group consisting of a halogen atom and a haloalkyl group; an aliphatic heterocyclyl group which may be substituted with one to four groups independently selected from the group consisting of a halogen atom, a cyano group, an alkyl group, a haloalkyl group, an alkylene group, and an alkoxy group; and an alkanoyl group,
   a heteroaryl moiety of the 5-membered monocyclic heteroaryl group which may be substituted represented by the ring C is a 5-membered monocyclic heteroaryl having 1 to 4 heteroatoms independently selected from the group consisting of oxygen, nitrogen, and sulfur,
   in the substituent of the group represented by the ring C, the aryl group in the selected group is phenyl, and
   in the substituent of the group represented by the ring C, an aliphatic heterocyclic moiety of the aliphatic heterocyclyl group which may be substituted in the selected group is a 4- to 9-membered monocyclic or bicyclic aliphatic heterocyclic ring having 1 to 3 heteroatoms independently selected from the group consisting of oxygen, nitrogen, and sulfur.
(33) In addition, another preferred aspect of the present invention includes a compound selected from the group consisting of:
   (3R)-4-[2-(2-fluorophenyl)sulfonyl-2-azaspiro[3.3]heptan-6-yl]-3-methyl-8-(trifhioromethyl)-2,3-dihydropyrido [3,4-f] [1,4] oxazepin-5-one;
   (3R)-4-[2-(2-fluorophenyl)sulfonyl-2-azaspiro[3.3]heptan-6-yl]-3-methyl-7-(trifhioromethyl)-2,3-dihydropyrido [3,2-f] [1,4] oxazepin-5-one;
   (3R)-4-[2-(2-fluorophenyl)sulfonyl-2-azaspiro[3.3]heptan-6-yl]-3-methyl-8-(trifhioromethoxy)-2,3-dihydropyrido [3,2-f] [1,4] oxazepin-5-one;
   (3R)-4-[2-(2-fluorophenyl)sulfonyl-2-azaspiro[3.3]heptan-6-yl]-3-methyl-8-(trifhioromethyl)-2,3-dihydropyrido [3,2-f] [1,4] oxazepin-5-one;
   (6R)-2-(1-fluorocyclopropyl)-5-[2-(2-fluorophenyl)sulfonyl-2-azaspiro[3.3]heptan-6-yl]-6-methyl-7,8-dihydro-6H-thiazolo[5,4-c]azepin-4-one;
   (7R)-2-[(1S*,5R*)-3-azabicyclo[3.1.0]hexan-3-yl]-6-[2-(2-fluorophenyl)sulfonyl-2-azaspiro[3.3]heptan-6-yl]-7-methyl-7,8-dihydropyrimido[5,4-f] oxazepin- 5 -one;
   (6R)-7-[2-(2-fluorophenyl)sulfonyl-2-azaspiro[3.3]heptan-6-yl]-2,6-dimethyl-5,6-dihydrothiazolo [5,4-f][1,4] oxazepin- 8-one;
   (6R)-2-(1,1-difluoroethyl)-7-[2-(2-fluorophenyl)sulfonyl-2-azaspiro[3.3]heptan-6-yl]-6-methyl-5,6-dihydrothiazolo[5,4-f] [1,4]oxazepin-8-one;
   (6R)-2-cyclopropyl-7-[2-(2-fluorophenyl)sulfonyl-2-azaspiro[3.3]heptan-6-yl]-6-methyl-5,6-dihydrothiazolo[5,4-f] [1,4]oxazepin-8-one;
   (6R)-2-(1-fluorocyclopropyl)-7-[2-(2-fluorophenyl)sulfonyl-2-azaspiro[3.3]heptan-6-yl]-6-methyl-5,6-dihydrothiazolo[5,4-f] [1,4]oxazepin-8-one;
   (6R)-2-cyclopropyl-5-[2-(2-fluorophenyl)sulfonyl-2-azaspiro[3.3]heptan-6-yl]-6-methyl-7,8-dihydro-6H-pyrazolo[1,5-a] [1,4]diazepin-4-one;
   (6R)-2-(1-fluorocyclopropyl)-5-[2-(2-fluorophenyl)sulfonyl-2-azaspiro[3.3]heptan-6-yl]-6-methyl-7,8-dihydro-6H-pyrazolo[1,5-a][1,4]diazepin-4-one;
   (6R)-5-[2-(2-fluorophenyl)sulfonyl-2-azaspiro[3.3]heptan-6-yl]-6-methyl-2-(trifluoromethyl)-7,8-dihydro-6H-pyrazolo[1,5-a] [1,4]diazepin-4-one;
   (3R)-4-[2-(2-fluorophenyl)sulfonyl-2-azaspiro[3.3]heptan-6-yl]-3,8-dimethyl-2,3-dihydropyrido[3,4-f] [1,4]oxazepin-5-one;
   (3R)-8-cyclopropyl-4-[2-(2-fluorophenyl)sulfonyl-2-azaspiro[3.3]heptan-6-yl]-3-methyl-2,3-dihydropyrido[3,4-f] [1,4]oxazepin-5-one;
   (3R)-8-cyclopropyl-4-[2-(2-fluorophenyl)sulfonyl-2-azaspiro[3.3]heptan-6-yl]-3-methyl-2,3-dihydropyrido[3,2-f] [1,4]oxazepin-5-one;
   (7R)-6-[2-(2-fluorophenyl)sulfonyl-2-azaspiro[3.3]heptan-6-yl]-2,7-dimethyl-7,8-dihydropyrimido[5,4-f] [1,4]oxazepin-5-one;
   (7R)-2-cyclopropyl-6-[2-(2-fluorophenyl)sulfonyl-2-azaspiro[3.3]heptan-6-yl]-7-methyl-7,8-dihydropyrimido[5,4-f][1,4]oxazepin-5-one;
   (3R)-4-[2-(2-fluorophenyl)sulfonyl-2-azaspiro[3.3]heptan-6-yl]-3-methyl-8-(trifluoromethyl)-2,3-dihydropyrido[2,3-f] [1,4]oxazepin-5-one; and
   (3R)-4-[2-(2-fluorophenyl)sulfonyl-2-azaspiro[3.3]heptan-6-yl]-3-methyl-8-(trifluoromethyl)-2,3-dihydro-1H-pyrido[2,3-f][1,4]diazepin-5-one,
      or a pharmacologically acceptable salt thereof.
(34) Another aspect of the present invention includes the compound or the pharmacologically acceptable salt thereof according to the aspect (1), wherein the compound of Formula [I] is a compound represented by the following Formula [III]: 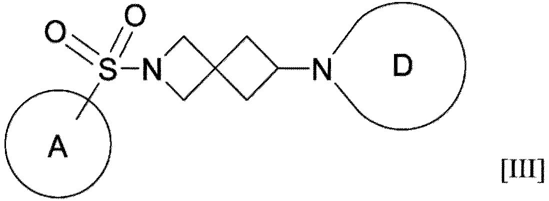 wherein,
   a ring A represents an aryl group which may be substituted, a heteroaryl group which may be substituted, or an aliphatic heterocyclyl group which may be substituted, and
   a ring D represents an aliphatic heterocyclyl group which may be substituted and may have a double bond in a part of a ring.
(35) Another aspect of the present invention includes the compound or the pharmacologically acceptable salt thereof according to the aspect (34), wherein in the group represented by Formula [III],
   each of substituents of the aryl group which may be substituted, the heteroaryl group which may be substituted, or the aliphatic heterocyclyl group which may be substituted represented by the ring A is one to three groups independently selected from the group consisting of a halogen atom, a cyano group, an alkyl group, an alkoxyalkyl group, a haloalkyl group, an alkoxy group, a haloalkoxy group, an alkanoyl group, an alkoxycarbonyl group, an amino group which may be substituted with one to two alkyl groups, an alkylthio group, a sulfonyl group which may be substituted with an alkyl group, an aminocarbonyl group which may be substituted with one to two alkyl groups, a nitro group, and aryl group,
   an aryl moiety of the aryl group which may be substituted represented by the ring A is a 6- to 10-membered monocyclic or bicyclic aryl,
   a heteroaryl moiety of the heteroaryl group which may be substituted represented by the ring A is a 5- to 10-membered monocyclic or bicyclic heteroaryl having 1 to 4 heteroatoms independently selected from the group consisting of oxygen, nitrogen, and sulfur,
   an aliphatic heterocyclic moiety of the aliphatic heterocyclyl group which may be substituted represented by the ring A is a 5- to 10-membered monocyclic or bicyclic aliphatic heterocyclic ring having 1 to 3 heteroatoms independently selected from the group consisting of oxygen, nitrogen, and sulfur,
   each of substituents of the aliphatic heterocyclyl group which may be substituted and may have a double bond in a part of a ring represented by the ring D is one to eight groups independently selected from the group consisting of an oxo group; a an alkyl group which may be substituted; a cycloalkyl group; an aryl group; a heteroaryl group; an aliphatic heterocyclyl group; an alkoxy group; a cycloalkoxy group; an alkylthio group; an amino group; an alkoxycarbonyl group; an alkanoyl group; an alkylsulfonyl group; a halogen atom; a hydroxyl group; and cyano group, and
   an aliphatic heterocyclic moiety of the aliphatic heterocyclyl group which may be substituted and may have a double bond in a part of a ring represented by the ring D is a 5- to 7-membered monocyclic aliphatic heterocyclic ring having 1 to 3 heteroatoms independently selected from the group consisting of oxygen, nitrogen, and sulfur.
(36) In addition, still another aspect of the present invention includes the compound or the pharmacologically acceptable salt thereof according to the aspect (34), wherein in the group represented by Formula [III],
   the ring A is a phenyl group which may be substituted or a monocyclic heteroaryl group which may be substituted,
   each of substituents of the phenyl group which may be substituted or the monocyclic heteroaryl group which may be substituted represented by the ring A is one to two groups independently selected from the group consisting of a halogen atom, an alkyl group, a haloalkyl group, an alkoxy group, and a nitro group,
   a heteroaryl moiety of the monocyclic heteroaryl group which may be substituted represented by the ring A is a 5- or 6-membered monocyclic heteroaryl having 1 to 4 heteroatoms independently selected from the group consisting of oxygen, nitrogen, and sulfur,
   each of substituents of the aliphatic heterocyclyl group which may be substituted and may have a double bond in a part of a ring represented by the ring D is one to eight groups independently selected from the group consisting of an oxo group; a an alkyl group which may be substituted; a cycloalkyl group; an aryl group; a heteroaryl group; an aliphatic heterocyclyl group; an alkoxy group; a cycloalkoxy group; an alkylthio group; an amino group; an alkoxycarbonyl group; an alkanoyl group; an alkylsulfonyl group; a halogen atom; a hydroxyl group; and cyano group,
   in each of the substituents of the groups represented by the ring D, each of substituents of the alkyl group which may be substituted in the selected group is one to five groups independently selected from the group consisting of a cycloalkyl group which may be substituted with an oxo group, a haloalkyl group, or a halogen atom; an aryl group which may be substituted with an alkyl group or a halogen atom; a heteroaryl group which may be substituted with an alkyl group; a heterocyclyl group which may be substituted with an oxo group; a halogen atom; a hydroxyl group; a cyano group; an alkoxy group; a cycloalkoxy group; a haloalkoxy group; an amino group; an alkylamino group; a dialkylamino group; a cycloalkylamino group; and alkylthio group, and
   an aliphatic heterocyclic moiety of the aliphatic heterocyclyl group which may be substituted and may have a double bond in a part of a ring represented by the ring D is a 5- to 7-membered monocyclic aliphatic heterocyclic ring having 1 to 3 heteroatoms independently selected from the group consisting of oxygen, nitrogen, and sulfur.
(37) In addition, still another aspect of the present invention includes the compound or the pharmacologically acceptable salt thereof according to the aspect (34), wherein in the group represented by Formula [III],
   the ring A is a phenyl group which may be substituted or a monocyclic heteroaryl group which may be substituted,
   each of substituents of the phenyl group which may be substituted or the monocyclic heteroaryl group which may be substituted represented by the ring A is one to two groups independently selected from the group consisting of a halogen atom, an alkyl group, a haloalkyl group, an alkoxy group, and a nitro group,
   a heteroaryl moiety of the monocyclic heteroaryl group which may be substituted represented by the ring A is pyrrolyl, furanyl, thienyl, imidazolyl, pyrazolyl, oxazolyl, isoxazolyl, thiazolyl, isothiazolyl, triazolyl, tetrazolyl, pyridyl, pyrazinyl, pyrimidinyl, pyridazinyl, thiazinyl, or triazinyl,
   each of substituents of the aliphatic heterocyclyl group which may be substituted and may have a double bond in a part of a ring represented by the ring D is one to eight groups independently selected from the group consisting of an oxo group; a an alkyl group which may be substituted; a cycloalkyl group; an aryl group; a heteroaryl group; an aliphatic heterocyclyl group; an alkoxy group; a cycloalkoxy group; an alkylthio group; an amino group; an alkoxycarbonyl group; an alkanoyl group; an alkylsulfonyl group; a halogen atom; a hydroxyl group; and cyano group,
   in each of the substituents of the groups represented by the ring D, each of substituents of the alkyl group which may be substituted in the selected group is one to five groups independently selected from the group consisting of a cycloalkyl group which may be substituted with an oxo group, a haloalkyl group, or a halogen atom; an aryl group which may be substituted with an alkyl group or a halogen atom; a heteroaryl group which may be substituted with an alkyl group; a heterocyclyl group which may be substituted with an oxo group; a halogen atom; a hydroxyl group; a cyano group; an alkoxy group; a cycloalkoxy group; a haloalkoxy group; an amino group; an alkylamino group; a dialkylamino group; a cycloalkylamino group; and alkylthio group, and
   an aliphatic heterocyclic moiety of the aliphatic heterocyclyl group which may be substituted and may have a double bond in a part of a ring represented by the ring D is azolidinyl, diazolidinyl, azinanyl, oxazinanyl, diazinanyl, thiazinanyl, azepanil, oxazepanil, diazepanil, or thiazepanil.
(38) In addition, still another aspect of the present invention includes the compound or the pharmacologically acceptable salt thereof according to the aspect (34), wherein in the group represented by Formula [III],
   the ring A is a phenyl group which may be substituted,
   each of substituents of the phenyl group which may be substituted represented by the ring A is a halogen atom,
   each of substituents of the aliphatic heterocyclyl group which may be substituted and may have a double bond in a part of a ring represented by the ring D is one to three groups independently selected from the group consisting of an oxo group; a an alkyl group which may be substituted; an aryl group; a heteroaryl group; an aliphatic heterocyclyl group; and an alkylsulfonyl group,
   in each of the substituents of the groups represented by the ring D, each of substituents of the alkyl group which may be substituted in the selected group is a group independently selected from the group consisting of an oxo group; a cycloalkyl group which may be substituted with a halogen atom; an aryl group; a heteroaryl group; a halogen atom; and an alkoxy group, and
   an aliphatic heterocyclic moiety of the aliphatic heterocyclyl group which may be substituted and may have a double bond in a part of a ring represented by the ring D is azolidinyl, diazolidinyl, azinanyl, oxazinanyl, diazinanyl, thiazinanyl, azepanil, oxazepanil, or diazepanil.
(39) Another aspect of the present invention includes the compound or the pharmacologically acceptable salt thereof according to the aspect (1), wherein the compound of Formula [I] is a compound represented by the following Formula [IV]: 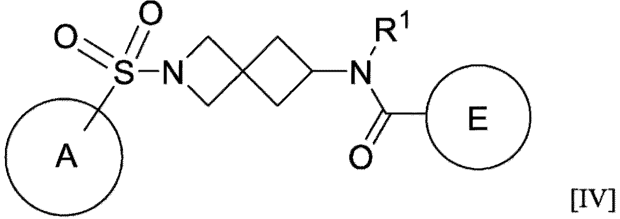 wherein,
   a ring A represents an aryl group which may be substituted, a heteroaryl group which may be substituted, or an aliphatic heterocyclyl group which may be substituted,
   R¹ represents an alkyl group which may be substituted, a cycloalkyl group which may be substituted, an alkenyl group which may be substituted, a cycloalkenyl group which may be substituted, an alkynyl group which may be substituted, an aryl group which may be substituted, a heteroaryl group which may be substituted, an aliphatic heterocyclyl group which may be substituted, an amino group which may be substituted, an alkoxy group which may be substituted, or an alkylthio group which may be substituted, and
   a ring E represents an aryl group which may be substituted, a heteroaryl group which may be substituted, or an aliphatic heterocyclyl group which may be substituted and may have a double bond in a part of a ring.
(40) Another aspect of the present invention includes the compound or the pharmacologically acceptable salt thereof according to the aspect (39), wherein in the group represented by Formula [IV],
   each of substituents of the aryl group which may be substituted, the heteroaryl group which may be substituted, or the aliphatic heterocyclyl group which may be substituted represented by the ring A is one to three groups independently selected from the group consisting of a halogen atom, a cyano group, an alkyl group, an alkoxyalkyl group, a haloalkyl group, an alkoxy group, a haloalkoxy group, an alkanoyl group, an alkoxycarbonyl group, an amino group which may be substituted with one to two alkyl groups, an alkylthio group, a sulfonyl group which may be substituted with an alkyl group, an aminocarbonyl group which may be substituted with one to two alkyl groups, a nitro group, and aryl group,
   an aryl moiety of the aryl group which may be substituted represented by the ring A is a 6- to 10-membered monocyclic or bicyclic aryl,
   a heteroaryl moiety of the heteroaryl group which may be substituted represented by the ring A is a 5- to 10-membered monocyclic or bicyclic heteroaryl having 1 to 4 heteroatoms independently selected from the group consisting of oxygen, nitrogen, and sulfur,
   an aliphatic heterocyclic moiety of the aliphatic heterocyclyl group which may be substituted represented by the ring A is a 5- to 10-membered monocyclic or bicyclic aliphatic heterocyclic ring having 1 to 3 heteroatoms independently selected from the group consisting of oxygen, nitrogen, and sulfur,
   R¹ is an alkyl group which may be substituted, a cycloalkyl group which may be substituted, an alkenyl group which may be substituted, a cycloalkenyl group which may be substituted, or an alkynyl group which may be substituted,
   each of substituents of the alkyl group which may be substituted, the alkenyl group which may be substituted, and the alkynyl group which may be substituted in the group represented by R¹ is a cycloalkyl group or a cycloalkenyl group,
   each of substituents of the cycloalkyl group which may be substituted, and the cycloalkenyl group which may be substituted in the group represented by R¹ is an alkyl group, an alkenyl group or an alkynyl group,
   an aryl moiety of the aryl group which may be substituted represented by the ring E is a 6- to 10-membered monocyclic or bicyclic aryl,
   a heteroaryl moiety of the heteroaryl group which may be substituted represented by the ring E is a 5- to 10-membered monocyclic or bicyclic heteroaryl having 1 to 4 heteroatoms independently selected from the group consisting of oxygen, nitrogen, and sulfur,
   an aliphatic heterocyclic moiety of the aliphatic heterocyclyl group which may be substituted and may have a double bond in a part of a ring represented by the ring E is a 5- to 10-membered monocyclic or bicyclic aliphatic heterocyclic ring having 1 to 3 heteroatoms independently selected from the group consisting of oxygen, nitrogen, and sulfur, and
   each of substituents of the aryl group which may be substituted, the heteroaryl group which may be substituted, or the aliphatic heterocyclyl group which may be substituted and may have a double bond in a part of a ring represented by the ring E is one to three groups independently selected from the group consisting of an alkyl group; a cycloalkyl group; an aryl group; a heteroaryl group; a halogen atom; a haloalkyl group; a hydroxyl group; a cyano group; an alkoxy group; a cycloalkoxy group; an aryloxy group; a haloalkoxy group; an amino group; an alkylamino group; a dialkylamino group; a cycloalkylamino group; and an alkylthio group.
(41) Another aspect of the present invention includes the compound or the pharmacologically acceptable salt thereof according to the aspect (39), wherein in the group represented by Formula [IV],
   the ring A is a phenyl group which may be substituted or a monocyclic heteroaryl group which may be substituted,
   each of substituents of the phenyl group which may be substituted or the monocyclic heteroaryl group which may be substituted represented by the ring A is one to two groups independently selected from the group consisting of a halogen atom, an alkyl group, a haloalkyl group, an alkoxy group, a nitro group, and aryl group,
   a heteroaryl moiety of the monocyclic heteroaryl group which may be substituted represented by the ring A is a 5- to 6-membered monocyclic heteroaryl having 1 to 4 heteroatoms independently selected from the group consisting of oxygen, nitrogen, and sulfur,
   R¹ is an alkyl group which may be substituted, a cycloalkyl group which may be substituted, an alkenyl group which may be substituted, a cycloalkenyl group which may be substituted, or an alkynyl group which may be substituted,
   each of substituents of the alkyl group which may be substituted, the alkenyl group which may be substituted, and the alkynyl group which may be substituted in the group represented by R¹ is a cycloalkyl group or a cycloalkenyl group,
   each of substituents of the cycloalkyl group which may be substituted, and the cycloalkenyl group which may be substituted is an alkyl group, an alkenyl group or an alkynyl group,
   the ring E is an aryl group which may be substituted, or a heteroaryl group which may be substituted,
   an aryl moiety of the aryl group which may be substituted represented by the ring E is a 6- to 10-membered monocyclic or bicyclic aryl,
   a heteroaryl moiety of the heteroaryl group which may be substituted represented by the ring E is a 5- to 10-membered monocyclic or bicyclic heteroaryl having 1 to 4 heteroatoms independently selected from the group consisting of oxygen, nitrogen, and sulfur, and
   each of substituents of the aryl group which may be substituted, and the heteroaryl group which may be substituted represented by the ring E is one to three groups independently selected from the group consisting of an alkyl group; a cycloalkyl group; an aryl group; a heteroaryl group; a halogen atom; a haloalkyl group; a hydroxyl group; a cyano group; an alkoxy group; a cycloalkoxy group; an aryloxy group; a haloalkoxy group; an amino group; an alkylamino group; a dialkylamino group; a cycloalkylamino group; and an alkylthio group.
(42) Another aspect of the present invention includes the compound or the pharmacologically acceptable salt thereof according to the aspect (39), wherein in the group represented by Formula [IV],
   the ring A is a phenyl group which may be substituted or a monocyclic heteroaryl group which may be substituted,
   each of substituents of the phenyl group which may be substituted or the monocyclic heteroaryl group which may be substituted represented by the ring A is one to two groups independently selected from the group consisting of a halogen atom, an alkyl group, a haloalkyl group, an alkoxy group, a nitro group, and an aryl group,
   a heteroaryl moiety of the monocyclic heteroaryl group which may be substituted represented by the ring A is pyrrolyl, furanyl, thienyl, imidazolyl, pyrazolyl, oxazolyl, isoxazolyl, thiazolyl, isothiazolyl, triazolyl, tetrazolyl, pyridyl, pyrazinyl, pyrimidinyl, pyridazinyl, thiazinyl, or triazinyl,
   R¹ is an alkyl group,
   the ring E represents an aryl group which may be substituted, or a heteroaryl group which may be substituted,
   an aryl moiety of the aryl group which may be substituted represented by the ring E is phenyl or naphthyl,
   a heteroaryl moiety of the heteroaryl group which may be substituted represented by the ring E is pyridyl, azaindolyl, imidazopyridyl or benzoimidazolyl, and
   each of substituents of the aryl group which may be substituted, and the heteroaryl group which may be substituted represented by the ring E is a group independently selected from the group consisting of an alkyl group; a cycloalkyl group; an aryl group; a heteroaryl group; a halogen atom; a haloalkyl group; a hydroxyl group; a cyano group; an alkoxy group; a cycloalkoxy group; an aryloxy group; a haloalkoxy group; an amino group; an alkylamino group; a dialkylamino group; a cycloalkylamino group; and an alkylthio group.
(43) Another aspect of the present invention includes the compound or the pharmacologically acceptable salt thereof according to the aspect (39), wherein in the group represented by Formula [IV],
   the ring A is a phenyl which may be substituted or thienyl group which may be substituted,
   each of substituents of the phenyl group which may be substituted or the thienyl group which may be substituted represented by the ring A is one to two groups independently selected from the group consisting of an aryl group, a halogen atom, an alkyl group, and an alkoxy group,
   R¹ is a propyl group,
   the ring E is a phenyl group which may be substituted, or a heteroaryl group which may be substituted,
   a heteroaryl moiety of the heteroaryl group which may be substituted represented by the ring E is pyridyl, azaindolyl, imidazopyridyl or benzoimidazolyl, and
   each of substituents of the phenyl group which may be substituted, and the heteroaryl group which may be substituted represented by the ring E is a group independently selected from the group consisting of an alkyl group, a dialkylamino group, a cycloalkylamino group and an alkoxy group.
(44) In addition, still another preferred aspect of the present invention includes a pharmaceutical composition containing the compound or the pharmacologically acceptable salt thereof according to any one of the aspects (1) to (43) as an active ingredient.
(45) Another aspect of the present invention includes the pharmaceutical composition according to the aspect (44), wherein the pharmaceutical composition is used for preventing or treating various diseases and/or symptoms associated therewith that are ameliorated by increasing a central TRH concentration, or for improving prognosis of these diseases and/or symptoms associated therewith.
(46) In addition, still another aspect of the invention includes the pharmaceutical composition according to the aspect (44), wherein the pharmaceutical composition is a TRH-DE inhibitor.
(47) In addition, still another aspect of the present invention includes the pharmaceutical composition according to the aspect (45) or (46), wherein the pharmaceutical composition is an agent for preventing or treating spinocerebellar degeneration, chronic pain, a sleep disorder, or other psychoneurotic diseases and/or symptoms associated therewith.
(48) In addition, still another aspect of the present invention includes the pharmaceutical composition according to the aspect (47), wherein the chronic pain is neuropathic pain or nociceptive pain.
(49) In addition, still another aspect of the present invention includes the pharmaceutical composition according to the aspect (48), wherein the neuropathic pain is pain associated with diabetic neuropathy, postherpetic neuralgia, pain associated with peripheral neuropathy due to chemotherapy, trigeminal neuralgia, complex regional pain syndrome, post-stroke pain, spinal cord injury pain, neuralgia, or pain associated with nerve damage.
(50) In addition, still another aspect of the present invention includes the pharmaceutical composition according to the aspect (48), wherein the nociceptive pain is pain associated with rheumatoid arthritis, pain associated with osteoarthritis deformans, postoperative pain, or myofascial pain.
(51) In addition, still another aspect of the present invention includes the pharmaceutical composition according to the aspect (47), wherein the sleep disorder is hypersomnia, a circadian rhythm disorder, sleep-disordered breathing, or other sleep disorders.
(52) In addition, still another aspect of the present invention includes the pharmaceutical composition according to the aspect (51), wherein the hypersomnia is narcolepsy, idiopathic hypersomnia, or repetitive hypersomnia.
(53) In addition, still another aspect of the present invention includes the pharmaceutical composition according to the aspect (51), wherein the circadian rhythm disorder is a sleep disorder due to shift work, delayed sleep phase syndrome, advanced sleep phase syndrome, non-24-hour sleep-wake syndrome, or an irregular sleep-wake pattern.
(54) In addition, still another aspect of the present invention includes the pharmaceutical composition according to the aspect (51), wherein the sleep-disordered breathing is sleep apnea syndrome.
(55) In addition, still another aspect of the present invention includes the pharmaceutical composition according to the aspect (51), wherein the other sleep disorders are jet lag syndrome, desynchronosis syndrome, restless legs syndrome, a periodic limb movement disorder, hypersomnia associated with insomnia associated with a neurological disorder or a mental disorder, or a rapid eye movement (REM) sleep behavior disorder.
(56) In addition, still another aspect of the present invention includes the pharmaceutical composition according to the aspect (47), wherein the other psychoneurotic diseases and/or the symptoms associated therewith are spinal muscular atrophy, amyotrophic lateral sclerosis, Parkinson's disease, Alzheimer's disease, chronic fatigue syndrome, vascular dementia, a bipolar disorder, depression, a spinal cord injury, cerebral infarction, head trauma, a pervasive consciousness disorder, a language disorder, fatigability, dysfunction due to nerve damage, or dysfunction due to surgery.
(57) In addition, still another aspect of the present invention includes a treatment method for various diseases and/or symptoms associated therewith that are ameliorated by increasing a central TRH concentration, the treatment method including administering a therapeutically effective amount of the compound or the pharmacologically acceptable salt thereof according to any one of the aspects (1) to (43) to a patient.
(58) In addition, another aspect of the present invention includes the treatment method according to the aspect (57), wherein the various diseases and/or the symptoms associated therewith that are ameliorated by increasing a central TRH concentration are spinocerebellar degeneration, pain, a sleep disorder, or other psychoneurotic diseases and/or symptoms associated therewith.

In a case where the compound [1], [11], [III] or [IV] of the present invention has an asymmetric carbon atom in the molecule, the compound [1], [11], [III] or [IV] may exist as a plurality of stereoisomers (that is, diastereomeric isomers and optical isomers) based on the asymmetric carbon atom, but the present invention encompasses any one of these stereoisomers and a mixture thereof.

The compound [1], [11], [III] or [IV] of the present invention encompasses a compound labeled with an isotope (for example, ³H, ¹³C, ¹⁴C, ¹⁵N, ¹⁸F, ³²P, ³⁵S, ¹²⁵I, or the like) or the like and a deuterated product.

The compound [1], [11], [III] or [IV] of the present invention or the pharmacologically acceptable salt thereof has a TRH-DE inhibitory activity, and is thus useful for prevention or treatment for various diseases and/or symptoms associated therewith that can be ameliorated by increasing a central TRH concentration, or for improving prognosis of these diseases. Examples of such diseases include spinocerebellar degeneration, chronic pain, a sleep disorder, or other psychoneurotic diseases and/or symptoms associated therewith, and in particular, chronic pain, a sleep disorder, and the like are preferable.

Examples of the spinocerebellar degeneration include hereditary spinocerebellar degeneration and sporadic (non-hereditary) spinocerebellar degeneration.

Examples of the hereditary spinocerebellar degeneration include autosomal recessive hereditary diseases and/or symptoms associated therewith, and autosomal dominant hereditary diseases and/or symptoms associated therewith.

Examples of the autosomal recessive hereditary diseases and/or the symptoms associated therewith include Friedreich's ataxia, ataxia with vitamin E deficiency, aprataxin deficiency, senataxin deficiency, and Charlevoix-Saguenay type spastic ataxia. Examples of the autosomal dominant hereditary diseases and/or the symptoms associated therewith include spinocerebellar ataxia type 1 (SCA1), spinocerebellar ataxia type 2 (SCA2), spinocerebellar ataxia type 3 (SCA3), spinocerebellar ataxia type 6 (SCA6), spinocerebellar ataxia type 31 (SCA31), and dentatorubral pallidoluysian atrophy.

Examples of the sporadic (non-hereditary) spinocerebellar degeneration include pure cerebellar ataxia type spinocerebellar degeneration and multiple system disorder type spinocerebellar degeneration.

Examples of the pure cerebellar ataxia type spinocerebellar degeneration include cortical cerebellar atrophy.

Examples of the multiple system disorder type spinocerebellar degeneration include multiple system atrophy.

Examples of the multiple system atrophy include olivopontocerebellar atrophy, nigrostriatal degeneration, and Shy-Drager syndrome.

Examples of the chronic pain include neuropathic pain, nociceptive pain, and mixed pain. Examples of the neuropathic pain include pain associated with diabetic neuropathy, postherpetic neuralgia, pain associated with peripheral neuropathy due to chemotherapy, trigeminal neuralgia, complex regional pain syndrome, post-stroke pain, spinal cord injury pain, neuralgia, and pain associated with nerve damage. Examples of the nociceptive pain include pain associated with rheumatoid arthritis, pain associated with osteoarthritis deformans, postoperative pain, and myofascial pain. Examples of the mixed pain include cancer pain, fibromyalgia syndrome, chronic back pain, and phantom limb pain.

Examples of the sleep disorder include hypersomnia, a circadian rhythm disorder, sleep-disordered breathing, and hypersomnia associated with other sleep disorders. Examples of the hypersomnia include narcolepsy, idiopathic hypersomnia, and repetitive hypersomnia. Examples of the circadian rhythm disorder include a sleep disorder due to shift work, delayed sleep phase syndrome, advanced sleep phase syndrome, non-24-hour sleep-wake syndrome, and an irregular sleep-wake pattern. Examples of the sleep-disordered breathing include sleep apnea syndrome. Examples of the other sleep disorders include jet lag syndrome, desynchronosis syndrome, restless legs syndrome, a periodic limb movement disorder, hypersomnia associated with insomnia associated with a neurological disorder or a mental disorder, and a rapid eye movement (REM) sleep behavior disorder.

Examples of the other psychoneurotic diseases and/or symptoms associated therewith include the following:
hereditary ataxia (such as congenital cerebellar ataxia and Machado-Joseph disease),
spinal muscular atrophy and related syndromes (such as spinal muscular atrophy, spinal and bulbar muscular atrophy, amyotrophic lateral sclerosis, and cervical spondylotic muscular atrophy),
extrapyramidal disorders and abnormal movements (such as Parkinson's disease and Parkinson's syndrome),
other degenerative diseases of the nervous system (such as Alzheimer's disease and progressive leukoencephalopathy),
intercurrent and paroxysmal disorders (such as epilepsy, migraine and cluster headache syndrome),
cerebral palsy and other paralytic syndromes (such as cerebral palsy, hemiplegia, paraplegia, and quadriplegia),
chronic fatigue syndrome,
symptomatic organic mental disorders (such as Alzheimer's disease, vascular dementia, and delirium),
mental and behavioral disorders due to the use of psychoactive substances (such as psycho-behavioral disorders due to alcohol use and psycho-behavioral disorders due to drug use),
schizophrenia, schizophrenia-type disorders, and delusional disorders,
mood disorders (such as bipolar disorders, depression, and persistent mood disorders),
eating disorders (such as anorexia nervosa and bulimia nervosa),
neurotic disorders, stress-related disorders, and somatic symptom disorders (such as phobic anxiety disorders, obsessive-compulsive disorder, and somatic symptom disorders),
psychological developmental disorders (autism spectrum disorders),
behavioral and emotional disorders that usually occur in childhood and adolescence (such as hyperactivity disorders and tic disorders),
obesity,
adrenoleukodystrophy,
a spinal cord injury,
cerebral infarction,
a head injury,
delayed consciousness disorders,
symptoms and signs of cognition, perception, emotional state, and behavior (such as nervousness, emotional anxiety and agitation, lethargy, and dullness of emotion)
language disorders,
fatigability,
dysfunction due to nerve damage,
dysfunction due to surgery, and the like.

The compound [I], [II], [III] or [IV] of the present invention or the pharmacologically acceptable salt thereof has an excellent inhibitory activity on TRH-DE as described above. The inhibitory activity on TRH-DE can be confirmed according to the assay method of an experimental example "2. TRH-DE inhibition test" described below. In addition, the efficacy of the compound [1], [11], [III] or [IV] of the present invention or the pharmacologically acceptable salt thereof is also confirmed in a central nervous system activation test (evaluation using a SCANET device in accordance with the method described in Japanese Patent No. 4817281) using mechanical stimulation hyperalgesia (a randall-selitto method) or a locomotor activity as an index in a Chronic constriction injury (CCI) model rat (prepared according to the method described in Pain 1988; 33:87-107), which is a neuropathic pain model.

The compound [I], [II], [III] or [IV] of the present invention can be used for pharmaceutical use either in a free form or in a pharmacologically acceptable salt form. Examples of the pharmacologically acceptable salt include inorganic acid salts such as hydrochloride, sulfate, phosphate, and hydrobromide, and organic acid salts such as acetate, fumarate, oxalate, citrate, methanesulfonate, benzenesulfonate, tosylate, and maleate.

The compound [I], [II], [III] or [IV] of the present invention or the pharmacologically acceptable salt thereof contains any of an intramolecular salt or an adduct thereof, a solvate or a hydrate thereof, and the like.

The compound [I], [II], [III] or [IV] of the present invention or the pharmacologically acceptable salt thereof can be orally or parenterally administered alone or as a pharmaceutical composition containing the compound or the pharmacologically acceptable salt thereof, and a pharmacologically acceptable carrier. The pharmacologically acceptable carrier may be a carrier commonly used in the art, and examples thereof include a diluent, a binder (syrup, gum arabic, gelatin, sorbitol, tragacanth, polyvinylpyrrolidone, or the like), an excipient (lactose, sucrose, cornstarch, potassium phosphate, sorbitol, glycine, or the like), a lubricant (magnesium stearate, talc, polyethylene glycol, silica, or the like), a disintegrant (potato starch), and a wetting agent (sodium lauryl sulfate or the like).

A dosage form of such a pharmaceutical composition is not particularly limited, and examples thereof include common pharmaceutical formulations such as a tablet, granules, a capsule, powder, an injection, an inhalant, and a suppository.

A dose of the compound [I], [II], [III] or [IV] of the present invention or the pharmacologically acceptable salt thereof varies depending on an administration method, an age, body weight, condition, or the like of a patient, and in the case of parenteral administration, the dose is usually 0.001 mg/kg to 100 mg/kg and preferably 0.03 mg/kg to 30 mg/kg per day. In the case of oral administration, the dose is usually 0.001 mg/kg to 100 mg/kg and preferably 0.03 mg/kg to 30 mg/kg per day.

The compound of the present invention or the pharmacologically acceptable salt thereof can be produced, for example, as follows.

### Synthesis Method A1

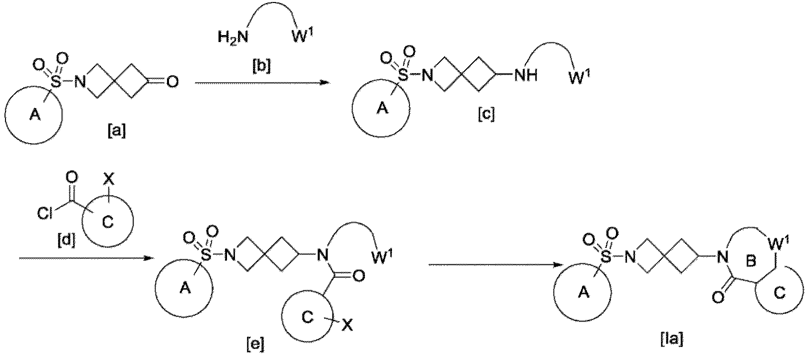

wherein, W¹ represents a hydroxyl group or an amino group which may be protected by a protective group,
X represents a leaving group such as a halogen atom,
a ring A represents an aryl group which may be substituted, a heteroaryl group which may be substituted, or an aliphatic heterocyclyl group which may be substituted,
a ring B represents an aliphatic heterocyclyl group which may be substituted and may have a double bond in a part of a ring, and
a ring C represents an aryl group which may be substituted and may be partially hydrogenated, a heteroaryl group which may be substituted and may be partially hydrogenated, a cycloalkyl group which may be substituted, or an aliphatic heterocyclyl group which may be substituted.

Among the target compounds [1] of the present invention, a compound represented by General Formula [Ia] can be produced, for example, as follows.

First, a compound represented by General Formula [a] is subjected to a reductive amination reaction with a compound represented by General Formula [b] to obtain a compound represented by General Formula [c]. The target product [1a] can be produced by an intramolecular SnAr reaction of the compound represented by General Formula [e] obtained by amidation of a compound represented by General Formula [c] and a compound represented by General Formula [d].

The reductive amination reaction between the compound [a] and the compound [b] or the salt thereof can be performed in an appropriate solvent in the presence of, for example, a reducing agent and an acid or a Lewis acid, according to a normal method. As the solvent, any solvent may be used as long as it does not interfere with the present reaction, and examples thereof include halogenated aliphatic hydrocarbons such as methylene chloride, alcohols such as methanol, ethers such as tetrahydrofuran, aromatic hydrocarbons such as toluene, and a mixture thereof. Examples of the reducing agent include sodium triacetoxyborohydride, sodium borohydride, hydrogen, and a palladium catalyst (for example, a palladium catalyst supported on activated carbon or the like). Examples of the acid include acetic acid. Examples of the Lewis acid include titanium(IV) chloride and tetraisopropyl titanate. The amount of the compound [b] used can be 1 to 10 equivalents, and preferably 1 to 5 equivalents, with respect to the compound [a]. The amount of the reducing agent used can be 1 to 10 equivalents, and preferably 1 to 3 equivalents, with respect to the compound [a]. The amount of the acid or Lewis acid used can be 1 to 10 equivalents, and preferably 1 to 3 equivalents, with respect to the compound [a]. The present reaction can be performed at - 10°C to 100°C, and preferably 10°C to 50°C.

The reaction between the compound [c] and the compound [d] can be performed in an appropriate solvent in the presence or absence of a base according to a normal method. As the solvent, any solvent may be used as long as it does not interfere with the present reaction, and examples thereof include ethers such as tetrahydrofuran, amides such as N,N-dimethylformamide, acetonitrile, and a mixture thereof. Examples of the base include triethylamine, diisopropylethylamine, and diazabicycloundecene. The amount of the compound [c] used can be 1.0 to 10.0 equivalents, and preferably 1.0 to 3.0 equivalents, with respect to the compound [d]. The present reaction can be performed at -78°C to room temperature, and preferably 0°C to room temperature.

The intramolecular SnAr reaction of the compound [e] can be performed in an appropriate solvent in the presence of a base according to a normal method. As the solvent, any solvent may be used as long as it does not interfere with the present reaction, and examples thereof include ethers such as tetrahydrofuran, amides such as N,N-dimethylformamide, acetonitrile, water, and a mixture thereof. Examples of the base include alkali metal hydrides such as sodium hydride, alkali metal alkoxides such as sodium tert-butoxide, organic amines such as diisopropylethylamine, and sodium hydroxide. The amount of the base used can be 1.0 to 10.0 equivalents, and preferably 1.0 to 3.0 equivalents, with respect to the compound [e]. The present reaction can be performed at -78°C to room temperature, and preferably 0°C to room temperature. Note that in the reaction between the compound [c] and the compound [d], the intramolecular SnAr reaction of the compound [e] is continuously performed in the same reaction system, and the compound [Ia] can also be obtained. In addition, in the reaction between the compound [c] and the compound [d], the compound [e] can be obtained by reacting an excessive amount of the compound [d] with a hydroxyl group or an amino group which may be protected by a protective group represented by W¹, and then performing hydrolysis.

### Synthesis Method A2

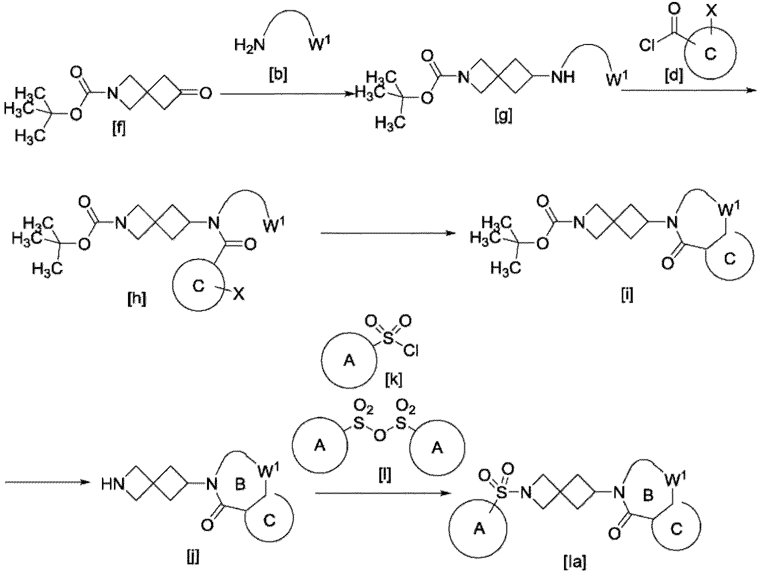

wherein, symbols represent the same meanings as described above.

Among the target compounds [1] of the present invention, a compound represented by General Formula [Ia] can be produced, for example, as follows.

A compound [i] is obtained in the same manner as that of Synthesis Method A1 using a compound [f], and then the compound [i] is deprotected to obtain a compound [j]. The target product [Ia] can be produced by a reaction of the compound [j] or the salt thereof with sulfonic anhydride or sulfonyl chloride.

A reductive amination reaction between the compound [f] and the compound [b] or the salt thereof can be performed in the same manner as the reaction between the compound [a] and the compound [b] in Synthesis Method A1.

A reaction between a compound [g] and the compound [d] can be performed in the same manner as the reaction between the compound [c] and the compound [d] in Synthesis Method A1.

A reaction from a compound [h] to the compound [i] can be performed in the same manner as the reaction from the compound [e] to the compound [Ia] in Synthesis Method A1.

A deprotection reaction of the compound [i] can be performed in an appropriate solvent in the presence of an acid or a Lewis acid according to a normal method. As the solvent, any solvent may be used as long as it does not interfere with the present reaction, and examples thereof include ethers such as 1,4-dioxane and tetrahydrofuran, halogenated aliphatic hydrocarbons such as chloroform, aromatic hydrocarbons such as toluene, acetonitrile, and a mixture thereof. Examples of the acid include hydrochloric acid, sulfuric acid, and trifluoroacetic acid. Examples of the Lewis acid include triethylsilyl trifluoromethanesulfonate. The amount of the acid or Lewis acid used can be 1.0 to 10.0 equivalents, and preferably 1.0 to 2.0 equivalents, with respect to the compound [i]. The present reaction can be performed at -78°C to room temperature, and preferably 0°C to room temperature.

The reaction between the compound [j] or the salt thereof and sulfonic anhydride or sulfonyl chloride can be performed in an appropriate solvent in the presence or absence of a base according to a normal method. As the solvent, any solvent may be used as long as it does not interfere with the present reaction, and examples thereof include ethers such as tetrahydrofuran, amides such as dimethylformamide, and a mixture thereof. Examples of the base include amines such as triethylamine. The amount of sulfonic anhydride or sulfonyl chloride used can be 1.0 to 10.0 equivalents, and preferably 1.0 to 3.0 equivalents, with respect to the compound [j] or the salt thereof. The amount of the base used can be 1.0 to 10.0 equivalents, and preferably 1.0 to 3.0 equivalents, with respect to the compound [j]. The present reaction can be performed at -78°C to room temperature, and preferably 0°C to room temperature.

### Synthesis Method B

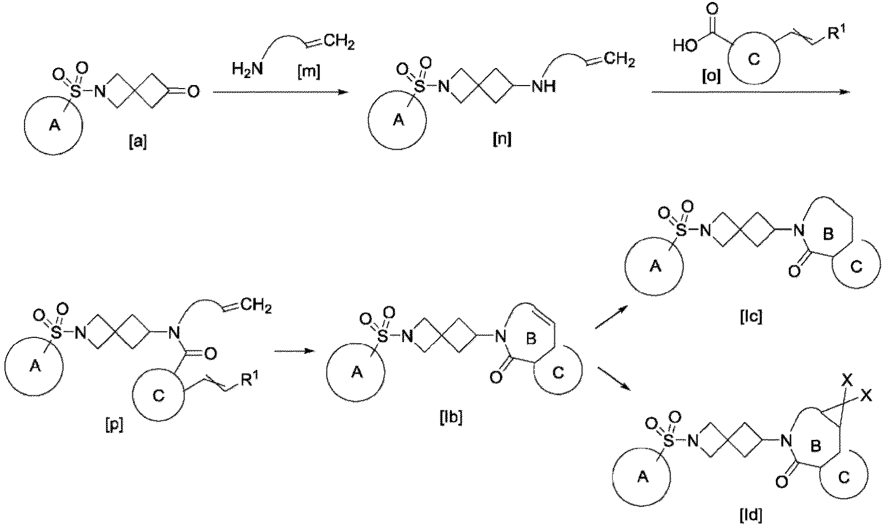

wherein, R¹ represents a hydrogen atom or an alkyl group, and other symbols represent the same meanings as described above.

Among the target compounds [1] of the present invention, a compound represented by General Formula [Ib], [Ic], or [Id] can be produced, for example, as follows.

The compound [a] and a compound [m] are subjected to a reductive amination reaction to obtain a compound [n]. The target product [Ib] can be produced by a ring-closing metathesis reaction of a compound [p] obtained by a reaction of the compound [n] and a compound [o]. In addition, the target product [Ic] can be produced by reducing the obtained compound [Ib]. The compound [Ib] is subjected to cyclopropylation, such that the target product [1d] can be produced.

The reaction between the compound [a] and the compound [m] can be performed in the same manner as the reaction between the compound [a] and the compound [b] in Synthesis Method A1.

The reaction between the compound [n] and the compound [o] can be performed in an appropriate solvent in the presence of, for example, a condensing agent and a base, according to a normal method. As the solvent, any solvent may be used as long as it does not interfere with the present reaction, and examples thereof include amides such as dimethylformamide, ethers such as tetrahydrofuran, halogenated aliphatic hydrocarbons such as chloroform, aromatic hydrocarbons such as toluene, acetonitrile, and a mixture thereof. Examples of the condensing agent include o-(7-azabenzotriazol-1-yl)-N,N,N',N'-tetramethyluronium hexafluorophosphate (HATU) and 1-ethyl-3-(3-dimethylaminopropyl)carbodiimide hydrochloride salt. Examples of the base include amines such as diisopropylethylamine. The amount of the condensing agent used can be 1.0 to 5.0 equivalents, and preferably 1.2 to 3.0 equivalents, with respect to the compound [n]. The amount of the base used can be 0 to 10 equivalents, and preferably 1.0 to 3.0 equivalents, with respect to the compound [n]. The present reaction can be performed at 0°C to 100°C, and preferably room temperature.

The ring-closing metathesis reaction of the compound [p] can be performed in an appropriate solvent in the presence of a catalyst according to a normal method. As the solvent, any solvent may be used as long as it does not interfere with the present reaction, and examples thereof include ethers such as tetrahydrofuran, halogenated aliphatic hydrocarbons such as chloroform, aromatic hydrocarbons such as toluene, acetonitrile, and a mixture thereof. Examples of the catalyst include a Grubbs catalyst (first generation or second generation) and a Hoveyda-Grubbs catalyst (first generation or second generation). The amount of the catalyst used can be 0.01 to 5.0 equivalents, and preferably 0.1 to 1.0 equivalent, with respect to the compound [p]. The present reaction can be performed at 0 to 100°C, and preferably room temperature.

A reduction reaction for the compound [Ib] can be performed by a reaction performed in an appropriate solvent in the presence of a catalyst under a hydrogen atmosphere according to a normal method. As the solvent, any solvent may be used as long as it does not interfere with the present reaction, and examples thereof include alcohols such as methanol, esters such as ethyl acetate, ethers such as tetrahydrofuran, and a mixture thereof. Examples of the catalyst include a palladium/carbon catalyst. The amount of the catalyst used can be 0.3 to 1.0, and preferably 0.3 in terms of weight ratio, with respect to the compound [Ib]. The present reaction can be performed at 0°C to room temperature, and preferably room temperature.

The reaction from the compound [Ib] to the compound [1d] can be performed in an appropriate solvent in the presence of (trifluoromethyl)trimethylsilane and sodium iodide according to a normal method. Examples of the solvent include ethers such as tetrahydrofuran. The amount of (trifluoromethyl)trimethylsilane used can be 1 to 10 equivalents, and preferably 3 to 5 equivalents, with respect to the compound [Ib]. The amount of sodium iodide used can be 0.1 to 5 equivalents, and preferably 0.3 to 1.0 equivalent, with respect to the compound [Ib]. The present reaction can be performed at room temperature to 150°C, and preferably 50°C to 80°C.

### Synthesis Method C

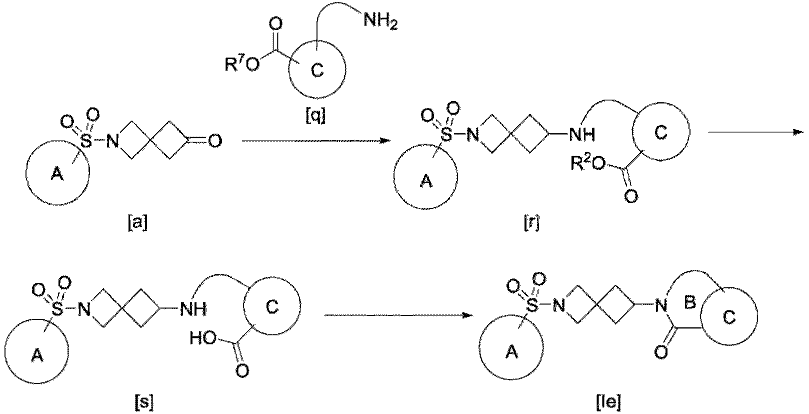

wherein, R² represents a protective group such as an alkyl group, and other symbols represent the same meanings as described above.

Among the target compounds [1] of the present invention, a compound represented by General Formula [Ie] can be produced, for example, as follows.

The compound [a] and a compound [q] or a salt thereof are subjected to a reductive amination reaction to obtain a compound [r]. The target product [Ie] can be produced by hydrolyzing the compound [r] to obtain a compound [s] or a salt thereof, and then performing an intramolecular condensation reaction.

A reductive amination reaction between the compound [a] and the compound [q] or the salt thereof can be performed in the same manner as the reaction between the compound [a] and the compound [b] in Synthesis Method A1.

The hydrolysis of the compound [r] can be performed in an appropriate solvent in the presence of a base and water according to a normal method. As the solvent, any solvent may be used as long as it does not interfere with the present reaction, and examples thereof include alcohols such as ethanol and ethers such as tetrahydrofuran. Examples of the base include alkali metal hydroxides such as sodium hydroxide. The present reaction can be performed at 0°C to room temperature, and preferably room temperature. The amount of the base used can be 1.0 to 10.0 equivalents, and preferably 1.0 to 3.0 equivalents, with respect to the compound [r].

The intramolecular condensation reaction of the compound [s] or the salt thereof can be performed in an appropriate solvent in the presence of, for example, a condensing agent and a base, according to a normal method. As the solvent, any solvent may be used as long as it does not interfere with the present reaction, and examples thereof include amides such as dimethylformamide, ethers such as tetrahydrofuran, halogenated aliphatic hydrocarbons such as chloroform, aromatic hydrocarbons such as toluene, acetonitrile, and a mixture thereof. Examples of the condensing agent include o-(7-azabenzotriazol-1-yl)-N,N,N',N'-tetramethyluronium hexafluorophosphate (HATU) and 1-ethyl-3-(3-dimethylaminopropyl)carbodiimide hydrochloride salt. Examples of the base include amines such as diisopropylethylamine. The amount of the condensing agent used can be 1.0 to 5.0 equivalents, and preferably 1.2 to 3.0 equivalents, with respect to the compound [s]. The present reaction can be performed at 0°C to 100°C, and preferably room temperature.

### Synthesis Method D

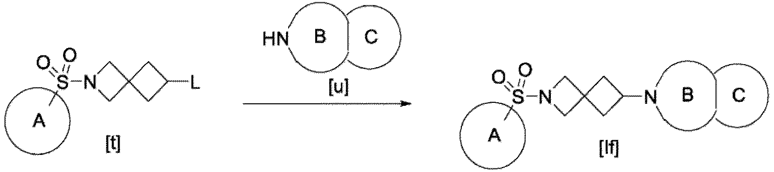

wherein, L represents a leaving group such as 4-methyl benzene sulfonate or benzene sulfonate, and other symbols represent the same meanings as described above.

Among the target compounds [1] of the present invention, a compound represented by General Formula [If] can be produced, for example, by a nucleophilic substitution reaction between a compound [t] and a compound [u].

The nucleophilic substitution reaction between the compound [t] and the compound [u] can be performed in an appropriate solvent in the presence of, for example, a base, according to a normal method. As the solvent, any solvent may be used as long as it does not interfere with the present reaction, and examples thereof include amides such as dimethylformamide, ethers such as tetrahydrofuran, halogenated aliphatic hydrocarbons such as chloroform, aromatic hydrocarbons such as toluene, acetonitrile, and a mixture thereof. Examples of the base include alkali metal hydrides such as sodium hydride and potassium carbonate. The amount of the compound [u] used can be 1.0 to 5.0 equivalents, and preferably 1.2 to 3.0 equivalents, with respect to the compound [t]. The present reaction can be performed at 0°C to 200°C, and preferably 100°C to 150°C.

### Synthesis Method E

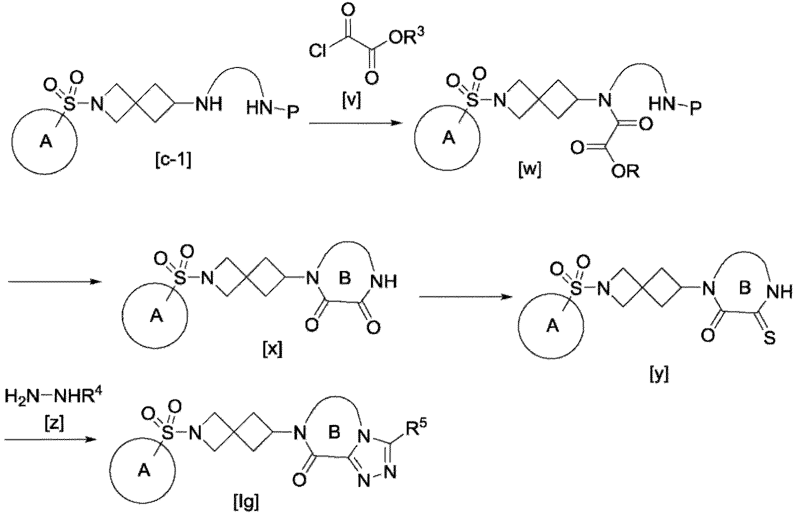

wherein, P represents a protective group such as a tert-butyloxycarbonyl group or a benzyloxycarbonyl group, R³ represents an alkyl group, R⁴ represents alkyl group, R⁵ represents alkyl group, and other symbols represent the same meanings as described above.

Among the target compounds [1] of the present invention, a compound represented by General Formula [Ig] can be produced, for example, as follows.

A compound [x] is obtained by deprotection accompanied by an intramolecular cyclization reaction of a compound [w] obtained by a condensation reaction between a compound represented by General Formula [c-1], among the compounds represented by General Formula [c] in Synthesis Method A1, and a compound [v]. The target product [Ig] can be produced by obtaining a compound [y] by thioamidation of the compound [x], and then performing a reaction with a hydrazine derivative [z] or a salt thereof.

The reaction between the compound [c-1] and the compound [v] can be performed in the same manner as the reaction between the compound [c] and the compound [d] in Synthesis Method A1.

The deprotection accompanied by the intramolecular cyclization reaction of the compound [w] can be performed in the same manner as the cyclization reaction of the compound [e] in Synthesis Method A1.

The compound [y] can be produced by reacting the compound [x] in a solvent in the presence of a sulfurizing agent and in the presence of a base.

Examples of the sulfurizing agent include Lawesson's reagent (2,4-bis(4-methoxyphenyl)-1,3,2,4-dithiadiphosphetane-2,4-disulfide). Examples of the base include amines such as triethylamine, N,N-diisopropylethylamine, and pyridine. As the solvent, any solvent may be used as long as it does not affect the reaction, and examples thereof include aromatic hydrocarbons such as toluene and xylene, and ether such as tetrahydrofuran, 1,4-dioxane, or 1,2-dimethoxyethane.

The amount of the sulfurizing agent used in the present reaction is 0.4 to 2.0 equivalents, and preferably 0.5 to 0.7 equivalents, with respect to the compound [x]. The amount of the base used is 1.0 to 20 equivalents, and preferably 2.0 to 7.0 equivalents, with respect to the compound [x]. The present reaction can be performed at 50°C to 180°C, and preferably 80°C to 130°C.

The reaction between the compound [y] and the compound [z] or the salt thereof can be performed, for example, in an appropriate solvent in the presence or absence of a base. As the salt of the compound [z], for example, a salt with an inorganic acid such as hydrochloride or sulfate can be used. As the solvent, any solvent may be used as long as it does not interfere with the present reaction, and examples thereof include amides such as N-methylpyrrolidone, ethers such as tetrahydrofuran, alcohols such as methanol, dimethyl sulfoxide, water, and a mixture thereof. A base may be added to accelerate the reaction. Examples of such a base include diisopropylethylamine, diazabicycloundecene, and sodium carbonate, and diisopropylethylamine is preferable. The amount of the compound [z] or the salt thereof used can be 1.0 to 10 equivalents, and preferably 2.0 to 7.0 equivalents, with respect to the compound [y]. The present reaction can be performed at room temperature to 250°C, and preferably 120°C to 180°C.

### Synthesis Method F

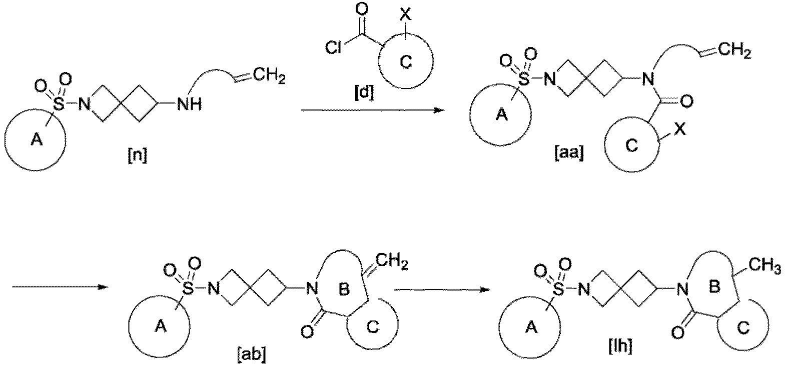

wherein, symbols represent the same meanings as described above.

Among the target compounds [1] of the present invention, a compound represented by General Formula [Ih] can be produced, for example, as follows.

Amidation of the compound [n] and the compound [d] is performed to obtain a compound [aa]. The target product [1h] can be produced by cyclization of the compound [aa] by an intramolecular coupling reaction to obtain a compound [ab], and then performing reduction.

First, the amidation of the compound [n] and the compound [d] can be performed in the same manner as the reaction between the compound [c] and the compound [d] in Synthesis Method A1.

The intramolecular coupling reaction of the compound [aa] can be performed in an appropriate solvent in the presence of a base, a phosphonium-based ligand, and a palladium catalyst and in the presence or absence of water according to a normal method. As the solvent, any solvent may be used as long as it does not interfere with the present reaction, and examples thereof include ethers such as dioxane, amides such as dimethylformamide, aromatic hydrocarbons such as toluene, and a mixture thereof. Examples of the phosphonium-based ligand include bromotri(pyrrolidino)phosphonium hexafluorophosphate (PyBROP) and benzotriazol-1-yloxy-trisdimethylaminophosphonium (BOP). Examples of the base include amines such as triethylamine, alkali metal carbonates such as sodium carbonate, and potassium phosphate. Examples of the palladium catalyst include bis(triphenylphosphine)palladium dichloride, tetrakis(triphenylphosphine)dipalladium, palladium acetate, and 1,1'-bis(diphenylphosphino)ferrocene palladium dichloride. The amount of the phosphonium-based condensing agent used can be 1.0 to 2.0 equivalents, and preferably 1.2 to 1.5 equivalents, with respect to the compound [aa]. The amount of the base used can be 2.0 to 6.0 equivalents, and preferably 3.0 to 4.0 equivalents, with respect to the compound [aa]. The amount of the palladium catalyst used can be 0.01 to 1.0 equivalent, and preferably 0.1 to 0.5 equivalents, with respect to the compound [aa]. The present reaction can be performed at room temperature to 150°C, and preferably 80°C to 120°C.

The reduction reaction of the compound [ab] can be performed in the same manner as the production of the compound [1d] in Synthesis Method B.

### Synthesis Method G

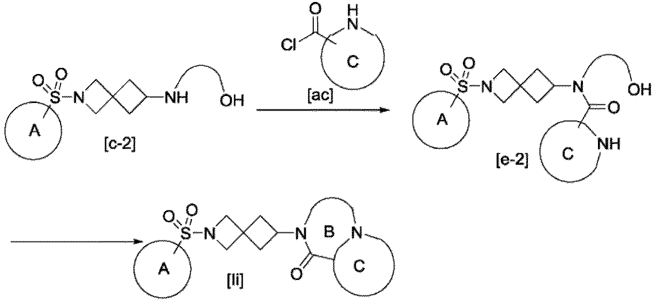

wherein, symbols represent the same meanings as described above.

Among the target compounds [1] of the present invention, a compound represented by General Formula [Ii] can be produced, for example, as follows.

The target product [11] can be produced by cyclization through an intramolecular Mitsunobu reaction of a compound [e-2] obtained by an amidation reaction between the compound represented by General Formula [c-2], among the compounds represented by General Formula [c] in Synthesis Method A1, and a compound [ac].

The amidation reaction between the compound [c-2] and the compound [ac] can be performed in the same manner as the reaction between the compound [c] and the compound [d] in Synthesis Method A1.

The cyclization through the intramolecular Mitsunobu reaction of the compound [e-2] can be performed in an appropriate solvent in the presence of an azodicarboxylic acid derivative and a phosphine derivative according to a normal method. As the solvent, any solvent may be used as long as it does not affect the present reaction, and examples thereof include ethers such as tetrahydrofuran and 1,4-dioxane, aromatic hydrocarbons such as toluene, nitriles such as acetonitrile, and a mixture thereof. Examples of the azodicarboxylic acid derivative include dialkyl azodicarboxylate such as diethyl azodicarboxylate or diisopropyl azodicarboxylate; and azodicarbonamide such as N,N,N',N'-tetramethylazodicarboxamide. Examples of the phosphine derivative include triarylphosphine such as triphenylphosphine and trialkylphosphine such as tributylphosphine. The amount of the azodicarboxylic acid derivative used can be 1.0 to 5.0 equivalents, and preferably 2.0 to 3.0 equivalents, with respect to the compound [e-2]. The amount of the phosphine derivative used can be 1.0 to 5.0 equivalents, and preferably 2.0 to 3.0 equivalents in terms of molar ratio, with respect to the compound [e-2]. The present reaction can be performed at 0°C to 120°C, and preferably room temperature to 80°C.

### Synthesis Method H1

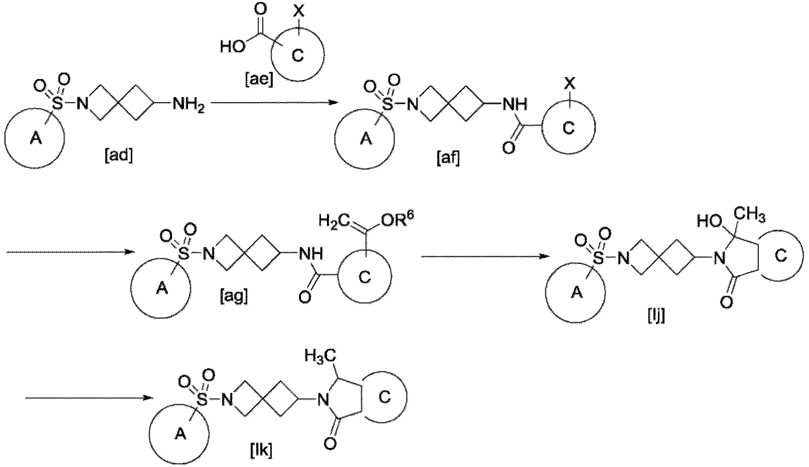

wherein, R⁶ represents a protective group such as an alkyl group, and other symbols represent the same meanings as described above.

Among the target compounds [1] of the present invention, a compound represented by General Formula [Ij] or [Ik] can be produced, for example, as follows.

A coupling reaction can be performed on a compound [af] obtained by an amidation reaction between a compound [ad] and the compound [c-2] to obtain a compound [ag]. The target product [1j] can be produced by a cyclization reaction of the compound [ag]. Furthermore, the target product [Ik] can also be produced by subjecting the target product [Ij] to a dehydration reaction.

First, the amidation reaction between the compound [ad] and the compound [c-2] can be performed in the same manner as the reaction between the compound [n] and the compound [o] in Synthesis Method B.

Next, the coupling reaction of the compound [af] can be performed in an appropriate solvent in the presence of a base, a phosphonium-based ligand, a palladium catalyst, and an organotin compound according to a normal method. As the solvent, any solvent may be used as long as it does not interfere with the present reaction, and examples thereof include ethers such as dioxane, amides such as dimethylformamide, aromatic hydrocarbons such as toluene, and a mixture thereof. Examples of the base include alkali metal carbonates such as sodium carbonate, potassium phosphate, and cesium fluoride. Examples of the phosphonium-based ligand include bromotri(pyrrolidino)phosphonium hexafluorophosphate (PyBROP) and benzotriazol-1-yloxy-trisdimethylaminophosphonium (BOP). Examples of the palladium catalyst include bis(triphenylphosphine)palladium dichloride, tetrakis(triphenylphosphine)dipalladium, palladium acetate, and 1,1'-bis(diphenylphosphino)ferrocene palladium dichloride. Examples of the organotin compound include (1-ethoxyethenyl)tributylstannane. The amount of the phosphonium-based condensing agent used can be 1.0 to 2.0 equivalents, and preferably 1.2 to 1.5 equivalents, with respect to the compound [af]. The amount of the base used can be 2.0 to 6.0 equivalents, and preferably 3.0 to 4.0 equivalents, with respect to the compound [af]. The amount of the palladium catalyst used can be 0.01 to 1.0 equivalent, and preferably 0.1 to 0.5 equivalents, with respect to the compound [af]. The amount of the organotin compound used can be 1.0 to 5.0 equivalents, and preferably 1.2 to 2.5 equivalents, with respect to the compound [af]. The present reaction can be performed at room temperature to 150°C, and preferably 80°C to 120°C.

The cyclization reaction of the compound [ag] can be performed by treating the compound [ag] with an acid in an appropriate solvent. As the solvent, any solvent may be used as long as it does not interfere with the present reaction, and examples thereof include esters such as ethyl acetate, halogenated aliphatic hydrocarbons such as chloroform, alcohols such as methanol, and a mixture thereof. Examples of the acid include hydrochloric acid and trifluoroacetic acid.

The dehydration reaction of the target product [1j] can be performed in an appropriate solvent in the presence of a reducing agent and a Lewis acid according to a normal method. As the solvent, any solvent may be used as long as it does not interfere with the present reaction, and examples thereof include ethers such as tetrahydrofuran, halogenated aliphatic hydrocarbons such as chloroform, aromatic hydrocarbons such as toluene, acetonitrile, and a mixture thereof. Examples of the reducing agent include triethylsilane. Examples of the Lewis acid include a boron trifluoride-ethyl ether complex. The amount of the reducing agent used can be 1.0 to 15.0 equivalents, and preferably 1.2 to 7.0 equivalents, with respect to the compound [Ij]. The amount of the reducing agent used can be 1.0 to 10.0 equivalents, and preferably 1.2 to 5.0 equivalents, with respect to the compound [Ij]. The present reaction can be performed at -100°C to room temperature, and preferably -50°C to 0°C.

### Synthesis Method H2

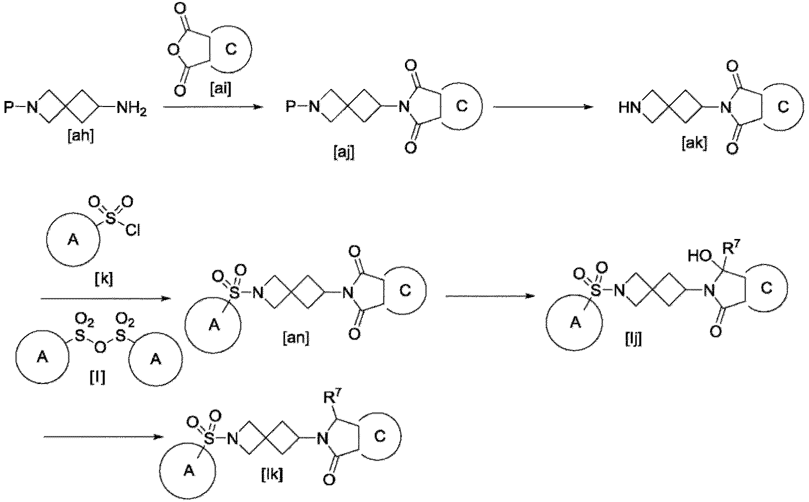

wherein, R⁷ represents an alkyl group, and other symbols represent the same meanings as described above.

Among the target compounds [1] of the present invention, a compound represented by General Formula [Ij] or [Ik] can be produced, for example, as follows.

A compound [aj] obtained by performing a reaction between a compound [ah] and a compound [ai] can be subjected to deprotection to obtain a compound [ak] or a salt thereof. A compound [an] can be obtained by sulfonamidation of the compound [ak] or the salt thereof. The target product [1j] can be produced by subjecting the compound [an] to a nucleophilic addition reaction. Furthermore, the target product [Ik] can also be produced by subjecting the target product [Ij] to a dehydration reaction similar to Synthesis Method H1.

First, the reaction between the compound [ah] and the compound [ai] can be performed by removing generated water from the reaction system using a Dean-Stark apparatus or the like in an appropriate solvent in the presence or absence of a base according to a normal method. As the solvent, any solvent may be used as long as it does not interfere with the present reaction, and examples thereof include ethers such as tetrahydrofuran, amides such as N,N-dimethylformamide, aromatic hydrocarbons such as toluene, and acetonitrile. Examples of the base include amines such as triethylamine. The amount of the compound [ai] used can be 1.0 to 5.0 equivalents, and preferably 1.1 to 1.5 equivalents, with respect to the compound [ah]. The amount of the base used can be 0 to 5.0 equivalents, and preferably 0.2 to 1.5 equivalents, with respect to the compound [ah]. The present reaction can be performed at room temperature to 180°C, and preferably 110°C to 150°C.

Next, the deprotection of the compound [aj] can be performed in the same manner as the reaction from the compound [i] to the compound [j] in Synthesis Method A2.

A sulfonamidation reaction of the compound [ak] can be performed in the same manner as the reaction from the compound [j] to the compound [Ia] in Synthesis Method A2.

The nucleophilic addition reaction of the compound [an] can be performed in an appropriate solvent in the presence of a Grignard reagent (organomagnesium halide) according to a normal method. As the solvent, any solvent may be used as long as it does not interfere with the present reaction, and examples thereof include ethers such as tetrahydrofuran, aromatic hydrocarbons such as toluene, aliphatic hydrocarbons such as hexane, and a mixture thereof. The amount of the Grignard reagent used can be 1.0 to 10.0 equivalents, and preferably 1.0 to 5.0 equivalents, with respect to the compound [an]. The present reaction can be performed at -78°C to room temperature, and preferably 0°C to room temperature.

### Synthesis Method I

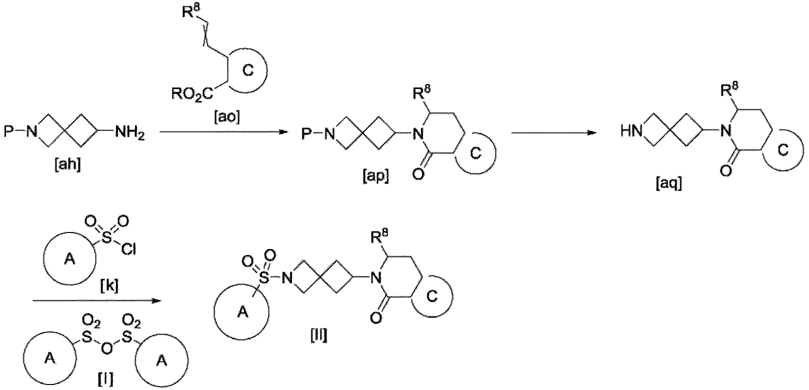

wherein, R⁸ represents an alkyl group such as a methyl group, and other symbols represent the same meanings as described above.

Among the target compounds [I] of the present invention, a compound represented by General Formula [Il] can be produced, for example, as follows.

A compound [ap] obtained by performing a reaction between the compound [ah] and a compound [ao] can be subjected to deprotection to obtain a compound [aq] or a salt thereof. The target product [Il] can be produced by sulfonamidation of the compound [aq] or the salt thereof.

First, the reaction between the compound [ah] and the compound [ao] can be performed in an appropriate solvent according to a normal method. As the solvent, any solvent may be used as long as it does not interfere with the present reaction, and examples thereof include amides such as N-methylpyrrolidone, ethers such as tetrahydrofuran, alcohols such as methanol, dimethyl sulfoxide, and a mixture thereof. The amount of the compound [ao] used can be 0.2 to 5.0 equivalents, and preferably 0.3 to 2.0 equivalents, with respect to the compound [ah]. The present reaction can be performed at room temperature to 180°C, and preferably room temperature to 130°C.

Next, the deprotection of the compound [ap] can be performed in the same manner as the reaction from the compound [i] to the compound [j] in Synthesis Method A2.

A sulfonamidation reaction of the compound [aq] can be performed in the same manner as the reaction from the compound [j] to the compound [Ia] in Synthesis Method A2.

### Synthesis Method J

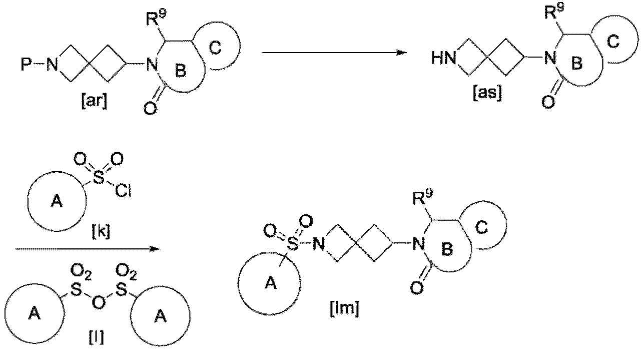

wherein, R⁹ represents an alkyl group such as a methyl group, and other symbols represent the same meanings as described above.

Among the target compounds [1] of the present invention, a compound represented by General Formula [1m] can be produced, for example, as follows.

A compound [as] or a salt thereof can be obtained by performing deprotection of a compound [ar]. The target product [1m] can be produced by sulfonamidation of the compound [as] or the salt thereof.

First, the deprotection of the compound [ar] can be performed in the same manner as the reaction from the compound [i] to the compound [j] in Synthesis Method A2.

Next, the sulfonamidation reaction of the compound [as] can be performed in the same manner as the reaction from the compound [j] to the compound [Ia] in Synthesis Method A2.

### Synthesis Method K

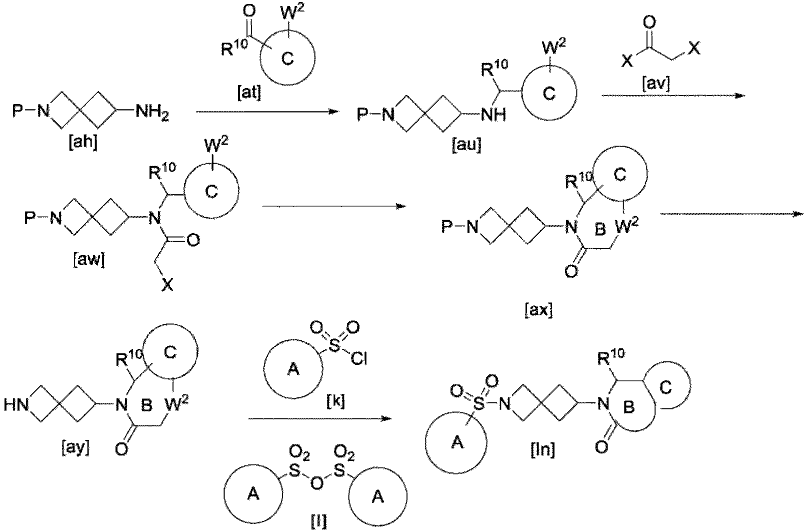

wherein, R¹⁰ represents an alkyl group such as a methyl group, W² represents a hydroxyl group or an amino group, and other symbols represent the same meanings as described above.

Among the target compounds [1] of the present invention, a compound represented by General Formula [In] can be produced, for example, as follows.

A compound [au] can be obtained by performing a reductive alkylation reaction between the compound [ah] and a compound [at]. A compound [ax] can be obtained by amidation and cyclization of the compound [au]. The target product [In] can be produced by performing deprotection of the compound [ax] and sulfonamidation.

First, the reductive alkylation reaction between the compound [ah] and the compound [at] can be performed in the same manner as the reaction between the compound [a] and the compound [b] in Synthesis Method A1.

Next, an amidation reaction between the compound [au] and a compound [av] can be performed in the same manner as the reaction between the compound [c] and the compound [d] in Synthesis Method A1.

A cyclization reaction of a compound [aw] can be performed in an appropriate solvent in the presence of a base according to a normal method. As the solvent, any solvent may be used as long as it does not interfere with the present reaction, and examples thereof include ethers such as tetrahydrofuran, amides such as N,N-dimethylformamide, acetonitrile, water, and a mixture thereof. Examples of the base include alkali metal hydrides such as sodium hydride, alkali metal alkoxides such as sodium tert-butoxide, organic amines such as diisopropylethylamine, and alkali metal carbonates such as sodium hydroxide and sodium carbonate. The amount of the base used can be 1.0 to 10.0 equivalents, and preferably 1.0 to 3.0 equivalents, with respect to the compound [aw]. The present reaction can be performed at -78°C to room temperature, and preferably 0°C to room temperature.

A deprotection reaction of the compound [ax] can be performed in the same manner as the reaction from the compound [i] to the compound [j] in Synthesis Method A2.

A sulfonamidation reaction of the compound [ay] can be performed in the same manner as the reaction from the compound [j] to the compound [Ia] in Synthesis Method A2.

### Synthesis Method L

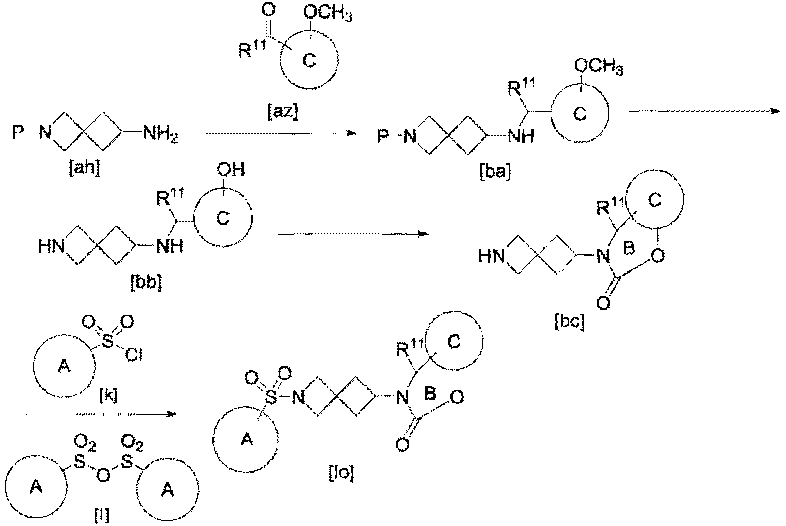

wherein, R¹¹ represents an alkyl group such as a methyl group, and other symbols represent the same meanings as described above.

Among the target compounds [1] of the present invention, a compound represented by General Formula [Io] can be produced, for example, as follows.

A compound [ba] can be obtained by performing a reductive alkylation reaction between the compound [ah] and a compound [az]. A compound [be] can be obtained by performing deprotection of the compound [ba] to obtain a compound [bb] followed by intramolecular cyclization of the compound [bb]. The target product [Io] can be produced by performing sulfonamidation of the compound [be].

First, the reductive alkylation reaction between the compound [ah] and the compound [az] can be performed in the same manner as the reaction between the compound [a] and the compound [b] in Synthesis Method A1.

Next, a deprotection reaction of the compound [ba] can be performed in an appropriate solvent in the presence of a Lewis acid according to a normal method. As the solvent, any solvent may be used as long as it does not interfere with the present reaction, and examples thereof include ethers such as 1,4-dioxane and tetrahydrofuran, halogenated aliphatic hydrocarbons such as chloroform, aromatic hydrocarbons such as toluene, acetonitrile, and a mixture thereof. Examples of the Lewis acid include boron tribromide. The amount of the Lewis acid used can be 1.0 to 10.0 equivalents, and preferably 1.0 to 2.0 equivalents, with respect to the compound [ba]. The present reaction can be performed at -100°C to room temperature, and preferably -78°C to room temperature.

An intramolecular cyclization reaction of a compound [bb] can be performed by reacting carbonyldiimidazole in a suitable solvent (e.g., N,N-dimethylformamide) in the presence of a base (e.g., diisopropylethylamine).

A sulfonamidation reaction of the compound [be] can be performed in the same manner as the reaction from the compound [j] to the compound [Ia] in Synthesis Method A2.

### Synthesis Method M

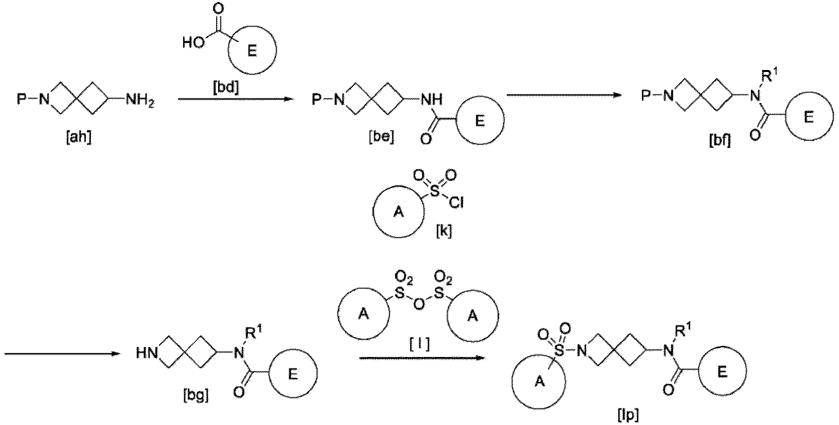

wherein, P represents a protective group such as a tert-butoxycarbonyl group, and other symbols represent the same meanings as described above.

Among the target compounds [1] of the present invention, a compound represented by General Formula [1p] can be produced, for example, as follows.

A compound [be] obtained by an amidation reaction of a compound [ah] with a compound [bd] is subject to alkylation to obtain a compound [bf], which is then deprotected to obtain a compound [bg] or a salt thereof. The target product [1p] can be produced by performing sulfonamidation of the compound [bg] or the salt thereof.

First, the amidation reaction between the compound [ah] and the compound [bd] can be performed in the same manner as the reaction between the compound [n] and the compound [o] in Synthesis Method B.

Next, the alkylation reaction of the compound [be] can be performed by reacting the compound [be] with an alkylating agent to achieve N-Alkylation. For example, the N-alkylation can be performed by reacting the compound [be] with methyl iodide, propyl iodide or the like in a solvent (e.g., acetonitrile or the like) in the presence of a base (e.g., sodium hydride or the like).

The deprotection reaction of the compound [bf] can be performed in the same manner as the reaction from the compound [i] to the compound [j] in Synthesis Method A2.

The reaction of the compound [bg] or the salt thereof with sulfonic anhydride or sulfonyl chloride can be performed in the same manner as the reaction of the compound [j] with sulfonic anhydride or sulfonyl chloride in Synthesis Method A2.

### Synthesis Method N

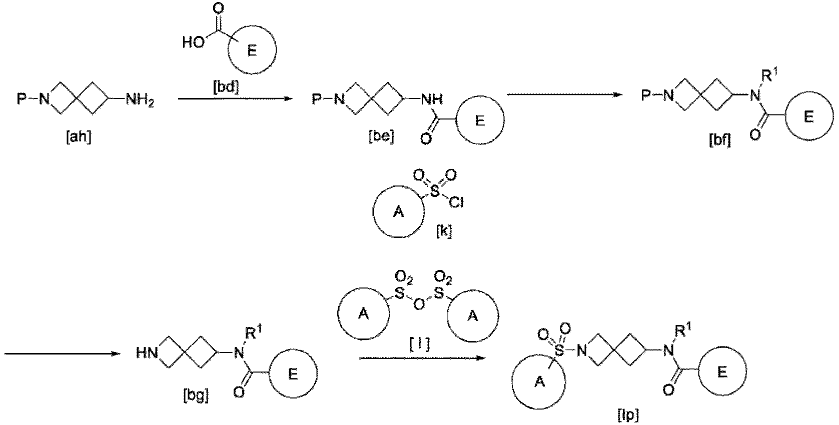

wherein, Ns represents a nosyl group (2-nitrobenzenesulfonyl group), and other symbols represent the same meanings as described above.

Among the target compounds [1] of the present invention, a compound represented by General Formula [Iq] can be produced, for example, as follows.

N-alkylation is performed on a compound [bh] obtained by the reaction of a compound [ah] with NsCl (2-nitrobenzenesulfonyl chloride) to obtain a compound [bi], which is then deprotected to obtain a compound [bj] or salt thereof. The compound [bj] or the salt thereof can be sulfonamidated to obtain a compound [bk]. A compound [bl] or a salt thereof can be obtained by performing a deprotection (denosylation) of the compound [bk]. The target product [Iq] can be produced by performing an amidation reaction of the compound [bl] or a salt thereof with a compound [bd].

First, the reaction of the compound [ah] with NsCl can be performed in a suitable solvent (e.g. dichloromethane or the like) in the presence of a base (e.g. triethylamine or the like) according to a normal method.

Next, the N-alkylation reaction of the compound [bh] can be performed in the same manner as the reaction from the compound [be] to the compound [bf] in Synthesis Method M.

The deprotection reaction of the compound [bi] can be performed in the same manner as the reaction from the compound [i] to the compound [j] in Synthesis Method A2.

The reaction of the compound [bj] or the salt thereof with sulfonic anhydride or sulfonyl chloride can be performed in the same manner as the reaction of the compound [j] with sulfonic anhydride or sulfonyl chloride in Synthesis Method A2.

The deprotection (denosylation) reaction of the compound [bk] can be performed by reacting the compound [bk] with a thiol compound (e.g. mercaptoacetic acid) in a suitable solvent (e.g. N,N-dimethylformamide) in the presence of a base (e.g. lithium hydroxide) according to a normal method.

The amidation reaction of the compound [bl] or the salt thereof can be performed in the same manner as the reaction from the compound [n] to the compound [o] in Synthesis Method B.

### Synthesis Method O

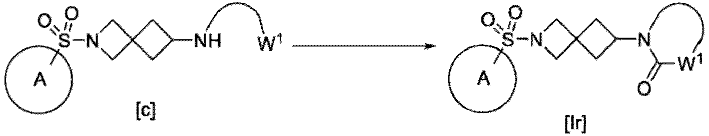

wherein, the symbols represent the same meanings as described above.

Among the target compounds [1] of the present invention, a compound represented by General Formula [1r] can be produced by an intramolecular cyclization reaction of a compound [c].

The intramolecular cyclization of the compound [c] can be performed by reacting the compound [c] with carbonyldiimidazole in a suitable solvent (e.g. N,N-dimethylformamide) and in the presence of a base (e.g. diisopropylethylamine) according to a normal method.

### Synthesis Method P

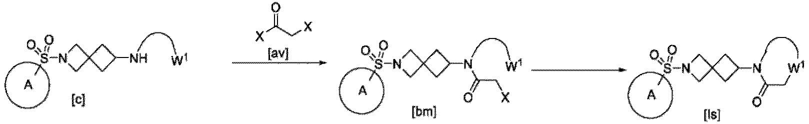

wherein, the symbols represent the same meanings as described above.

Among the target compounds [1] of the present invention, a compound represented by General Formula [Is] can be produced, for example, by cyclizing a compound [bm] obtained by amidating a compound [c].

First, the amidation reaction of the compound [c] with a compound [av] can be performed in the same manner as the reaction of the compound [au] with the compound [av] in Synthesis Method K. As a solvent, halogenated aliphatic hydrocarbons such as chloroform and methylene chloride, or a mixture thereof may be used in addition to the same solvent as above.

The cyclization reaction of the compound [bm] can be performed in the same manner as the cyclization reaction of the compound [aw] in Synthesis Method K.

### Synthesis Method Q

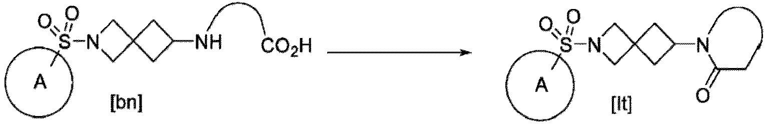

wherein, the symbols represent the same meanings as described above.

Among the target compounds [1] of the present invention, a compound represented by General Formula [It] can be produced by an intramolecular condensation reaction of a compound [bn].

The intramolecular condensation reaction of the compound [bn] can be performed in the same manner as the intramolecular condensation reaction of the compound [s] in Synthesis Method C.

### Synthesis Method R

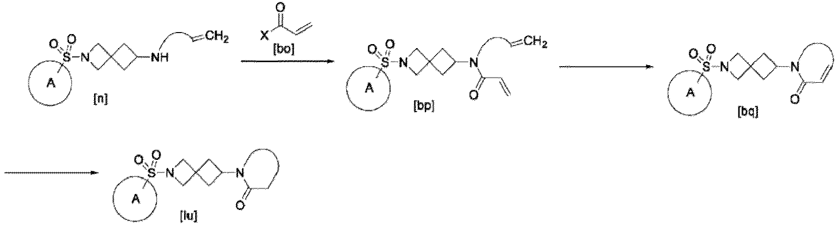

wherein, the symbols represent the same meanings as described above.

Among the target compounds [1] of the present invention, a compound represented by General Formula [1u] can be can be produced, for example, as follows.

A compound [bq] can be obtained by ring-closing metathesis reaction of a compound [bp] obtained by amidation of a compound [n]. The obtained compound [bq] can be reduced to produce the target product [1u].

First, the amidation reaction of the compound [n] with a compound [bo] can be performed in the same manner as the reaction of the compound [au] with the compound [av] in Synthesis Method K. Halogenated aliphatic hydrocarbons such as chloroform and methylene chloride, or a mixture thereof may be used as a solvent in addition to the same solvent as above.

The ring-closing metathesis reaction of the compound [bp] can be performed in the same manner as the ring-closing metathesis reaction of the compound [p] in Synthesis Method B.

The reduction reaction of the compound [bq] can be performed in the same manner as the reduction reaction for the compound [Ib] in Synthesis Method B.

### Synthesis Method a of Intermediate

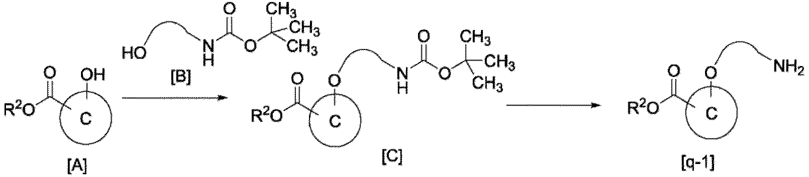

wherein, symbols represent the same meanings as described above.

Among the compounds represented by General Formula [q] in Synthesis Method C, a compound represented by General Formula [q-1] can be produced by deprotection of a compound [C] obtained by a Mitsunobu reaction between a compound [A] and a compound [B].

The Mitsunobu reaction between the compound [A] and the compound [B] can be performed in the presence of an azodicarboxylic acid derivative and a phosphine derivative. As the solvent, any solvent may be used as long as it does not affect the present reaction, and examples thereof include ethers such as tetrahydrofuran and 1,4-dioxane, aromatic hydrocarbons such as toluene, nitriles such as acetonitrile, and a mixture thereof.

Examples of the azodicarboxylic acid derivative include dialkyl azodicarboxylate such as diethyl azodicarboxylate or diisopropyl azodicarboxylate; and azodicarbonamide such as N,N,N',N'-tetramethylazodicarboxamide.

Examples of the phosphine derivative include triarylphosphine such as triphenylphosphine and trialkylphosphine such as tributylphosphine.

The amount of the compound [B] used can be 1.0 to 15.0 equivalents, and preferably 2.0 to 3.0 equivalents, with respect to the compound [A].

The amount of the azodicarboxylic acid derivative used can be 1.0 to 15.0 equivalents, and preferably 2.0 to 3.0 equivalents, with respect to the compound [A].

The amount of the phosphine derivative used can be 1.0 to 15.0 equivalents, and preferably 2.0 to 3.0 equivalents, with respect to the compound [A]. The present reaction can be performed at 0°C to 100°C, and preferably room temperature.

Deprotection of the compound [C] can be performed in the same manner as the reaction from the compound [j] to the compound [k] in Synthesis Method A2.

### Synthesis Method b of Intermediate

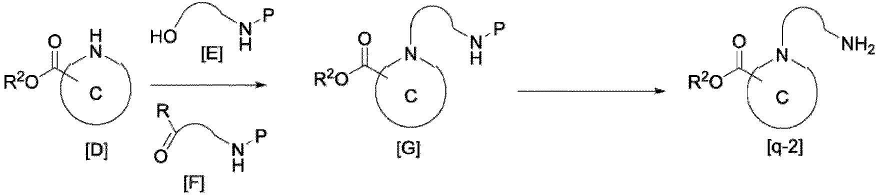

wherein, symbols represent the same meanings as described above.

Among the compounds represented by General Formula [q] in Synthesis Method C, a compound represented by General Formula [q-2] can be produced by deprotection of a compound [F] obtained by a Mitsunobu reaction between a compound [D] and a compound [E] or a reductive amination reaction with the compound [F].

The Mitsunobu reaction between the compound [D] and the compound [E] can be performed in the same manner as the reaction between the compound [A] and the compound [B] in Synthesis Method a of an intermediate.

The reductive amination reaction between the compound [D] and the compound [F] can be performed in the same manner as the reaction between the compound [a] and the compound [b] in Synthesis Method A1.

Deprotection of the compound [F] can be performed in the same manner as the reaction from the compound [i] to the compound [j] in Synthesis Method A2.

### Synthesis Method c of Intermediate

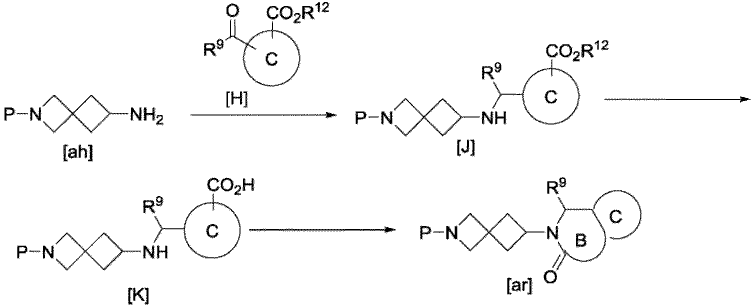

wherein, R¹² represents a protective group such as a methyl group, and other symbols represent the same meanings as described above.

The compound represented by General Formula [ar] in Synthesis Method J can be produced by a cyclization reaction of a compound [K] obtained by deprotection of a compound [J] obtained by a reductive alkylation reaction between the compound [ah] and a compound [H].

The reductive alkylation reaction between the compound [ah] and the compound [H] can be performed in the same manner as the reaction between the compound [a] and the compound [b] in Synthesis Method A1.

A deprotection reaction of the compound [J] can be performed in the same manner as the reaction from the compound [r] to the compound [s] in Synthesis Method C.

A cyclization reaction of the compound [K] can be performed in the same manner as the reaction from the compound [s] to the compound [Ie] in Synthesis Method C.

The raw material compound in the method can be produced in the same manner as a known method and/or a method described in Examples described below.

Note that the introduction of the protective group into the functional group and the removal of the protective group for the functional group can be performed with reference to a known method (PROTECTIVE GROUPS in ORGANIC SYNTHESIS (by Theodora W. Greene, Peter G. M. Wuts) or the like).

In addition, the compound and the intermediate compound of the present invention produced by the method described above can be further structurally converted into another target compound or intermediate by a method described in Examples described below and/or a known method or a combination thereof. Specific examples thereof include the following methods.

### (1) Conversion of Halogen Atom into Alkyl Group

A compound having a halogen atom is coupled with, for example, an alkylboronic acid derivative or an organozinc reactant, such that the halogen atom can be converted into a corresponding alkyl group.

### (2) Conversion of Halogen Atom into Acyl Group

A compound having a halogen atom is subjected to the same reaction as the reaction from the compound [af] to the compound [ag] in General Synthesis Method H1, and then, the compound is subjected to an acid treatment, such that the halogen atom can be converted into a corresponding acyl group.

### (3) Conversion of Halogen Atom into Amino Group

A compound having a halogen atom is coupled with, for example, an amine derivative using a palladium catalyst and a ligand, such that the halogen atom can be converted into a corresponding amino group.

### (4) Conversion of Halogen Atom into Alkoxy Group and Amino Group

A compound having a halogen atom on aryl is reacted with a compound having nucleophilicity, for example, sodium methoxide and amines, such that the halogen atom can be converted into a corresponding alkoxy group and amino group, respectively.

### (5) Conversion of Carbonyl Group into Dihalogenated Alkyl Group

A carbonyl group can be converted into a corresponding dihalogenated alkyl group, for example, by a reaction with a halogenating agent. For example, a compound having a corresponding dihalogenated alkyl group can be produced by reacting a corresponding starting compound having a carbonyl group with a halogenating agent (for example, a fluorinating agent such as bis(2-methoxyethyl)aminosulfur trifluoride) in a solvent (for example, dichloromethane, ethanol, or the like).

### (6) Conversion of Hydroxy Group into Alkoxy Group

A hydroxy group can be converted into a corresponding alkoxy group by reacting the hydroxy group with an alkylating agent. For example, a compound having a corresponding alkoxy group can be produced by reacting a corresponding starting compound having a hydroxy group with an alkylating agent (for example, a methylating agent such as methyl iodide) in a solvent (for example, dichloromethane or the like) in the presence of a base (for example, sodium hydride or the like).

### (7) N-Alkylation

N-alkylation can be performed by reacting a nitrogen-containing compound with an alkylating agent. For example, N-alkylation can be performed by a nitrogen-containing compound with methyl iodide in a solvent (for example, acetonitrile or the like) in the presence of a base (for example, potassium carbonate or the like).

Alternatively, N-alkylation can be performed by reacting a nitrogen-containing compound with an aldehyde (for example, formaldehyde) in a solvent (for example, dichloromethane or the like) in the presence of a base (for example, N,N-diisopropylethylamine or the like) and in the presence of a reducing agent (for example, sodium triacetoxyborohydride or the like).

### (8) N-Acylation

N-acylation can be performed by reacting a nitrogen-containing compound with an acylating agent. For example, N-acylation can be performed by reacting a nitrogen-containing compound with an acylating agent (for example, acetyl chloride) in a solvent (for example, chloroform) in the presence of a base (for example, N,N-diisopropylethylamine).

### (9) Halogenation of Aromatic Derivative

A compound having an aromatic ring can be halogenated by reacting the compound with a halogenating agent. For example, a compound can be halogenated by reacting the compound with a halogenating agent (for example, N-bromosuccinimide) in a solvent (for example, acetonitrile).

### (10) Conversion of Halogen Atom into Nitrile Group

A compound having a halogen atom is reacted with zinc cyanide under a palladium catalyst, such that the halogen atom can be converted into a nitrile group. For example, a compound having a halogen atom is reacted with zinc cyanide in a solvent (for example, N,N-dimethylformamide) in the presence of a palladium catalyst (for example, tetrakistriphenylphosphinepalladium), such that the halogen atom can be converted into a nitrile group.

### (11) Conversion of Bromine into Iodine

An aryl bromide compound is reacted with copper iodide, such that bromine can be converted into iodine. For example, an aryl bromide compound can be reacted with copper iodide or sodium iodide in a solvent (for example, 1,4-dioxane) in the presence of a ligand (for example, trans-N,N'-dimethylcyclohexane-1,2-amine), such that bromine can be converted into iodine.

### (12) Conversion of Iodine into Trifluoromethyl Group

An aryl iodide compound is reacted with a trifluoromethylating agent, such that iodine can be converted into a trifluoromethyl group. For example, an aryl iodide compound is reacted with a trifluoromethylating agent (for example, methyl difluoro(fluorosulfonyl)acetate) in a solvent (for example, N,N-dimethylformamide) in the presence of copper iodide, such that iodine can be converted into a corresponding trifluoromethyl group.

### (13) Difluoromethylation by C-H Activation

A difluoromethyl form can be produced by reacting a compound having an aromatic group capable of C-H activation with a difluoromethylating reagent. For example, a compound having an aromatic group is reacted with a difluoromethylating reagent (for example, difluoromethanesulfinic acid) in a solvent (for example, dimethyl sulfoxide) in the presence of tert-butyl hydroperoxide, such that conversion to a corresponding difluoromethyl group can be performed.

In the present specification, THF refers to tetrahydrofuran, and DMF refers to dimethylformamide. HATU refers to o-(7-azabenzotriazol-1-yl)-N,N,N',N'-tetramethyluronium hexafluorophosphate.

The compound of the present invention produced as described above or the raw material compound thereof is isolated and purified in a free form thereof or as a salt thereof. The salt can be produced by performing a commonly used salt formation treatment. The isolation and purification can be performed by applying general chemical operations such as extraction, concentration, crystallization, filtration, recrystallization, and various types of chromatography.

In a case where the compound of the present invention or the pharmacologically acceptable salt thereof is present as an optical isomer based on asymmetric carbon, the compound can be separated into individual optical isomers by a general optical resolution means (for example, a fractional crystallization method or a division method using a chiral column). In addition, an optical isomer can also be synthesized using an optically pure starting material. Furthermore, an optical isomer can also be synthesized by stereoselectively performing each reaction using an asymmetric auxiliary group or an asymmetric catalyst.

The compound of the present invention or the pharmacologically acceptable salt thereof is not limited to the following Examples, and examples thereof include the following compounds.

### Examples

### 1. Preparation of Example Compounds

### Example 1

### Production of (3R)-4-[2-(2-fluorophenyl)sulfonyl-2-azaspiro[3.3]heptan-6-yl]-3-methyl-8-(trifluoromethyl)-2,3-dihydro-1H-pyrido[4,3-e][1,4]diazepin-5-one

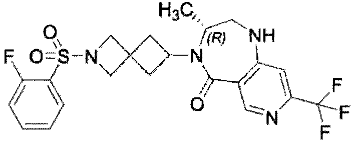

(1) To a mixture of 200 mg of 4-chloro-6-(trifluoromethyl)pyridine-3-carboxylic acid and 4.4 mL of methylene chloride, 0.113 mL of oxalyl chloride and 0.0068 mL of N,N-dimethylformamide were added. The reaction mixture was stirred at room temperature for 30 minutes, and then 0.075 mL of oxalyl chloride was added to the reaction mixture. The reaction mixture was stirred at room temperature for 15 minutes and then concentrated under reduced pressure to obtain a crude product of 4-chloro-6-(trifluoromethyl)pyridine-3-carbonyl chloride.
(2) To a mixture of the crude product of 4-chloro-6-(trifluoromethyl)pyridine-3-carbonyl chloride obtained in (1) above and 2.1 mL of acetonitrile, a mixture of 200 mg of benzyl N-[(2R)-2-[[2-(2-fluorophenyl)sulfonyl-2-azaspiro[3.3]heptan-6-yl]amino]propyl]carbamate and 2.1 mL of acetonitrile was added, and then 0.18 mL of triethylamine was added. The reaction mixture was stirred at room temperature for 2 hours. After brineand chloroform were added to the reaction mixture and the mixture was stirred, the organic layer was separated, and the mixture was concentrated under reduced pressure. The resulting residue was purified by silica gel column chromatography (solvent: hexane/ethyl acetate = 100/0 to 10/90) to obtain 178 mg of benzyl N-[(2R)-2-[[4-chloro-6-(trifluoromethyl)pyridin-3-carbonyl]-[2-(2-fluorophenyl)sulfonyl-2-azaspiro[3.3]heptan-6-yl]amino]propyl]carbamate as a yellow powder.
(3) To a mixture of 178 mg of the benzyl N-[(2R)-2-[[4-chloro-6-(trifluoromethyl)pyridin-3-carbonyl]-[2-(2-fluorophenyl)sulfonyl-2-azaspiro[3.3]heptan-6-yl]amino]propyl]carbamate obtained in (2) above and 1.3 mL of N,N-dimethylformamide, 16.0 mg of sodium hydride (60%) was added, and the mixture was stirred at room temperature for 1 hour. To the reaction mixture, 8.5 mg of sodium hydride (60%) was added. The reaction mixture was stirred at room temperature for 30 minutes, and then a saturated ammonium chloride aqueous solution was added thereto. Water was added to the reaction mixture, and extraction was performed with chloroform. The organic layer was separated, and the reaction mixture was concentrated under reduced pressure. The resulting residue was purified by reverse-phase HPLC (solvent: 10 mM ammonium carbonate aqueous solution/acetonitrile = 70/30 to 40/60) to obtain 107.6 mg of the title compound (yield 81%) as a yellow powder.
MS(ESI)m/z:499[M+H]⁺

### Examples 2 to 7:

Corresponding raw material compounds were treated in the same manner as that of Example 1 to obtain compounds shown in Table 1. However, * in diagrams represents racemic carbon. The same applies to the following table.

**[Table 1]**

| Example | Structural formula | Physical property value and the like |
|---|---|---|
| 2 | 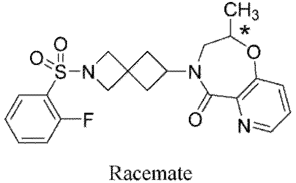 | MS(ESI) m/z;432 [M+H]+ |
| 3 | 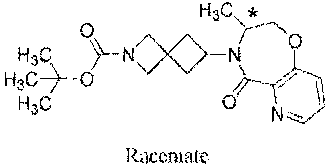 | MS(ESI) m/z;374 [M+H]+ |
| 4 | 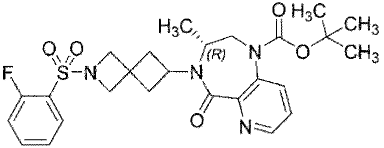 | MS (ESI) m/z;531 [M+H]+ |
| 5 | 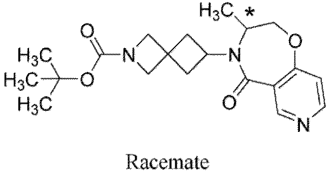 | MS(ESI) m/z;374 [M+H]+ |
| 6 | 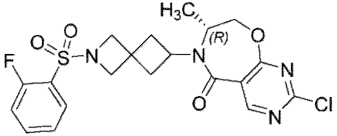 | MS(ESI) m/z;467/469 [M+H]+ |
| 7 | 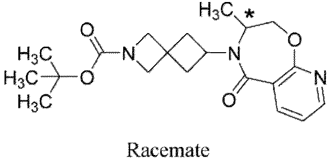 | MS(ESI) m/z;374 [M+H]+ |

### Example 8

### Production of (7R)-8-[2-(2-fluorophenyl)sulfonyl-2-azaspiro[3.3]heptan-6-yl]-7-methyl-6,7-dihydropyrazino[2,3-f][1,4]oxazepin-9-one

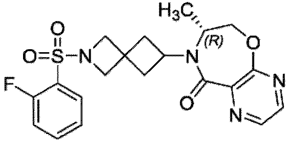

(1) To a mixture of 432 mg of 3-chloropyrazine-2-carboxylic acid and 7 mL of methylene chloride, 0.280 mL of oxalyl chloride and 0.020 mL of N,N-dimethylformamide were added. The reaction mixture was stirred at room temperature for 1 hour and then concentrated under reduced pressure to obtain a crude product of 3-chloropyrazine-2-carbonyl chloride.
(2) To a mixture of 307 mg of (2R)-2-[[2-(2-fluorophenyl)sulfonyl-2-azaspiro[3.3]heptan-6-yl]amino]propan-1-ol, 0.65 mL of triethylamine, and 5.0 mL of acetonitrile, a mixture of the total amount of the 3-chloropyrazine-2-carbonyl chloride obtained in (1) above and 3.0 mL of acetonitrile was added under ice-cooling. The reaction mixture was stirred at room temperature for 3.5 hours. To the reaction mixture, 8.0 mL of a 1 N sodium hydroxide aqueous solution was added. The reaction mixture was stirred at room temperature for 70 minutes. Brine and ethyl acetate were added to the reaction mixture, liquid separation was performed, and extraction from the aqueous layer was performed again with ethyl acetate. The organic layer was combined, washed with brine, and dried over sodium sulfate. The insoluble matter was filtered off, and the filtrate was concentrated under reduced pressure, thereby obtaining 425 mg of a crude product of 3-chloro-N-[2-(2-fluorophenyl)sulfonyl-2-azaspiro[3.3]heptan-6-yl]-N-[(1R)-2-hydroxy-1-methyl-ethyl]pyrazin-2-carboxamide as a yellowish brown powder.
(3) To a mixture of 425 mg of the crude product of 3-chloro-N-[2-(2-fluorophenyl)sulfonyl-2-azaspiro[3.3]heptan-6-yl]-N-[(1R)-2-hydroxy-1-methylethyl]pyrazin-2-carboxamide obtained in (2) above and 5.0 mL of N,N-dimethylformamide, 35.3 mg of sodium hydride (60%) was added, and the mixture was stirred for 40 minutes under ice-cooling. A saturated ammonium chloride aqueous solution was added to the reaction mixture, a saturated sodium bicarbonate aqueous solution and brine were added thereto, and extraction was performed twice with ethyl acetate. The organic layer was combined, washed with brine, and dried over sodium sulfate. The insoluble matter was filtered off, and the filtrate was concentrated under reduced pressure. The resulting residue was purified by NH silica gel column chromatography (solvent: hexane/ethyl acetate = 30/70 to 0/100). When fractions were collected and concentrated, a solid was precipitated. Therefore, hexane was added, suspension washing was performed, and the solid was collected by filtration. The resulting solid was dried under reduced pressure to obtain 258 mg of the title compound (yield 2 step 64%) as a colorless powder.
MS(ESI)m/z:433[M+H]⁺

### Examples 9 to 39:

Corresponding raw material compounds were treated in the same manner as that of Example 8 to obtain compounds shown in Table 2.

**[Table 2-1]**

| Example | Structural formula | Physical property value and the like |
|---|---|---|
| 9 | 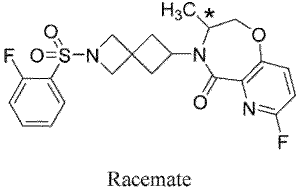 | MS(ESI) m/z;450 [M+H]+ |
| 10 | 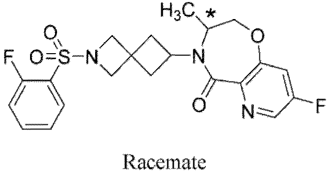 | MS(ESI) m/z;450 [M+H]+ |
| 11 | 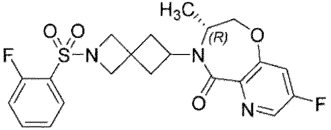 | MS(ESI) m/z;450 [M+H]+ |
| 12 | 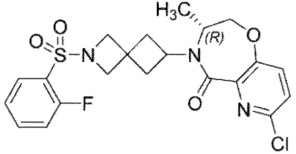 | MS(ESI) m/z;466/468 [M+H]+ |
| 13 | 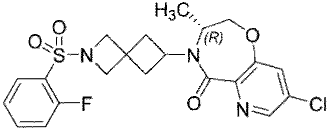 | MS(ESI) m/z;466/468 [M+H]+ |
| 14 | 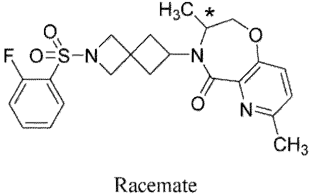 | MS(ESI) m/z;446 [M+H]+ |

**[Table 2-2]**

| | | |
|---|---|---|
| 15 | 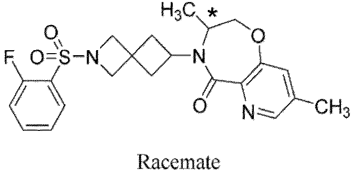 | MS(ESI) m/z;446 [M+H]+ |
| 16 | 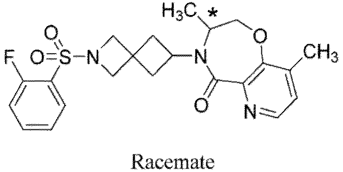 | MS(ESI) m/z;446 [M+H]+ |
| 17 | 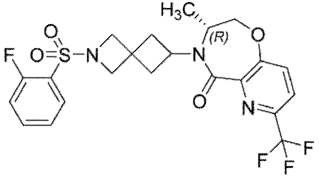 | MS(ESI) m/z;500 [M+H]+ |
| 18 | 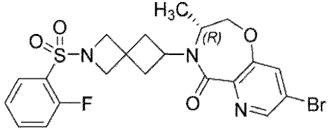 | MS(ESI) m/z;510/512 [M+H] + |
| 19 | 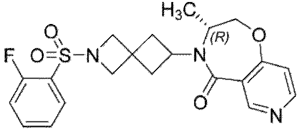 | MS(ESI) m/z;432 [M+H]+ |
| 20 | 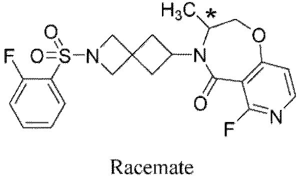 | MS(ESI) m/z;450 [M+H]+ |
| 21 | 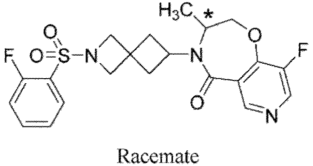 | MS(ESI) m/z;450 [M+H]+ |

**[Table 2-3]**

| | | |
|---|---|---|
| 22 | 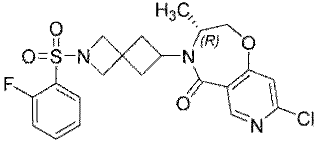 | MS(ESI) m/z;466/468 [M+H]+ |
| 23 | 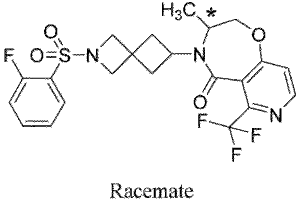 | MS(ESI) m/z;500 [M+H]+ |
| 24 | 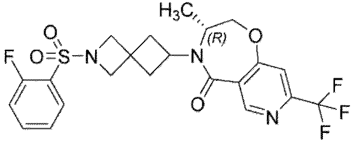 | MS(ESI) m/z;500 [M+H]+ |
| 25 | 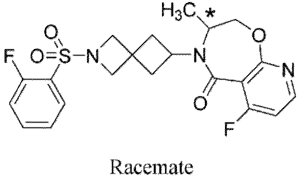 | MS(ESI) m/z;450 [M+H]+ |
| 26 | 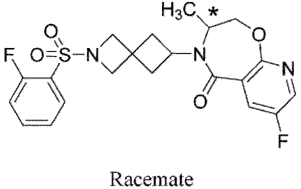 | MS(ESI) m/z;450 [M+H]+ |
| 27 | 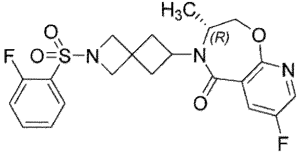 | MS(ESI) m/z;450 [M+H] + |

**[Table 2-4]**

| | | |
|---|---|---|
| 28 | 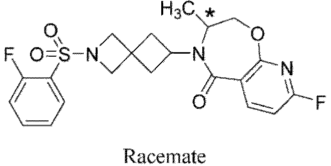 | MS(ESI) m/z;450 [M+H]+ |
| 29 | 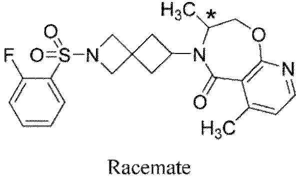 | MS(ESI) m/z;446 [M+H]+ |
| 30 | 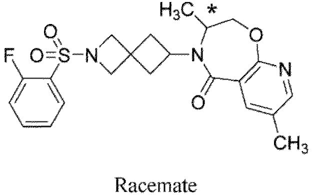 | MS(ESI) m/z;446 [M+H]+ |
| 31 | 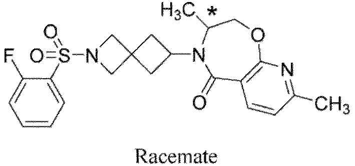 | MS(ESI) m/z;446 [M+H]+ |
| 32 | 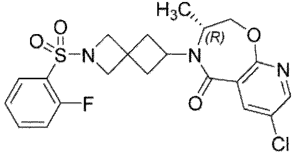 | MS(ESI) m/z;466/468 [M+H]+ |
| 33 | 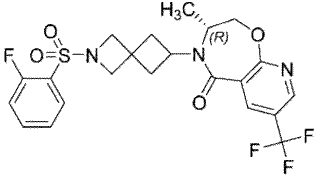 | MS(ESI) m/z;500 [M+H]+ |

**[Table 2-5]**

| | | |
|---|---|---|
| 34 | 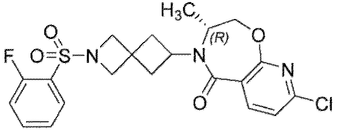 | MS(ESI) m/z;466/468 [M+H]+ |
| 35 | 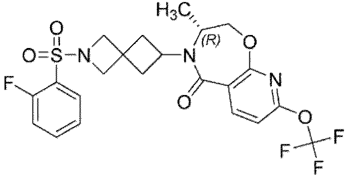 | MS(ESI) m/z;516 [M+H]+ |
| 36 | 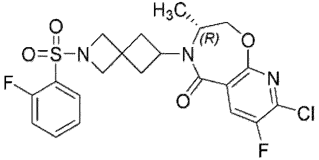 | MS(ESI) m/z;484/486 [M+H]+ |
| 37 | 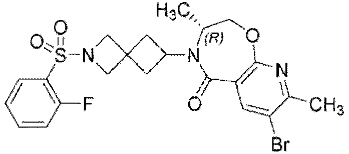 | MS(ESI) m/z;524/526 [M+H]+ |
| 38 | 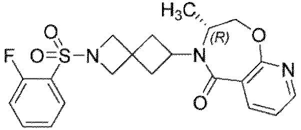 | MS(ESI) m/z;432 [M+H]+ |
| 39 | 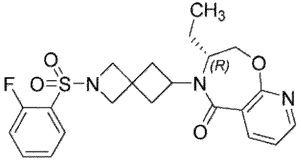 | MS(ESI) m/z;446 [M+H]+ |

### Example 40

### Production of (7R)-3-chloro-8-[2-(2-fluorophenyl)sulfonyl-2-azaspiro[3.3]heptan-6-yl]-7-methyl-6,7-dihydropyrazino[2,3-f] [1,4]oxazepin-9-one

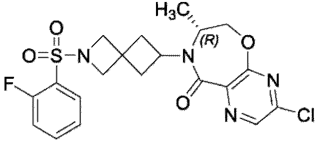

(1) To a mixture of 916 mg of 3,5-dichloropyrazine-2-carboxylic acid and 16 mL of methylene chloride, 0.530 mL of oxalyl chloride and 0.020 mL of N,N-dimethylformamide were added. The reaction mixture was stirred at room temperature for 1 hour and then concentrated under reduced pressure to obtain a crude product of 3,5-dichloropyrazine-2-carbonyl chloride.
(2) To a mixture of 525 mg of (2R)-2-[[2-(2-fluorophenyl)sulfonyl-2-azaspiro[3.3]heptan-6-yl]amino]propan-1-ol, 1.20 mL of triethylamine, and 15.0 mL of acetonitrile, a mixture of the crude product of 3,5-dichloropyrazine-2-carbonyl chloride obtained in (1) above and 5.0 mL of acetonitrile was added under ice-cooling. The reaction mixture was stirred at room temperature for 50 minutes. To the reaction mixture, 13.0 mL of a 1 N sodium hydroxide aqueous solution was added. The reaction mixture was stirred at room temperature for 1 hour, brine and ethyl acetate were added to the reaction mixture, liquid separation was performed, and extraction from the aqueous layer was performed again with ethyl acetate. The organic layer was combined, washed with brine, and dried over sodium sulfate. The insoluble matter was filtered off, and the filtrate was concentrated under reduced pressure. The resulting residue was purified by silica gel column chromatography (solvent: hexane/ethyl acetate = 25/75 to 0/100). Fractions were collected and concentrated under reduced pressure, the resulting residue was suspended and washed with 25 mL of a mixed solvent of diisopropyl ether:hexane = 2:3, and a solid was collected by filtration. The resulting solid was dried under reduced pressure to obtain 544 mg of the title compound (yield 73%) as a colorless powder.
MS(ESI)m/z:467/469[M+H]⁺

### Examples 41 to 43:

Corresponding raw material compounds were treated in the same manner as that of Example 40 to obtain compounds shown in Table 3.

**[Table 3]**

| Example | Structural formula | Physical property value and the like |
|---|---|---|
| 41 | 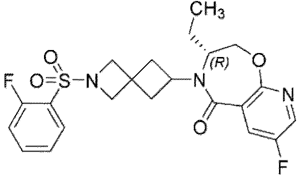 | MS(ESI) m/z;464 [M+H]+ |
| 42 | 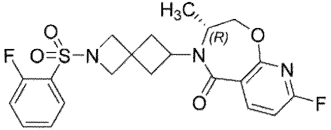 | MS(ESI) m/z;450 [M+H]+ |
| 43 | 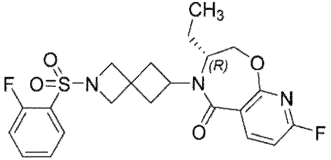 | MS(ESI) m/z;464 [M+H]+ |

### Example 44

### Production of (7R)-6-[2-(2-fluorophenyl)sulfonyl]-2-azaspiro[3.3]heptan-6-yl]-7-methyl-2-(trifluoromethyl)-7,8-dihydropyrimido[5,4-f] [1,4]oxazepin-5-one

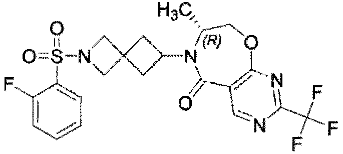

(1) To a mixture of 526 mg of 4-chloro-2-(trifluoromethyl)pyrimidine-5-carboxylic acid and 8.0 mL of methylene chloride, 0.300 mL of oxalyl chloride and 0.006 mL of N,N-dimethylformamide were added. The reaction mixture was stirred at room temperature for 3.5 hours and then concentrated under reduced pressure to obtain a crude product of 4-chloro-2-(trifluoromethyl)pyrimidine-5-carbonyl chloride.
(2) To a mixture of 770 mg of (2R)-2-[[2-(2-fluorophenyl)sulfonyl-2-azaspiro[3.3]heptan-6-yl]amino]propan-1-ol, 1.20 mL of triethylamine, and 6.0 mL of tetrahydrofuran, a mixture of the crude product of 4-chloro-2-(trifluoromethyl)pyrimidine-5-carbonyl chloride obtained in (1) above and 4.0 mL of tetrahydrofuran was added under ice-cooling. The reaction mixture was stirred at room temperature for 2.5 hours, a citric acid aqueous solution was added thereto, and then extraction was performed twice with ethyl acetate. The organic layer was combined, washed with brine, and dried over sodium sulfate. The insoluble matter was filtered off, and the filtrate was concentrated under reduced pressure. The resulting residue was purified again by silica gel column chromatography (solvent: hexane/ethyl acetate = 50/50 to 20/80) and then was subjected to silica gel column chromatography (solvent: hexane/ethyl acetate = 50/50 to 25/75), thereby obtaining 57.8 mg of the title compound (yield 4.9%) as a colorless powder.
MS(ESI)m/z:501[M+H]⁺

### Example 45

### Production of 2-[2-(2-fluorophenyl)sulfonyl-2-azaspiro[3,3]heptan-6-yl]-1-methyl-4,5-dihydro-1H-2-benzazepin-3-one

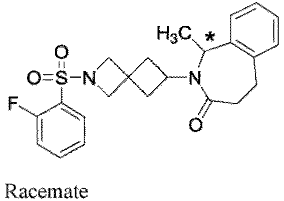

To a mixture of 28.2 mg of tert-butyl 6-(1-methyl-3-oxo-4,5-dihydro-1H-2-benzazepin-2-yl)-2-azaspiro[3,3]heptane-2-carbamate and 1 mL of methylene chloride, 0.018 mL of trimethylsilyl trifluoromethanesulfonate was added. The reaction mixture was stirred at room temperature for 30 minutes and then concentrated under reduced pressure. To the resulting residue, 1 mL of methylene chloride, 23 mg of 2-fluorobenzenesulfonyl chloride, and 0.050 mL of triethylamine were added. The reaction mixture was stirred at room temperature for 2 hours, a saturated sodium bicarbonate aqueous solution was added thereto, and then extraction was performed twice with chloroform. The organic layers were combined and dried over sodium sulfate. The insoluble matter was filtered off, and the filtrate was concentrated under reduced pressure. The resulting residue was purified by silica gel column chromatography (solvent: hexane/ethyl acetate = 100/0 to 0/100) to obtain 258 mg of the title compound (yield 2 step 37%) as a pale yellow viscous product.
MS(ESI)m/z:429[M+H]⁺

### Examples 46 to 69:

Corresponding raw material compounds were treated in the same manner as that of Example 45 to obtain compounds shown in Table 4.

**[Table 4-1]**

| Example | Structural formula | Physical property value and the like |
|---|---|---|
| 46 | 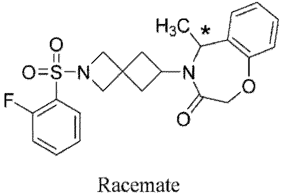 | MS (ESI) m/z;431 [M+H]+ |
| 47 | 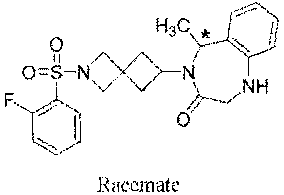 | MS(ESI) m/z;430 [M+H]+ |
| 48 | 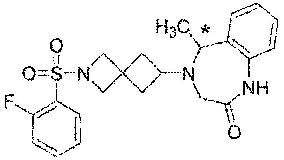 | MS(ESI) m/z;430 [M+H]+ |
| 49 | 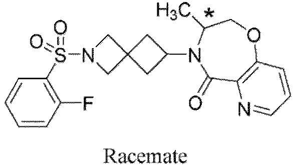 | MS(ESI) m/z;432 [M+H]+ |
| 50 | 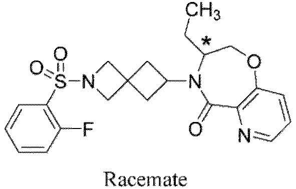 | MS(ESI) m/z;446 [M+H]+ |

**[Table 4-2]**

| | | |
|---|---|---|
| 51 | 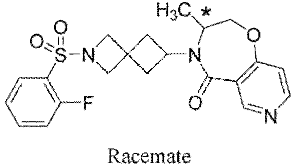 | MS(ESI) m/z;432 [M+H]+ |
| 52 | 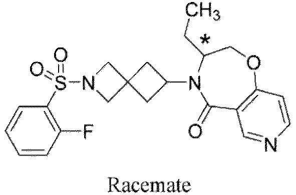 | MS(ESI) m/z;446 [M+H]+ |
| 53 | 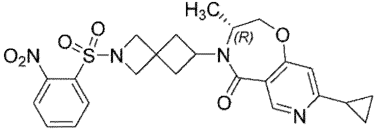 | MS(ESI) m/z;499 [M+H] + |
| 54 | 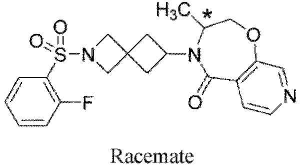 | MS(ESI) m/z;432 [M+H]+ |
| 55 | 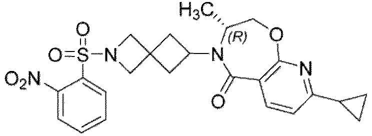 | MS(ESI) m/z;499 [M+H]+ |
| 56 | 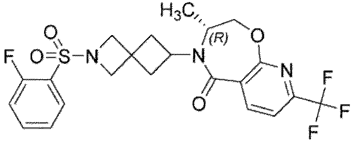 | MS(ESI) m/z;500 [M+H]+ |
| 57 | 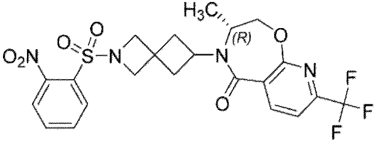 | MS(ESI) m/z;527 [M+H]+ |

**[Table 4-3]**

| | | |
|---|---|---|
| 58 | 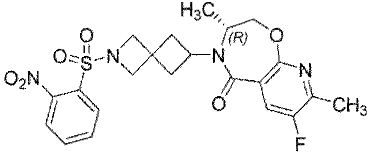 | MS (ESI) m/z;491 [M+H]+ |
| 59 | 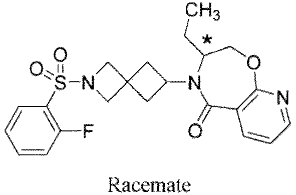 | MS(ESI) m/z;446 [M+H]+ |
| 60 | 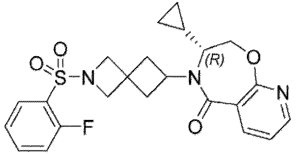 | MS(ESI) m/z;458 [M+H]+ |
| 61 | 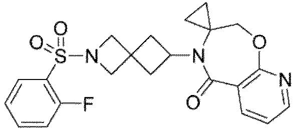 | MS(ESI) m/z;444 [M+H]+ |
| 62 | 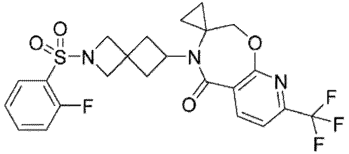 | MS(ESI) m/z;512 [M+H]+ |
| 63 | 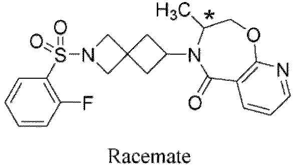 | MS(ESI) m/z;432 [M+H]+ |
| 64 | 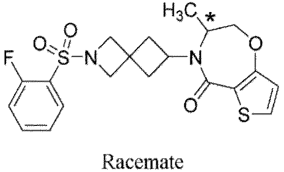 | MS(ESI) m/z;437 [M+H]+ |

**[Table 4-4]**

| | | |
|---|---|---|
| 65 | 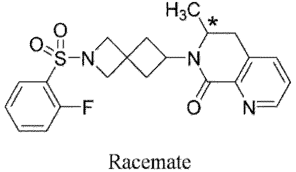 | MS(ESI) m/z;416 [M+H]+ |
| 66 | 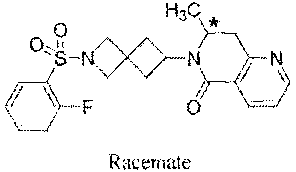 | MS(ESI) m/z;416 [M+H]+ |
| 67 | 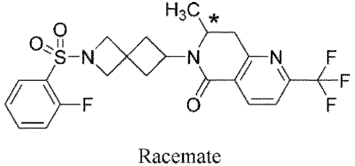 | MS(ESI) m/z;484 [M+H]+ |
| 68 | 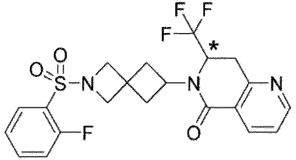 | MS(ESI) m/z;470 [M+H]+ |
| 69 | 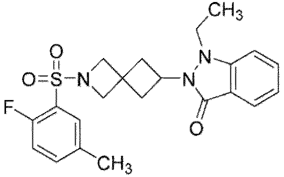 | MS(ESI) m/z;430 [M+H]+ |

### Example 70

### Production of 4-[2-(2-fluoro-5-methyl-phenyl)sulfonyl-2-azaspiro[3.3]heptan-6-yl]-3-methyl-2,3-dihydropyrido[3,2-f] [1,4]oxazepin-5-one

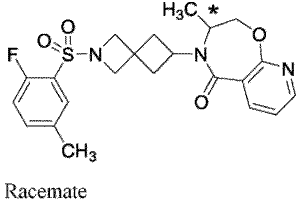

A mixture of 24 mg of 4-(2-azaspiro[3.3]heptan-6-yl)-3-methyl-2,3-dihydropyrido[3,2-f][1,4]oxazepin-5-one, 17.7 mg of 2-fluoro-5-methyl-benzenesulfonyl chloride, 0.0638 mL of N,N-diisopropylethylamine, and 1 mL of chloroform was stirred at room temperature overnight. Water was added to the reaction mixture, extraction was performed with chloroform, and the organic layer was concentrated. Dimethyl sulfoxide and methanol were added to resulting residue, and the mixture was purified by reverse-phase HPLC (solvent: 10 mM ammonium carbonate aqueous solution/acetonitrile = 30/70 to 0/100), thereby obtaining 26.9 mg of the title compound (yield 69%).
MS(ESI)m/z:446[M+H]⁺

### Examples 71 to 81:

Corresponding raw material compounds were treated in the same manner as that of Example 70 to obtain compounds shown in Table 5.

**[Table 5]**

| Example | Structural formula | Physical property value and the like |
|---|---|---|
| 71 | 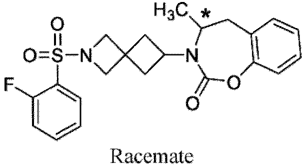 | MS(ESI) m/z;431 [M+H]+ |
| 72 | 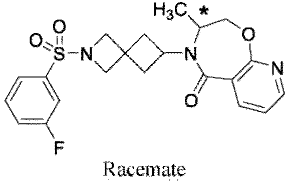 | MS(ESI) m/z;432 [M+H]+ |
| 73 | 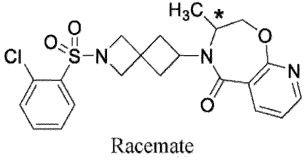 | MS(ESI) m/z;448 [M+H]+ |
| 74 | 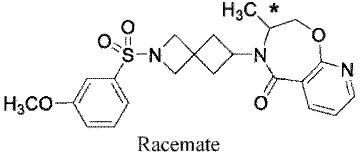 | MS(ESI) m/z;444 [M+H]+ |
| 75 | 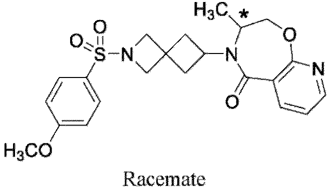 | MS(ESI) m/z;444 [M+H]+ |
| 76 | 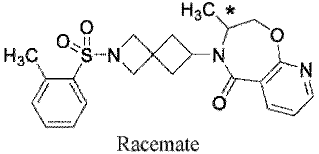 | MS(ESI) m/z;428 [M+H]+ |
| 77 | 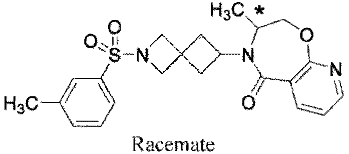 | MS(ESI) m/z;428 [M+H]+ |
| 78 | 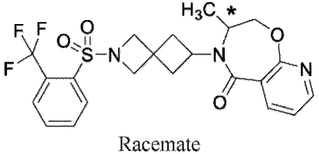 | MS(ESI) m/z;482 [M+H]+ |
| 79 | 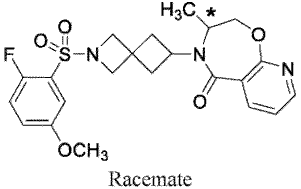 | MS(ESI) m/z;462 [M+H]+ |
| 80 | 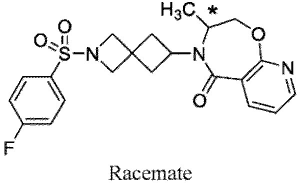 | MS(ESI) m/z;432 [M+H]+ |
| 81 | 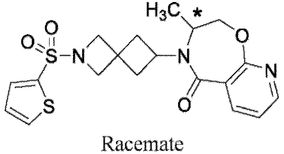 | MS(ESI) m/z;420 [M+H]+ |

### Example 82

### Production of 2-[2-(benzenesulfonyl)-2-azaspiro[3.3]heptan-6-yl]isoindolin-1,3-dione

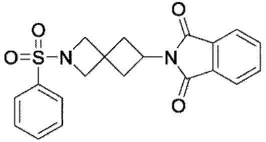

In a 100 mL eggplant flask, 670 mg of tert-butyl 6-(1,3-dioxoisoindolin-2-yl)-2-azaspiro[3.3]heptane-2-carboxylate was weighed, and 6.0 mL of dichloromethane was added. Next, 672 mg of trimethylsilyl trifluoromethanesulfonate was added, and the mixture was stirred at room temperature for 30 minutes. The reaction solution was concentrated under reduced pressure to obtain 917 mg of a crude product of 2-(2-azaspiro[3.3]heptan-6-yl)isoindoline-1,3-dione trifluoromethanesulfonate as a colorless solid. In a 100 mL eggplant flask, 917 mg of the resulting crude product, and 7.0 mL of dichloromethane and 980 µL of triethylamine were added. Next, 871 mg of benzenesulfonic anhydride was added, and the mixture was stirred at room temperature for 20 minutes. A saturated sodium bicarbonate solution and ethyl acetate were added to the reaction solution, and the organic layer was extracted. The organic layer was washed with brine and then dried over magnesium sulfate. The insoluble matter was filtered, and then the solution was concentrated under reduced pressure. 0.86 g of the resulting pale yellow oily product was purified by silica gel column chromatography (solvent: hexane/ethyl acetate = 90/10 to 65/35) to obtain 649 mg of the title compound (yield 2 step 87%) as a colorless powder.
MS(ESI)m/z:383[M+H]⁺

### Example 83:

A corresponding raw material compound was treated in the same manner as that of Example 82 to obtain a compound shown in Table 6.

**[Table 6]**

| Example | Structural formula | Physical property value and the like |
|---|---|---|
| 83 | 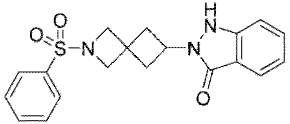 | MS(ESI) m/z;370 [M+H]+ |

### Example 84

### Production of 4-[2-(benzenesulfonyl)-2-azaspiro[3.3]heptan-6-yl]-3-methyl-2,3-dihydropyrido[3,2-f] [1,4]oxazepin-5-one

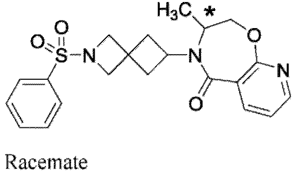

A mixture of 25 mg of 4-(2-azaspiro[3.3]heptan-6-yl)-3-methyl-2,3-dihydropyrido[3,2-f] [1,4]oxazepin-5-one, 32.7 mg of benzenesulfonic anhydride, and 1 mL of chloroform was stirred at room temperature overnight. The reaction mixture was concentrated, methanol was added, and then the mixture was purified by reverse-phase HPLC (solvent: 10 mM ammonium carbonate aqueous solution/acetonitrile = 70/30 to 40/60), thereby obtaining 24.3 mg of the title compound (yield 64%).
MS(ESI)m/z:414[M+H]⁺

### Example 85

### Production of 6-[2-(2-fluorophenyl)sulfonyl-2-azaspiro[3.3]heptan-6-yl]-7-methyl-2-(trifluoromethyl)-8,9-dihydro-7H-pyrido[3, 2-c]azepin-5-one

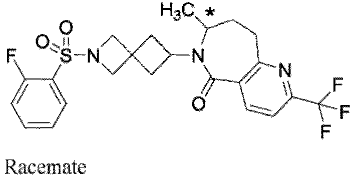

175 mg of 6-[2-(2-fluorophenyl)sulfonyl-2-azaspiro[3.3]heptan-6-yl]-7-methyl-2-(trifluoromethyl)-7H-pyrido[3,2-c]azepin-5-one, 80 mg of 10% palladium carbon, 6.0 mL of ethanol, and 1.5 mL of tetrahydrofuran were weighed, and the mixture was stirred under a hydrogen atmosphere at room temperature for 2 hours. The reaction mixture was diluted with chloroform, the insoluble matter was removed by celite filtration, and then the filtrate was concentrated under reduced pressure. The resulting residue was purified by silica gel column chromatography (solvent: hexane/ethyl acetate = 90/10 to 50/50) to obtain 160.4 mg of the title compound (yield 91%) as a colorless powder.
MS(ESI)m/z:498[M+H]⁺

### Examples 86 to 108:

Corresponding raw material compounds were treated in the same manner as that of Example 85 to obtain compounds shown in Table 7.

**[Table 7-1]**

| Example | Structural formula | Physical property value and the like |
|---|---|---|
| 86 | 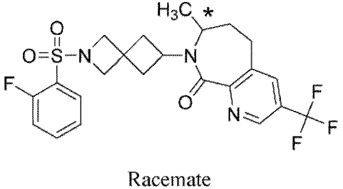 | MS(ESI) m/z;498 [M+H]+ |
| 87 | 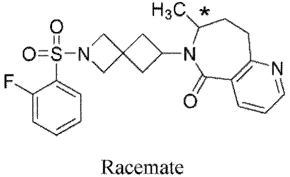 | MS(ESI) m/z;430 [M+H]+ |
| 88 | 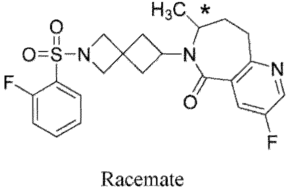 | MS(ESI) m/z;448 [M+H]+ |
| 89 | 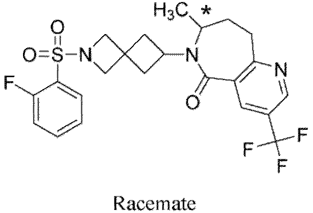 | MS(ESI) m/z;498 [M+H]+ |
| 90 | 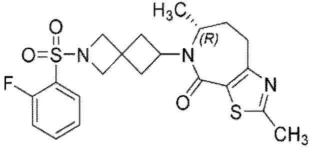 | MS(ESI) m/z;450 [M+H]+ |
| 91 | 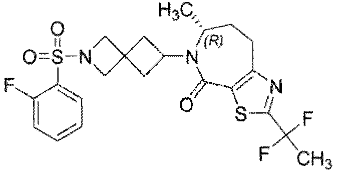 | MS(ESI) m/z;500 [M+H]+ |

**[Table 7-2]**

| | | |
|---|---|---|
| 92 | 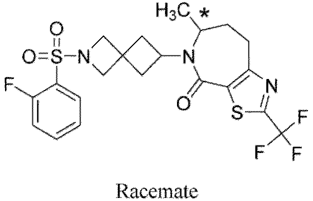 | MS(ESI) m/z;504 [M+H]+ |
| 93 | 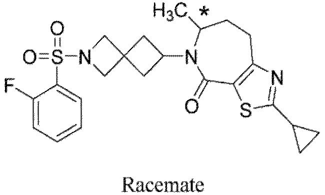 | MS(ESI) m/z;476 [M+H]+ |
| 94 | 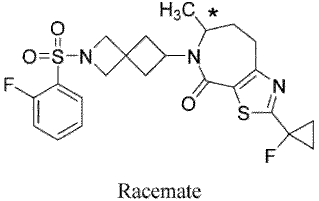 | MS(ESI) m/z;494 [M+H]+ |
| 95 | 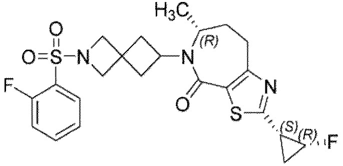 | MS(ESI) m/z;494 [M+H]+ |
| 96 | 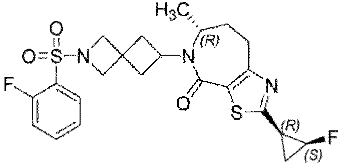 | MS(ESI) m/z;494 [M+H]+ |
| 97 | 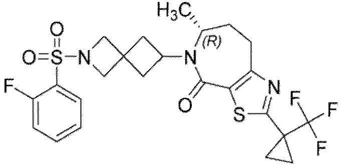 | MS(ESI) m/z;544 [M+H]+ |

**[Table 7-3]**

| | | |
|---|---|---|
| 98 | 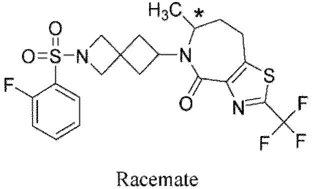 | MS(ESI) m/z;504 [M+H]+ |
| 99 | 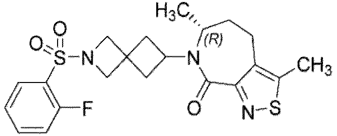 | MS(ESI) m/z;450 [M+H]+ |
| 100 | 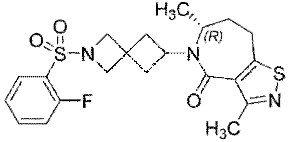 | MS(ESI) m/z;450 [M+H]+ |
| 101 | 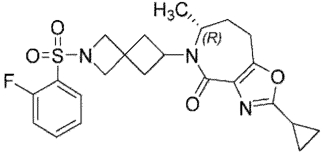 | MS(ESI) m/z;460 [M+H]+ |
| 102 | 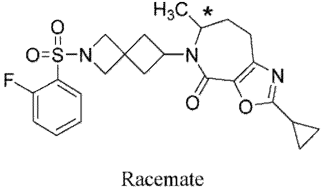 | MS(ESI) m/z;460 [M+H]+ |
| 103 | 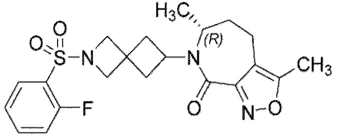 | MS(ESI) m/z;434 [M+H]+ |
| 104 | 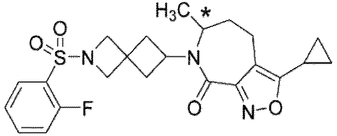 | MS(ESI) m/z;460 [M+H]+ |

**[Table 7-4]**

| | | |
|---|---|---|
| 105 | 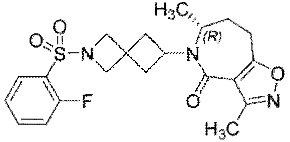 | MS(ESI) m/z;434 [M+H]+ |
| 106 | 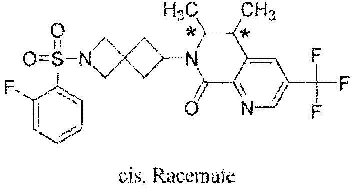 | MS(ESI) m/z;498 [M+H]+ |
| 107 | 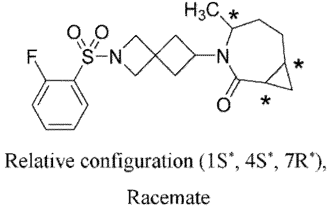 | MS(ESI) m/z;393 [M+H]+ |
| 108 | 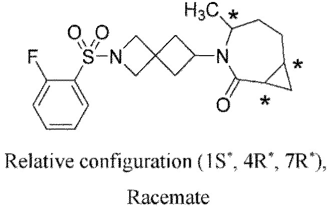 | MS(ESI) m/z;393 [M+H]+ |

### Example 109

### Production of (6R)-2-(1-fluorocyclopropyl)-5-[2-(2-fluorophenyl)sulfonyl-2-azaspiro[3.3]heptan-6-yl]-6-methyl-7,8-dihydro-6H-thiazolo[5,4-c]azepin-4-one

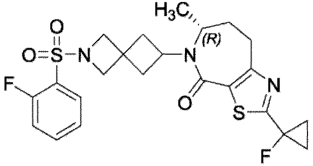

and Example 110

### Production of (6R)-5-[2-(2-fluorophenyl)sulfonyl-2-azaspiro[3.3]heptan-6-yl]-2-isopropyl-6-methyl-7,8-dihydro-6H-thiazolo[5,4-c]azepin-4-one

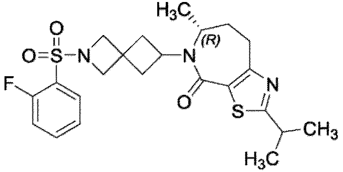

In a 100 mL eggplant flask, 175 mg of (6R)-2-(1-fluorocyclopropyl)-5-[2-(2-fluorophenyl)sulfonyl-2-azaspiro[3.3]heptan-6-yl]-6-methyl-6H-thiazolo[5,4-c]azepin-4-one was weighed, and 1.0 mL of tetrahydrofuran and 4.0 mL of ethanol were added and dissolved. Next, 90.1 mg of 10% palladium carbon (type M-hydrous) was added, and the mixture was stirred under a hydrogen atmosphere at room temperature for 135 minutes. Chloroform was added to the reaction solution to dilute the mixture, and the mixture was filtered through celite. Washing was performed with chloroform, and then the filtrate was concentrated under reduced pressure. 0.17 g of the resulting pale yellow amorphous solid was purified by reverse-phase HPLC (solvent: 10 mM ammonium carbonate aqueous solution/acetonitrile = 60/40 to 50/50) to obtain 127 mg of (6R)-2-(1-fluorocyclopropyl)-5-[2-(2-fluorophenyl)sulfonyl-2-azaspiro[3.3]heptan-6-yl]-6-methyl-7,8-dihydro-6H-thiazolo[5,4-c]azepin-4-one (yield 72%).
MS(ESI)m/z:494[M+H]⁺

At the same time, 19.3 mg of (6R)-5-[2-(2-fluorophenyl)sulfonyl-2-azaspiro[3.3]heptan-6-yl]-2-isopropyl-6-methyl-7,8-dihydro-6H-thiazolo[5,4-c]azepin-4-one (yield 11%) was obtained.
MS(ESI)m/z:478[M+H]⁺

### Example 111

### Production of 6-[2-(2-fluorophenyl)sulfonyl-2-azaspiro[3.3]heptan-6-yl]-7-methyl-2-(trifluoromethyl)-7H-pyrido[3, 2-c]azepin-5-one

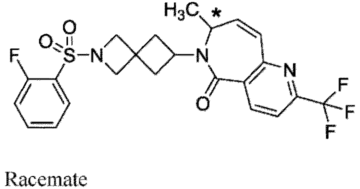

A mixture of 346 mg of N-[2-(2-fluorophenyl)sulfonyl-2-azaspiro[3.3]heptan-6-yl]-N-(1-methylallyl)-2-vinyl-pyridine-3-carboxamide, 40 mg of a second generation Grubbs catalyst, and 6.0 mL of toluene was stirred at 60°C for 1.5 hours. To the reaction mixture, 19.8 mg of a second generation Grubbs catalyst was added, and the mixture was stirred at 60°C for 30 minutes. The reaction mixture was returned at room temperature, celite and silica gel were filtered, and the filtrate was concentrated under reduced pressure. The resulting residue was purified by silica gel column chromatography (solvent: hexane/ethyl acetate = 90/10 to 60/40) to obtain 262.3 mg of the title compound as a colorless powder.
MS(ESI)m/z:496[M+H]⁺

### Examples 112 to 132:

Corresponding raw material compounds were treated in the same manner as that of Example 111 to obtain compounds shown in Table 8.

**[Table 8-1]**

| Example | Structural formula | Physical property value and the like |
|---|---|---|
| 112 | 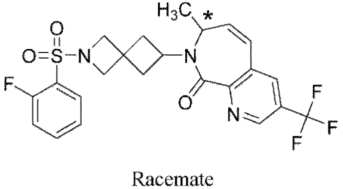 | MS (ESI) m/z;496 [M+H]+ |
| 113 | 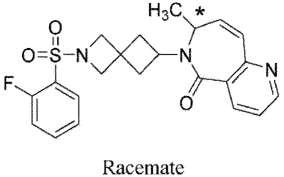 | MS (ESI) m/z;428 [M+H]+ |
| 114 | 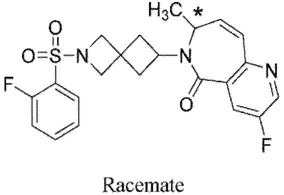 | MS(ESI) m/z;446 [M+H]+ |
| 115 | 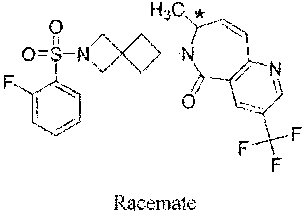 | MS (ESI) m/z;496 [M+H] + |
| 116 | 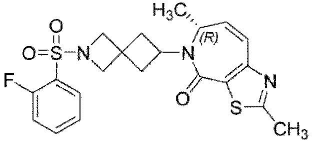 | MS (ESI) m/z;448 [M+H] + |
| 117 | 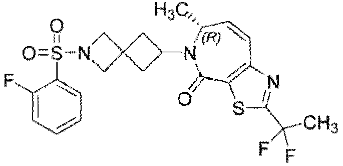 | MS (ESI) m/z;498 [M+H] + |

**[Table 8-2]**

| | | |
|---|---|---|
| 118 | 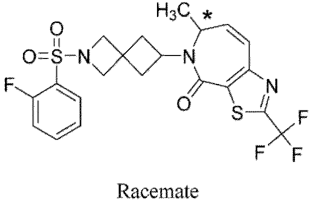 | MS(ESI) m/z;502 [M+H]+ |
| 119 | 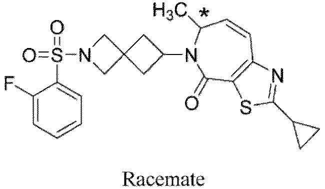 | MS (ESI) m/z;474 [M+H]+ |
| 120 | 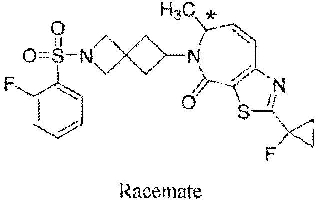 | MS (ESI) m/z;492 [M+H]+ |
| 121 | 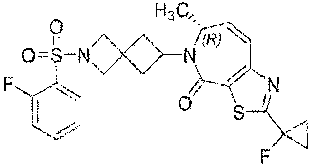 | MS(ESI) m/z;492 [M+H]+ |
| 122 | 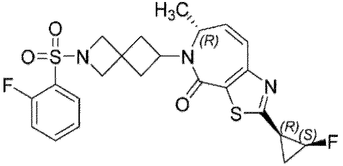 | MS(ESI) m/z;492 [M+H]+ |
| 123 | 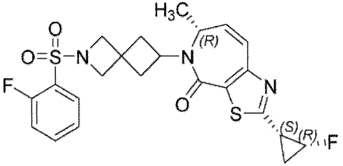 | MS(ESI) m/z;492 [M+H]+ |

**[Table 8-3]**

| | | |
|---|---|---|
| 124 | 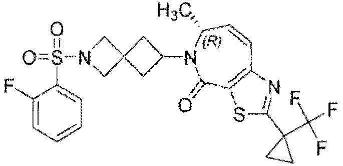 | MS(ESI) m/z;542 [M+H]+ |
| 125 | 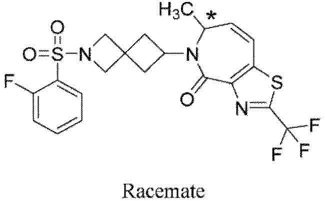 | MS(ESI) m/z;502 [M+H]+ |
| 126 | 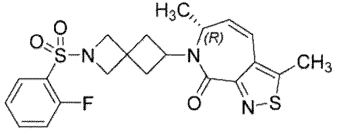 | MS(ESI) m/z;448 [M+H]+ |
| 127 | 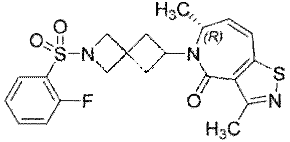 | MS(ESI) m/z;448 [M+H]+ |
| 128 | 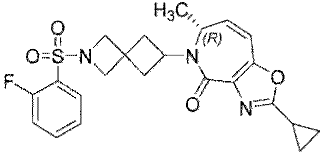 | MS (ESI) m/z;458 [M+H]+ |
| 129 | 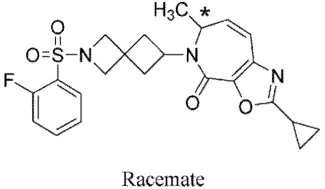 | MS(ESI) m/z;458 [M+H]+ |
| 130 | 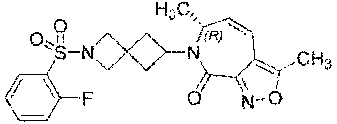 | MS(ESI) m/z;432 [M+H]+ |

**[Table 8-4]**

| | | |
|---|---|---|
| 131 | 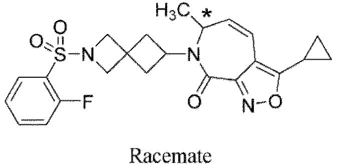 | MS (ESI) m/z;458 [M+H]+ |
| 132 | 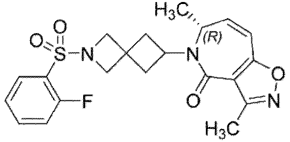 | MS (ESI) m/z;432 [M+H]+ |

### Example 133

### Production of (2R*,4S*,5R*)-10-cyclopropyl-3,3-difluoro-6-[2-(2-fluorophenyl)sulfonyl-[3.3]heptan-6-yl]-5-methyl-9-thia-6,11-diazatricyclo[6.3.0.02.4]undec-1(8),10-dien-7-one

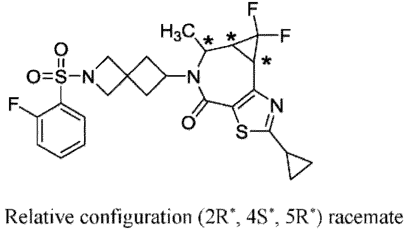

In a 100 mL eggplant flask, 128 mg of 2-cyclopropyl-5-[2-(2-fluorophenyl)sulfonyl-2-azaspiro[3.3]heptan-6-yl]-6-methyl-6H-thiazolo[5,4-c]azepin-4-one was weighed, and 4.0 mL of tetrahydrofuran was added. Next, 15.9 mg of sodium iodide and 120 µL of (trifluoromethyl)trimethylsilane were added, and the mixture was heated and stirred under reflux for 110 minutes. Thereafter, 180 µL of (trifluoromethyl)trimethylsilane was additionally added, and the mixture was heated and stirred under reflux for 150 minutes. Next, the reaction solution was transferred to a 5 mL test tube for microwave reaction, 1.0 mL of tetrahydrofuran, 14.6 mg of sodium iodide, and 180 µL of (trifluoromethyl)trimethylsilane were additionally added, and the mixture was stirred at 100°C for 1 hour using a microwave reactor. The reaction solution was diluted with ethyl acetate, washing was performed with brine, and then drying was performed with magnesium sulfate. The insoluble matter was filtered, and then the solution was concentrated under reduced pressure. 141 mg of the resulting yellowish brown viscous oily product was purified by reverse-phase HPLC (solvent: 10 mM ammonium carbonate aqueous solution/acetonitrile = 70/30 to 40/60) to obtain 25.9 mg of the title compound (yield 18%) as a colorless amorphous solid.
MS(ESI)m/z:524[M+H]⁺

### Example 134

### Production of (3R)-4-[2-(2-fluorophenyl)sulfonyl-2-azaspiro[3.3]heptan-6-yl]-3-methyl-8-morpholino-2,3-dihydropyrido[3,4-f] [1,4]oxazepin-5-one

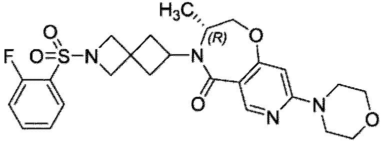

100 mg of (3R)-8-chloro-4-[2-(2-fluorophenyl)sulfonyl]-2-azaspiro[3.3]heptan-6-yl]-3-methyl-2,3-dihydropyrido[3,4-f][1,4]oxazepin-5-one, 21 mg of morpholine, 10 mg of dicyclohexyl-[2-(2,4,6-triisopropylphenyl)phenyl]phosphane, 6 mg of tris(dibenzylideneacetone)dipalladium, and 31 mg of sodium tert-butoxide were suspended in 1.5 mL of 1,4-dioxane, and the mixture was stirred at an external temperature of 80°C for 2 hours and then stirred at an external temperature of 90°C for 2 hours. 21 mg of morpholine was added, and the mixture was stirred at an external temperature of 90°C for 2 hours. To the reaction solution, 10 mg of dicyclohexyl-[2-(2,4,6-triisopropylphenyl)phenyl]phosphane and 6 mg of tris(dibenzylideneacetone)dipalladium were added, and the mixture was stirred at an external temperature of 90°C for 1 hour. To the reaction solution, 3 mg of sodium tert-butoxide was added, and the mixture was stirred at an external temperature of 90°C for 1 hour. A saturated ammonium chloride aqueous solution and chloroform were added to the reaction solution for extraction, the solvent was distilled off, and the mixture was purified by reverse-phase HPLC (solvent: 10 mM ammonium carbonate aqueous solution/acetonitrile = 70/30 to 40/60) and then purified by silica gel column chromatography (solvent: hexane/ethyl acetate = 50/50 to 0/100), thereby obtaining 32 mg of the title compound (yield 29%) as a colorless powder.
MS(ESI)m/z:517[M+H]⁺

### Examples 135 to 161:

Corresponding raw material compounds were treated in the same manner as that of Example 134 to obtain compounds shown in Table 9.

**[Table 9-1]**

| Example | Structural formula | Physical property value and the like |
|---|---|---|
| 135 | 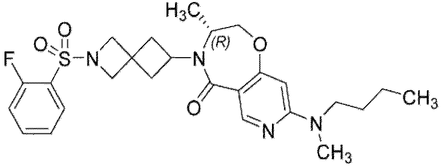 | MS(ESI) m/z;517 [M+H]+ |
| 136 | 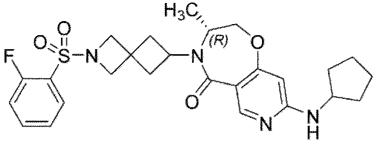 | MS(ESI) m/z;515 [M+H]+ |
| 137 | 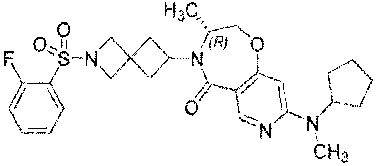 | MS(ESI) m/z;529 [M+H]+ |
| 138 | 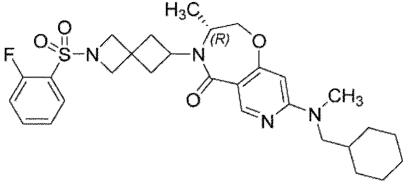 | MS(ESI) m/z;557 [M+H]+ |
| 139 | 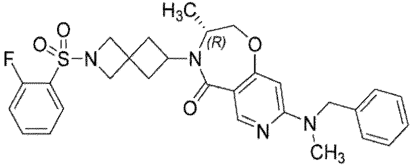 | MS(ESI) m/z;551 [M+H]+ |
| 140 | 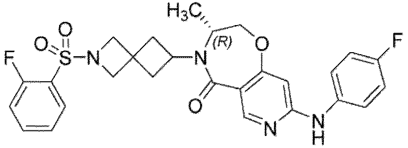 | MS(ESI) m/z;541 [M+H]+ |

**[Table 9-2]**

| | | |
|---|---|---|
| 141 | 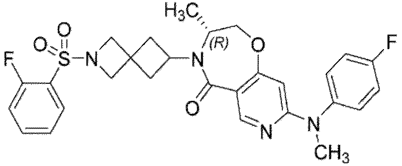 | MS(ESI) m/z;555 [M+H]+ |
| 142 | 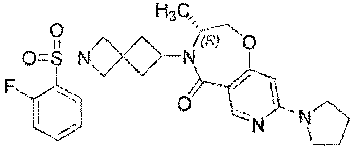 | MS(ESI) m/z;501 [M+H]+ |
| 143 | 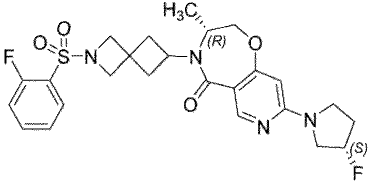 | MS(ESI) m/z;519 [M+H]+ |
| 144 | 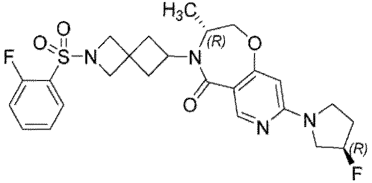 | MS(ESI) m/z;519 [M+H]+ |
| 145 | 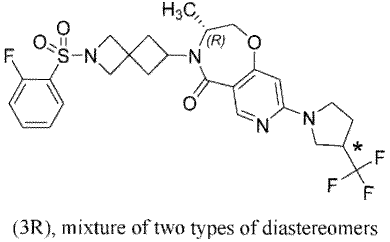 | MS(ESI) m/z;569 [M+H]+ |
| 146 | 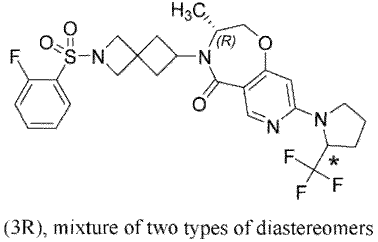 | MS(ESI) m/z;569 [M+H]+ |

**[Table 9-3]**

| | | |
|---|---|---|
| 147 | 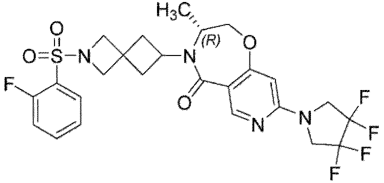 | MS(ESI) m/z;573 [M+H]+ |
| 148 | 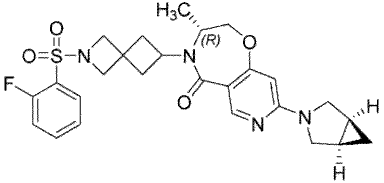 | MS(ESI) m/z;513 [M+H]+ |
| 149 | 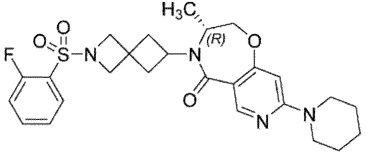 | MS(ESI) m/z;515 [M+H]+ |
| 150 | 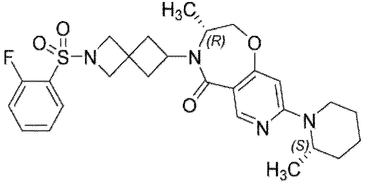 | MS(ESI) m/z;529 [M+H]+ |
| 151 | 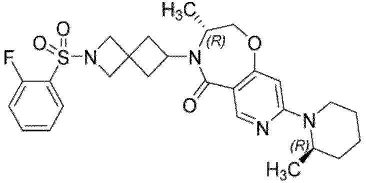 | MS(ESI) m/z;529 [M+H]+ |
| 152 | 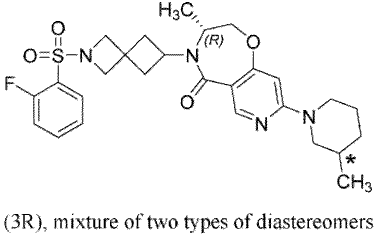 | MS(ESI) m/z;529 [M+H]+ |

**[Table 9-4]**

| | | |
|---|---|---|
| 153 | 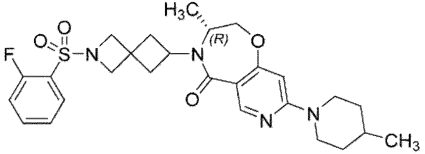 | MS(ESI) m/z;529 [M+H]+ |
| 154 | 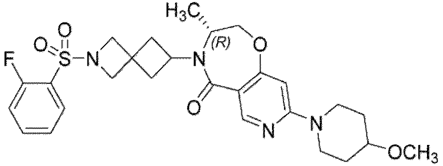 | MS(ESI) m/z;545 [M+H]+ |
| 155 | 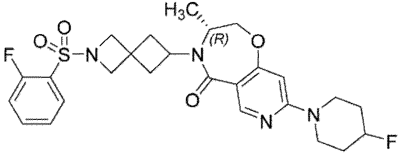 | MS(ESI) m/z;533 [M+H]+ |
| 156 | 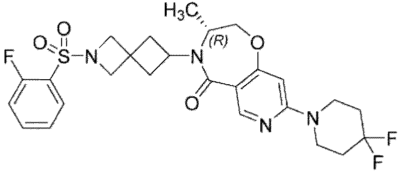 | MS(ESI) m/z;551 [M+H]+ |
| 157 | 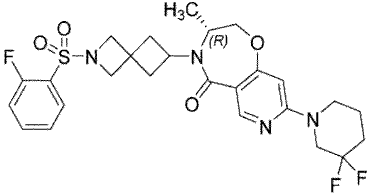 | MS(ESI) m/z;551 [M+H]+ |
| 158 | 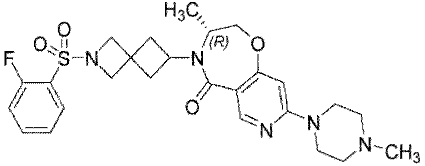 | MS(ESI) m/z;530 [M+H]+ |

**[Table 9-5]**

| | | |
|---|---|---|
| 159 | 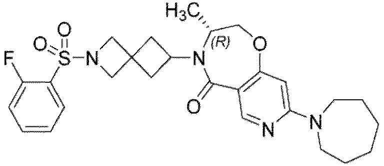 | MS(ESI) m/z;529 [M+H]+ |
| 160 | 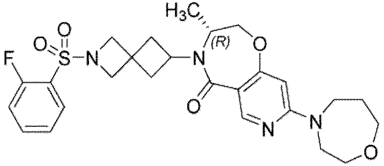 | MS(ESI) m/z;531 [M+H]+ |
| 161 | 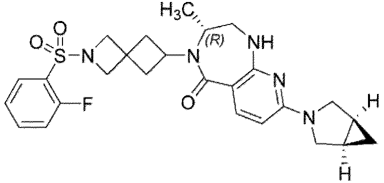 | MS(ESI) m/z;512 [M+H]+ |

### Example 162

### Production of (3R)-4-[2-(2-fluorophenyl)sulfonyl-2-azaspiro[3.3]heptan-6-yl]-8-[(3S)-3-fluoropyrrolidin-1-yl]-3-methyl-2,3-dihydro-1H-pyrido[2,3-e][1,4]diazepin-5-one

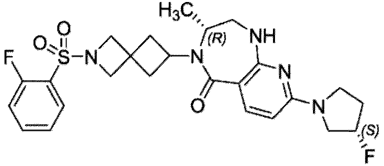

88 mg of (3R)-8-chloro-4-[2-(2-fluorophenyl)sulfonyl-2-azaspiro[3.3]heptan-6-yl]-3-methyl-2,3-dihydropyrido[3,4-f] [1,4]oxazepin-5-on e, 37 mg of (3S)-3-fluoropyrrolidine hydrochloride, 28 mg of tris(dibenzylideneacetone)dipalladium, 22 mg of dicyclohexyl-[2-(2,4,6-triisopropylphenyl)phenyl]phosphane, and 57 mg of sodium t-butoxide were suspended in 2 mL of 1,4-dioxane, and the mixture was stirred at 90°C for 2.5 hours. Water was added to the reaction mixture, and extraction was performed with ethyl acetate. The organic layer was washed with water and brine, filtration was performed after drying with sodium sulfate, and the filtrate was concentrated under reduced pressure. The resulting residue was purified by silica gel column chromatography (solvent: hexane/ethyl acetate = 60/40 to 20/80). The resulting residue was purified again by NH silica gel column chromatography (solvent: hexane/ethyl acetate = 60/40 to 30/70) to obtain a crude product of the title compound.

In addition, a crude product containing benzyl (3R)-4-[2-(2-fluorophenyl)sulfonyl-2-azaspiro[3.3]heptan-6-yl]-8-[(3S)-3-fluoropyrrolidin-1-yl]-3-methyl-5-oxo-2,3-dihydropyrido[2,3-e][1,4]diazepine-1-carboxylate was obtained. 2 mL of methanol and 2 mL of a 1 mol/L sodium hydroxide aqueous solution were added thereto, and the mixture was stirred at 70°C for 2.5 hours. Water was added to the reaction mixture, and extraction was performed with ethyl acetate. The organic layer was washed with water and brine, filtration was performed after drying with sodium sulfate, and the filtrate was concentrated under reduced pressure. The resulting residue was combined with the crude product of the title compound obtained by the previous purification by column chromatography and purified again by silica gel column chromatography (solvent: hexane/ethyl acetate = 60/40 to 30/70) to obtain 13 mg of the title compound (yield 17%) as a colorless powder.
MS(ESI)m/z:518[M+H]⁺

### Example 163

### Production of (3R)-4-[2-(2-fluorophenyl)sulfonyl-2-azaspiro[3.3]heptan-6-yl]-8-methoxy-3-methyl-2,3-dihydropyrido[3,2-f] [1,4]oxazepin-5-one

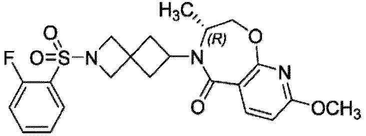

and Example 164

### Production of (3R)-8-methoxy-4-[2-(2-methoxyphenyl)sulfonyl-2-azaspiro[3.3]heptan-6-yl]-3-methyl-2,3-dihydropyrido[3,2-f] [1,4]oxazepin-5-one

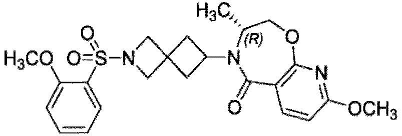

77.8 mg of (3R)-8-chloro-4-[2-(2-fluorophenyl)sulfonyl]-2-azaspiro[3.3]heptan-6-yl]-3-methyl-2,3-dihydropyrido[3,2-f][1,4]oxazepin-5-one was dissolved in 1.6 mL of methanol, 47.8 µL of sodium methoxide (5 mol/L methanol aqueous solution) was added, and the mixture was heated and refluxed for 3 hours. To the reaction mixture, 31.9 µL of sodium methoxide (5 mol/L methanol aqueous solution) was added, and the mixture was heated and refluxed for 0.5 hours. The mixture was cooled at room temperature, and the reaction mixture was concentrated under reduced pressure. Water was added to the resulting residue, and extraction was performed with ethyl acetate. The organic layer was dried over magnesium sulfate, filtration was performed, and then the filtrate was concentrated under reduced pressure. The resulting residue was purified by silica gel column chromatography (solvent: hexane/ethyl acetate = 50/50 to 33/67 to 25/75) to obtain 45.7 mg of (3R)-4-[2-(2-fluorophenyl)sulfonyl-2-azaspiro[3.3]heptan-6-yl]-8-methoxy-3-methyl-2,3-dihydropyrido[3,2-f][1,4]oxazepin-5-one (yield 62%) as a colorless powder.
MS(ESI)m/z:462[M+H]⁺

At the same time, 14.1 mg of (3R)-8-methoxy-4-[2-(2-methoxyphenyl)sulfonyl-2-azaspiro[3.3]heptan-6-yl]-3-methyl-2,3-dihydropyrido[3,2-f][1,4]oxazepin-5-one (yield 19%) was obtained as a colorless powder.
MS(ESI)m/z:474[M+H]⁺

### Examples 165 and 166:

Corresponding raw material compounds were treated in the same manner as those of Examples 163 and 164 to obtain compounds shown in Table 10.

**[Table 10]**

| Example | Structural formula | Physical property value and the like |
|---|---|---|
| 165 | 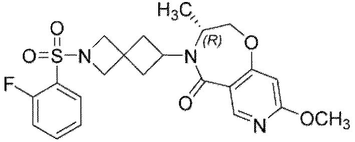 | MS(ESI) m/z;462 [M+H]+ |
| 166 | 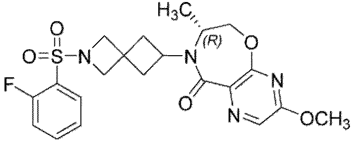 | MS(ESI) m/z;463 [M+H]+ |

### Example 167

### Production of (3R)-7-fluoro-4-[2-(2-fluorophenyl)sulfonyl-2-azaspiro[3.3]heptan-6-yl]-8-methoxy-3-methyl-2,3-dihydropyrido[3,2-f] [1,4]oxazepin-5-one

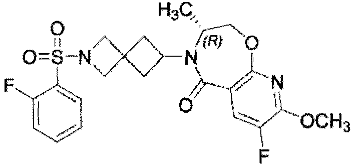

(1) In a 50 mL eggplant flask, 403 mg of 2,6-dichloro-5-fluoro-pyridine-3-carboxylic acid was weighed, and 7 mL of dichloromethane was added. Next, 175 µL of oxalyl chloride and 20 µL of N,N-dimethylformamide were added, and the mixture was stirred at room temperature for 75 minutes. The reaction solution was concentrated under reduced pressure to obtain a crude product of 2,6-dichloro-5-fluoro-pyridine-3-carboxylic acid chloride.
(2) The crude product obtained in (1) was dissolved in 9.0 mL of acetonitrile in a 50 mL eggplant flask, 206 mg of (2R)-2-[[2-(2-fluorophenyl)sulfonyl-2-azaspiro[3.3]heptan-6-yl]amino]propan-1-ol and 350 µL of triethylamine were added, and the mixture was stirred at room temperature for 100 minutes. Thereafter, 5.0 mL of a 1 N sodium hydroxide aqueous solution was added to the reaction mixture, and the mixture was stirred at room temperature for 80 minutes. Ethyl acetate, a saturated sodium bicarbonate solution, and brine were added to the reaction mixture, and the organic layer was extracted. The organic layer was washed with brine and then dried over magnesium sulfate. The insoluble matter was filtered, and then the solution was concentrated under reduced pressure. 0.40 g of the resulting residue was purified by silica gel column chromatography (solvent: hexane/ethyl acetate = 65/35 to 30/70) to obtain 260 mg of 2,6-dichloro-5-fluoro-N-[2-(2-fluorophenyl)sulfonyl-2-azaspiro[3.3]heptan-6-yl]-N-[(1R)-2-hydroxy-1-methyl-ethyl]pyridine-3-carboxamide (yield 2 step 80%) as a pale yellow solid.
   MS(ESI)m/z:520[M+H]⁺
(3) In a 100 mL eggplant flask, 259 mg of the 2,6-dichloro-5-fluoro-N-[2-(2-fluorophenyl)sulfonyl-2-azaspiro[3.3]heptan-6-yl]-N-[(1R)-2-hydroxy-1-methylethyl]pyridine-3-carboxamide obtained in (2) was weighed, and 5.0 mL of N,N-dimethylformamide was added. 26.0 mg of sodium hydride (60%) was added under ice-cooling, the mixture was stirred under ice-cooling for 1 hour, 23.7 mg of sodium hydride (60%) was additionally added, and the mixture was stirred under ice-cooling for 45 minutes. Next, 100 µL of methanol was added, and the mixture was stirred under ice-cooling for 15 minutes. A saturated ammonium chloride aqueous solution and brine were added to the reaction solution, and extraction was performed with ethyl acetate. The organic layer was washed with brine and then dried over magnesium sulfate. The insoluble matter was filtered, and then the solution was concentrated under reduced pressure. 0.35 g of the resulting pale yellow oily product was purified by silica gel column chromatography (solvent: hexane/ethyl acetate = 70/30 to 25/75) to obtain 104 mg of a colorless oily product. The colorless oily product was purified again by reverse-phase HPLC (solvent: 10 mM ammonium carbonate aqueous solution/acetonitrile = 70/30 to 40/60) to obtain 76.1 mg of the title compound (yield 32%) as a colorless amorphous solid.
MS(ESI)m/z:480[M+H]⁺

### Example 168

### Production of (7R)-2-[(1S*,5R*)-3-azabicyclo[3.1.0]hexan-3-yl]-6-[2-(2-fluorophenyl)sulfonyl]-2-azaspiro[3.3]heptan-6-yl]-7-methyl-7, 8-dihydropyrimido [5,4-f][1,4]oxazepin-5-one

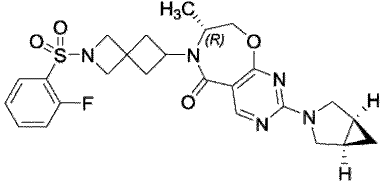

50 mg of (7R)-2-chloro-6-[2-(2-fluorophenyl)sulfonyl-2-azaspiro[3.3]heptan-2-yl]-7-methyl-7,8-dihydropyrimido[5,4-f] [1,4]oxazepin-5- one, 16 mg of 3-azabicyclo[3.1.0]hexane hydrochloride, and 46 µL of N,N-diisopropylethylamine were dissolved in 1 mL of N,N-dimethylformamide, and the mixture was stirred at room temperature for 0.5 hours. Water was added to the reaction mixture, and extraction was performed with ethyl acetate. The organic layer was washed with water and brine, filtration was performed after drying with sodium sulfate, and the filtrate was concentrated under reduced pressure. The resulting residue was purified by silica gel column chromatography (solvent: hexane/ethyl acetate = 80/20 to 0/100) to obtain 42 mg of the title compound (yield 91%) as a colorless powder.
MS(ESI)m/z:514[M+H]⁺

### Example 169:

A corresponding raw material compound was treated in the same manner as that of Example 168 to obtain a compound shown in Table 11.

**[Table 11]**

| Example | Structural formula | Physical property value and the like |
|---|---|---|
| 169 | 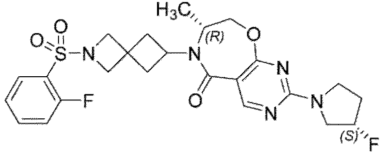 | MS (ESI) m/z;520 [M+H] + |

### Example 170

### Production of (6R)-5-[2-(2-fluorophenyl)sulfonyl-2-azaspiro[3.3]heptan-6-yl]-1,6-dimethyl-6,7-dihydropyrazolo[4,3-f] [1,4]oxazepin-4-one

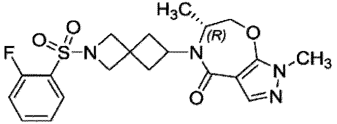

(1) To a mixture of 750 mg of ethyl 5-[(2R)-2-[[2-(2-fluorophenyl)sulfonyl-2-azaspiro[3.3]heptan-6-yl]amino]propoxy]-1-methyl-pyrazole-4-carboxylate, 4.70 mL of ethanol, and 4.70 mL of tetrahydrofuran, 4.70 mL of a 1 N sodium hydroxide aqueous solution was added. The reaction mixture was stirred at 70°C for 2 hours. After cooling to room temperature, the solvent was distilled off under reduced pressure to obtain a crude product of sodium 5-[(2R)-2-[[2-(2-fluorophenyl)sulfonyl-2-azaspiro[3.3]heptan-6-yl]amino]propoxy]-1-methyl-pyrazole-4-carboxylate.
(2) To a mixture of 10 mL of N,N-dimethylformamide of the crude product obtained in (1) above, 890 mg of HATU and 0.410 mL of N,N-diisopropylethylamine were added. The reaction mixture was stirred at room temperature for 30 minutes. Water and ethyl acetate were added to the reaction mixture, liquid separation was performed, and extraction from the aqueous layer was performed again with ethyl acetate. The organic layers were combined, washed with brine, and dried over magnesium sulfate. The insoluble matter was filtered off, and the filtrate was concentrated under reduced pressure. The resulting residue was purified by silica gel column chromatography (solvent: ethyl acetate/methanol = 100/0 to 95/5). When fractions were collected and concentrated, a solid was precipitated. Therefore, a mixed solvent of ethyl acetate and hexane (4:6) was added, suspension washing was performed, and the solid was collected by filtration. The resulting solid was dried under reduced pressure to obtain 301 mg of the title compound (yield 2 step 44%) as a colorless powder.
   MS(ESI)m/z:435[M+H]⁺

### Examples 171 to 233:

Corresponding raw material compounds were treated in the same manner as that of Example 170 to obtain compounds shown in Table 12.

**[Table 12-1]**

| Example | Structural formula | Physical property value and the like |
|---|---|---|
| 171 | 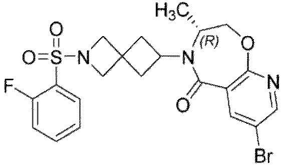 | MS(ESI) m/z;510/512 [M+H]+ |
| 172 | 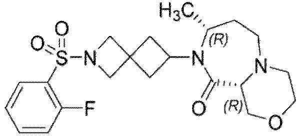 | MS(ESI) m/z;438 [M+H]+ |
| 173 | 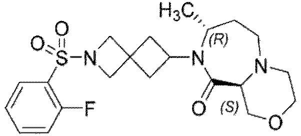 | MS(ESI) m/z;438 [M+H]+ |
| 174 | 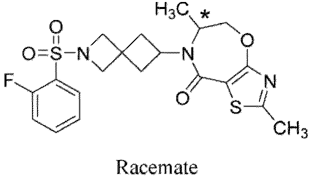 | MS(ESI) m/z;452 [M+H]+ |
| 175 | 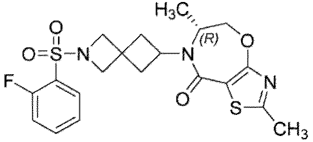 | MS(ESI) m/z;452 [M+H]+ |
| 176 | 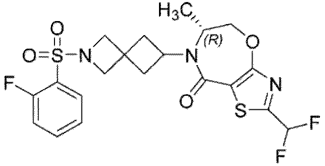 | MS(ESI) m/z;488 [M+H]+ |

**[Table 12-2]**

| | | |
|---|---|---|
| 177 | 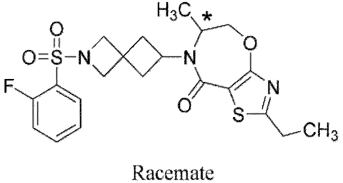 | MS(ESI) m/z;466 [M+H]+ |
| 178 | 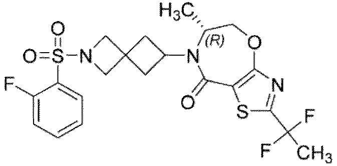 | MS(ESI) m/z;502 [M+H]+ |
| 179 | 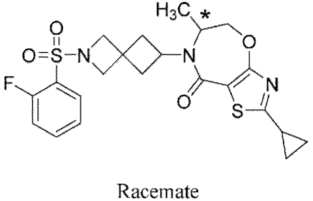 | MS(ESI) m/z;478 [M+H]+ |
| 180 | 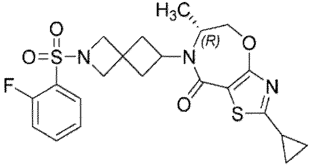 | MS(ESI) m/z;478 [M+H]+ |
| 181 | 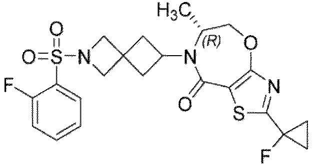 | MS(ESI) m/z;496 [M+H]+ |
| 182 | 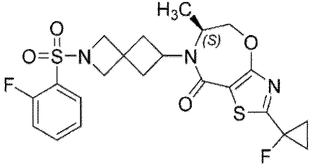 | MS(ESI) m/z;496 [M+H]+ |

**[Table 12-3]**

| | | |
|---|---|---|
| 183 | 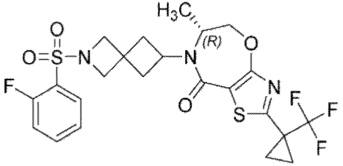 | MS(ESI) m/z;546 [M+H]+ |
| 184 | 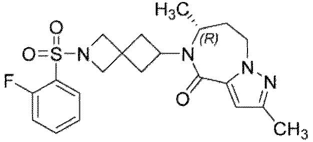 | MS(ESI) m/z;433 [M+H] + |
| 185 | 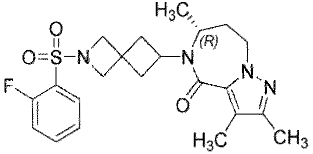 | MS(ESI) m/z;447 [M+H] + |
| 186 | 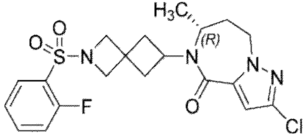 | MS(ESI) m/z;453[M+H]+ |
| 187 | 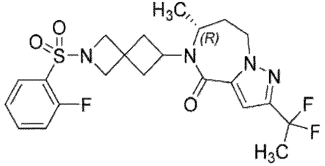 | MS(ESI) m/z;483 [M+H] + |
| 188 | 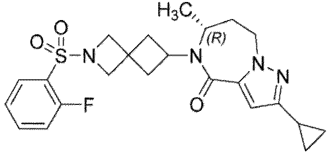 | MS(ESI) m/z;459[M+H]+ |
| 189 | 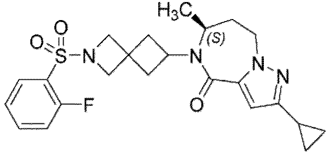 | MS(ESI) m/z;459[M+H]+ |

**[Table 12-4]**

| | | |
|---|---|---|
| 190 | 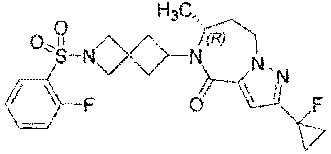 | MS(ESI) m/z;477[M+H]+ |
| 191 | 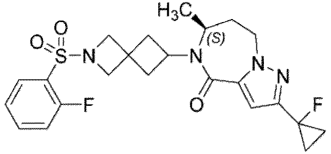 | MS(ESI) m/z;477[M+H]+ |
| 192 | 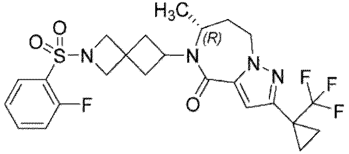 | MS(ESI) m/z;527[M+H]+ |
| 193 | 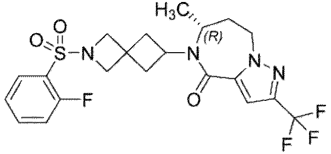 | MS(ESI) m/z;487[M+H]+ |
| 194 | 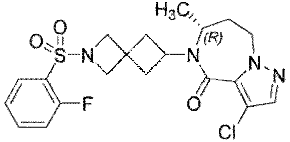 | MS(ESI) m/z;453/455 [M+H]+ |
| 195 | 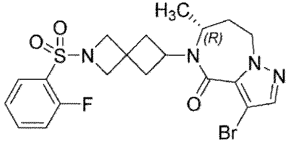 | MS(ESI) m/z;497/499 [M+H]+ |
| 196 | 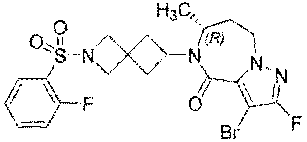 | MS(ESI) m/z;515/517 [M+H]+ |

**[Table 12-5]**

| | | |
|---|---|---|
| 197 | 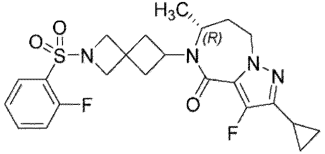 | MS(ESI) m/z;477 [M+H]+ |
| 198 | 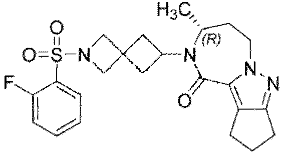 | MS(ESI) m/z;459 [M+H]+ |
| 199 | 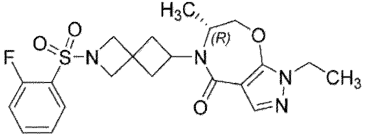 | MS(ESI) m/z;449 [M+H]+ |
| 200 | 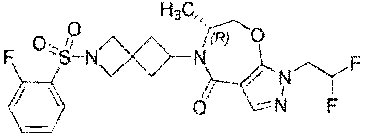 | MS(ESI) m/z;485 [M+H]+ |
| 201 | 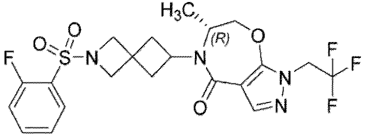 | MS(ESI) m/z;503 [M+H]+ |
| 202 | 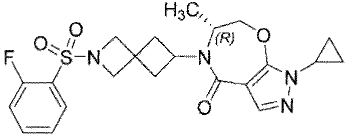 | MS(ESI) m/z;461 [M+H]+ |
| 203 | 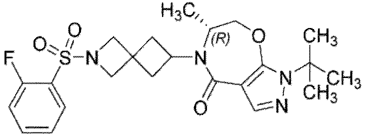 | MS(ESI) m/z;477 [M+H]+ |
| 204 | 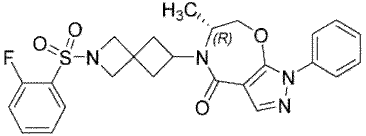 | MS(ESI) m/z;497 [M+H]+ |

**[Table 12-6]**

| | | |
|---|---|---|
| 205 | 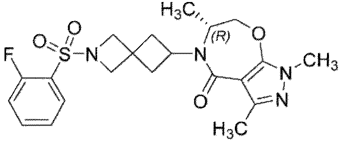 | MS(ESI) m/z;449 [M+H]+ |
| 206 | 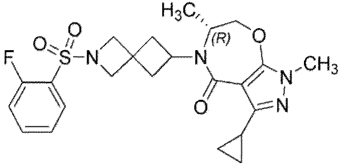 | MS(ESI) m/z;475 [M+H]+ |
| 207 | 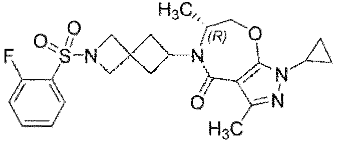 | MS(ESI) m/z;475 [M+H]+ |
| 208 | 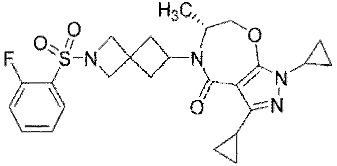 | MS (ESI) m/z;501 [M+H] + |
| 209 | 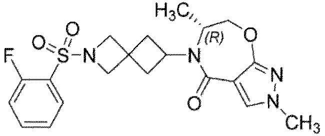 | MS(ESI) m/z;435 [M+H]+ |
| 210 | 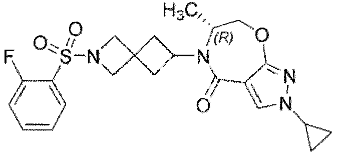 | MS(ESI) m/z;461 [M+H]+ |
| 211 | 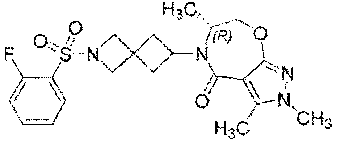 | MS(ESI) m/z;449 [M+H]+ |

**[Table 12-7]**

| | | |
|---|---|---|
| 212 | 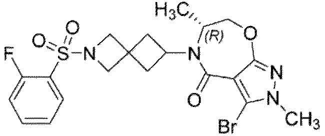 | MS(ESI) m/z;513/515 [M+H]+ |
| 214 | 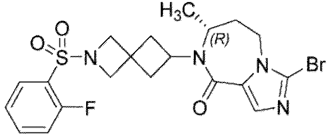 | MS(ESI) m/z;497/499 [M+H]+ |
| 215 | 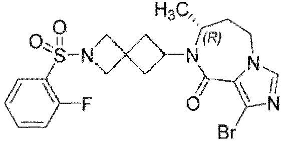 | MS(ESI) m/z;497/499 [M+H]+ |
| 216 | 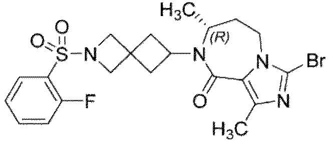 | MS(ESI) m/z;511/513 [M+H]+ |
| 217 | 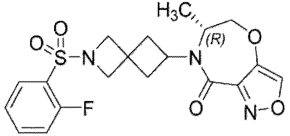 | MS(ESI) m/z;422 [M+H]+ |
| 218 | 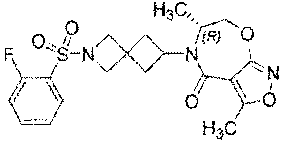 | MS(ESI) m/z;436 [M+H]+ |
| 219 | 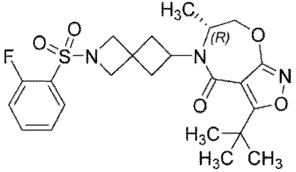 | MS(ESI) m/z;478 [M+H]+ |

**[Table 12-8]**

| | | |
|---|---|---|
| 220 | 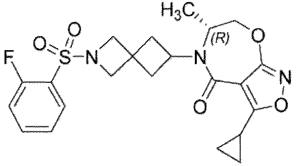 | MS(ESI) m/z;462 [M+H]+ |
| 221 | 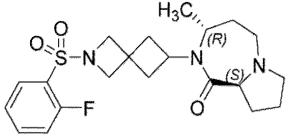 | MS(ESI) m/z;422 [M+H]+ |
| 222 | 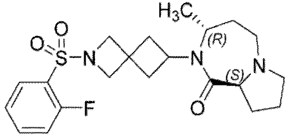 | MS(ESI) m/z;422 [M+H]+ |
| 223 | 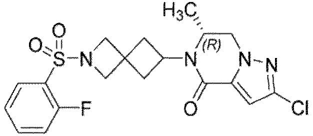 | MS(ESI) m/z;439/441 [M+H]+ |
| 224 | 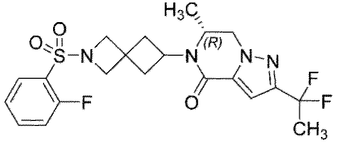 | MS(ESI) m/z;469 [M+H]+ |
| 225 | 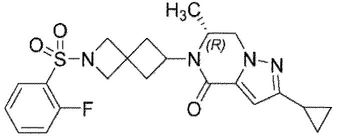 | MS(ESI) m/z;445 [M+H]+ |
| 226 | 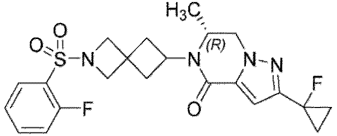 | MS(ESI) m/z;463 [M+H]+ |
| 227 | 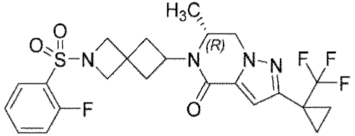 | MS(ESI) m/z;513 [M+H]+ |

**[Table 12-9]**

| | | |
|---|---|---|
| 228 | 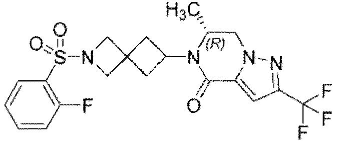 | MS(ESI) m/z;473 [M+H]+ |
| 229 | 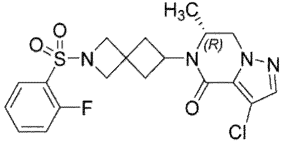 | MS(ESI) m/z;439/441 [M+H]+ |
| 230 | 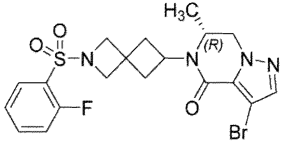 | MS(ESI) m/z;483/485 [M+H]+ |
| 231 | 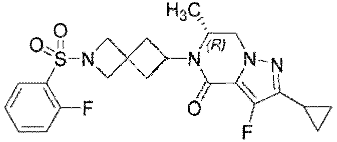 | MS(ESI) m/z; 463 [M+H]+ |
| 232 | 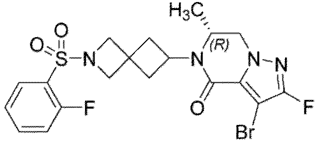 | MS(ESI) m/z;501/503 [M+H]+ |
| 233 | 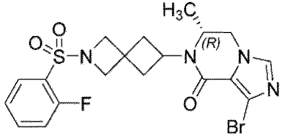 | MS(ESI) m/z;483/485 [M+H]+ |

### Example 234

### Production of (6R)-2-[(1S,2R)-2-fluorocyclopropyl]-7-[2-(2-fluorophenyl)sulfonyl-2-azaspiro[3.3]heptan-6-yl]-6-methyl-5,6-dihydrothiazolo[5,4-f] [1,4]oxazepin-8-one

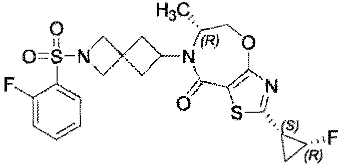

and Example 235

### Production of (6R)-2-[(1R,2R)-2-fluorocyclopropyl]-7-[2-(2-fluorophenyl)sulfonyl-2-azaspiro[3.3]heptan-6-yl]-6-methyl-5,6-dihydrothiazolo[5,4-f] [1,4]oxazepin-8-one

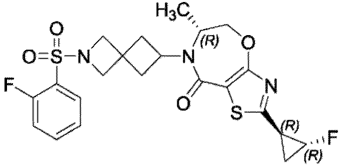

In a 100 mL eggplant flask, 390 mg of 2-[(1S,2R)-2-fluorocyclopropyl]-4-[(2R)-2-[[2-(2-fluorophenyl)sulfonyl-2-azaspiro[3.3]heptan-6-yl]amino]propoxy]thiazol-5-carboxylic acid ethyl ester was weighed, and 1.5 mL of ethanol and 2.5 mL of tetrahydrofuran were added and dissolved. Next, 1.50 mL of a 1 N sodium hydroxide aqueous solution was added, and the mixture was stirred at room temperature for 12 hours. To the reaction solution, 1.50 mL of 1 N hydrochloric acid was added, and the solvent was concentrated under reduced pressure. 4.0 mL of N,N-dimethylformamide was added thereto. Next, 364 mg of HATU and 350 µL of N,N-diisopropylethylamine were sequentially added, and the mixture was stirred at room temperature for 140 minutes. Ethyl acetate, a saturated sodium bicarbonate solution, and brine were added to the reaction solution, and the organic layer was extracted. The organic layer was washed with brine and then dried over magnesium sulfate. The insoluble matter was filtered, and then the solution was concentrated under reduced pressure. 0.62 g of the orange oily product was purified by silica gel column chromatography (solvent: hexane/ethyl acetate = 70/30 to 25/75). By thin-layer chromatography (solvent: hexane/ethyl acetate = 1:2), spots on the high polarity side (Rf to 0.2) were collected and concentrated to obtain 125 mg of a pale yellow amorphous solid. The pale yellow amorphous solid was purified by reverse-phase HPLC (solvent: 10 mM ammonium carbonate aqueous solution/acetonitrile = 70/30 to 40/60) to obtain 91.0 mg of the title compound (6R)-2-[(1S,2R)-2-fluorocyclopropyl]-7-[2-(2-fluorophenyl)sulfonyl-2-azaspiro[3.3]heptan-6-yl]-6-methyl-5,6-dihydrothiazolo[5,4-f][1,4]oxazepin-8-one (yield 2 step 26%) as a colorless amorphous solid.
MS(ESI)m/z:496[M+H]⁺

At the same time, in silica gel column chromatography, by thin-layer chromatography (solvent: hexane/ethyl acetate = 1:2), spots on the low polarity side (Rf to 0.4) were collected and concentrated, thereby obtaining 230 mg of the title compound (6R)-2-[(1R,2R)-2-fluorocyclopropyl]-7-[2-(2-fluorophenyl)sulfonyl-2-azaspiro[3.3]heptan-6-yl]-6-methyl-5,6-dihydrothiazolo[5,4-f] [1,4]oxazepin-8-one (yield 2 step 64%) as a colorless amorphous solid.
MS(ESI)m/z:496[M+H]⁺

### Example 236

### Production of (6R)-2-[(1R,2S)-2-fluorocyclopropyl]-7-[2-(2-fluorophenyl)sulfonyl-2-azaspiro[3.3]heptan-6-yl]-6-methyl-5,6-dihydrothiazolo[5,4-f] [1,4]oxazepin-8-one

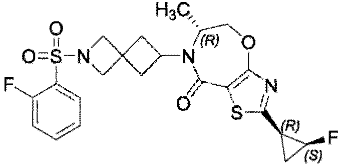

and Example 237

### Production of (6R)-2-[(1S,2S)-2-fluorocyclopropyl]-7-[2-(2-fluorophenyl)sulfonyl-2-azaspiro[3.3]heptan-6-yl]-6-methyl-5,6-dihydrothiazolo[5,4-f] [1,4]oxazepin-8-one

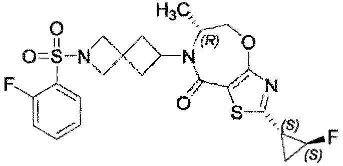

Using 387 mg of 2-[(1R,2S)-2-fluorocyclopropyl]-4-[(2R)-2-[[2-(2-fluorophenyl)sulfonyl-2-azaspiro[3.3]heptan-6-yl]amino]propoxy]thiazole-5-carboxylic acid ethyl ester, the reaction was performed according to the method of Example 234 to obtain 0.58 g of a crude product as an orange oily product. The orange oily product was purified by silica gel column chromatography (solvent: hexane/ethyl acetate = 70/30 to 25/75). By thin-layer chromatography (solvent: hexane/ethyl acetate = 1:2), spots on the high polarity side (Rf to 0.3) were collected and concentrated to obtain 174 mg of a yellow amorphous solid. The yellow amorphous solid was purified by reverse-phase HPLC (solvent: 10 mM ammonium carbonate aqueous solution/acetonitrile = 70/30 to 40/60) to obtain 119 mg of the title compound (6R)-2-[(1R,2S)-2-fluorocyclopropyl]-7-[2-(2-fluorophenyl)sulfonyl-2-azaspiro[3.3]heptan-6-yl]-6-methyl-5,6-dihydrothiazolo[5,4-f] [1,4]oxazepin-8-one (yield 2 step 34%) as a colorless amorphous solid.
MS(ESI)m/z:496[M+H]⁺

At the same time, in silica gel column chromatography, by thin-layer chromatography (solvent: hexane/ethyl acetate = 1:2), spots on the low polarity side (Rf to 0.5) were collected and concentrated, thereby obtaining 186 mg of the title compound (6R)-2-[(1S,2S)-2-fluorocyclopropyl]-7-[2-(2-fluorophenyl)sulfonyl-2-azaspiro[3.3]heptan-6-yl]-6-methyl-5,6-dihydrothiazolo[5,4-f] [1,4]oxazepin-8-one (yield 2 step 53%) as a colorless amorphous solid.
MS(ESI)m/z:496[M+H]⁺

### Example 238

### Production of (6R)-7-[2-(2-fluorophenyl)sulfonyl-2-azaspiro[3.3]heptan-6-yl]-6-methyl-3-(trifluoromethyl)-5,6-dihydroimidazo[1,5-a]pyrazin-8-one

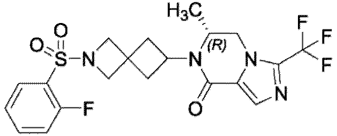

Into a 30 mL eggplant flask, 57.9 mg of N-[2-(2-fluorophenyl)sulfonyl-2-azaspiro[3.3]heptan-6-yl]-N-[(1R)-2-hydroxy-1-methyl-ethyl]-2-(trifluoromethyl)-1H-imidazole-5-carboxamide and 38 mg of triphenylphosphine were put and dissolved in 1 mL of toluene and 1 mL of tetrahydrofuran. 34 mg of bis(2-methoxyethyl) azodicarboxylate was added, and the mixture was stirred at room temperature for 0.5 hours. Brine was added thereto, and extraction was performed with ethyl acetate. The organic layer was filtered and concentrated. The resulting residue was purified by silica gel chromatography (solvent: hexane/ethyl acetate = 70/30 to 10/90) and further purified by reverse-phase preparative HPLC (solvent: 10 mM ammonium carbonate aqueous solution/acetonitrile = 70/30 to 40/60), thereby obtaining 5.3 mg of the title compound (yield 9.5%) as a pale yellow viscous oily product.
MS(ESI)m/z:473[M+H]⁺

### Example 239

### Production of 4-[2-(2-fluorophenyl)sulfonyl-2-azaspiro[3.3]heptan-6-yl]-3-methyl-2,3-dihydro-1,4-benzoxazepin-5-one

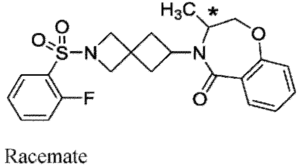

150 mg of 3-methyl-3,4-dihydro-2H-1,4-benzoxazepin-5-one was dissolved in 5 mL of N,N-dimethylformamide, and 34 mg of sodium hydride (60%) was added thereto. 300 mg of [2-(2-fluorophenyl)sulfonyl-2-azaspiro[3.3]heptan-6-yl]-4-methylbenzenesulfonate was added, and the mixture was stirred at 80°C for 2 hours. The temperature was raised to 110°C, and the mixture was stirred for 1hour. The temperature was raised to 130°C, and the mixture was stirred for 10 hours. Water was added to the reaction mixture, and extraction was performed with ethyl acetate. The organic layer was washed with water and brine, filtration was performed after drying with magnesium sulfate, and the filtrate was concentrated under reduced pressure. The resulting residue was dissolved in dimethyl sulfoxide and purified by reverse-phase HPLC (solvent: 10 mM ammonium carbonate aqueous solution/acetonitrile = 60/40 to 30/70), and then purified by silica gel column chromatography (solvent: hexane/ethyl acetate = 75/25 to 55/45), thereby obtaining 7 mg of the title compound (yield 2%) as a colorless powder.
MS(ESI)m/z:431 [M+H]⁺

### Examples 240 to 244:

Corresponding raw material compounds were treated in the same manner as that of Example 239 to obtain compounds shown in Table 13.

**[Table 13]**

| Example | Structural formula | Physical property value and the like |
|---|---|---|
| 240 | 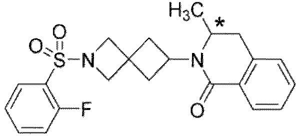 | MS(ESI) m/z;415 [M+H]+ |
| 241 | 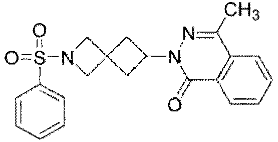 | MS(ESI) m/z;396 [M+H]+ |
| 242 | 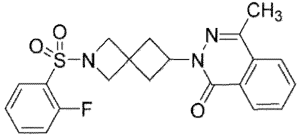 | MS(ESI) m/z;414 [M+H]+ |
| 243 | 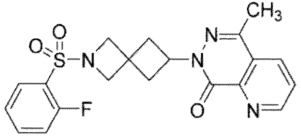 | MS(ESI) m/z;415 [M+H]+ |
| 244 | 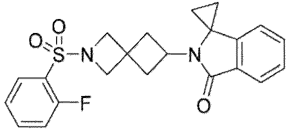 | MS(ESI) m/z;413 [M+H]+ |

### Example 245

### Production of (3R)-1-ethyl-4-[2-(2-fluorophenyl)sulfonyl-2-azaspiro[3.3]heptan-6-yl]-3-methyl-2,3-dihydropyrido[3,2-e][1,4]diazepin-5-one

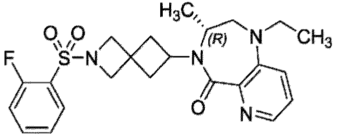

20 mg of (3R)-4-[2-(2-fluorophenyl)sulfonyl-2-azaspiro[3.3]heptan-6-yl]-3-methyl-2,3-dihydro-1H-pyrido[3,2-e][1,4]diazepin-5-one was dissolved in 1 mL of dichloromethane, 3.9 µL of acetaldehyde was added, the mixture was stirred at room temperature for 15 minutes, 16.7 mg of sodium triacetoxyborohydride was added, and the mixture was stirred for 1 hour. To the reaction solution, 26.2 µL of acetaldehyde and 19.7 mg of sodium triacetoxyborohydride were added, and the mixture was stirred at room temperature for 1 hour. To the reaction solution, 13.1 µL of acetaldehyde and 19.7 mg of sodium triacetoxyborohydride were added, and the mixture was stirred at room temperature for 1 hour. A saturated sodium bicarbonate aqueous solution was added to the reaction mixture, the mixture was stirred, the organic layer was separated with Phase-separator^{®}, and then the organic layer was concentrated under reduced pressure. The resulting residue was purified by NH silica gel column chromatography (solvent: ethyl acetate/methanol = 100/0 to 90/10) to obtain 19.9 mg of the title compound (yield 93%) as a light yellow powder.
MS(ESI)m/z:459[M+H]⁺

### Example 246:

A corresponding raw material compound was treated in the same manner as that of Example 245 to obtain a compound shown in Table 14.

**[Table 14]**

| Example | Structural formula | Physical property value and the like |
|---|---|---|
| 246 | 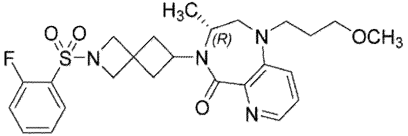 | MS(ESI) m/z;503 [M+H]+ |

### Example 247

### Production of (3R)-4-[2-(2-fluorophenyl)sulfonyl-2-azaspiro[3.3]heptan-6-yl]-1,3-dimethyl-8-(trifluoromethyl)-2,3-dihydropyrido[2,3-e] [1,4]diazepin-5-one

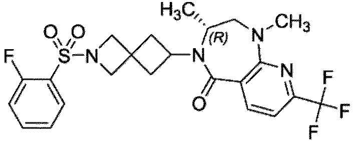

Into a 10 mL eggplant flask, 16.2 mg of (3R)-4-[2-(2-fluorophenyl)sulfonyl-2-azaspiro[3.3]heptan-6-yl]-3-methyl-8-(trifluoromethyl)-2,3-dihydro-1H-pyrido[2,3-e][1,4]diazepin-5-one was put and dissolved in 0.5 mL of N,N-dimethylformamide. 4 mg of sodium hydride and 0.010 mL of iodomethane were added under ice-cooling, and then the mixture was stirred at room temperature for 18 hours. A saturated ammonium chloride aqueous solution and ethyl acetate were added to the reaction mixture, and liquid separation was performed. The organic layer was dried over sodium sulfate, filtered, and concentrated. The resulting residue was dissolved in dimethyl sulfoxide and purified by reverse-phase preparative HPLC (solvent: 10 mM ammonium carbonate aqueous solution/acetonitrile = 50/50 to 20/80) to obtain 7.7 mg of the title compound (yield 46%) as a pale yellow powder.
MS(ESI)m/z:513[M+H]⁺

### Example 248:

A corresponding raw material compound was treated in the same manner as that of Example 247 to obtain a compound shown in Table 15.

**[Table 15]**

| Example | Structural formula | Physical property value and the like |
|---|---|---|
| 248 | 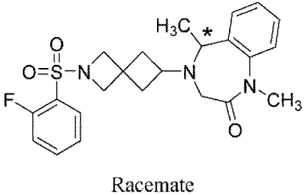 | MS(ESI) m/z;444 [M+H]+ |

### Example 249

### Production of 6-[2-(2-fluorophenyl)sulfonyl-2-azaspiro[3.3]heptan-6-yl]-2-methoxy-7-methyl-8,9-dihydro-7H-pyrido[3,2-c]azepin-5-one

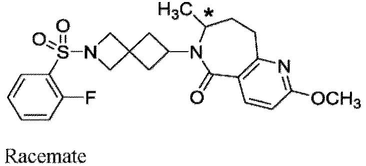

40 mg of 6-[2-(2-fluorophenyl)sulfonyl-2-azaspiro[3.3]heptan-6-yl]-7-methyl-1,7,8,9-tetrahydropyrido[3,2-c]azepin-2,5-dione was dissolved in 1 mL of N,N-dimethylformamide, and 5.4 mg of sodium hydride (60%) was added. 11.2 µL of methyl iodide was added thereto, and the mixture was stirred at room temperature for 0.5 hours. Water was added to the reaction mixture, and extraction was performed with ethyl acetate. The organic layer was washed with brine, filtration was performed after drying with magnesium sulfate, and the filtrate was concentrated under reduced pressure. The resulting residue was dissolved in dimethyl sulfoxide and purified by reverse-phase HPLC (solvent: 10 mM ammonium carbonate aqueous solution/acetonitrile = 70/30 to 40/60) to obtain 36 mg of the title compound (yield 87%) as a colorless powder.
MS(ESI)m/z:460[M+H]⁺

### Example 250

### Production of 2-[2-benzenesulfonyl-2-azaspiro[3.3]heptan-6-yl]-1-ethyl-indazol-3-one

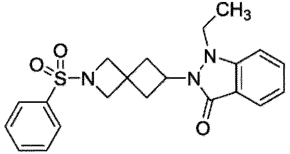

80 mg of 2-[2-benzenesulfonyl-2-azaspiro[3.3]heptan-6-yl]-1H-indazol-3-one was dissolved in 2 mL of N,N-dimethylformamide, 60 mg of potassium carbonate and 26.0 µL of ethyl iodide were added thereto, and the mixture was stirred at 100°C for 1.5 hours. The reaction solution was dissolved in dimethyl sulfoxide and purified by reverse-phase HPLC (solvent: 10 mM ammonium carbonate aqueous solution/acetonitrile = 70/30 to 40/60) to obtain 65 mg of the title compound (yield 76%) as a brown oily product.
MS(ESI)m/z:398[M+H]⁺

### Example 251

### Production of (3R)-8-chloro-4-[2-(2-fluorophenyl)sulfonyl-2-azaspiro[3.3]heptan-6-yl]-3-methyl-2,3-dihydro-1H-pyrido[2,3-e] [1,4]diazepin-5-one

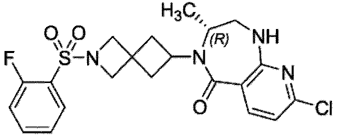

and Example 252

### Production of benzyl (3R)-8-chloro-4-[2-(2-fluorophenyl)sulfonyl-2-azaspiro[3.3]heptan-6-yl]-3-methyl-5-oxo-2,3-dihydropyrido[2,3-e] [1,4]diazepine-1-carboxylate

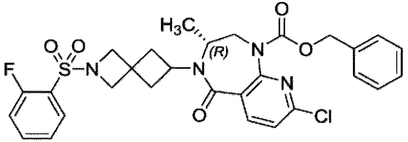

(1) 430 mg of 2,6-dichloropyridine-3-carboxylic acid was dissolved in 11 mL of dichloromethane, 284 µL of oxalyl chloride was added, 17.3 µL of N,N-dimethylformamide was added, and then the mixture was stirred at room temperature for 30 minutes. The reaction solution was concentrated under reduced pressure to obtain a crude product of 2,6-dichloropyridine-3-carbonyl chloride as a yellow viscous product.
(2) The crude product of 2,6-dichloropyridine-3-carbonyl chloride obtained in (1) above was dissolved in 5.4 mL of acetonitrile, a solution of 500 mg of benzyl N-[(2R)-2-[[2-(2-fluorophenyl)sulfonyl-2-azaspiro[3.3]heptan-6-yl]amino]propyl]carbamate and acetonitrile (5.4 mL) was added, 452 µL of triethylamine was added, and the mixture was stirred at room temperature for 30 minutes. A saturated sodium bicarbonate aqueous solution and chloroform were added to the reaction mixture, the mixture was stirred, the organic layer was separated with Phase-separator^{®}, and then the organic layer was concentrated under reduced pressure. The resulting residue was purified by silica gel column chromatography (solvent: hexane/ethyl acetate = 100/0 to 20/80) to obtain 579 mg of benzyl N-[(2R)-2-[(2,6-dichloropyridin-3-carbonyl)-[2-(2-fluorophenyl)sulfonyl-2-azaspiro[3.3]heptan-6-yl]amino]propyl]carbamate as a yellow powder.
(3) 579 mg of the benzyl N-[(2R)-2-[(2,6-dichloropyridin-3-carbonyl)-[2-(2-fluorophenyl)sulfonyl-2-azaspiro[3.3]heptan-6-yl]amino]propyl]carbamate obtained in (2) above was dissolved in 4.6 mL of N,N-dimethylformamide, 54.7 mg of sodium hydride (60%) was added, and the mixture was stirred at room temperature for 5 hours. A saturated ammonium chloride aqueous solution was added to the reaction solution to stop the reaction. Water was added to the reaction mixture, and extraction was performed with ethyl acetate. The organic layer was washed with water and brine and then separated with Phase-separator^{®}, and then the organic layer was concentrated under reduced pressure. The resulting residue was purified by NH silica gel column chromatography (solvent: hexane/ethyl acetate = 50/50 to 15/85) and then purified again by NH silica gel column chromatography (solvent: hexane/ethyl acetate = 50/50 to 20/80) to obtain 81.1 mg of (3R)-8-chloro-4-[2-(2-fluorophenyl)sulfonyl-2-azaspiro[3.3]heptan-6-yl]-3-methyl-2,3-dihydro-1H-pyrido[2,3-e][1,4]diazepin-5-one (two stage yield 16%) as a colorless powder.
   MS(ESI)m/z:465/467 [M+H]⁺

At the same time, 47.6 mg of benzyl (3R)-8-chloro-4-[2-(2-fluorophenyl)sulfonyl-2-azaspiro[3.3]heptan-6-yl]-3-methyl-2,3-dihydro[2,3-e][1,4]diazepine-1-carboxylate (two stage yield 8.7%) was obtained as a yellow powder.
MS(ESI)m/z:599/601[M+H]⁺

### Examples 253 to 257:

Corresponding raw material compounds were treated in the same manner as that of Example 251 to obtain compounds shown in Table 16.

**[Table 16]**

| Example | Structural formula | Physical property value and the like |
|---|---|---|
| 253 | 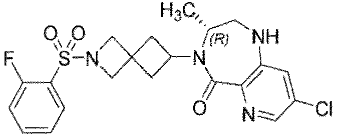 | MS(ESI) m/z;465/467[M+H]+ |
| 254 | 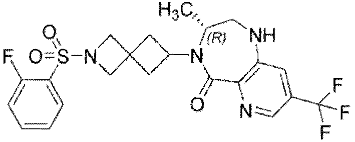 | MS(ESI) m/z;499[M+H]+ |
| 255 | 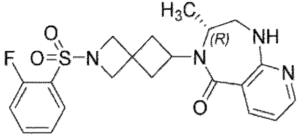 | MS(ESI) m/z;431[M+H]+ |
| 256 | 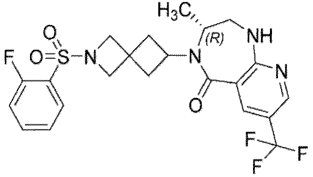 | MS(ESI) m/z;499[M+H]+ |
| 257 | 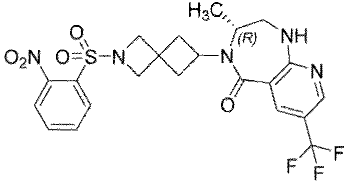 | MS(ESI) m/z;526[M+H]+ |

### Example 258

### Production of (3R)-8-cyclopropyl-4-[2-(2-fluorophenyl)sulfonyl-2-azaspiro[3.3]heptan-6-yl]-3-methyl-2,3-dihydro-1H-pyrido[2,3-e][1,4]diazepin-5-one

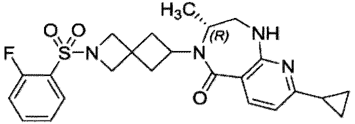

1 mL of toluene and 60 µL of water were added to 45 mg of benzyl (3R)-8-chloro-4-[2-(2-fluorophenyl)sulfonyl-2-azaspiro[3.3]heptan-6-yl]-3-methyl-5-oxo-2,3-dihydropyrido[2,3-e][1,4]diazepine-1-carboxylate, 9.7 mg of cyclopropyl boronic acid, 5.5 mg of bis(tricyclohexylphosphine)palladium(II) dichloride, and 47.8 mg of potassium phosphate were added, and the mixture was stirred at 130°C for 2 hours. To the reaction solution, 6.5 mg of cyclopropyl boronic acid and 5.5 mg of bis(tricyclohexylphosphine)palladium(II) dichloride were additionally added, and the mixture was stirred at 130°C for 2 hours. The reaction solution was cooled to room temperature, water and chloroform were added, the mixture was stirred, the organic layer was separated with Phase-separator^{®}, and then the organic layer was concentrated under reduced pressure. The resulting residue was dissolved in 2 mL of methanol, 0.75 mL of a 1 N sodium hydroxide aqueous solution was added, and the mixture was stirred at room temperature overnight. Thereafter, 0.38 mL of a 1 N sodium hydroxide aqueous solution was additionally added to the reaction solution, and the mixture was stirred at 80°C for 3 hours. The reaction solution was cooled to room temperature, water and chloroform were added, the mixture was stirred, the organic layer was separated with Phase-separator^{®}, and then the organic layer was concentrated under reduced pressure. The resulting residue was purified by silica gel column chromatography (solvent: hexane/ethyl acetate = 100/0 to 20/80). The resulting residue was purified by reverse-phase HPLC (solvent: 10 mM ammonium carbonate aqueous solution/acetonitrile = 60/40 to 30/70) to obtain 13.5 mg of the title compound (yield 2 step 38%) as a colorless powder.
MS(ESI)m/z:471[M+H]⁺

### Example 259

### Production of 4-[2-(2-fluorophenyl)sulfonyl-2-azaspiro[3.3]heptan-6-yl]-3-methyl-8-(trifluoromethyl)-2,3-dihydropyrido[3,2-f] [1,4]thiazepin-5-one

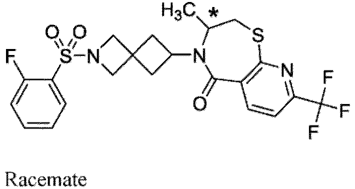

Into a 50 mL eggplant flask, 380.4 mg of 2-chloro-N-[2-[2-[[2-chloro-6-(trifluoromethyl)pyridin-3-carbonyl]-[2-(2-fluorophenyl)sulfonyl-2-azaspiro[3.3]heptan-6-yl]amino]propyldisulfanyl]-1-methyl-ethyl]-N-[2-(2-fluorophenyl)sulfonyl-2-azaspiro[3.3]heptan-6-yl]-6-(trifluoromethyl)pyridine-3-carboxamide was put and dissolved in 5 mL of dimethylformamide. 70 mg of sodium hydride was added under ice-cooling, and then the temperature was raised to room temperature. Subsequently, 60 mg of sodium borohydride was added, and the mixture was stirred at room temperature for 1 hour. A saturated ammonium chloride aqueous solution was added, and extraction was performed with ethyl acetate. The organic layer was dried over sodium sulfate, filtered, and concentrated. The resulting residue was dissolved in dimethyl sulfoxide and purified by reverse-phase preparative HPLC (solvent: 10 mM ammonium carbonate aqueous solution/acetonitrile = 60/40 to 30/70) to obtain 14.7 mg of the title compound (yield 8.3%) as a pale yellow powder.
MS(ESI)m/z:516[M+H]⁺

### Example 260

### Production of (7R)-8-[2-(2-fluorophenyl)sulfonyl-2-azaspiro[3.3]heptan-6-yl]-3,7-dimethyl-6,7-dihydro-5H-[1,2,4]triazolo[4,3-a][1,4]diazepin-9-one

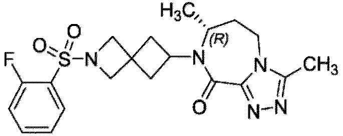

To a mixture of 60.0 mg of (7R)-1-[2-(2-fluorophenyl)sulfonyl-2-azaspiro[3.3]heptan-6-yl]-7-methyl-3-thioxo-1,4-diazepin-2-one and 2.00 mL of 1-butanol, 90.0 mg of acetohydrazine was added. The reaction mixture was stirred at 160°C for 6 hours. After cooling to room temperature, water and chloroform were added to the reaction mixture, liquid separation was performed, and extraction from the aqueous layer was performed again with chloroform. The organic layers were combined, washed with brine, and dried over magnesium sulfate. The insoluble matter was filtered off, and the filtrate was concentrated under reduced pressure. The resulting residue was purified by silica gel column chromatography (solvent: chloroform/methanol = 100/0 to 95/5). When fractions were collected and concentrated, a solid was precipitated. Therefore, ethyl acetate was added, suspension washing was performed, and the solid was collected by filtration. The resulting solid was dried under reduced pressure to obtain 23.1 mg of the title compound (yield 37%) as a colorless powder.
MS(ESI)m/z:434[M+H]⁺

### Examples 261 to 265:

Corresponding raw material compounds were treated in the same manner as that of Example 260 to obtain compounds shown in Table 17.

**[Table 17]**

| Example | Structural formula | Physical property value and the like |
|---|---|---|
| 261 | 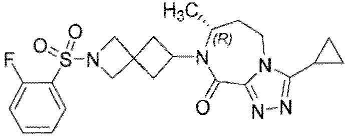 | MS(ESI) m/z;460 [M+H]+ |
| 262 | 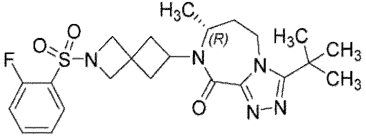 | MS(ESI) m/z;476 [M+H]+ |
| 263 | 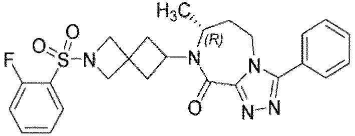 | MS(ESI) m/z;496 [M+H]+ |
| 264 | 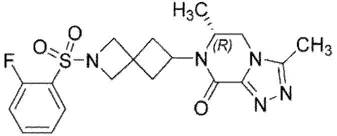 | MS(ESI) m/z;420 [M+H]+ |
| 265 | 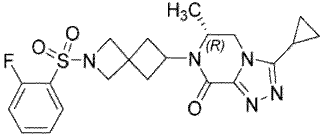 | MS(ESI) m/z;446 [M+H]+ |

### Example 266

### Production of 2-[2-(2-fluorophenyl)sulfonyl-2-azaspiro[3.3]heptan-6-yl]-3-methyl-isoindolin-1-one

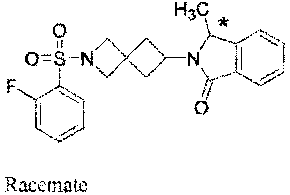

In a 10 mL eggplant flask, 82.3 mg of 2-[2-(2-fluorophenyl)sulfonyl-2-azaspiro[3.3]heptan-6-yl]-3-hydroxy-3-methyl-isoindolin-1-one was weighed and dissolved in 2.0 mL of dichloromethane. The resultant was cooled in a dry ice-acetone bath (external temperature -78°C), 130 µL of triethylsilane and then 50 µL of a borontrifluoride diethyl ether complex were added. Thereafter, the temperature was raised, and the mixture was stirred under ice-cooling for 40 minutes. Brine was added to the reaction solution, and extraction was performed with ethyl acetate. The organic layer was washed with brine and then dried over magnesium sulfate. The insoluble matter was filtered, and then the solution was concentrated under reduced pressure. 0.09 g of the resulting colorless oily product was purified by silica gel column chromatography (solvent: hexane/ethyl acetate = 80/20 to 50/50) to obtain 56.4 mg of the title compound (yield 71%) as a colorless powder.
MS(ESI)m/z:401[M+H]⁺

### Examples 267 to 274:

Corresponding raw material compounds were treated in the same manner as that of Example 266 to obtain compounds shown in Table 18.

**[Table 18-1]**

| Example | Structural formula | Physical property value and the like |
|---|---|---|
| 267 | 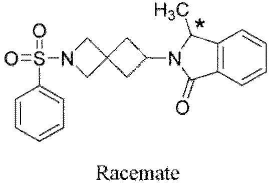 | MS (ESI) m/z;383 [M+H]+ |
| 268 | 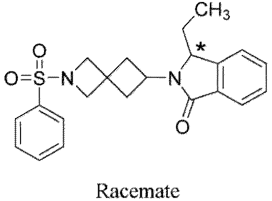 | MS(ESI) m/z;397 [M+H]+ |
| 269 | 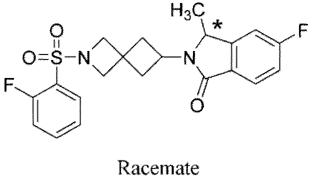 | MS(ESI) m/z;419 [M+H]+ |
| 270 | 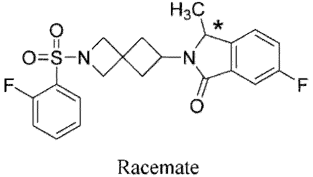 | MS(ESI) m/z;419 [M+H]+ |
| 271 | 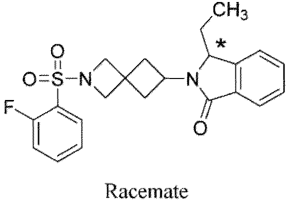 | MS(ESI) m/z;415 [M+H]+ |

**[Table 18-2]**

| | | |
|---|---|---|
| 272 | 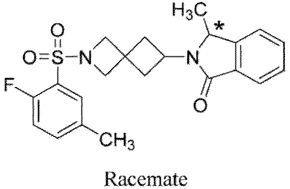 | MS(ESI) m/z;415 [M+H]+ |
| 273 | 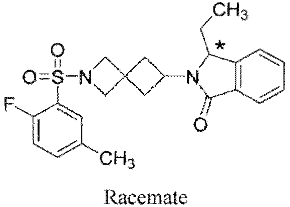 | MS(ESI) m/z;429 [M+H]+ |
| 274 | 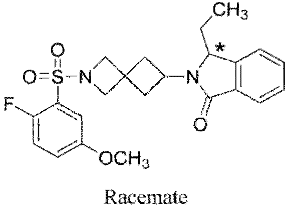 | MS(ESI) m/z;445 [M+H]+ |

### Example 275

### Production of 6-[2-(2-fluorophenyl)sulfonyl-2-azaspiro[3.3]heptan-6-yl]-5-hydroxy-5-methyl-3-(trifluoromethyl)pyrrolo[3,4-b]pyridin-7-one

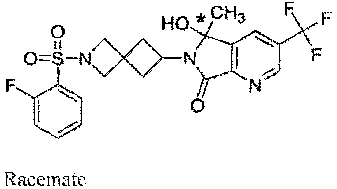

In a 100 mL eggplant flask, 579 mg of 3-bromo-N-[2-(2-fluorophenyl)sulfonyl-2-azaspiro[3.3]heptan-6-yl]-5-(trifluoromethyl)pyridine-2-carboxamide was weighed, and 8.0 mL of 1,4-dioxane was added. Next, 600 µL of tributyl(1-ethoxyvinyl)tin and 196 mg of tetrakis triphenylphosphine palladium were added, and the mixture was heated and stirred under a nitrogen atmosphere at 100°C for 24 hours. The reaction solution was cooled to room temperature, and then the reaction solution was diluted with ethyl acetate and subjected to celite filtration. The organic layer was washed with brine and then dried over magnesium sulfate. The insoluble matter was filtered, and the filtrate was concentrated under reduced pressure. 1.41 g of the resulting yellowish brown oily product was purified by NH silica gel column chromatography (solvent: hexane/ethyl acetate = 80/20 to 45/55) to obtain 455 mg of a crude product of 3-(1-ethoxyvinyl)-N-[2-(2-fluorophenyl)sulfonyl-2-azaspiro[3.3]heptan-6-yl]-5-(trifluoromethyl)pyridine-2-carboxamide as a pale yellow solid. In a 100 mL eggplant flask, 455 mg of the resulting crude product was weighed and dissolved in 10 mL of tetrahydrofuran. Next, 2.0 mL of 1 N hydrochloric acid was added, and the mixture was stirred at room temperature for 3 hours. Ethyl acetate and a saturated sodium bicarbonate aqueous solution were added to the reaction mixture, and the organic layer was extracted. The organic layer was washed with brine and then dried over magnesium sulfate. The insoluble matter was filtered, and then the solution was concentrated under reduced pressure. The resulting pale yellow solid was suspended and washed with 20 mL of diisopropyl ether, and a solid was collected by filtration and dried under reduced pressure, thereby obtaining 373 mg of the title compound (yield 2 step 69%) as a colorless powder.
MS(ESI)m/z:486[M+H]⁺

### Examples 276 to 282:

Corresponding raw material compounds were treated in the same manner as that of Example 275 to obtain compounds shown in Table 19.

**[Table 19-1]**

| Example | Structural formula | Physical property value and the like |
|---|---|---|
| 276 | 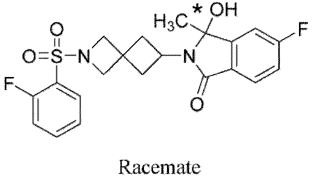 | MS (ESI) m/z;435 [M+H]+ |
| 277 | 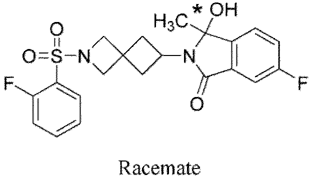 | MS (ESI) m/z;435 [M+H]+ |
| 278 | 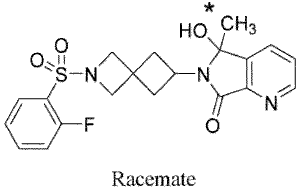 | MS (ESI) m/z;418 [M+H]+ |
| 279 | 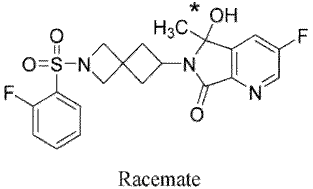 | MS (ESI) m/z;436 [M+H]+ |
| 280 | 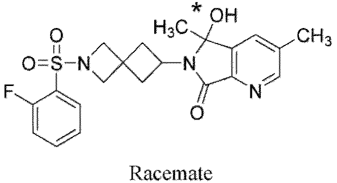 | MS(ESI) m/z;432 [M+H]+ |

**[Table 19-2]**

| | | |
|---|---|---|
| 281 | 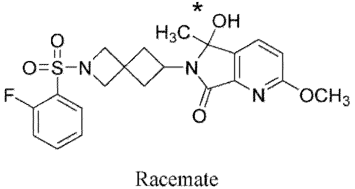 | MS (ESI) m/z;448 [M+H]+ |
| 282 | 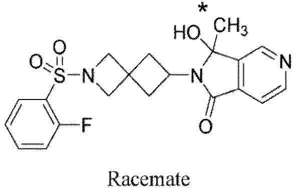 | MS (ESI) m/z;418 [M+H]+ |

### Example 283

### Production of 2-[2-(2-fluorophenyl)sulfonyl-2-azaspiro[3.3]heptan-6-yl]-3-hydroxy-3-methyl-isoindolin-1-one

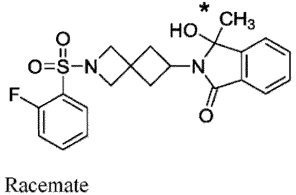

In a 50 mL eggplant flask, 223 mg of 2-[2-(2-fluorophenyl)sulfonyl-2-azaspiro[3.3]heptan-6-yl]isoindolin-1,3-dione was weighed, 6.0 mL of tetrahydrofuran was added, and the mixture was stirred under ice-cooling. Next, 2.0 mL of a 1 mol/L methylmagnesium bromide tetrahydrofuran solution was added, and the mixture was stirred under ice-cooling for 105 minutes. An ammonium chloride aqueous solution and brine were added to the reaction mixture. Extraction was performed with ethyl acetate, washing was performed with brine, and drying was performed with magnesium sulfate. The insoluble matter was filtered, and then the solution was concentrated under reduced pressure. 0.29 g of the resulting colorless solid was purified by silica gel column chromatography (solvent: hexane/ethyl acetate = 75/25 to 30/70) to obtain 216 mg of the title compound (yield 93%) as a colorless powder.
MS(ESI)m/z:417[M+H]⁺

### Examples 284 to 289:

Corresponding raw material compounds were treated in the same manner as that of Example 283 to obtain compounds shown in Table 20.

**[Table 20]**

| Example | Structural formula | Physical property value and the like |
|---|---|---|
| 284 | 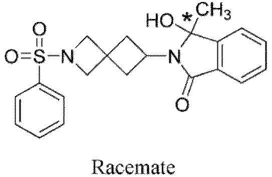 | MS(ESI) m/z;399 [M+H]+ |
| 285 | 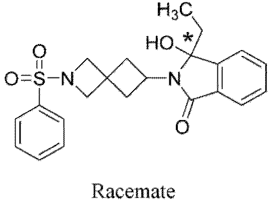 | MS(ESI) m/z;413 [M+H]+ |
| 286 | 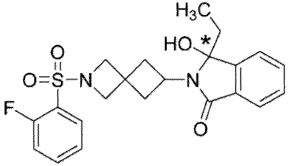 | MS(ESI) m/z;431 [M+H]+ |
| 287 | 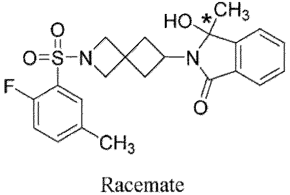 | MS(ESI) m/z;431 [M+H]+ |
| 288 | 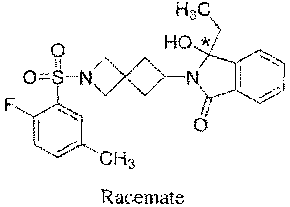 | MS(ESI) m/z;445 [M+H]+ |
| 289 | 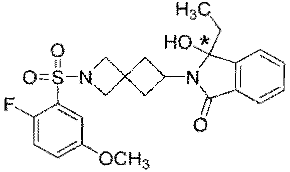 | MS(ESI) m/z;461 [M+H]+ |

### Example 290

### Production of 6-[2-(2-fluorophenyl)sulfonyl-2-azaspiro[3.3]heptan-6-yl]-5-methyl-3-(trifluoromethyl)-5H-pyrrolo[3,4-b]pyridin-7-one

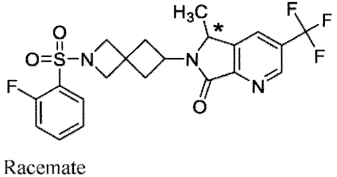

In a 50 mL eggplant flask, 321 mg of 6-[2-(2-fluorophenyl)sulfonyl-2-azaspiro[3.3]heptan-6-yl]-5-hydroxy-5-methyl-3-(trifluoromethyl)pyrrolo[3,4-b]pyridin-7-one was weighed, and 6.0 mL of dichloromethane was added. Next, 219 mg of methanesulfonic anhydride and 500 µL of triethylamine were added, and the mixture was stirred at room temperature for 1 hour. The reaction solution was diluted with ethyl acetate, washing was performed with a saturated sodium bicarbonate solution and brine, and then drying was performed with magnesium sulfate. The insoluble matter was filtered off, and the solution was concentrated under reduced pressure, thereby obtaining 327 mg of a crude product of 6-[2-(2-fluorophenyl)sulfonyl-2-azaspiro[3.3]heptan-6-yl]-5-methylen-3-(trifluoromethyl)pyrrolo[3,4-b]pyridin-7-one as a colorless solid. In a 100 mL eggplant flask, 327 mg of the resulting crude product was weighed, and 8.0 mL of ethanol and 2.0 mL of tetrahydrofuran were added. Next, 304 mg of a 5% palladium/carbon(hydrous) was added, and the mixture was stirred under a hydrogen atmosphere at room temperature for 7 hours. The reaction solution was diluted with 20 mL of chloroform, and then the mixture was filtered through celite. Washing was performed with 40 mL of chloroform, and then the filtrate was concentrated under reduced pressure. 0.34 g of the resulting pale yellow oily product was purified by NH silica gel column chromatography (solvent: hexane/ethyl acetate = 55/45 to 30/70) to obtain 251 mg of the title compound (yield 2 step 81%) as a colorless solid.
MS(ESI)m/z:470[M+H]⁺

### Examples 291 to 295:

Corresponding raw material compounds were treated in the same manner as that of Example 290 to obtain compounds shown in Table 21.

**[Table 21]**

| Example | Structural formula | Physical property value and the like |
|---|---|---|
| 291 | 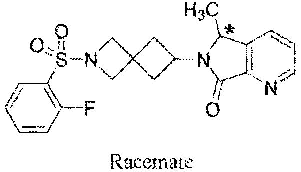 | MS(ESI) m/z;402 [M+H]+ |
| 292 | 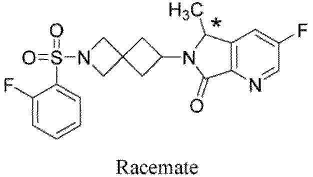 | MS(ESI) m/z;420 [M+H]+ |
| 293 | 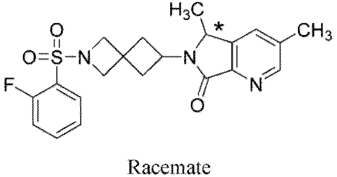 | MS(ESI) m/z;416 [M+H]+ |
| 294 | 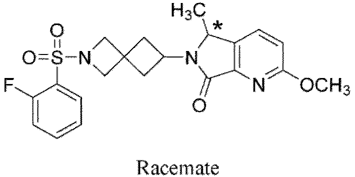 | MS(ESI) m/z;432 [M+H]+ |
| 295 | 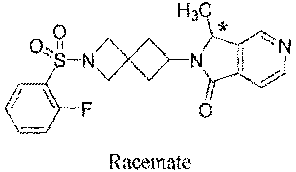 | MS(ESI) m/z;402 [M+H]+ |

### Example 296

### Production of 2-[2-(2-fluorophenyl)sulfonyl-2-azaspiro[3.3]heptan-6-yl]-1-methyl-1H-pyrrolo[3,4-c]pyridin-3-one

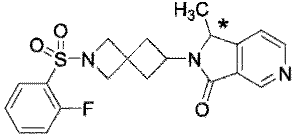

150 mg of 2-(2-fluorophenyl)sulfonyl-2-azaspiro[3.3]heptan-6-amine and 100 mg of methyl 4-acetylpyridine-3-carboxylate were dissolved in 5 mL of tetrahydrofuran, 473 mg of tetraisopropyl orthotitanate was added, and the mixture was stirred at room temperature for 14 hours. 471 mg of sodium triacetoxyborohydride was added to the reaction solution, the temperature was raised to 50°C, and the mixture was stirred for 1 hour. The reaction solution was diluted with chloroform, NH silica gel was added, and the mixture was concentrated under reduced pressure. The resultant was purified by NH silica gel column chromatography (solvent: hexane/ethyl acetate = 50/50 to 0/100) to obtain 155 mg of the title compound (yield 70%) as a colorless oily product.
MS(ESI)m/z:402[M+H]⁺

### Example 297

### Production of (6R)-3-bromo-7-[2-(2-fluorophenyl)sulfonyl-2-azaspiro[3.3]heptan-6-yl]-6-methyl-5,6-dihydroisoxazolo[3,4-f][1,4]oxazepin-8-one

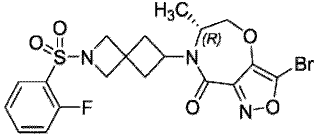

163 mg of (6R)-7-[2-(2-fluorophenyl)sulfonyl-2-azaspiro[3.3]heptan-6-yl]-6-methyl-5,6-dihydroisoxazolo[3,4-f][1,4]oxazepin-8-one was dissolved in 3 mL of acetonitrile, 1-bromopyrrolidine-2,5-dione was added, and the mixture was stirred at room temperature for 2 hours. Further, the mixture was stirred at 50°C for 3 hours, at room temperature for 15 hours, and at 50°C for 2 hours, water was added to the reaction mixture, and then extraction was performed with ethyl acetate. The organic layer was washed with water and brine, dried over magnesium sulfate, and then filtered. The filtrate was concentrated under reduced pressure, and the resulting residue was purified by silica gel column chromatography (solvent: hexane/ethyl acetate = 75/25 to 20/80) to obtain 69 mg of the title compound (yield 36%) as a colorless powder.
MS(ESI)m/z:500/502[M+H]⁺

### Examples 298 and 299:

Corresponding raw material compounds and reagents were treated in the same manner as that of Example 297 to obtain compounds shown in Table 22.

**[Table 22]**

| Example | Structural formula | Physical property value and the like |
|---|---|---|
| 298 | 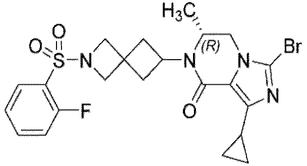 | MS(ESI) m/z;523/525 [M+H]+ |
| 299 | 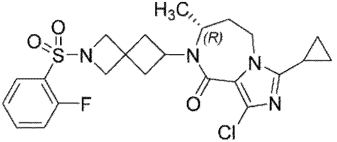 | MS(ESI) m/z;493/495 [M+H]+ |

### Example 300

### Production of 9,9-Difluoro-6-[2-(2-fluorophenyl)sulfonyl-2-azaspiro [3.3]heptan-6-yl]-7-methyl-7,8-dihydropyrido [3,2-c] azepin-5-one

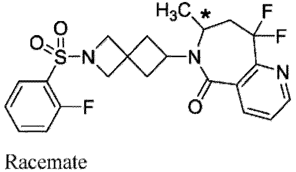

and Example 301

### Production of 9-fluoro-6-[2-(2-fluorophenyl)sulfonyl-2-azaspiro[3.3]heptan-6-yl]-7-methyl-7H-pyrido [3,2-c] azepin-5-one

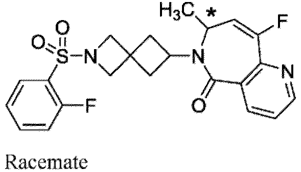

80 mg of 6-[2-(2-fluorophenyl)sulfonyl-2-azaspiro[3.3]heptan-6-yl]-7-methyl-7,8-dihydropyrido[3,2-c]azepin-5,9-dione was dissolved in 2 mL of chloroform, and 0.166 mL of bis(2-methoxyethyl)aminosulfur trifluoride was added. The mixture was stirred at 50°C for 1 hour. A saturated sodium hydride aqueous solution was added to the reaction mixture, and extraction was performed with chloroform. The organic layer was washed with water and brine, filtration was performed after drying with magnesium sulfate, and the filtrate was concentrated under reduced pressure. The resulting residue was purified by silica gel column chromatography (solvent: hexane/ethyl acetate = 50/50 to 35/65 to 30/70) to obtain 34 mg of 9,9-difluoro-6-[2-(2-fluorophenyl)sulfonyl-2-azaspiro[3.3]heptan-6-yl]-7-methyl-7,8-dihydropyrido[3,2-c]azepin-5-one (yield 40%) as a colorless powder.
MS(ESI)m/z:466[M+H]⁺

In addition, 9 mg of 9-fluoro-6-[2-(2-fluorophenyl)sulfonyl-2-azaspiro[3.3]heptan-6-yl]-7-methyl-7H-pyrido[3,2-c]azepin-5-one (yield 11%) was obtained as a colorless powder.
MS(ESI)m/z:446[M+H]⁺

### Example 302

### Production of (3R)-1-acetyl-4-[2-(2-fluorophenyl)sulfonyl-2-azaspiro[3.3]heptan-6-yl]-3-methyl-2,3-dihydropyrido[3,2-e] [1,4]diazepin-5-one

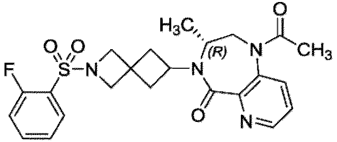

15 mg of (3R)-4-[2-(2-fluorophenyl)sulfonyl-2-azaspiro[3.3]heptan-6-yl]-3-methyl-2,3-dihydro-1H-pyrido[3,2-e][1,4]diazepin-5-one was dissolved in 1 mL of chloroform, 18.1 µL of N,N-diisopropylethylamine was added, and the mixture was ice-cooled. 5.0 µL of acetyl chloride was added to the reaction mixture, and the mixture was stirred at room temperature for 1 hour. 2.5 µL of acetyl chloride was added to the reaction mixture, and the mixture was stirred at room temperature for 1 hour. A saturated sodium bicarbonate aqueous solution was added to the reaction mixture, the mixture was stirred, the organic layer was separated with Phase-separator^{®}, and then the organic layer was concentrated under reduced pressure. The resulting residue was purified by NH silica gel column chromatography (solvent: ethyl acetate/methanol = 100/0 to 95/5) and then purified again by silica gel column chromatography (solvent: ethyl acetate/methanol = 100/0 to 90/10), thereby obtaining 10.7 mg of the title compound (yield 65%) as a colorless powder.
MS(ESI)m/z:473[M+H]⁺

### Examples 303 to 308:

Corresponding raw material compounds were treated in the same manner as that of Example 302 to obtain compounds shown in Table 23.

**[Table 23]**

| Example | Structural formula | Physical property value and the like |
|---|---|---|
| 303 | 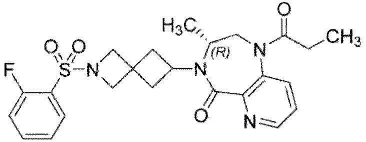 | MS(ESI) m/z;487 [M+H]+ |
| 304 | 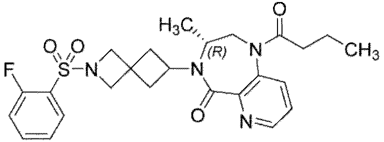 | MS(ESI) m/z;501 [M+H]+ |
| 305 | 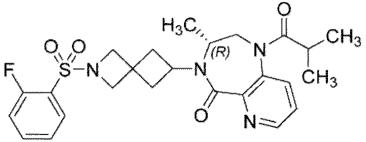 | MS(ESI) m/z;501 [M+H]+ |
| 306 | 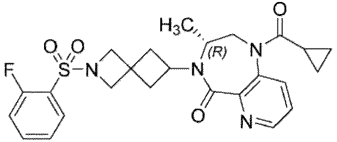 | MS(ESI) m/z;499 [M+H]+ |
| 307 | 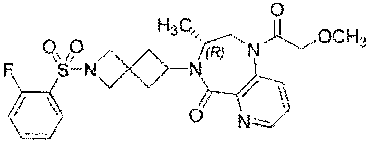 | MS(ESI) m/z;503 [M+H]+ |
| 308 | 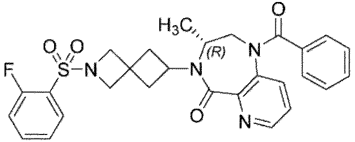 | MS(ESI) m/z;535 [M+H]+ |

### Example 309

### Production of (7R)-8-[2-(2-fluorophenyl)sulfonyl-2-azaspiro[3.3]heptan-6-yl]-7-methyl-6,7-dihydro-5H-imidazo[1,5-a][1,4]diazepin-9-one

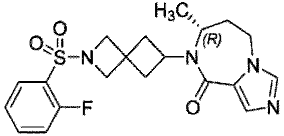

and Example 310

### Production of (7R)-1-cyclohexyl-8-[2-(2-fluorophenyl)sulfonyl-2-azaspiro[3.3]heptan-6-yl]-7-methyl-6,7-dihydro-5H-imidazo[1,5-a][1,4]diazepin-9-one

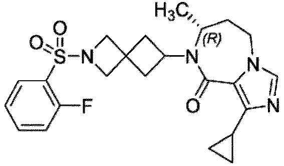

100 mg of (7R)-1-bromo-8-[2-(2-fluorophenyl)sulfonyl-2-azaspiro[3.3]heptan-6-yl]-7-methyl-6,7-dihydro-5H-imidazo[1,5-a][1,4]diazepin-9-one, 25.9 mg of cyclopropaneboronic acid, 14.8 mg of dichlorobis(tricyclohexylphosphine)palladium, and 85.4 mg of potassium phosphate were suspended in 2 mL of toluene and 0.4 mL of water, and the mixture was stirred at 110°C for 7 hours.

To the reaction mixture, 25.9 mg of cyclopropaneboronic acid, 14.8 mg of dichlorobis(tricyclohexylphosphine)palladium, and 85.4 mg of potassium phosphate were added, and the mixture was stirred at 110°C for 4 hours. The reaction mixture was returned to room temperature, water was added, and extraction was performed with chloroform. The organic layer was dried over magnesium sulfate, filtration was performed, and then the filtrate was concentrated under reduced pressure. To the resulting residue, 44.6 mg of potassium cyclopropyl trifluoroborate, 6.8 mg of palladium acetate, 7.2 mg of butyldi-1-adamantylphosphine, and 131 mg of cesium carbonate were added, and the mixture was suspended in 2 mL of toluene and 0.3 mL of water. The mixture was stirred at 110°C for 8 hours. Water was added to the reaction mixture, and extraction was performed with chloroform. The organic layer was washed with water and brine, filtration was performed after drying with magnesium sulfate, and the filtrate was concentrated under reduced pressure. The resulting residue was dissolved in dimethyl sulfoxide, purified by reverse-phase HPLC (solvent: 10 mM ammonium carbonate aqueous solution/acetonitrile = 70/30 to 40/60) and then purified by NH silica gel column chromatography (solvent: ethyl acetate/methanol = 100/0 to 94/6), thereby obtaining 5.4 mg of (7R)-8-[2-(2-fluorophenyl)sulfonyl-2-azaspiro[3.3]heptan-6-yl]-7-methyl-6,7-dihydro-SH-imidazo[1,5-a] [1,4]diazepin-9-one (yield 6.4%) as a colorless powder.
MS(ESI)m/z:419[M+H]⁺

In addition, the resultant was purified again by silica gel column chromatography (solvent: ethyl acetate/methanol = 100/0 to 90/10) to obtain 3.2 mg of (7R)-1-cyclohexyl-8-[2-(2-fluorophenyl)sulfonyl-2-azaspiro[3.3]heptan-6-yl]-7-methyl-6,7-dihydro-5H-imidazo[1,5-a][1,4]diazepin-9-one (yield 3.5%) as a colorless powder.
MS(ESI)m/z:459[M+H]⁺

### Examples 311 to 330:

Corresponding raw material compounds and reagents were treated in the same manner as that of Example 310 to obtain compounds shown in Table 24.

**[Table 24-1]**

| Example | Structural formula | Physical property value and the like |
|---|---|---|
| 311 | 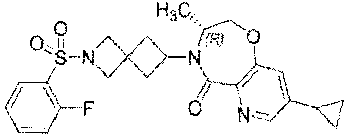 | MS(ESI) m/z;472 [M+H]+ |
| 312 | 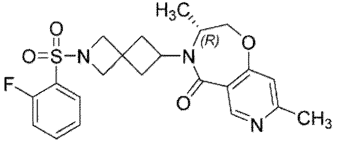 | MS(ESI) m/z;446 [M+H]+ |
| 313 | 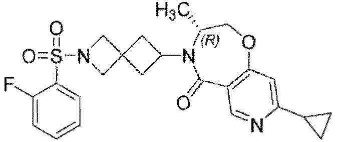 | MS(ESI) m/z;472 [M+H]+ |
| 314 | 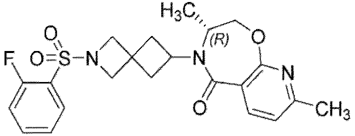 | MS(ESI) m/z;446 [M+H]+ |
| 315 | 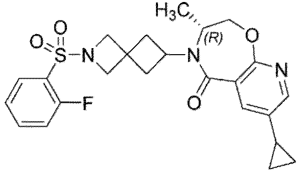 | MS(ESI) m/z;472 [M+H]+ |
| 316 | 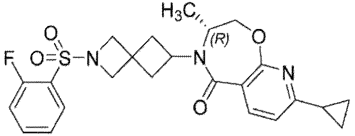 | MS(ESI) m/z;472 [M+H]+ |
| 317 | 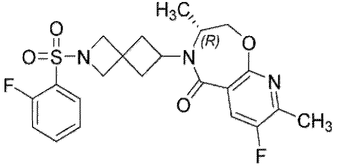 | MS(ESI) m/z;464 [M+H]+ |

**[Table 24-2]**

| | | |
|---|---|---|
| 318 | 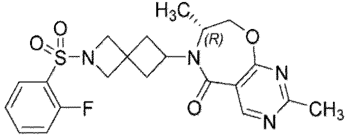 | MS(ESI) m/z;447 [M+H]+ |
| 319 | 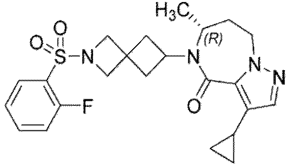 | MS(ESI) m/z;459 [M+H]+ |
| 320 | 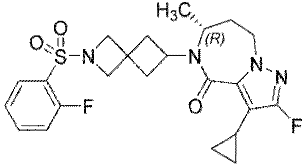 | MS(ESI) m/z;477 [M+H]+ |
| 321 | 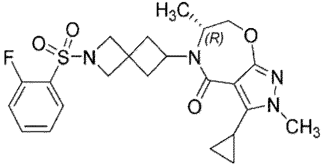 | MS(ESI) m/z;475 [M+H]+ |
| 322 | 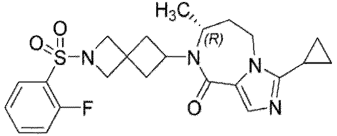 | MS(ESI) m/z;459 [M+H]+ |
| 323 | 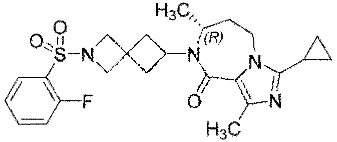 | MS(ESI) m/z;473 [M+H]+ |
| 324 | 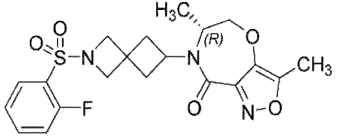 | MS(ESI) m/z;436 [M+H]+ |

**[Table 24-3]**

| | | |
|---|---|---|
| 325 | 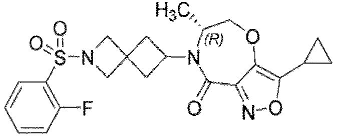 | MS(ESI) m/z;462 [M+H]+ |
| 326 | 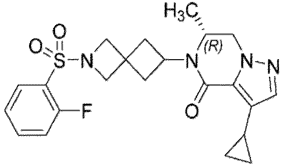 | MS(ESI) m/z;445 [M+H]+ |
| 327 | 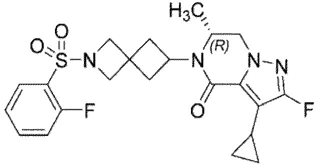 | MS(ESI) m/z;463 [M+H]+ |
| 328 | 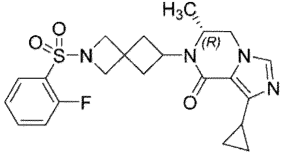 | MS(ESI) m/z;445 [M+H]+ |
| 329 | 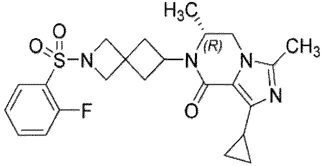 | MS(ESI) m/z;459 [M+H]+ |
| 330 | 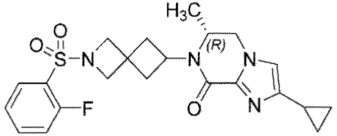 | MS(ESI) m/z;445 [M+H]+ |

### Example 331

### Production of (7R)-2-cyclopropyl-6-[2-(2-fluorophenyl)sulfonyl-2-azaspiro[3.3]heptan-6-yl]-7-methyl-7,8-dihydropyrimido[5,4-f] [1,4]oxazepin-5-one

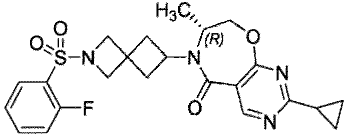

To a mixture of 91 mg of (7R)-2-chloro-6-[2-(2-fluorophenyl)sulfonyl-2-azaspiro[3.3]heptan-6-yl]-7-methyl-7,8-dihydropyrimido[5,4-f][1,4]oxazepin-5-one, 10 mg of [(2-dicyclohexylphosphino-2',6'-bis(N,N-dimethylamino)-1,1'-biphenyl)-2-(2'-amino-1,1'-biphenyl)]palladium methane sulfonate, and 2 mL of tetrahydrofuran, 0.80 mL of a 0.5 mol/L bromo(cyclopropyl)zinc tetrahydrofuran solution was added under a nitrogen atmosphere, and the mixture was stirred at 60°C for 1.5 hours. To the reaction mixture, 22 mg of [(2-dicyclohexylphosphino-2',6'-bis(N,N-dimethylamino)-1,1'-biphenyl)-2-(2'-amino-1,1'-biphenyl)]palladium methane sulfonate and 0.80 mL of a 0.5 mol/L bromo(cyclopropyl)zinc tetrahydrofuran solution were added, and the mixture was stirred at 60°C for 30 minutes. The reaction mixture was returned to room temperature, a saturated sodium bicarbonate aqueous solution was added, and extraction was performed twice with ethyl acetate. The organic layers were combined, washed with brine, and then dried over magnesium sulfate. The insoluble matter was filtered off, and the filtrate was concentrated under reduced pressure. The resulting residue was purified by silica gel column chromatography (solvent: hexane/ethyl acetate = 50/50 to 20/80), and then recrystallization was performed using hexane-ethyl acetate. A solid was collected by filtration and dried under reduced pressure to obtain 46 mg of the title compound (yield 50%) as a colorless powder.
MS(ESI)m/z:473[M+H]⁺

### Example 332

### Production of (3R)-4-[2-(2-fluorophenyl)sulfonyl-2-azaspiro[3.3]heptan-6-yl]-3-methyl-5-oxo-2,3-dihydropyrido[2,3-f][1,4]oxazepin-8-carbonitrile

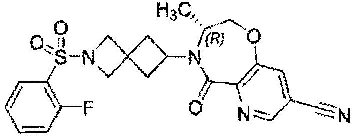

94 mg of (3R)-8-bromo-4-[2-(2-fluorophenyl)sulfonyl-2-azaspiro[3.3]heptan-6-yl]-3-methyl-2,3-dihydropyrido[2,3-f][1,4]oxazepin-5-one, 32 mg of zinc cyanide, and 21 mg of tetrakistriphenylphosphine palladium were suspended in 2 mL of dimethylformamide, and the mixture was stirred at 110°C for 11 hours. A saturated sodium bicarbonate aqueous solution was added to the reaction mixture, and extraction was performed with ethyl acetate. The organic layer was washed with water and brine, dried over magnesium sulfate, and then filtered. The filtrate was concentrated, the resulting residue was dissolved in 2 mL of dimethylformamide, 97 mg of zinc cyanide and 42 mg of tetrakistriphenylphosphine palladium were added, and the mixture was stirred at 120°C for 1.5 hours. A saturated sodium bicarbonate aqueous solution was added to the reaction mixture, and extraction was performed with ethyl acetate. The organic layer was washed with water and brine, dried over magnesium sulfate, and then filtered. The filtrate was concentrated, the resulting residue was dissolved in dimethyl sulfoxide, the resultant was purified by basic preparative HPLC (solvent: 10 mM ammonium carbonate aqueous solution/acetonitrile = 70/30 to 40/60) and then purified again by silica gel chromatography (solvent: ethyl acetate/methanol = 100/0 to 90/10), thereby obtaining 13 mg of the title compound (yield 15%) as a colorless powder.
MS(ESI)m/z:457[M+H]⁺

### Example 333

### Production of (3R)-4-[2-(2-fluorophenyl)sulfonyl-2-azaspiro[3.3]heptan-6-yl]-3-methyl-5-oxo-2,3-dihydropyrido[3,2-f][1,4]oxazepin-7-carbonitrile

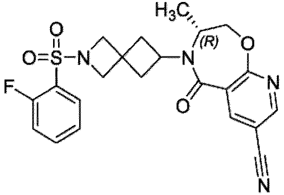

Into a 20 mL eggplant flask, 50.0 mg of (3R)-7-bromo-4-[2-(2-fluorophenyl)sulfonyl-2-azaspiro[3.3]heptan-6-yl]-3-methyl-2,3-dihydropyrido[3,2-f][1,4]oxazepin-5-one, 16 mg of a [1,1'-bis(diphenylphosphino)ferrocene]palladium(II) dichloride dichloromethane complex, and 5.3 mg of zinc were put, and 1 mL of dimethylformamide was added. 58 mg of zinc cyanide was added to the reaction mixture, and then the mixture was heated and stirred at 140°C for 20 hours. The reaction mixture was returned to room temperature, a saturated sodium bicarbonate aqueous solution and ethyl acetate were added, and liquid separation was performed. The organic layer was dried over sodium sulfate, filtered, and concentrated. The resulting residue was dissolved in dimethyl sulfoxide and purified by reverse-phase preparative HPLC (solvent: 10 mM ammonium carbonate aqueous solution/acetonitrile = 70/30 to 40/60) to obtain 8.3 mg of the title compound (yield 19%) as a pale yellow powder.
MS(ESI)m/z:457[M+H]⁺

### Example 334:

Corresponding raw material compounds were treated in the same manner as that of Example 333 to obtain compounds shown in Table 25.

**[Table 25]**

| Example | Structural formula | Physical property value and the like |
|---|---|---|
| 334 | 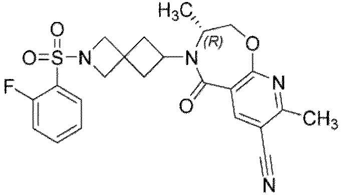 | MS(ESI) m/z;471 [M+H]+ |

### Example 335

### Production of (3R)-8-acetyl-4-[2-(2-fluorophenyl)sulfonyl-2-azaspiro[3.3]heptan-6-yl]-3-methyl-2,3-dihydropyrido[2,3-f][1,4]oxazepin-5-one

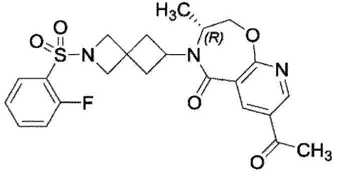

120 mg of (3R)-8-bromo-4-[2-(2-fluorophenyl)sulfonyl-2-azaspiro[3.3]heptan-6-yl]-3-methyl-2,3-dihydropyrido[2,3-f][1,4]oxazepin-5-one, 0.119 mL of tributyl(1-ethoxyvinyl)tin, and 27 mg of tetrakistriphenylphosphine palladium were dissolved in 3 mL of 1,4-dioxane, and the mixture was stirred at 100°C for 9 hours. After cooling at room temperature, 0.5 mL of a 1 mol/L hydrochloric acid aqueous solution was added to the reaction solution, and the mixture was stirred at room temperature for 1 hour. Water was added to the reaction mixture, and extraction was performed with ethyl acetate. The organic layer was washed with brine, filtration was performed after drying with magnesium sulfate, and the filtrate was concentrated under reduced pressure. The resulting residue was dissolved in dimethyl sulfoxide and purified by reverse-phase HPLC (solvent: 10 mM ammonium carbonate aqueous solution/acetonitrile = 70/30 to 40/60) to obtain 26 mg of the title compound (yield 23%) as a yellow powder.
MS(ESI)m/z:474[M+H]⁺

### Example 336:

A corresponding raw material compound was treated in the same manner as that of Example 335 to obtain a compound shown in Table 26.

**[Table 26]**

| Example | Structural formula | Physical property value and the like |
|---|---|---|
| 336 | 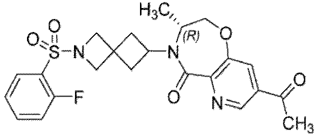 | MS(ESI) m/z;474 [M+H]+ |

### Example 337

### Production of (3R)-4-[2-(2-fluorophenyl)sulfonyl-2-azaspiro[3.3]heptan-6-yl]-3-methyl-8-(trifluoromethyl)-2,3-dihydropyrido[2,3-f] [1,4]oxazepin-5-one

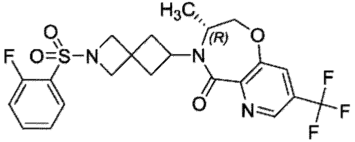

(1) 150 mg of (3R)-8-bromo-4-[2-(2-fluorophenyl)sulfonyl-2-azaspiro[3.3]heptan-6-yl]-3-methyl-2,3-dihydropyrido[2,3-f][1,4]oxazepin-5-one, 88 mg of sodium iodide, and 84 mg of trans-N,N'-dimethylcyclohexane-1,2-diamine were suspended in 3 mL of 1,4-dioxane, and 5.6 mg of copper iodide was added. The reaction mixture was stirred at 110°C for 1.5 hours. The reaction mixture was returned to room temperature and diluted with ethyl acetate, the insoluble matter was filtered off, and washing was performed by adding a 1 mol/L hydrochloric acid aqueous solution to the filtrate. The organic layer was separated and dried over sodium sulfate, filtration was performed, and then the filtrate was concentrated under reduced pressure. The resulting residue was purified by silica gel column chromatography (solvent: hexane/ethyl acetate = 70/30 to 20/80) to obtain 42 mg of (3R)-4-[2-(2-fluorophenyl)sulfonyl-2-azaspiro[3.3]heptan-6-yl]-8-iodo-3-methyl-2,3-dihydropyrido[2,3-f][1,4]oxazepin-5-one (yield 26%) as a colorless powder.
   MS(ESI)m/z:558[M+H]⁺
(2) 40 mg of the (3R)-4-[2-(2-fluorophenyl)sulfonyl-2-azaspiro[3.3]heptan-6-yl]-8-iodo-3-methyl-2,3-dihydropyrido[2,3-f] [1,4]oxazepin- 5-one obtained in (1) above was dissolved in 2 mL of N,N-dimethylformamide, 69 mg of methyl 2,2-difluoro-2-(fluoro sulfonyl) acetate and 41 mg of copper iodide were added, and the mixture was stirred at 110°C for 1 hour. To the reaction solution, 138 mg of methyl 2,2-difluoro-2-(fluorosulfonyl)acetate and 82 mg of copper iodide were added, and the mixture was stirred at 110°C for 3 hours. Further, to the reaction solution, 138 mg of methyl 2,2-difluoro-2-(fluorosulfonyl)acetate and 82 mg of copper iodide were added, and the mixture was stirred at 110°C for 3 hours. The reaction mixture was returned to room temperature, water was added, and extraction was performed with ethyl acetate. The organic layer was washed with water and brine, filtration was performed after drying with magnesium sulfate, and the filtrate was concentrated under reduced pressure. The resulting residue was purified by silica gel column chromatography (solvent: hexane/ethyl acetate = 70/30 to 25/75) to obtain 17 mg of the title compound (yield 47%) as a colorless powder.
   MS(ESI)m/z:500[M+H]⁺

### Example 338

### Production of (3R)-4-[2-(2-fluorophenyl)sulfonyl-2-azaspiro[3.3]heptan-6-yl]-3-methyl-2,3-dihydro-1H-pyrido[3,2-e] [1,4]diazepin-5-one

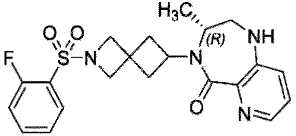

280 mg of tert-butyl (3R)-4-[2-(2-fluorophenyl)sulfonyl-2-azaspiro[3.3]heptan-6-yl]-3-methyl-5-oxo-2,3-dihydropyrido[3,2-e][1,4]diazepine-1-carboxylate was dissolved in 3 mL of dichloromethane, 107 µL of trimethylsilyl trifluoromethanesulfonate was added, and the mixture was stirred at room temperature for 6 hours. The reaction mixture was concentrated under reduced pressure. The resulting residue was dissolved in methanol, a saturated sodium bicarbonate aqueous solution was added, and the mixture was stirred for 5 minutes. Chloroform was added to the reaction mixture, the mixture was stirred, the organic layer was separated with Phase-separator^{®}, and then the organic layer was concentrated under reduced pressure. The resulting residue was purified by NH silica gel column chromatography (solvent: ethyl acetate/methanol = 97/3 to 90/10) to obtain 134 mg of the title compound (yield 59%) as a colorless powder.
MS(ESI)m/z:431[M+H]⁺

### Example 339

### Production of 6-[2-(2-fluorophenyl)sulfonyl-2-azaspiro[3.3]heptan-6-yl]-7-methyl-1,7,8,9-tetrahydropyrido[3,2-c]azepin-2,5-dione

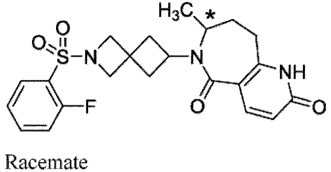

and Example 340

### Production of 6-[2-(2-fluorophenyl)sulfonyl-2-azaspiro[3.3]heptan-6-yl]-9-hydroxy-7-methyl- 8,9-dihydro-7H-pyrido [3,2-c] azepin-5-one

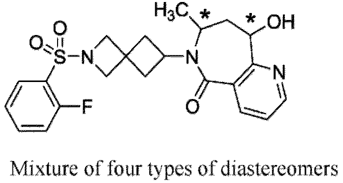

245 mg of 6-[2-(2-fluorophenyl)sulfonyl-2-azaspiro[3.3]heptan-6-yl]-7-methyl-1-oxide-8,9-dihydro-7H-pyrido[3,2-c]azepin-1-ium-5-one was dissolved in 3 mL of acetic anhydride, and the mixture was stirred at 80°C for 1 hour. The reaction mixture was concentrated, the resulting residue was dissolved in 3 mL of methanol, 91 mg of potassium carbonate was added, and the mixture was stirred at room temperature for 1 hour. Water was added to the reaction mixture, and extraction was performed with ethyl acetate. The aqueous layer was extracted with chloroform. The organic layer was dried over sodium sulfate, filtration was performed, and then the filtrate was concentrated under reduced pressure. The resulting residue was purified by NH silica gel column chromatography (solvent: hexane/ethyl acetate = 80/20 to 20/80, and then chloroform/methanol = 90/10) to obtain 90 mg of 6-[2-(2-fluorophenyl)sulfonyl-2-azaspiro [3.3]heptan-6-yl] -7-methyl-1,7,8,9-tetrahydropyrido [3,2-c] azepin-2,5-dione (two stage yield 37%) as a colorless powder.
MS(ESI)m/z:446[M+H]⁺

At the same time, 135 mg of 6-[2-(2-fluorophenyl)sulfonyl-2-azaspiro[3.3]heptan-6-yl]-9-hydroxy-7-methyl-8,9-dihydro-7H-pyrido[3,2-c]azepin-5-one (diastereomeric mixing ratio of 65:35, two stage yield 55%) was obtained as a colorless powder.
MS(ESI)m/z:446[M+H]⁺

### Example 341

### Production of (7R)-3-(difluoromethyl)-8-[2-(2-fluorophenyl)sulfonyl-2-azaspiro[3.3]heptan-6-yl]-7-methyl-6,7-dihydropyrazino[2,3-f] [1,4]oxazepin-9-one

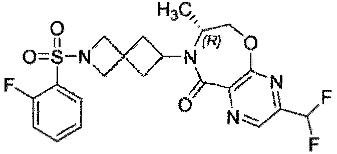

In a 50 mL eggplant flask, 150 mg of (7R)-8-[2-(2-fluorophenyl)sulfonyl-2-azaspiro[3.3]heptan-6-yl]-6,7-dihydropyrazino[2,3-f][1,4]oxazepin-9-one was weighed, and 2.0 mL of dimethyl sulfoxide was added and dissolved. Next, 0.80 mL of water, 250 mg of zinc bis(difluoromethanesulfinate), and 35 µL of trifluoroacetic acid were added. Next, 250 µL of a 70% tert-butyl hydroperoxide aqueous solution was added, and the mixture was stirred at room temperature for 2 hours. Thereafter, the mixture was heated and stirred at 50°C for 145 minutes. The reaction solution was cooled to room temperature, 226 mg of zinc bis(difluoromethanesulfinate) and 200 µL of a 70% tert-butyl hydroperoxide aqueous solution were additionally added, and then the mixture was heated and stirred at 50°C for 4 hours. To the reaction solution, a sodium bisulfite aqueous solution, saturated ammonium chloride, a saturated sodium bicarbonate aqueous solution, and brine were added, and extraction was performed with ethyl acetate. The organic layer was washed with brine and then dried over magnesium sulfate. The insoluble matter was filtered, and then the filtrate was concentrated under reduced pressure. 175 mg of the resulting pale yellow oily product was purified by reverse-phase HPLC (solvent: 10 mM ammonium carbonate aqueous solution/acetonitrile = 70/30 to 40/60) to obtain 37.3 mg of a mixture of the title compound and (7R)-2-(difluoromethyl)-8-[2-(2-fluorophenyl)sulfonyl-2-azaspiro[3.3]heptan-6-yl]-7-methyl-6,7-dihydropyrazino[2,3 -f][1,4]oxazepin-9-one (content ratio 92:8) (yield 21%) as a pale yellow amorphous solid.
MS(ESI)m/z:483[M+H]⁺

### Example 342

### Production of (7R)-3-cyclopropyl-1-(difluoromethyl)-8-[2-(2-fluorophenyl)sulfonyl-2-azaspiro[3.3]heptan-6-yl]-7-methyl-6,7-dihydro-5H-imidazo[1,5-a][1,4]diazepin-9-one

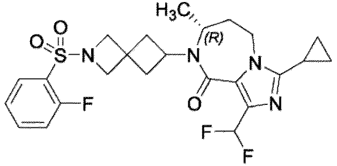

125 mg of (7R)-3-cyclohexyl-8-[2-(2-fluorophenyl)sulfonyl-2-azaspiro[3.3]heptan-6-yl]-7-methyl-6,7-dihydro-SH-imidazo[1,5-a] [1,4]diazepin-9-one and 201 mg of zinc difluoromethanesulfinate were suspended in 2 mL of dimethyl sulfoxide, 0.19 mL of tert-butyl hydroperoxide was added, and the mixture was stirred at 50°C for 3 hours. To the reaction solution, 201 mg of zinc difluoromethanesulfinate and 0.19 mL of tert-butyl hydroperoxide were added, and the mixture was stirred at 50°C for 3 hours. The temperature was raised to 70°C, and the mixture was stirred for 7 hours. A saturated sodium bicarbonate aqueous solution was added to the reaction mixture, and extraction was performed three times with ethyl acetate. The organic layer was washed with brine, filtration was performed after drying with sodium sulfate, and the filtrate was concentrated under reduced pressure. The resulting residue was purified by silica gel column chromatography (solvent: chloroform/methanol = 100/0 to 85/15) and further purified by reverse-phase HPLC (solvent: 10 mM ammonium carbonate aqueous solution/acetonitrile = 60/40 to 30/70), thereby obtaining 7 mg of the title compound (yield 5%) as a colorless powder.
MS(ESI)m/z:509[M+H]⁺

### Example 343

### Production of 2-[2-(2-fluorophenyl)sulfonyl-2-azaspiro[3.3]heptan-6-yl]-3-methyl-6-(trifluoromethyl)-3,4-dihydro-2,7-naphthyridin-1-one

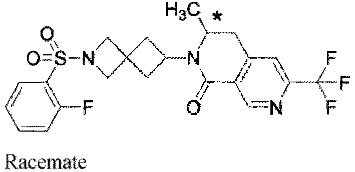

179 mg of 2-(2-fluorophenyl)sulfonyl-2-azaspiro[3.3]heptan-6-amine was dissolved in 3 mL of ethanol, 120 mg of ethyl 4-[(E)-prop-1-enyl]-6-(trifluoromethyl)pyridine-3-carboxylate was added, and the mixture was stirred at 50°C for 4 hours. The temperature was raised to 90°C, and the mixture was stirred for 1 hour. After cooling to room temperature, the reaction mixture was concentrated under reduced pressure, the resulting residue was dissolved in 2 mL of N-methylpyrrolidone, and the mixture was stirred at 130°C for 3.5 hours. After cooling to room temperature, an ammonium chloride aqueous solution was added to the reaction mixture, and extraction was performed with ethyl acetate. The organic layer was dried over magnesium sulfate, filtration was performed, and then the filtrate was concentrated under reduced pressure. The resulting residue was purified by silica gel column chromatography (solvent: hexane/ethyl acetate = 67/33 to 33/67) to obtain 69.7 mg of the title compound (yield 31%) as a light yellow powder.
MS(ESI)m/z:484[M+H]⁺

### Example 344

Production of 7-chloro-3-[2-(2-fluorophenyl)sulfonyl-2-azaspiro[3.3]heptan-6-yl]-2,2-dimethyl-pyrido[3,4-e][1,3]oxazin-4-one

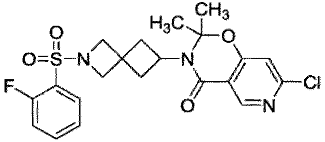

In a 20 mL eggplant flask, 121 mg of (3R)-8-chloro-4-[2-(2-fluorophenyl)sulfonyl-2-azaspiro[3.3]heptan-6-yl]-3-methyl-2,3-dihydropyrido[3,4-f][1,4]oxazepin-5-one was weighed, and 2.0 mL of N,N-dimethylformamide and 100 µL of ethanol were added. 21.8 mg of sodium hydride (60%) was added under ice-cooling, and the mixture was stirred under ice-cooling for 50 minutes. A saturated ammonium chloride aqueous solution and brine were added to the reaction solution, and extraction was performed with ethyl acetate. The organic layer was washed with brine and then dried over magnesium sulfate. The insoluble matter was filtered, and then the solution was concentrated under reduced pressure. 177 mg of the resulting colorless oily product was purified by silica gel column chromatography (solvent: hexane/ethyl acetate = 65/35 to 35/65) to obtain 11.6 mg of the title compound (yield 10%) as a colorless powder.
MS(ESI)m/z:466/468[M+H]⁺

### Example 345

### Production of (3R)-2-[2-(2-fluorophenyl)sulfonyl-2-azaspiro[3.3]heptan-6-yl]-3-methyl-6-(trifluoromethyl)-3,4-dihydropyrrolo[1,2-a]pyrazin-1-one

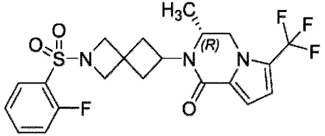

(1) 20 mg of ethyl 1-[(2R)-2-(tert-butoxycarbonylamino)propyl]-5-(trifluoromethyl)pyrrole-2-carboxylate was dissolved in 1 mL of dichloromethane, 12.2 mg of trimethylsilyl trifluoromethanesulfonate was added, and the mixture was stirred at room temperature for 1 hour. The reaction mixture was concentrated under reduced pressure to obtain a crude product of ethyl 1-[(2R)-2-aminopropyl]-5-(trifluoromethyl)pyrrole-2-carboxylate trifluoromethanesulfonate.
(2) The crude product of ethyl 1-[(2R)-2-aminopropyl]-5-(trifluoromethyl)pyrrole-2-carboxylate trifluoromethanesulfonate obtained in (1) above and 17.7 mg of 2-(2-fluorophenyl)sulfonyl-2-azaspiro[3.3]heptan-6-one were dissolved in 1 mL of dichloromethane, and 9.2 µL of triethylamine was added. 17.5 mg of sodium triacetoxyborohydride and 3.8 µL of acetic acid were added thereto, and the mixture was stirred at room temperature for 3 hours. A saturated sodium bicarbonate aqueous solution was added to the reaction mixture, and extraction was performed with chloroform. The organic layer was washed with water and brine, filtration was performed after drying with magnesium sulfate, and the filtrate was concentrated under reduced pressure. The resulting residue was dissolved in 1 mL of ethanol, and the mixture was stirred at 100°C for 2 hours. The reaction mixture was returned to room temperature and diluted with dimethyl sulfoxide, and the reactant was purified by reverse-phase HPLC (solvent: 10 mM ammonium carbonate aqueous solution/acetonitrile = 60/40 = 30/70), thereby obtaining 13 mg of the title compound (yield 50%) as a colorless powder.
   MS(ESI)m/z:472[M+H]⁺

### Example 346

### Production of (3R)-4-[2-(2-fluorophenyl)sulfonyl-2-azaspiro[3.3]heptan-6-yl]-3-methyl-8-(trifluoromethyl)-2,3-dihydro-1H-pyrido[2,3-e][1,4]diazepin-5-one

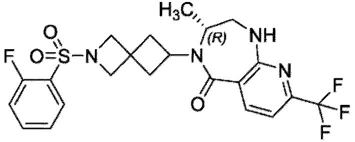

Into a 10 mL eggplant flask, 82.1 mg of t-butyl (3R)-4-[2-(2-fluorophenyl)sulfonyl-2-azaspiro[3.3]heptan-6-yl]-3-methyl-5-oxo-8-(trifluoromethyl)-2,3-dihydropyrido[2,3-e][1,4]diazepine-1-carboxylate was put and dissolved in 1 mL of dichloromethane. 0.030 mL of trimethylsilyl trifluoromethanesulfonic acid was added at room temperature, and the mixture was stirred for 1 hour. A saturated sodium bicarbonate aqueous solution was added to the reaction mixture, and extraction was performed with chloroform. The organic layer was dried over sodium sulfate, filtered, and concentrated. The resulting residue was purified by silica gel chromatography (solvent: hexane/ethyl acetate = 90/10 to 30/70) to obtain 51.0 mg of the title compound (yield 76%) as a colorless powder.
MS(ESI)m/z:499[M+H]⁺

### Example 347

A corresponding raw material compound was treated in the same manner as that of Example 170 to obtain a compound shown in Table 27.

**[Table 27]**

| Example | Structural formula | Physical property value and the like |
|---|---|---|
| 347 | 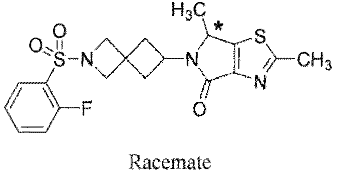 | MS(ESI) m/z;422 [M+H]+ |

### Examples 348 to 365:

The racemic mixture or the diastereomeric mixture produced in each of Examples was resolved by chiral high performance liquid chromatography (chiral HPLC) or chiral supercritical fluid chromatography (chiral SFC) to obtain compounds shown in Table 28.

**[Table 28-1]**

| Example | Physical property value and the like | Structural formula |
|---|---|---|
| | | Analysis conditions and the like |
| 348 | MS(ESI) m/z;432 [ M+H]+ | 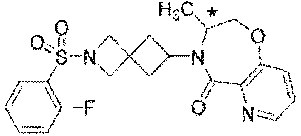 Single enantiomer |
| | | Column: DAICEL CHIRALPAK IG-3 (3 µm, 4.6 × 150 mm) |
| | | Transfer phase: Methyl tert-butyl ether/methanol/diethylamine (70/30/0.1) |
| | | Flow rate: 0.5 mL/min |
| | | Temperature: 25°C |
| | | Analysis channel: UV 291 nm |
| | | Retention time (min): 9.194 |
| 349 | MS(ESI) m/z;432 [ M+H]+ | 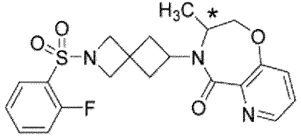 Single enantiomer opposite to that of Example 348 |
| | | Column: DAICEL CHIRALPAK IG-3 (3 µm, 4.6 × 150 mm) |
| | | Transfer phase: Methyl tert-butyl ether/methanol/diethylamine (70/30/0.1) |
| | | Flow rate: 0.5 mL/min |
| | | Temperature: 25°C |
| | | Analysis channel: UV 291 nm |
| | | Retention time (min): 12.863 |

**[Table 28-2]**

| | | |
|---|---|---|
| 350 | MS(ESI) m/z;498 [ M+H]+ | 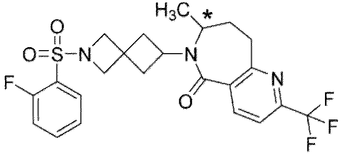 Single enantiomer |
| | | Column: CHIRALPAK IF-3 (4.6 ^{×} 150 mm) |
| | | Transfer phase: Methyl tert-butyl ether/methanol/diethylamine (95/5/0.1) |
| | | Flow rate: 0.5 mL/min |
| | | Temperature: 25°C |
| | | Analysis channel: PDA 267.0 nm |
| | | Retention time (min): 9.788 |
| 351 | MS(ESI) m/z;498 [ M+H]+ | 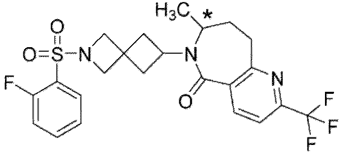 Single enantiomer opposite to that of Example 350 |
| | | Column: CHIRALPAK IF-3 (4.6 × 150 mm) |
| | | Transfer phase: Methyl tert-butyl ether/methanol/diethylamine (95/5/0.1) |
| | | Flow rate: 0.5 mL/min |
| | | Temperature: 25°C |
| | | Analysis channel: PDA 267.0 nm |
| | | Retention time (min): 12.360 |

**[Table 28-3]**

| | | |
|---|---|---|
| 352 | MS(ESI) m/z;504 [ M+H]+ | 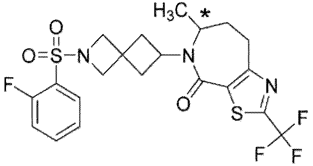 Single enantiomer |
| | | Column: CHIRALPAK IG-3 (4.6 ^{×} 150 mm) |
| | | Transfer phase: Methanol/acetonitrile/diethylamine (90/10/0.1) |
| | | Flow rate: 0.5 mL/min |
| | | Temperature: 25°C |
| | | Analysis channel: PDA 267.0 nm |
| | | Retention time (min): 9.984 |
| 353 | MS(ESI) m/z;504 [ M+H]+ | 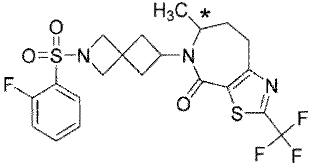 Single enantiomer opposite to that of Example 352 |
| | | Column: CHIRALPAK IG-3 (4.6 ^{×} 150 mm) |
| | | Transfer phase: Methanol/acetonitrile/diethylamine (90/10/0.1) |
| | | Flow rate: 0.5 mL/min |
| | | Temperature: 25°C |
| | | Analysis channel: PDA 267.0 nm |
| | | Retention time (min): 13.213 |

**[Table 28-4]**

| | | |
|---|---|---|
| 354 | MS(ESI) m/z;476 [ M+H]+ | 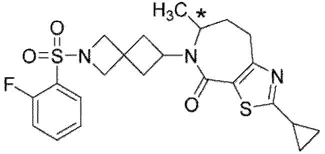 Single enantiomer |
| | | Column: CHIRALPAK IE-3 (4.6 ^{×} 150 mm) |
| | | Transfer phase: Ethanol/acetonitrile/diethylamine (93/7/0.1) |
| | | Flow rate: 0.5 mL/min |
| | | Temperature: 25°C |
| | | Analysis channel: PDA 274.0 nm |
| | | Retention time (min): 11.335 |
| 355 | MS(ESI) m/z;476 [ M+H]+ | 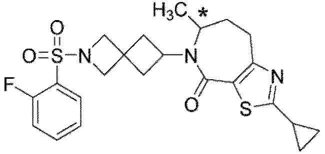 Single enantiomer opposite to that of Example 354 |
| | | Column: CHIRALPAK TE-3 (4.6 ^{×} 150 mm) |
| | | Transfer phase: Ethanol/acetonitrile/diethylamine (93/7/0.1) |
| | | Flow rate: 0.5 mL/min |
| | | Temperature: 25°C |
| | | Analysis channel: PDA 274.0 nm |
| | | Retention time (min): 13.297 |

**[Table 28-5]**

| | | |
|---|---|---|
| 356 | MS(ESI) m/z;484 [ M+H]+ | 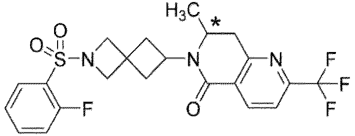 Single enantiomer |
| | | Column: CHIRALPAK IG-3 (4.6 ^{×} 150 mm) |
| | | Transfer phase: Methanol/acetonitrile/diethylamine (90/10/0.1) |
| | | Flow rate: 0.5 mL/min |
| | | Temperature: 25°C |
| | | Analysis channel: PDA 275.0 nm |
| | | Retention time (min): 12.122 |
| 357 | MS(ESI) m/z;484 [ M+H]+ | 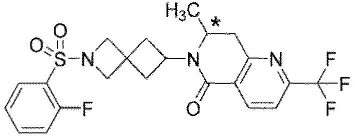 Single enantiomer opposite to that of Example 351 |
| | | Column: CHIRALPAK IG-3 (4.6 ^{×} 150 mm) |
| | | Transfer phase: Methanol/acetonitrile/diethylamine (90/10/0.1) |
| | | Flow rate: 0.5 mL/min |
| | | Temperature: 25°C |
| | | Analysis channel: PDA 275.0 nm |
| | | Retention time (min): 8.444 |

**[Table 28-6]**

| | | |
|---|---|---|
| 358 | MS(ESI) m/z;401 [ M+H]+ | 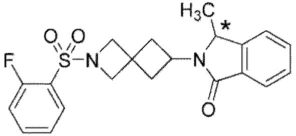 Single enantiomer |
| | | Column: CHIRALPAK IA-3 (3 µm, 4.6 ^{×} 150 mm) |
| | | Transfer phase: Hexane/ethanol (40/60) |
| | | Flow rate: 0.5 mL/min |
| | | Temperature: 25°C |
| | | Analysis channel: 200 to 400 nm |
| | | Retention time (min): 11.844 |
| 359 | MS(ESI) m/z;401 [ M+H]+ | 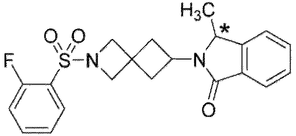 Single enantiomer opposite to that of Example 358 |
| | | Column: CHIRALPAK IA-3 (3 µm, 4.6 ^{×} 150 mm) |
| | | Transfer phase: Hexane/ethanol (40/60) |
| | | Flow rate: 0.5 mL/min |
| | | Temperature: 25°C |
| | | Analysis channel: 200 to 400 nm |
| | | Retention time (min): 13.789 |

**[Table 28-7]**

| | | |
|---|---|---|
| 360 | MS(ESI) m/z;402 [ M+H]+ | 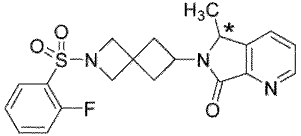 Single enantiomer |
| | | Column: CHIRALPAK IC-3 (3 µm, 4.6 ^{×} 150 mm) |
| | | Transfer phase: Methyl tert-butyl ether/methanol/diethylamine (50/50/0.1) |
| | | Flow rate: 0.5 mL/min |
| | | Temperature: 25°C |
| | | Analysis channel: 200 to 400 nm |
| | | Retention time (min): 12.161 |
| 361 | MS(ESI) m/z;402 [ M+H]+ | 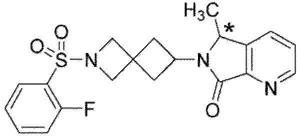 Single enantiomer opposite to that of Example 360 |
| | | Column: CHIRALPAK IC-3 (3 µm, 4.6 ^{×} 150 mm) |
| | | Transfer phase: Methyl tert-butyl ether/methanol/diethylamine (50/50/0.1) |
| | | Flow rate: 0.5 mL/min |
| | | Temperature: 25°C |
| | | Analysis channel: 200 to 400 nm |
| | | Retention time (min): 13.783 |

**[Table 28-8]**

| | | |
|---|---|---|
| 362 | MS(ESI) m/z;420 [ M+H]+ | 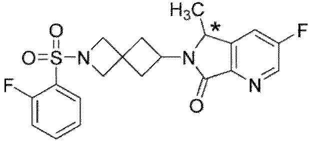 Single enantiomer |
| | | Column: CHIRALPAK IC-3 (3 µm, 4.6 ^{×} 150 mm) |
| | | Transfer phase: Methyl tert-butyl ether/methanol/diethylamine (60/40/0.1) |
| | | Flow rate: 0.5 mL/min |
| | | Temperature: 25°C |
| | | Analysis channel: 200 to 400 nm |
| | | Retention time (min): 12.326 |
| 363 | MS(ESI) m/z;420 [ M+H]+ | 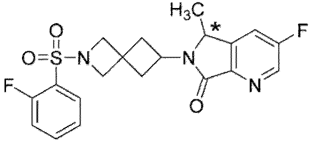 Single enantiomer opposite to that of Example 362 |
| | | Column: CHIRALPAK IC-3 (3 µm, 4.6 ^{×} 150 mm) |
| | | Transfer phase: Methyl tert-butyl ether/methanol/diethylamine (60/40/0.1) |
| | | Flow rate: 0.5 mL/min |
| | | Temperature: 25°C |
| | | Analysis channel: 200 to 400 nm |
| | | Retention time (min): 13.974 |

**[Table 28-9]**

| | | |
|---|---|---|
| 364 | MS(ESI) m/z;470 [ M+H]+ | 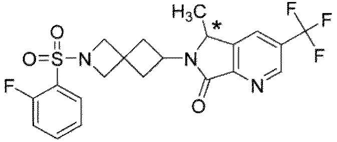 Single enantiomer |
| | | Column: CHIRALPAK IA-3 (4.6 ^{×} 150 mm) |
| | | Transfer phase: Methyl tert-butyl ether/ethanol/diethylamine (85/15/0.1) |
| | | Flow rate: 0.5 mL/min |
| | | Temperature: 25°C |
| | | Analysis channel: PDA 274.0 nm |
| | | Retention time (min): 9.513 |
| 365 | MS(ESI) m/z;470 [ M+H]+ | 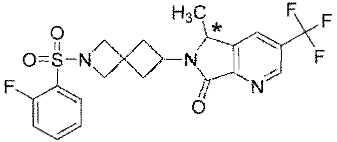 Single enantiomer opposite to that of Example 364 |
| | | Column: CHIRALPAK IA-3 (4.6 ^{×} 150 mm) |
| | | Transfer phase: Methyl tert-butyl ether/ethanol/diethylamine (85/15/0.1) |
| | | Flow rate: 0.5 mL/min |
| | | Temperature: 25°C |
| | | Analysis channel: PDA 274.0 nm |
| | | Retention time (min): 12.288 |

### Examples 366 to 368:

Corresponding raw material compounds were treated in the same manner as that of Example 1 to obtain compounds shown in Table 29.

**[Table 29]**

| Example | Structural formula | Physical property value and the like |
|---|---|---|
| 366 | 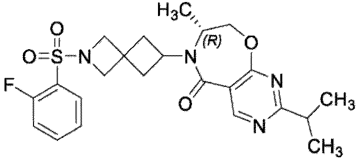 | MS(ESI) m/z;474 [M+H]+ |
| 367 | 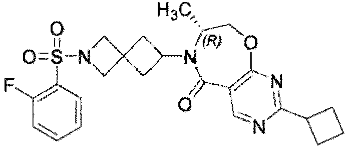 | MS(ESI) m/z;487 [M+H]+ |
| 368 | 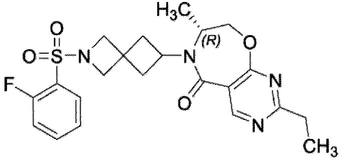 | MS(ESI) m/z;461 [M+H]+ |

### Example 369

### Production of (7R)-6-[2-(2-fluorophenyl)sulfonyl-2-azaspiro[3.3]heptan-6-yl]-7-methyl-2-propan-2-yloxy-7,8-dihydropyrimid[5,4-f][1,4]oxazepin-5-one

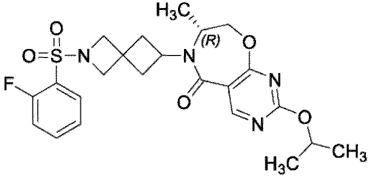

80 mg of (7R)-2-chloro-6-[2-(2-fluorophenyl)sulfonyl-2-azaspiro[3.3]heptan-6-yl]-7-methyl-7,8-dihydropyrimid[5,4-f][1,4]oxazepin-5-one was dissolved in 1.7 mL of N,N-dimethylformamide, 26.2 µL of 2-propanol and 13.7 mg of sodium hydride (60%) were added under ice-cooling, and the mixture was stirred at room temperature for 1 hour. A saturated ammonium chloride aqueous solution was added to the reaction mixture, and then extraction was performed with ethyl acetate. The organic layers were combined and washed with brine, filtration was performed after drying with sodium sulfate, and the filtrate was concentrated under reduced pressure. The resulting residue was purified by silica gel column chromatography (solvent: hexane/ethyl acetate = 60/40 to 20/80) to obtain 34 mg of (7R)-6-[2-(2-fluorophenyl)sulfonyl-2-azaspiro[3.3]heptan-6-yl]-7-methyl-2-propan-2-yloxy-7,8-dihydropyrimid[5,4-f][1,4]oxazepin-5-one (yield 40%) as a colorless powder.
MS(ESI)m/z:491[M+H]⁺

### Examples 370 to 379:

Corresponding raw material compounds were treated in the same manner as that of Example 369 to obtain compounds shown in Table 30.

**[Table 30]**

| Example | Structural formula | Physical property value and the like |
|---|---|---|
| 370 | 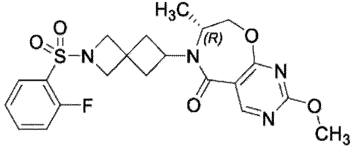 | MS(ESI) m/z;463 [M+H]+ |
| 371 | 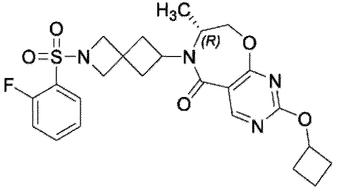 | MS(ESI) m/z;503 [M+H]+ |
| 372 | 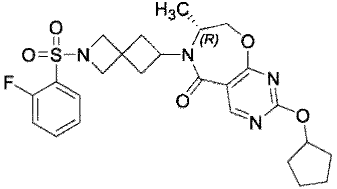 | MS(ESI) m/z;517 [M+H]+ |
| 373 | 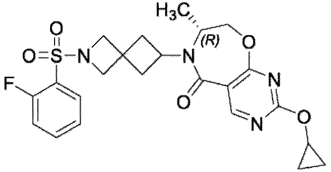 | MS(ESI) m/z;489 [M+H]+ |
| 374 | 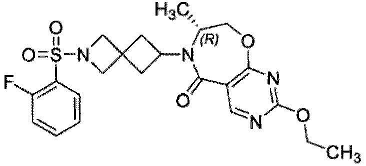 | MS(ESI) m/z;477 [M+H]+ |
| 375 | 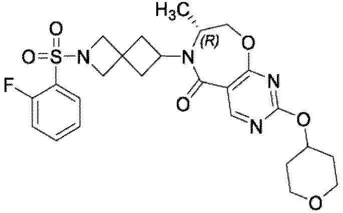 | MS(ESI) m/z;533 [M+H]+ |
| 376 | 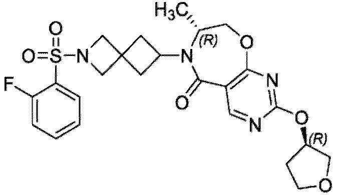 | MS(ESI) m/z;519 [M+H]+ |
| 377 | 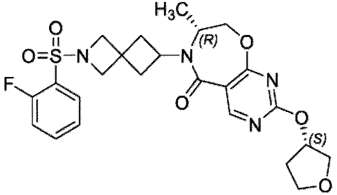 | MS(ESI) m/z;519 [M+H]+ |
| 378 | 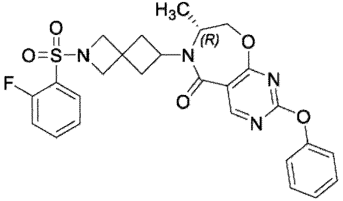 | MS(ESI) m/z>525 [M+H]+ |
| 379 | 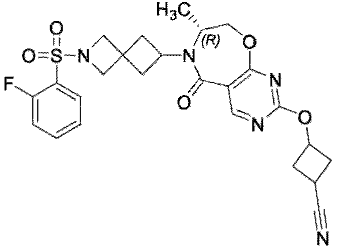 Relative configuration of cyclobutyl is cis and trans mixture | MS(ESI) m/z;528 [M+H]+ |

### Examples 380 to 412:

Corresponding raw material compounds were treated in the same manner as that of Example 168 to obtain compounds shown in Table 31.

**[Table 31]**

| Example | Structural formula | Physical property value and the like |
|---|---|---|
| 380 | 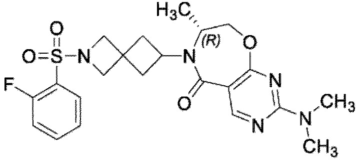 | MS(ESI) m/z;476 [M+H]+ |
| 381 | 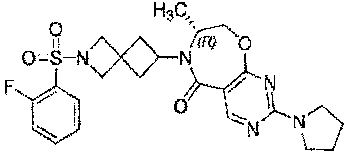 | MS(ESI) m/z;502 [M+H]+ |
| 382 | 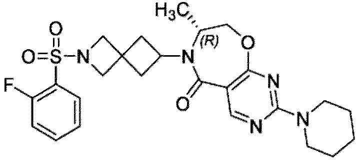 | MS(ESI) m/z;516 [M+H]+ |
| 383 | 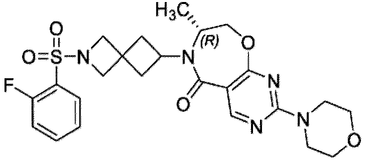 | MS(ESI) m/z;518 [M+H]+ |
| 384 | 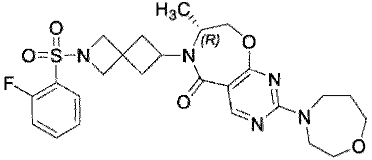 | MS(ESI) m/z;532 [M+H]+ |
| 385 | 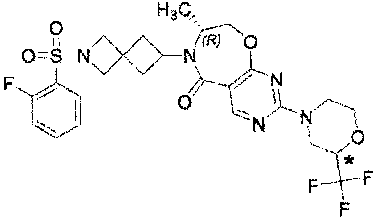 (3R), mixture of two types of diastereomers | MS(ESI) m/z;586 [M+H]+ |
| 386 | 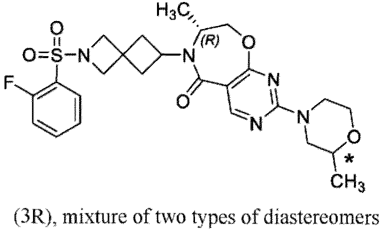 | MS(ESI) m/z;532 [M+H]+ |
| 387 | 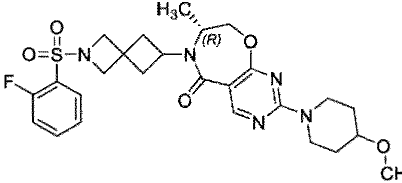 | MS(ESI) m/z;546 [M+H]+ |
| 388 | 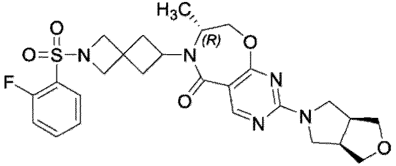 | MS(ESI) m/z;544 [M+H]+ |
| 389 | 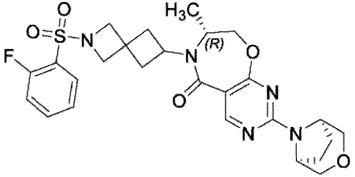 | MS(ESI) m/z;544 [M+H]+ |
| 390 | 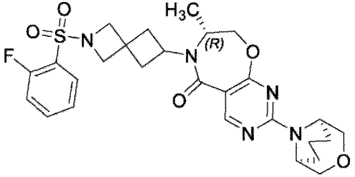 | MS(ESI) m/z; 558.5 [M+H] + |
| 391 | 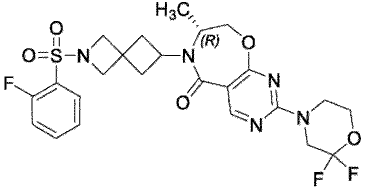 | MS(ESI) m/z;554 [M+H]+ |
| 392 | 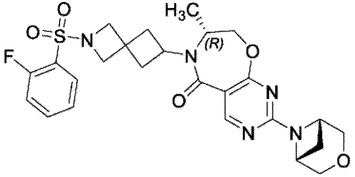 | MS(ESI) m/z;530 [M+H]+ |
| 393 | 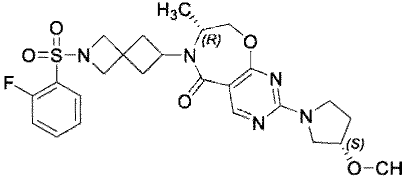 | MS(ESI) m/z;532 [M+H]+ |
| 394 | 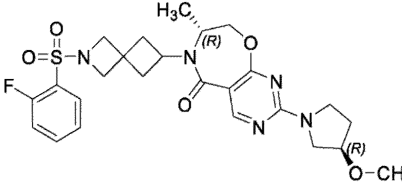 | MS(ESI) m/z;532 [M+H]+ |
| 395 | 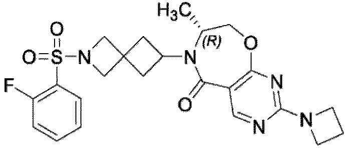 | MS(ESI) m/z;488 [M+H]+ |
| 396 | 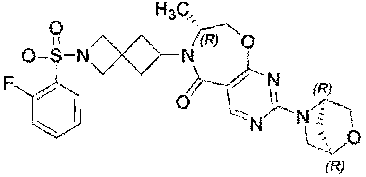 | MS(ESI) m/z;530 [M+H]+ |
| 397 | 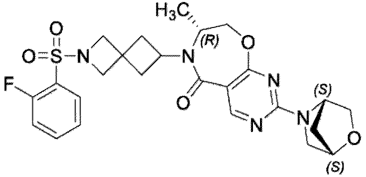 | MS(ESI) m/z;530 [M+H]+ |
| 398 | 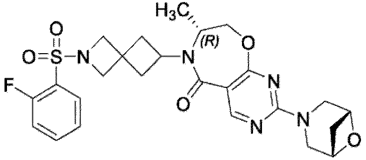 | MS(ESI) m/z;530 [M+H]+ |
| 399 | 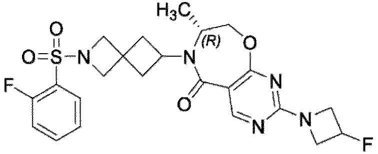 | MS(ESI) m/z;506 [M+H]+ |
| 400 | 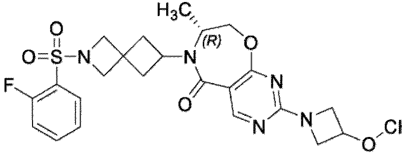 | MS(ESI) m/z;517 [M+H]+ |
| 401 | 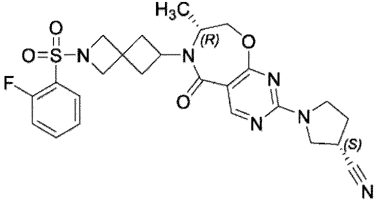 | MS(ESI) m/z;527 [M+H]+ |
| 402 | 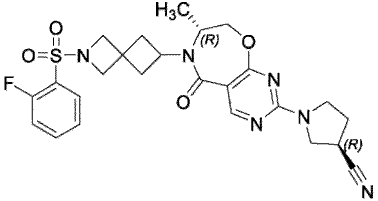 | MS(ESI) m/z;527 [M+H]+ |
| 403 | 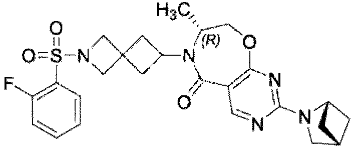 | MS(ESI) m/z;514 [M+H]+ |
| 404 | 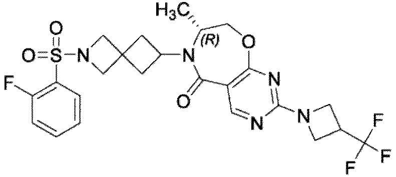 | MS(ESI) m/z;556 [M+H]+ |
| 405 | 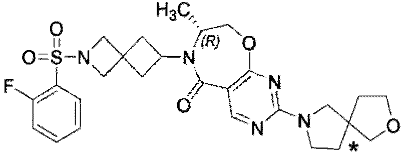 (3R), mixture of two types of diastereomers | MS(ESI) m/z;558 [M+H]+ |
| 406 | 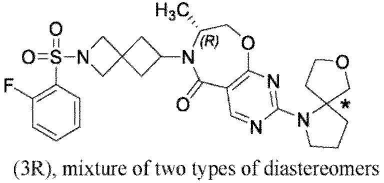 | MS(ESI) m/z;558 [M+H]+ |
| 407 | 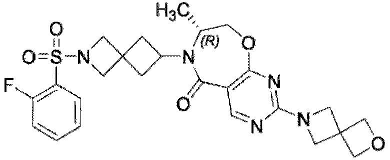 | MS(ESI) m/z;530 [M+H]+ |
| 408 | 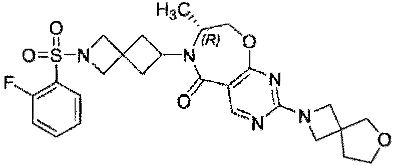 | MS(ESI) m/z;544 [M+H]+ |
| 409 | 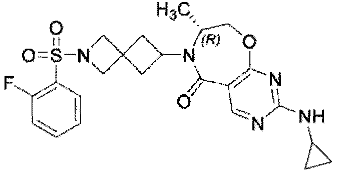 | MS(ESI) m/z;488 [M+H]+ |
| 410 | 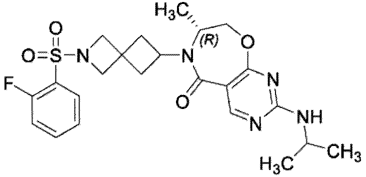 | MS(ESI) m/z;490 [M+H]+ |
| 411 | 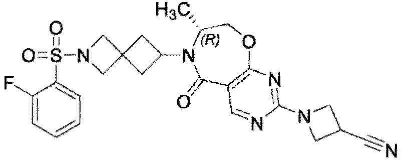 | MS(ESI) m/z;513 [M+H]+ |
| 412 | 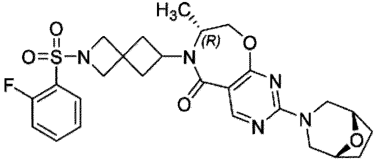 | MS(ESI) m/z;544 [M+H]+ |

### Example 413

A corresponding raw material compound was treated in the same manner as that of Example 70 to obtain a compound shown in Table 32.

**[Table 32]**

| | | |
|---|---|---|
| 413 | 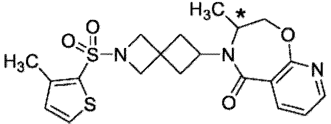 | MS(ESI) m/z;434 [M+H]+ |

### Example 414

### Production of 2-methoxy-N-[2-(3-methylphenyl)sulfonyl-2-azaspiro[3.3]heptan-6-yl]-N-propylpyridine-3-carboxamide

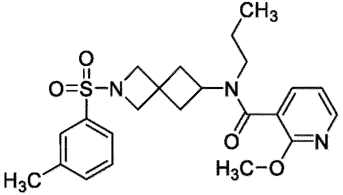

To a mixture of 50 mg of N-(2-azaspiro[3.3]heptan-6-yl)-2-methoxy-N-propylpyridine-3-carboxamide and 1.5 mL of methylene chloride, 0.0376 mL of 3-methylbenzenesulfonyl chloride and 0.072 mL of triethylamine was added and the mixture was stirred at room temperature for 2 hours. The reaction mixture was concentrated under reduced pressure, and then diluted in methanol, and the mixture was neutralized with 1M hydrochloric acid. Afterwards, the product was purified by reversed-phase HPLC (solvent: 10 mM aqueous ammonium carbonate solution/acetonitrile = 60/40 to 30/70) to obtain 51.4 mg of the title compound (yield 67.1%) as a colorless viscous material.
MS(ESI)m/z:444[M+H]⁺

### Examples 415 to 421

Corresponding raw material compounds were treated in the same manner as that of Example 414 to obtain compounds shown in Table 33.

**[Table 33]**

| Example | Structural formula | Physical property value and the like |
|---|---|---|
| 415 | 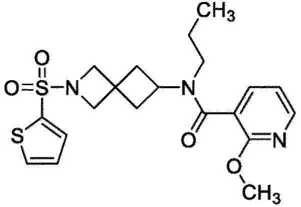 | MS(ESI) m/z; 436 [M+H]+ |
| 416 | 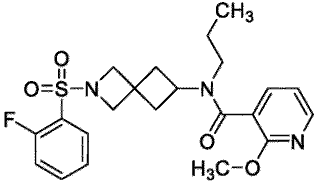 | MS(ESI) m/z; 448 [M+H]+ |
| 417 | 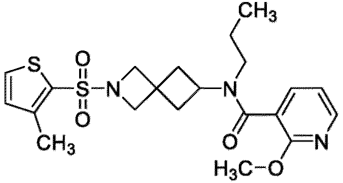 | MS(ESI) m/z; 450 [M+H]+ |
| 418 | 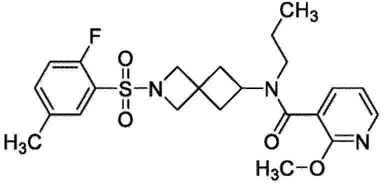 | MS(ESI) m/z; 462 [M+H]+ |
| 419 | 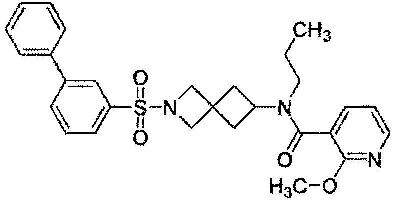 | MS(ESI) m/z; 506 [M+H]+ |
| 420 | 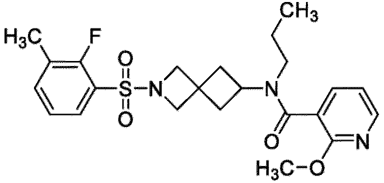 | MS(ESI) m/z; 462 [M+H]+ |
| 421 | 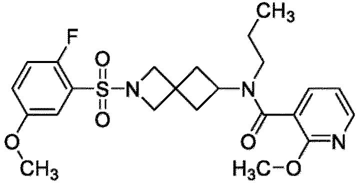 | MS(ESI) m/z; 478 [M+H]+ |

### Example 422

A corresponding raw material compound was treated in the same manner as that of Example 45 to obtain a compound shown in Table 34.

**[Table 34]**

| Example | Structural formula | Physical property value and the like |
|---|---|---|
| 422 | 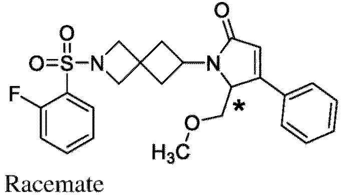 | MS(ESI) m/z; 457 [M+H]+ |

### Example 423

### Production of N-[2-(benzenesulfonyl)-2-azaspiro[3.3]heptan-6-yl]-2-methoxy-N-propylpyridine-3-carboxamide

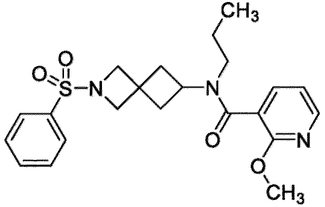

To a mixture of 74.8 mg of 2-(benzenesulfonyl)-N-propyl-2-azaspiro[3.3]heptan-6-amine and 55.0 mg of 2-methoxypyridine 3-carboxylic acid in 1.0 mL of N,N-dimethylformamide, 133 mg of HATU and 150 µL of diisopropylethylamine were added, and the mixture was stirred at room temperature for 1 hour and 45 minutes. Saturated aqueous sodium bicarbonate solution was added to the reaction mixture, the mixture was extracted with ethyl acetate, and the organic layer was concentrated. The resulting residue was purified by reversed-phase HPLC (solvent: 10 mM aqueous ammonium carbonate solution/ acetonitrile = 60/40 to 30/70) to obtain 94.6 mg (yield 87%) of the title compound as a colorless oil.
MS(ESI)m/z:430[M+H]⁺

### Examples 424 to 431

Corresponding raw material compounds were treated in the same manner as that of Example 423 to obtain compounds shown in Table 35.

**[Table 35]**

| Example | Structural formula | Physical property value and the like |
|---|---|---|
| 424 | 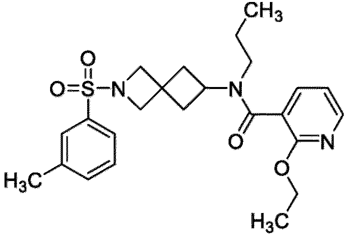 | MS(ESI) m/z; 458 [M+H]+ |
| 425 | 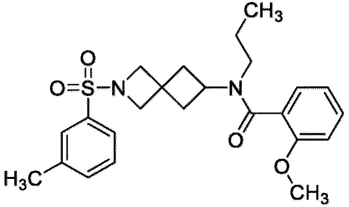 | MS(ESI) m/z; 443 [M+H]+ |
| 426 | 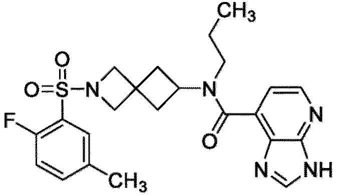 | MS(ESI) m/z; 472 [M+H]+ |
| 427 | 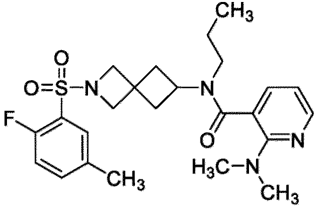 | MS(ESI) m/z; 475 [M+H]+ |
| 428 | 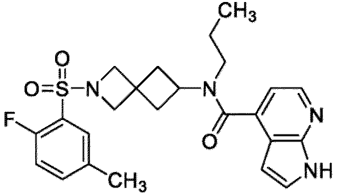 | MS(ESI) m/z; 471 [M+H]+ |
| 429 | 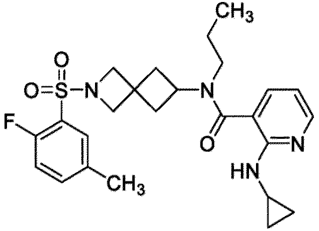 | MS(ESI) m/z; 487 [M+H]+ |
| 430 | 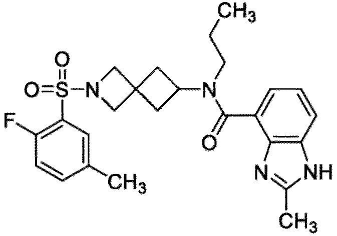 | MS(ESI) m/z; 485 [M+H]+ |
| 431 | 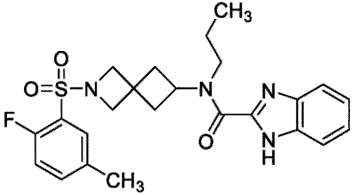 | MS(ESI) m/z; 471 [M+H]+ |

### Example 432.

### Production of 1-[2-(2-fluorophenyl)sulfonyl-2-azaspiro[3.3]heptan-6-yl]-7-methyl-1,3-diazepan-2-one

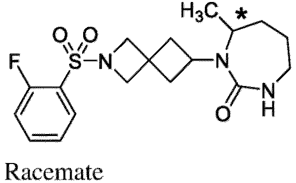

To a solution of 30 mg of 4-N-[2-(2-fluorophenyl)sulfonyl-2-azaspiro[3.3]heptan-6-yl]pentane-1,4-diamine in 0.42 mL of N,N-dimethylformamide, 0.044 mL of diisopropylethylamine and 15.1 mg of carbonyldiimidazole were added, and the mixture was stirred at room temperature for 1 hour. Then 9.6 mg of carbonyldiimidazole was added to the reaction mixture and the mixture was stirred at room temperature for 30 minutes. Water was added to the reaction mixture and the mixture was extracted with ethyl acetate. After washing the organic layer with water and brine, the organic layer was separated with Phase-separator^{®} and concentrated under reduced pressure. The resulting residue was purified by reversed-phase HPLC (solvent: 10 mM aqueous ammonium carbonate solution/acetonitrile = 80/20 to 50/50) to yield 5.3 mg of the title compound (yield 16%) as a colorless powder.

MS(ESI)m/z:382[M+H]⁺

### Example 433

### Production of 3-[2-(2-fluorophenyl)sulfonyl-2-azaspiro[3.3]heptan-6-yl]-4-methyl-1,3-oxazinan-2-one

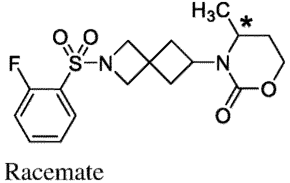

(1) To a mixture of 100 mg of 2-(2-fluorophenyl)sulfonyl-2-azaspiro[3.3]heptan-6-amine and 48.9 mg of 4-hydroxy-2-butanone in 1.85 mL of methylene chloride, 0.021 mL of acetic acid and 133.3 mg of sodium triacetoxyborohydride were added, and the mixture was stirred at room temperature for 1 hour. After adding saturated aqueous sodium bicarbonate solution to the reaction mixture and stirring, the organic layer was separated with Phase-separator^{®} and concentrated under reduced pressure to obtain a crude product of 3-[[2-(2-fluorophenyl)sulfonyl-2-azaspiro[3.3]heptan-6-yl]amino]butan-1-ol.
(2) To a solution of the crude product of 3-[[2-(2-fluorophenyl)sulfonyl-2-azaspiro[3.3]heptan-6-yl]amino]butan-1-ol obtained in the above (1) in 1.85 mL of methylene chloride, 0.154 mL of triethylamine and 120 mg of carbonyldiimidazole were added, and the mixture was stirred at room temperature for 1 hour. Then 9.0 mg of dimethylaminopyridine was added thereto and the mixture was stirred at room temperature all night. The reaction solution was concentrated under reduced pressure and the resulting residue was purified by silica gel column chromatography (solvent: ethyl acetate/methanol = 100/0 to 98/2) to obtain 115 mg of the title compound (two-step yield 78%) as a colorless oil.
   MS(ESI)m/z:369[M+H]⁺

### Example 434

### Production of 1-[2-(2-fluorophenyl)sulfonyl-2-azaspiro[3.3]heptan-6-yl]-6-methyl-1,3-diazinan-2-one

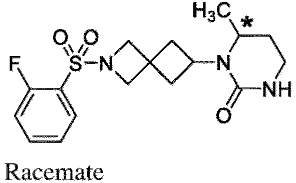

To a mixture of 250 mg of benzyl N-[3-[[2-(2-fluorophenyl)sulfonyl-2-azaspiro[3.3]heptan-6-yl]amino]butyl]carbamate, 133 mg of carbonyldiimidazole and 0.20 mL of triethylamine in 2.4 mL of tetrahydrofuran, 50 mg of 10% palladium carbon was added, and the mixture was stirred at room temperature for 1 hour and 30 minutes under a hydrogen atmosphere. After replacing the atomosphere in the reaction system with nitrogen, 133 mg of carbonyldiimidazole and 0.2 mL of triethylamine were added thereto, and the mixture was stirred at room temperature for 1 hour. Insoluble material was removed by filtration and the filtrate was concentrated under reduced pressure. To the resulting residue, 3.2 mL of methylene chloride and 0.067 mL of trimethylamine were added, and the mixture was stirred at room temperature for 1 hour, and then allowed to stand for 2 days. After concentrating the reaction mixture under reduced pressure, the resulting residue was purified by reversed-phase HPLC (solvent: 10 mM aqueous ammonium carbonate solution/acetonitrile = 80/20 to 50/50) to obtain 155 mg of the title compound (yield 87%) as a colorless powder.
MS(ESI)m/z:368[M+H]⁺

### Example 435

### Production of (5R)-1-[2-(2-fluorophenyl)sulfonyl-2-azaspiro[3.3]heptan-6-yl]-5-methylimidazolidin-2-one

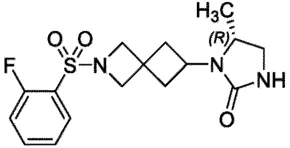

(1) To a mixture of 200mg of benzyl N-[(2R)-2-[[2-((2-fluorophenyl)sulfonyl-2-azaspiro[3.3]heptan-6-yl]amino]propyl]carbamate in tetrahydrofuran, 20 mg of 10% palladium carbon was added and the mixture was stirred for 1 hour at room temperature under a hydrogen atmosphere. Insoluble matter was removed by filtration and the filtrate was concentrated under reduced pressure to obtain a crude product of (2R)-N2-[2-(2-fluorophenyl)sulfonyl-2-azaspiro[3.3]heptan-6-yl]propane-1,2-diamine.
(2) To the crude product of (2R)-N2-[2-(2-fluorophenyl)sulfonyl-2-azaspiro[3.3]heptan-6-yl]propane-1,2-diamine obtained in the above (1), 4.3 mL of methylene chloride, 0.18 mL of triethylamine and 140 mg of carbonyldiimidazole were added, and the mixture was stirred at room temperature for 2 hours. To the reaction mixture was added 2.6 mg of dimethylaminopyridine and the mixture was stirred at room temperature all night. The reaction mixture was concentrated under reduced pressure and the resulting residue was purified by silica gel column chromatography (solvent: ethyl acetate/methanol = 100/0 to 95/5) to obtain 122 mg of the title compound (yield 76%) as a colorless viscous material.
   MS(ESI)m/z:354[M+H]⁺

### Example 436

### Production of (4R*,7S*)-1-[2-(2-fluorophenyl)sulfonyl-2-azaspiro[3.3]heptan-6-yl]-4,7-dimethylazepan-2-one

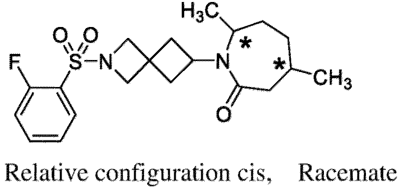

and Example 437.

### Production of (4R*,7R*)-1-[2-(2-fluorophenyl)sulfonyl-2-azaspiro[3.3]heptan-6-yl]-4,7-dimethylazepan-2-one

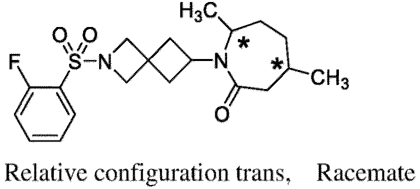

(1) To a mixture of 100 mg of 2-(2-fluorophenyl)sulfonyl-2-azaspiro[3.3]heptan-6-amine and 87.8 mg of 3-methyl-6-oxoheptanoic acid in 1.85 mL of methylene chloride, 0.021 mL of acetic acid and 133.3 mg of sodium triacetoxyborohydride were added, and the mixture was stirred at room temperature for 1 hour. Methanol was added to the reaction mixture, and the mixture was stirred and then concentrated under reduced pressure. The resulting residue was purified by reversed-phase HPLC (solvent: 10 mM aqueous ammonium carbonate solution/acetonitrile = 90/10 to 60/40) to obtain 6-[[2-(2-fluorophenyl)sulfonyl-2-azaspiro[3.3]heptan-6-yl]amino]-3-methylheptanoic acid as a diastereomeric mixture.
(2) The diastereomeric mixture of 6-[[2-(2-fluorophenyl)sulfonyl-2-azaspiro[3.3]heptan-6-yl]amino]-3-methyl-heptanoic acid obtained in the (1) was treated in the same manner as in Example 423 and the diastereomers were separated to obtain each of the title compounds.

### Example 436

40 mg of (4R*,7S*)-1-[2-(2-fluorophenyl)sulfonyl-2-azaspiro[3.3]heptan-6-yl]-4,7-dimethylazepan-2-one (2-step yield 39%)
MS(ESI)m/z:395[M+H]⁺

### Example 437

47.3 mg of (4R*,7R*)-1-[2-(2-fluorophenyl)sulfonyl-2-azaspiro[3.3]heptan-6-yl]-4,7-dimethylazepan-2-one (2-step yield 46%)
MS(ESI)m/z:395[M+H]⁺

### Examples 438 and 439

Corresponding raw compounds were treated in the same manner as in Examples 436 and 437 to obtain compounds shown in Table 36.

**[Table 36]**

| Example | Structural formula | Physical property value and the like |
|---|---|---|
| 438 | 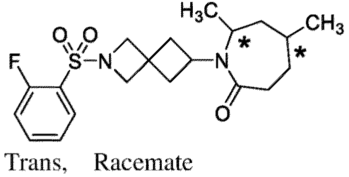 | MS(ESI) m/z; 395 [M+H]+ |
| 439 | 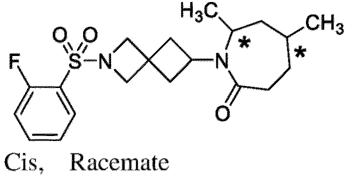 | MS(ESI) m/z; 395 [M+H]+ |

### Example 440

### Production of 1-[2-(2-fluorophenyl)sulfonyl-2-azaspiro[3.3]heptan-6-yl]-6-methylpiperidin-2-one

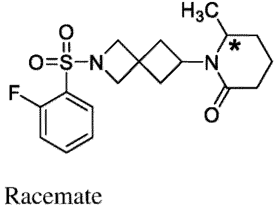

To a mixture of 161mg of 2-(2-fluorophenyl)sulfonyl-2-azaspiro[3.3]heptan-6-amine and 3.0 mL of acetonitrile, 0.15 mL of ethyl 5-oxohexanoate, 190 mg of sodium triacetoxyborohydride, and 0.025 mL of acetic acid were added, and the mixture was stirred at room temperature for 16 hours and 30 minutes. The reaction mixture was further heated to reflux all night. The reaction mixture was brought to room temperature and ethyl acetate was added thereto. The organic phase was washed sequentially with saturated aqueous sodium bicarbonate solution and brine. The organic phase was dried over magnesium sulfate, the insoluble material was removed by filtration, and the filtrate was concentrated under reduced pressure. The resulting residue was purified by reversed-phase HPLC (solvent: 10 mM aqueous ammonium carbonate solution/acetonitrile = 70/30 to 40/60) to obtain 120 mg of the title compound (yield 55%) as a colorless oil.
MS(ESI)m/z:367[M+H]⁺

### Example 441

### Production of 4-[2-(2-fluorophenyl)sulfonyl-2-azaspiro[3.3]heptan-6-yl]-5-methylthiomorpholin-3-one

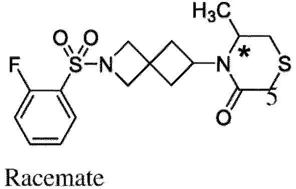

(1) To a mixture of 430 mg of ethyl 2-[2-[[2-(2-fluorophenyl)sulfonyl-2-azaspiro[3.3]heptan-6-yl]amino]propylsulfanyl]acetate, 2.0 mL of tetrahydrofuran and 2.0 mL of ethanol, 1.5 mL of 1M aqueous sodium hydroxide solution were added, and the mixture was stirred at room temperature all night. To the reaction mixture was added 1.5 mL of 1M hydrochloric acid and, and the mixture was concentrated under reduced pressure to obtain a crude product of 2-[2-[[2-(2-fluorophenyl)sulfonyl-2-azaspiro[3.3]heptan-6-yl]amino propylsulfanyl]acetic acid.
(2) The crude product obtained in (1) above was treated in the same manner as in Example 423 to obtain 352 mg of the title compound (yield 92%) as a colorless viscous material.
   MS(ESI)m/z:385[M+H]⁺

### Example 442

### Production of (7R)-1-[2-(2-fluorophenyl)sulfonyl-2-azaspiro[3.3]heptan-6-yl]-7-methyl-1,4-diazepan-2-one

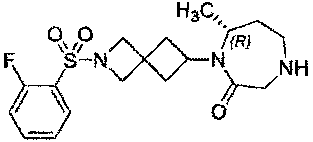

To a mixture of 1.39 g of tert-butyl (SR)-4-[2-(2-fluorophenyl)sulfonyl-2-azaspiro[3.3]heptan-6-yl]-5-methyl- 3-oxo-1,4-diazepan-1-carboxylate and 13.4 mL of methylene chloride, 0.55 mL of trimethylsilyl trifluoromethanesulfonate was added, and the mixture was stirred at room temperature for 30 minutes. After concentrating the reaction mixture under reduced pressure, saturated aqueous sodium bicarbonate solution and chloroform were added to the residue, and the mixture was stirred. The organic layer was separated with Phase-separator^{®} and concentrated under reduced pressure. The resulting residue was purified by NH silica gel column chromatography (solvent: ethyl acetate/methanol = 100/0 to 90/10) to obtain 729 mg of the title compound (yield 71%) as a colorless powder.
MS(ESI)m/z:382[M+H]⁺

### Example 443

### Production of (7R)-4-(cyclopropylmethyl)-1-[2-(2-fluorophenyl)sulfonyl-2-azaspiro[3.3]heptan-6-yl]-7-methyl-1,4-diazepan-2-one

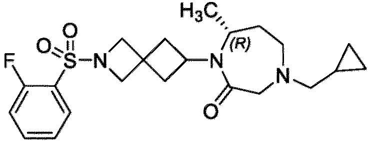

A mixture of 15 mg of (7R)-1-[2-(2-fluorophenyl)sulfonyl-2-azaspiro[3.3]heptan-6-yl]-7-methyl-1,4-diazepan-2-one, 3.58 mg of cyclopropanecarboxyaldehyde, 2.3 µL of acetic acid and 0.2 mL of methylene chloride was stirred at room temperature for 10 minutes, then 12.5 mg of sodium triacetoxyborohydride was added thereto, and the mixture was stirred at room temperature for 2 hours. To the reaction mixture were added 1 mL of methylene chloride and 1 mL of saturated aqueous sodium bicarbonate solution, and after stirring, the organic phase was separated with Phase-separator^{®} and concentrated. The resulting residue was purified by reversed-phase HPLC (solvent: 10 mM aqueous ammonium carbonate solution/acetonitrile = 70/30 to 40/60) to obtain 12.5mg of the title compound (yield 73%).
MS(ESI)m/z:436[M+H]⁺

### Examples 444 to 448

Corresponding raw material compounds were treated in the same manner as in Example 443 to obtain compounds shown in Table 37.

**[Table 37]**

| Example | Structural formula | Physical property value and the like |
|---|---|---|
| 444 | 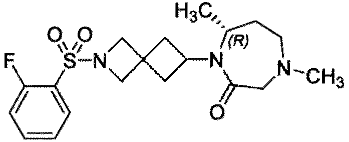 | MS(ESI) m/z; 396 [M+H]+ |
| 445 | 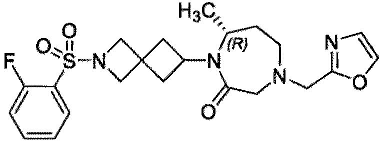 | MS(ESI) m/z; 463 [M+H]+ |
| 446 | 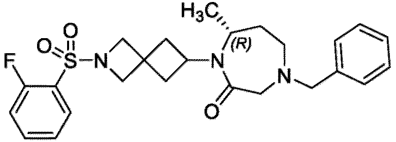 | MS(ESI) m/z; 472 [M+H]+ |
| 447 | 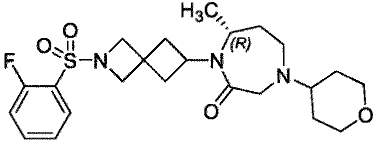 | MS(ESI) m/z; 466 [M+H]+ |
| 448 | 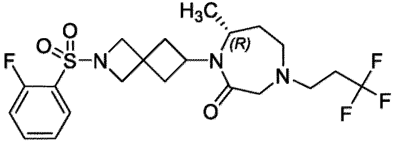 | MS(ESI) m/z; 478 [M+H]+ |

### Examples 449 to 454

(7R)-1-[2-(2-fluorophenyl)sulfonyl-2-azaspiro[3.3]heptan-6-yl]-7-methyl-1,4-diazepan-2-one and corresponding reagents were used and treated in the same manner as in Example 423 to obtain compounds shown in Table 38.

**[Table 38]**

| Example | Structural formula | Physical property value and the like |
|---|---|---|
| 449 | 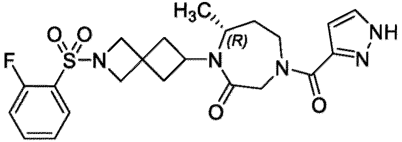 | MS(ESI) m/z; 476 [M+H]+ |
| 450 | 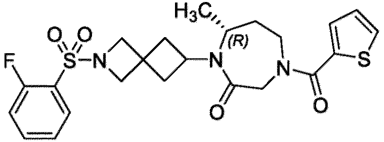 | MS(ESI) m/z; 492 [M+H]+ |
| 451 | 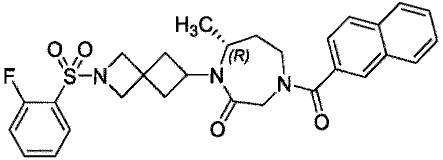 | MS(ESI) m/z; 536 [M+H]+ |
| 452 | 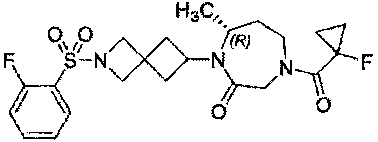 | MS(ESI) m/z; 468 [M+H]+ |
| 453 | 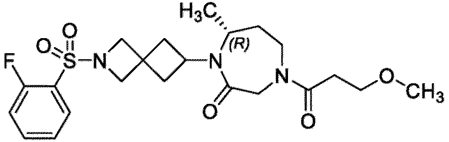 | MS(ESI) m/z; 468 [M+H]+ |
| 454 | 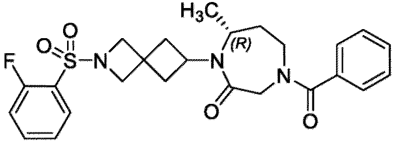 | MS(ESI) m/z; 486 [M+H]+ |

### Example 455

### Production of (7R)-1-[2-(2-fluorophenyl)sulfonyl-2-azaspiro[3.3]heptan-6-yl]-7-methyl-4-methylsulfonyl-1,4-diazepan-2-one

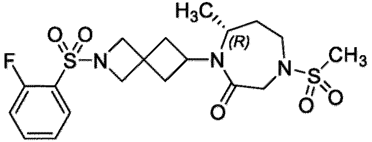

To a mixture of 15 mg of (7R)-1-[2-(2-fluorophenyl)sulfonyl-2-azaspiro[3.3]heptan-6-yl]-7-methyl-1,4-diazepan-2-one, 0.02 mL of diisopropylethylamine and 0.4 mL of methylene chloride, 13.7 mg of methanesulfonic anhydride was added, and the mixture was stirred at room temperature for 30 minutes. Water and chloroform were added to the reaction mixture and the mixture was stirred, then the organic layer was separated with Phase-separator^{®} and concentrated under reduced pressure. The resulting residue was purified by reversed-phase HPLC (solvent: 10 mM aqueous ammonium carbonate solution/acetonitrile = 70/30 to 40/60) to obtain 17.4 mg of the title compound (yield 96%) as a colorless powder.
MS(ESI)m/z:460[M+H]⁺

### Example 456

### Production of (7R)-1-[2-(2-fluorophenyl)sulfonyl-2-azaspiro[3.3]heptan-6-yl]-7-methyl-4-pyridin-2-yl-1,4-diazepan-2-one

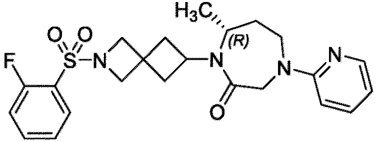

To a mixture of 1.8 mg of tris(dibenzylideneacetone)dipalladium, 1.7 mg of 1,3-bis(2,6-diisopropylphenyl)imidazolium chloride, 6.6 mg of sodium t-butoxide and 1mL of 1,4-dioxane, 15 mg of (7R)-1-[2-(2-fluorophenyl)sulfonyl-2-azaspiro[3.3]heptan-6-yl]-7-methyl-1,4-diazepan-2-one and 18.6 mg of 2-bromopyridine were added, and the mixture was stirred at room temperature for 3 hours, and then the mixture was heated to reflux for 4 hours. To the reaction mixture, 18.6 mg of 2-bromopyridine, 1.8 mg of tris(dibenzylideneacetone)dipalladium, and1.7 mg of 1,3-bis(2,6-diisopropylphenyl)imidazolium chloride were added, and the mixture was heated to reflux for 3 hours. The reaction mixture was brought to room temperature, water and chloroform were added thereto, and the mixture was stirred. The organic layer was separated with Phase-separator^{®} and concentrated under reduced pressure. The resulting residue was purified by NH silica gel column chromatography (solvent: hexane/ethyl acetate = 100/0 to 0/100) and then by reverse phase HPLC (solvent: 10 mM aqueous ammonium carbonate solution/acetonitrile = 60/40 to 30/70) again to obtain 7.5 mg of the title compound (yield 42%) as a colorless powder.
MS(ESI)m/z:459[M+H]⁺

### Example 457

### Production of 4-[2-(2-fluorophenyl)sulfonyl-2-azaspiro[3.3]heptan-6-yl]-5-methyl-1,4-oxazepan-3-one

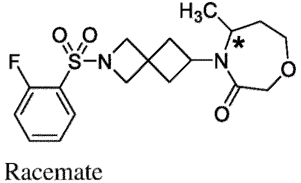

To a mixture of 80 mg of 2-chloro-N-[2-(2-fluorophenyl)sulfonyl-2-azaspiro[3.3]heptan-6-yl]-N-(4-hydroxybutan-2-yl)acetamide and 0.95 mL of N,N-dimethylformamide, 8.4 mg of sodium hydride (60%) was added under an ice-cold condition, and the mixture was stirred for 30 minutes. Water was added to the reaction mixture and the mixture was extracted with ethyl acetate. The organic phase was washed with water and brine, and the organic layer was separated with Phase-separator^{®} and concentrated under reduced pressure. The resulting residue was purified by reversed-phase HPLC (solvent: 10 mM aqueous ammonium carbonate solution/acetonitrile = 70/30 to 40/60) to obtain 2.6 mg of the title compound (yield 3.6%) as a pale yellow viscous material.
MS(ESI)m/z:383[M+H]⁺

### Example 458

### Production of (5R)-4-[2-(2-fluorophenyl)sulfonyl-2-azaspiro[3.3]heptan-6-yl]-5-methylmorpholin-3-one

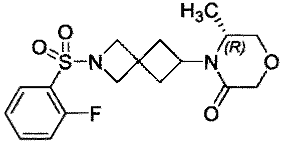

To a mixture of 50 mg of (2R)-2-[[2-(2-fluorophenyl)sulfonyl-2-azaspiro[3.3]heptan-6-yl]amino]propan-1-ol, 75 µL of triethylamine and 1.5 mL of tetrahydrofuran, 38 µL of 2-chloroacetyl chloride was added dropwise, and the mixture was stirred for 1 hour and 20 minutes. To the reaction mixture was added 90 µL of 28% sodium methoxide methanol solution, and the mixtuire was stirred for 2 hours. Then, 50 µL of 28% sodium methoxide methanol solution was added thereto, and the mixture was stirred overnight. Again 50 µL of 28% sodium methoxide methanol solution was added thereto, and the mixture was stirred for 4 hours. Water was added to the reaction mixture, the mixture was neutralized by 1M hydrochloric acid, and chloroform was added thereto and the mixture was stirred. The organic layer was separated with Phase-separator^{®} and concentrated under reduced pressure. The resulting residue was purified by reversed-phase HPLC (solvent: 10 mM aqueous ammonium carbonate solution/acetonitrile = 80/20 to 50/50) to obtain 29.4 mg of the title compound (yield 52%) as a yellow solid.
MS(ESI)m/z:369[M+H]⁺

### Example 459

### Production of 1-[2-(2-fluorophenyl)sulfonyl-2-azaspiro[3.3]heptan-6-yl]-7-methylazepan-2-one

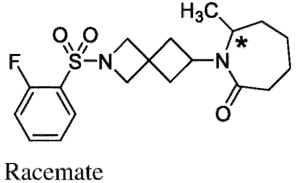

1-[2-(2-fluorophenyl)sulfonyl-2-azaspiro[3.3]heptan-6-yl]-2-methyl-3,4-dihydro-2H-azepine-7-one was used and treated in the same manner as in Example 85 to obtain the title compound.
MS(ESI)m/z:381[M+H]⁺

### Example 460.

### Production of (3R)-4-[2-(2-fluorophenyl)sulfonyl-2-azaspiro[3.3]heptan-6-yl]-3-methyl-1,4-oxazepan-5-one

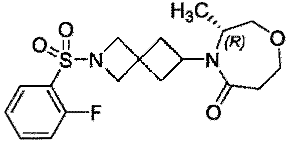

Tert-butyl 3-[(2R)-2-[[2-(2-fluorophenyl)sulfonyl-2-azaspiro[3.3]heptan-6-yl]amino]propoxy]propanoate was treated in the same manner in the order of Example 442 and Example 423 to obtain the title compound.
MS(ESI)m/z:383[M+H]⁺

In production of the compounds of Examples, as the raw material compound and the intermediate, a commercially available reagent, or a compound chemically modified from these reagents by an existing method and an equivalent method can be used. In addition, it is possible to produce the raw material compound and the intermediate using compounds described in the following Reference Examples.

### Reference Example 1

### Production of 2-(2-fluorophenyl)sulfonyl-2-azaspiro[3.3]heptan-6-one

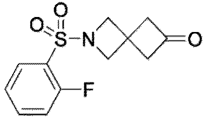

27.0 g of tert-butyl 6-oxo-2-azaspiro[3.3]-heptane-2-carboxylate was dissolved in 250 mL of dichloromethane, and 24.7 mL of trimethylsilyl trifluoromethanesulfonate was added dropwise under ice-cooling for 5 minutes. The temperature was raised to room temperature, and the mixture was stirred for 0.5 hours (reaction solution 1). 24.9 g of 2-fluorobenzenesulfonyl chloride was dissolved in 250 mL of dichloromethane, and 35.5 mL of triethylamine was added under ice-cooling. The reaction solution 1 was added dropwise thereto for 10 minutes. Thereafter, the temperature was raised to room temperature, and the mixture was stirred for 1 hour. The reaction solution was concentrated to halve the solvent amount. A 1 N sodium hydroxide aqueous solution was added to the organic layer and washed twice and then washed twice with 1 N hydrochloric acid. Washing was performed once again with a 1 N sodium hydroxide aqueous solution. The organic layer was washed with brine, dried over magnesium sulfate, and then concentrated under reduced pressure. The resulting residue was suspended and washed with hexane/ethyl acetate, a solid was collected by filtration, and drying was performed under reduced pressure, thereby obtaining 24.8 g of the title compound (yield 72%) as a brown powder.
MS(ESI)m/z:270[M+H]⁺

### Reference Example 2

### Production of 2-(2-fluorophenyl)sulfonyl-2-azaspiro[3.3]heptan-6-amine

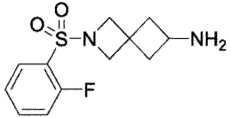

(1) In a 200 mL eggplant flask, 3.17 g of tert-butyl 6-amino-2-azaspiro[3.3]heptane-2-carboxylate was weighed, and 50 mL of dichloromethane and 3.5 mL of triethylamine were added and stirred under ice-cooling. Next, 4.03 g of 2-nitrobenzenesulfonyl chloride was added, and the mixture was stirred at room temperature for 15 hours. A saturated sodium bicarbonate aqueous solution was added to the reaction solution, and the mixture was stirred at room temperature for 5 minutes. A mixed solvent of ethyl acetate:hexane = 1:1 and brine were added to the reaction solution, and the organic layer was extracted. The aqueous layer was extracted again with a mixed solvent of ethyl acetate:hexane = 1:1, and the organic layer was mixed. The organic layers were combined, washed with brine, and then dried over magnesium sulfate. The insoluble matter was filtered, and then the filtrate was concentrated under reduced pressure. To 5.66 g of the resulting colorless solid, 60 mL of a mixed solvent of ethyl acetate:hexane = 1:5 was added and suspended, and a solid was collected by filtration and dried under reduced pressure, thereby obtaining 5.45 g of tert-butyl 6-[(2-nitrophenyl)sulfonylamino]-2-azaspiro[3.3]heptane-2-carboxylate (yield 92%) as a colorless powder.
   MS(ESI)m/z:396[M-H]⁻
(2) In a 200 mL eggplant flask, 5.45 g of the tert-butyl 6-[(2-nitrophenyl)sulfonylamino]-2-azaspiro[3.3]heptane-2-carboxylate obtained in (1) above was weighed, 45 mL of dichloromethane was added, and the mixture was stirred under ice-cooling. Next, 3.4 mL of trimethylsilyl trifluoromethanesulfonate was added, and thereafter, the mixture was stirred at room temperature for 80 minutes. The reaction solution was concentrated under reduced pressure to obtain 8.07 g of N-(2-azaspiro[3.3]heptan-6-yl)-2-nitro-benzenesulfonamide trifluoromethanesulfonate (yield 88%) as a colorless amorphous solid.
   MS(ESI)m/z:298[M+H]⁺
(3) In a 200 mL eggplant flask, 8.07 g of the N-(2-azaspiro[3.3]heptan-6-yl)-2-nitro-benzenesulfonamide trifluoromethanesulfonate obtained in (2) above was weighed, 60 mL of methylene chloride and 5.0 mL of triethylamine were added, and the mixture was stirred under ice-cooling. Next, 3.19 g of 2-fluorobenzenesulfonyl chloride was added, and the mixture was stirred under ice-cooling for 105 minutes. Thereafter, 2.5 mL of triethylamine and 0.50 g of 2-fluorobenzenesulfonyl chloride were additionally added, and the mixture was stirred at room temperature for 15 hours. A saturated sodium bicarbonate aqueous solution was added to the reaction solution, and extraction was performed with a mixed solvent of ethyl acetate:hexane = 1:1. The organic layer was washed with a saturated sodium bicarbonate aqueous solution and brine, and then dried over magnesium sulfate. The insoluble matter was filtered, and then the filtrate was concentrated under reduced pressure. 7.33 g of the resulting brown solid was suspended in 25 mL of chloroform, and then a solid was collected by filtration, washed with 5 mL of chloroform, and then dried under reduced pressure, thereby obtaining 2.54 g of N-[2-(2-fluorophenyl)sulfonyl-2-azaspiro[3.3]heptan-6-yl]-2-nitro-benzenesulfonamide as a colorless powder. The mother liquid after the collection by filtration was concentrated under reduced pressure, the resulting brown solid was suspended in 6 mL of chloroform, and then a solid was collected by filtration, washed with 4 mL of chloroform, and then dried under reduced pressure, thereby obtaining 1.57 g of N-[2-(2-fluorophenyl)sulfonyl-2-azaspiro[3.3]heptan-6-yl]-2-nitrobenzenesulfonamide as a colorless powder. The mother liquid after the collection by filtration was concentrated under reduced pressure, and 3.15 g of the resulting brown oily product was purified by silica gel column chromatography (solvent: hexane/ethyl acetate = 50/50 to 15/85), thereby obtaining 1.72 g of a pale yellow solid. The pale yellow solid was suspended in 12 mL of a mixed solvent of chloroform:hexane = 1:1, and then a solid was collected by filtration, washed with hexane, and dried under reduced pressure, thereby obtaining 1.39 g of N-[2-(2-fluorophenyl)sulfonyl-2-azaspiro[3.3]heptan-6-yl]-2-nitro-benzenesulfonamide as a colorless solid. The total acquisition amount of N-[2-(2-fluorophenyl)sulfonyl-2-azaspiro[3.3]heptan-6-yl]-2-nitro-benzenesulfonamide was 5.49 g.
   MS(ESI)m/z:456[M+H]⁺
(4) In a 100 mL eggplant flask, 1.38 g of the N-[2-(2-fluorophenyl)sulfonyl-2-azaspiro[3.3]heptan-6-yl]-2-nitro-benzenesulfonamide obtained in (3) above was weighed, and 17 mL of acetonitrile was added. Next, 1.48 g of cesium carbonate and 500 µL of 4-ethylthiophenol were added, and the mixture was stirred at room temperature for 80 minutes. Thereafter, 200 µL of 4-ethylthiophenol was additionally added, and the mixture was stirred at room temperature for 4 hours. Water and 15 mL of 1 mol/L hydrochloric acid were added to the reaction solution to set the pH to 2. Hexane was added thereto, and the aqueous layer was extracted. Water was added again to the organic layer, and the aqueous layer was extracted. The aqueous layers were combined, 16 mL of a 1 mol/L sodium hydroxide aqueous solution and a saturated sodium bicarbonate solution were added to set the pH to 9, and then extraction was performed with chloroform (100 mL × two times). The organic layer was dried over sodium sulfate, the insoluble matter was filtered, and then the solution was concentrated under reduced pressure, thereby obtaining 770 mg of the title compound (yield 94%) as a yellow oily product.
   MS(ESI)m/z:271[M+H]⁺

### Reference Example 3

### Production of (2R)-2-[[2-(2-fluorophenyl)sulfonyl-2-azaspiro[3.3]heptan-6-yl]amino]propan-1-ol

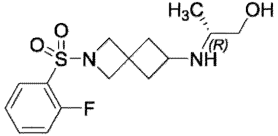

24.8 g of 2-(2-fluorophenyl)sulfonyl-2-azaspiro[3.3]heptan-6-one was dissolved in 300 mL of dichloromethane, and 8.98 g of (2R)-2-aminopropan-1-ol and 5.26 mL of acetic acid were added. Under ice-cooling, 39.0 g of sodium triacetoxyborohydride was slowly added in four portions. The temperature was raised to room temperature, and the mixture was stirred for 3 hours. A 1 N sodium hydroxide aqueous solution was added to the reaction mixture, and extraction was performed with chloroform. The organic layer was washed with brine, filtration was performed after drying with sodium sulfate, and the filtrate was concentrated under reduced pressure. (Crude Product 1) On the other hand, sodium chloride was added to the aqueous layer to saturate the aqueous layer, and extraction was performed three times with chloroform. The organic layers were combined, washed with brine, and dried over sodium sulfate, filtration was performed, and then the filtrate was concentrated under reduced pressure, thereby obtaining 5.37 g of the title compound (yield 18%) as a light yellow powder.

The crystals were added to the crude product 1 to crystallize, suspension washing were performed with hexane/ethyl acetate, the generated solid was collected by filtration, and drying was performed under reduced pressure, thereby obtaining 17.2 g of the title compound (yield 57%) as a white powder. The filtrate was concentrated under reduced pressure, the resulting residue was purified by silica gel chromatography (solvent: ethyl acetate/methanol = 100/0 to 92/8), fractions were collected and concentrated under reduced pressure, the resulting residue was suspended and washed with hexane/ethyl acetate, and a solid was collected by filtration and dried under reduced pressure, thereby obtaining 3.66 g of the title compound (yield 12%) as a white powder.
MS(ESI)m/z:329[M+H]⁺

### Reference Examples 4 to 27:

Corresponding raw material compounds were treated in the same manner as that of Reference Example 3 to obtain compounds shown in Table 39.

**[Table 39-1]**

| Reference Example | Structural formula | Physical property value and the like |
|---|---|---|
| 4 | 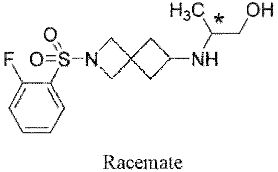 | MS(ESI) m/z;329 [M+H]+ |
| 5 | 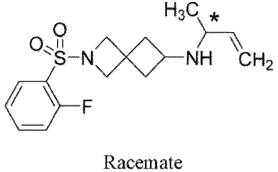 | MS(ESI) m/z;325 [M+H]+ |
| 6 | 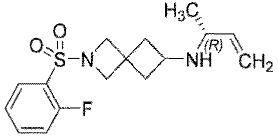 | MS(ESI) m/z;325 [M+H]+ |
| 7 | 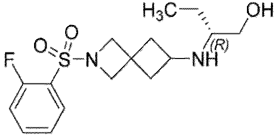 | MS(ESI) m/z;343 [M+H]+ |
| 8 | 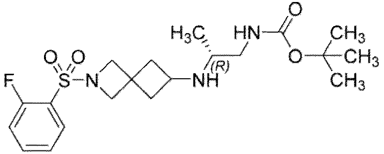 | MS(ESI) m/z;428 [M+H]+ |
| 9 | 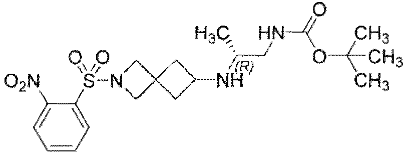 | MS(ESI) m/z;455 [M]+ |
| 10 | 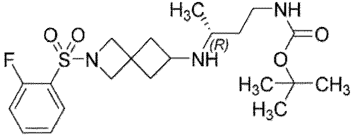 | MS(ESI) m/z;442 [M+H]+ |

**[Table 39-2]**

| | | |
|---|---|---|
| 11 | 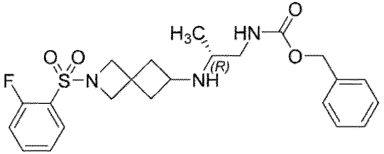 | MS(ESI) m/z;462 [M+H]+ |
| 12 | 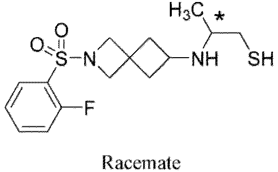 | MS(ESI) m/z;345 [M+H]+ |
| 13 | 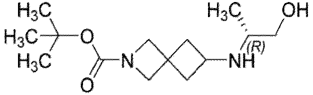 | MS(ESI) m/z;271 [M+H]+ |
| 14 | 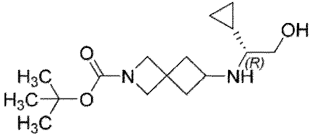 | MS(ESI) m/z;297 [M+H]+ |
| 15 | 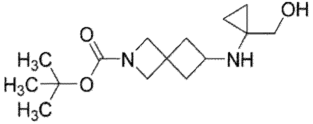 | MS(ESI) m/z;283 [M+H]+ |
| 16 | 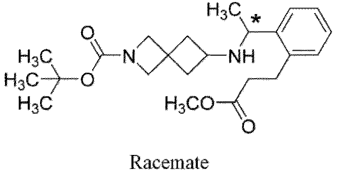 | MS(ESI) m/z;403 [M+H]+ |
| 17 | 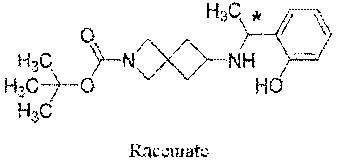 | MS(ESI) m/z;333 [M+H]+ |

**[Table 39-3]**

| | | |
|---|---|---|
| 18 | 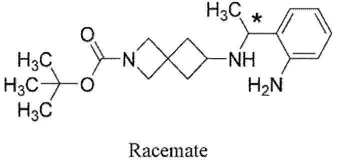 | MS(ESI) m/z;332 [M+H]+ |
| 19 | 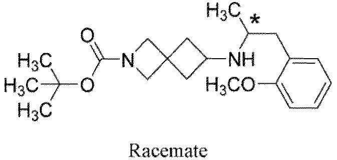 | MS(ESI) m/z;361 [M+H]+ |
| 20 | 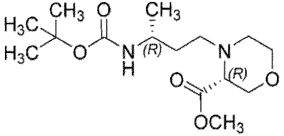 | MS(ESI) m/z;317 [M+H]+ |
| 21 | 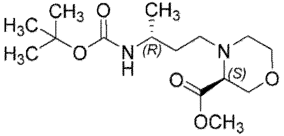 | MS(ESI) m/z;317 [M+H]+ |
| 22 | 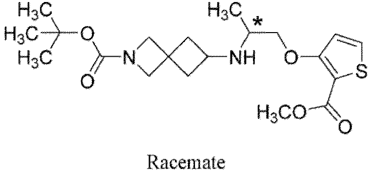 | MS(ESI) m/z;411 [M+H]+ |
| 23 | 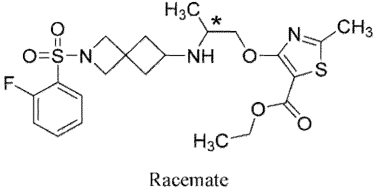 | MS(ESI) m/z;498 [M+H]+ |

**[Table 39-4]**

| | | |
|---|---|---|
| 24 | 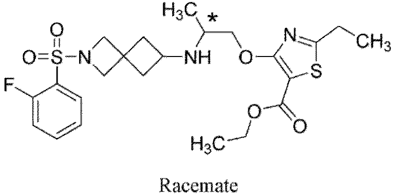 | MS(ESI) m/z;512 [M+H]+ |
| 25 | 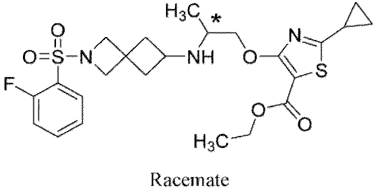 | MS(ESI) m/z;524 [M+H]+ |
| 26 | 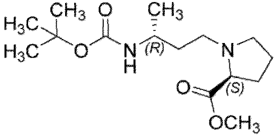 | MS(ESI) m/z;301 [M+H]+ |
| 27 | 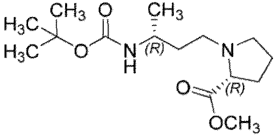 | MS(ESI) m/z;301 [M+H]+ |

### Reference Example 28

### Production of tert-butyl 6-(7-methyl-5-oxo-7,8-dihydro-1,6-naphthyridin-6-yl)-2-azaspiro [3.3]heptane-2-carboxylate

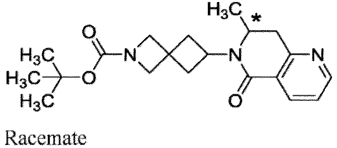

180 mg of tert-butyl 6-amino-2-azaspiro[3.3]heptane-2-carboxylate, 155 mg of tert-butyl 2-acetonylpyridine-3-carboxylate (purity 39 mass%, containing 7-methylpyrano[4,3-b]pyridin-5-one as an impurity), and 0.1 mL of acetic acid were dissolved in 3 mL of dichloromethane, 124 mg of sodium triacetoxyborohydride was added, and the mixture was stirred at room temperature for 20 hours. A saturated sodium bicarbonate aqueous solution was added to the reaction mixture, and extraction was performed with chloroform. The organic layer was washed with brine, filtration was performed after drying with sodium sulfate, and the filtrate was concentrated under reduced pressure. The resulting residue was dissolved in 3 mL of acetonitrile, and the mixture was stirred at 50°C for 1 hour. The temperature was raised to 80°C, and the mixture was stirred for 11 hours. The reaction solution was dissolved in dimethyl sulfoxide and purified by reverse-phase HPLC (solvent: 10 mM ammonium carbonate aqueous solution/acetonitrile = 70/30 to 40/60) to obtain 22 mg of the title compound (yield 21%) as a brown powder.
MS(ESI)m/z:358[M+H]⁺

### Reference Example 29

### Production of 1-[[2-(2-fluorophenyl)sulfonyl-2-azaspiro[3.3]heptan-6-yl]amino]propan-2-ol

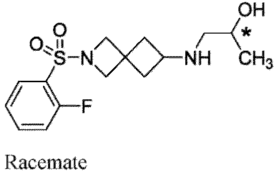

300 mg of 2-(2-fluorophenyl)sulfonyl-2-azaspiro[3.3]heptan-6-amine was dissolved in 1 mL of tetrahydrofuran, 155 µL of propylene oxide was added, and the mixture was stirred at room temperature for 15 hours. 466 µL of propylene oxide was added to the reaction solution, and the mixture was stirred at 60°C for 15 hours. 311 µL of propylene oxide was added to the reaction solution, and the mixture was stirred at 60°C for 2 hours. The reaction solution was dissolved in dimethyl sulfoxide and purified by reverse-phase HPLC (solvent: 10 mM ammonium carbonate aqueous solution/acetonitrile = 80/20 to 50/50) to obtain 168 mg of the title compound (yield 46%) as a colorless viscous compound.
MS(ESI)m/z:329[M+H]⁺

### Reference Examples 30 and 31:

Corresponding raw materials were treated in the same manner as that of Example 170 to obtain compounds shown in Table 40.

**[Table 40]**

| Reference Example | Structural formula | Physical property value and the like |
|---|---|---|
| 30 | 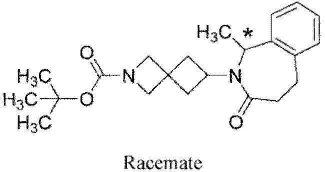 | MS(ESI) m/z;315 [M-tBu+H]+ |
| 31 | 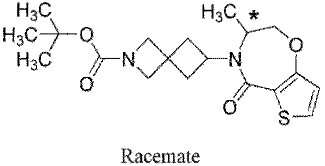 | MS(ESI) m/z;379 [M+H]+ |

### Reference Examples 32 to 35:

Corresponding raw materials were treated in the same manner as that of Example 1 to obtain compounds shown in Table 41.

**[Table 41]**

| Reference Example | Structural formula | Physical property value and the like |
|---|---|---|
| 32 | 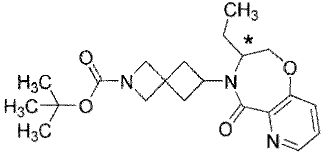 | MS(ESI) m/z;388 [M+H]+ |
| 33 | 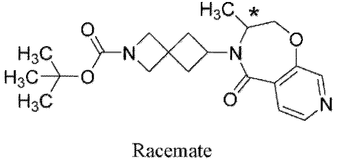 | MS(ESI) m/z;374 [M+H]+ |
| 34 | 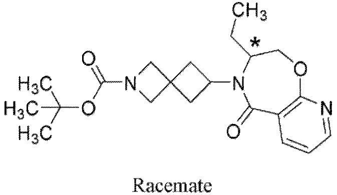 | MS(ESI) m/z;388 [M+H]+ |
| 35 | 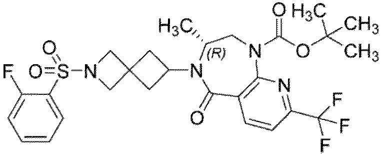 | MS(ESI) m/z;599[M+H]+ |

### Reference Examples 36 to 40:

Corresponding raw materials were treated in the same manner as that of Example 8 to obtain compounds shown in Table 42.

**[Table 42]**

| Reference Example | Structural formula | Physical property value and the like |
|---|---|---|
| 36 | 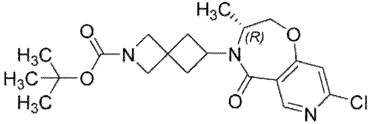 | MS(ESI) m/z;408/410 [M+H]+ |
| 37 | 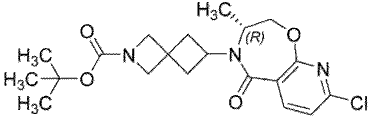 | MS(ESI) m/z;352/354 [M-tBu+H]+ |
| 38 | 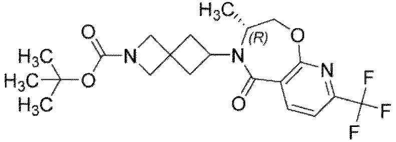 | MS(ESI) m/z;442 [M+H]+ |
| 39 | 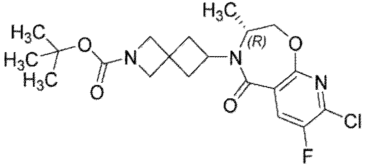 | MS(ESI) m/z;370/372 [M-tBu+H]+ |
| 40 | 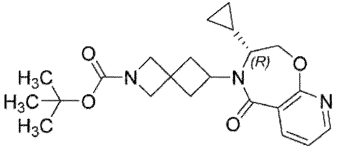 | MS(ESI) m/z;400 [M +H]+ |

### Reference Examples 41 and 42:

Corresponding raw materials were treated in the same manner as that of Example 44 to obtain compounds shown in Table 43.

**[Table 43]**

| Reference Example | Structural formula | Physical property value and the like |
|---|---|---|
| 41 | 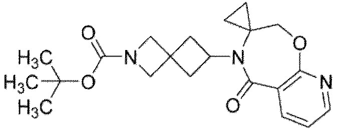 | MS(ESI) m/z;386 [M+H]+ |
| 42 | 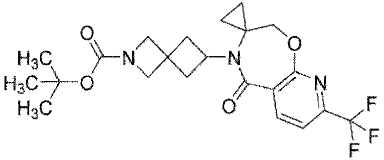 | MS(ESI) m/z;398 [M-tBu+H]+ |

### Reference Example 43

### Production of 4-(2-azaspiro[3.3]heptan-6-yl)-3-methyl-2,3-dihydropyrido[3,2-f][1,4]oxazepin-5-one

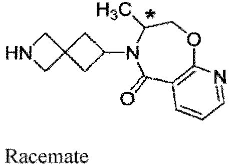

1.36 g of tert-butyl 6-(3-methyl-5-oxo-2,3-dihydropyrido[3,2-f][1,4]oxazepin-4-yl)-2-azaspiro[3.3]heptane-2-carboxylate was dissolved in 17 mL of dichloromethane, 0.710 mL of trimethylsilyl trifluoromethanesulfonate was added, and the mixture was stirred at room temperature for 1 hour. The reaction mixture was concentrated under reduced pressure. The resulting residue was suspended and washed with diethyl ether, the filtrate was removed, and the residue was dried under reduced pressure. The resultant was dissolved in water, a saturated sodium bicarbonate aqueous solution was added, the mixture was stirred for 15 minutes, and then extraction was performed with a mixed solution of chloroform and methanol. The organic layer was dried over magnesium sulfate and then filtered, and the filtrate was concentrated under reduced pressure and dried, thereby obtaining 864 mg of the title compound (yield 95%) as a yellow oily product.
MS(ESI)m/z:274[M+H]⁺

### Reference Example 44

### Production of 3-bromo-N-[2-(2-fluorophenyl)sulfonyl-2-azaspiro[3.3]heptan-6-yl]-N-(1-methylallyl)-5-(trifluoromethyl)pyridine-2-carboxamide

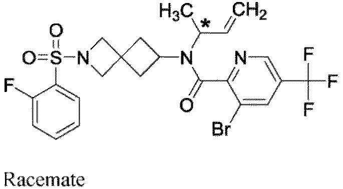

(1) In a 30 mL eggplant flask, 410 mg of 3-bromo-5-(trifluoromethyl)pyridine-2-carboxylic acid was weighed, and 6.0 mL of dichloromethane was added. The mixture was stirred under ice-cooling, 200 µL of oxalyl chloride and 10 µL of N,N-dimethylformamide were added, and the mixture was stirred at room temperature for 50 minutes. The reaction solution was concentrated under reduced pressure to obtain a crude product of 3-bromo-5-(trifluoromethyl)pyridine-2-carboxylic acid chloride as a yellow oily product.
(2) In a 10 mL eggplant flask, 459 mg of 2-(2-fluorophenyl)sulfonyl-N-(1-methylallyl)-2-azaspiro[3.3]heptan-6-amine was weighed, 6.0 mL of dichloromethane and 800 µL of triethylamine were added, and the mixture was stirred under ice-cooling. A solution obtained by dissolving the total amount of the crude product of 3-bromo-5-(trifluoromethyl)pyridine-2-carboxylic acid chloride obtained in (1) above in 4.0 mL of dichloromethane was added thereto, and the mixture was stirred under ice-cooling for 30 minutes and thereafter at room temperature for 30 minutes. Brine was added to the reaction mixture, and extraction was performed with ethyl acetate. The organic layer was washed with brine and then dried over magnesium sulfate. The insoluble matter was filtered, and then the solution was concentrated under reduced pressure. 0.84 g of the resulting orange oily product was purified by silica gel column chromatography (solvent: hexane/ethyl acetate = 80/20 to 55/45) to obtain 707 mg of the title compound (yield 87%) as a colorless powder.
   MS(ESI)m/z:576/578[M+H]⁺

### Reference Examples 45 to 52:

Corresponding raw material compounds were treated in the same manner as that of Reference Example 44 to obtain compounds shown in Table 44.

**[Table 44-1]**

| Reference Example | Structural formula | Physical property value and the like |
|---|---|---|
| 45 | 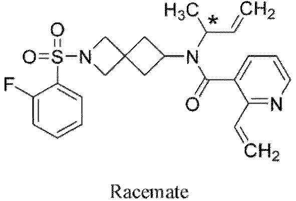 | MS(ESI) m/z;456 [M+H]+ |
| 46 | 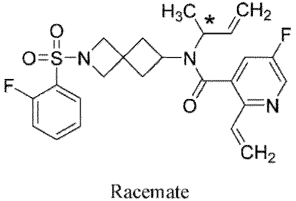 | MS(ESI) m/z;474 [M+H]+ |
| 47 | 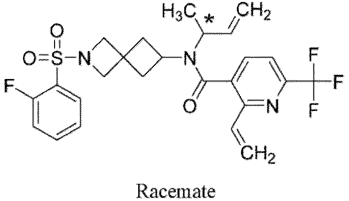 | MS(ESI) m/z;524 [M+H]+ |
| 48 | 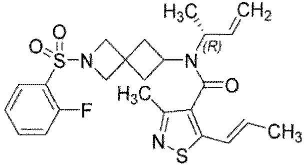 | MS(ESI) m/z;490 [M+H]+ |
| 49 | 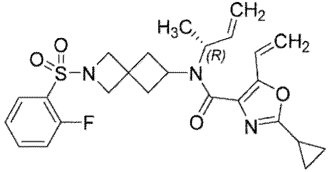 | MS(ESI) m/z;486 [M+H]+ |

**[Table 44-2]**

| | | |
|---|---|---|
| 50 | 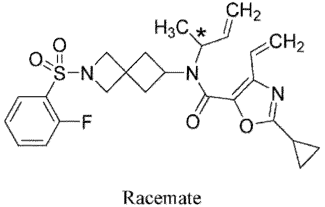 | MS(ESI) m/z;486 [M+H]+ |
| 51 | 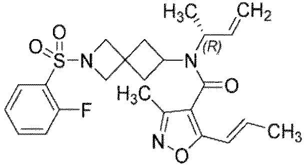 | MS(ESI) m/z;474 [M+H]+ |
| 52 | 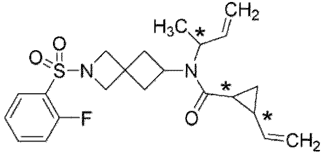 Relative configuration (1S*, 2S*) of cyclopropane, mixture of four types of diastereomers | MS(ESI) m/z;419 [M+H]+ |

### Reference Example 53

### Production of N-[2-(2-fluorophenyl)sulfonyl-2-azaspiro[3.3]heptan-6-yl]-N-(1-methylallyl)-2-(trifluoromethyl)-5-vinyl-thiazole-4-carboxamide

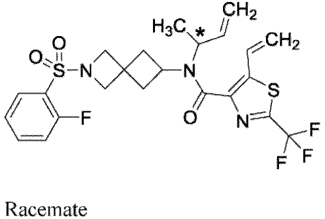

(1) 106 mg of ethyl 2-(trifluoromethyl)-5-vinyl-thiazole-4-carboxylate was dissolved in 3 mL of ethanol and 3 mL of tetrahydrofuran, 0.842 mL of a 1 mol/L sodium hydroxide aqueous solution was added, and the mixture was stirred at room temperature for 20 minutes. A 1 mol/L sodium hydroxide aqueous solution was added to the reaction mixture, and reverse-extraction was performed with ethyl acetate-hexane. The organic layer was washed with a 1 mol/L sodium hydroxide aqueous solution, the combined aqueous layers were acidified with a 6 mol/L hydrochloric acid aqueous solution, and then extraction was performed with ethyl acetate. The organic layer was dried over magnesium sulfate and then filtered, and the filtrate was concentrated under reduced pressure, thereby obtaining 25.5 mg of 2-(trifluoromethyl)-5-vinyl-thiazole-4-carboxylic acid (yield 27%) as a white powder.
(2) 25.5 mg of the 2-(trifluoromethyl)-5-vinyl-thiazole-4-carboxylic acid obtained in (1) above was subjected to toluene azeotropy and then dissolved in 2 mL of dichloromethane, 24.2 µL of oxalyl chloride and 8 µL of N,N-dimethylformamide were added, and then the mixture was stirred for 20 minutes. The reaction mixture was concentrated under reduced pressure to obtain a crude product of 2-(trifluoromethyl)-5-vinyl-thiazole-4-carbonyl chloride.
(3) The crude product of 2-(trifluoromethyl)-5-vinyl-thiazole-4-carbonyl chloride obtained in (2) above was dissolved in 3 mL of dichloromethane, 46.3 mg of 2-(2-fluorophenyl)sulfonyl-N-(1-methylallyl)-2-azaspiro[3.3]heptan-6-amine and 79.4 µL of triethylamine were added, and the mixture was stirred at room temperature for 1 hour. 50 mg of 2-(2-fluorophenyl)sulfonyl-N-(1-methylallyl)-2-azaspiro[3.3]heptan-6-amine was added, and the mixture was stirred at room temperature for 30 minutes. A saturated sodium bicarbonate aqueous solution was added to the reaction mixture, and extraction was performed with dichloromethane. The organic layer was washed with an ammonium chloride aqueous solution and brine, filtration was performed after drying with magnesium sulfate, and the filtrate was concentrated under reduced pressure. The resulting residue was purified by silica gel column chromatography (solvent: hexane/ethyl acetate = 75/25 to 67/33) to obtain 21.4 mg of the title compound (two stage yield 35%) as a light yellow oily product.
   MS(ESI)m/z:530[M+H]⁺

### ] Reference Example 54

### Production of N-[2-(2-fluorophenyl)sulfonyl-2-azaspiro [3.3]heptan-6-yl] -2-methyl-N-[(1R)-1-methylallyl]-4-[(E)-prop-1-enyl]thiazole-5-carboxamide

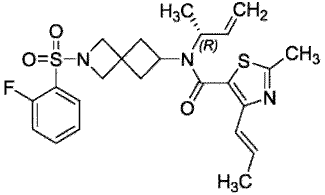

In a 50 mL eggplant flask, 214 mg of 2-(2-fluorophenyl)sulfonyl-N-[(1R)-methylallyl]-2-azaspiro[3.3]heptan-6-amine and 138 mg of 2-methyl-4-[(E)-prop-1-enyl]thiazole-5-carboxylic acid were weighed, and 4.0 mL of dimethylformamide was added. Next, 337 mg of HATU and 0.3 mL of triethylamine were added, and the mixture was stirred at room temperature for 16 hours. A saturated sodium bicarbonate aqueous solution and a mixed solvent of hexane/ethyl acetate (1:1) were added to the reaction mixture, and the organic layer was extracted. The organic layer was washed with brine and dried over magnesium sulfate. The insoluble matter was filtered, and then the solution was concentrated under reduced pressure. The resulting residue was purified by silica gel chromatography (solvent: hexane/ethyl acetate = 75/25 to 40/60) to obtain 259 mg of the title compound (yield 80%) as a pale orange viscous oily product.
MS(ESI)m/z:490[M+H]⁺

### Reference Examples 55 to 74:

Corresponding raw material compounds were treated in the same manner as that of Reference Example 54 to obtain compounds shown in Table 45.

**[Table 45-1]**

| Reference Example | Structural formula | Physical property value and the like |
|---|---|---|
| 55 | 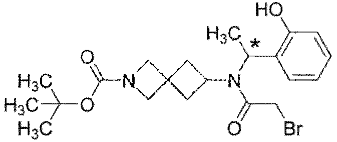 | MS(ESI) m/z;397/399 [M-tBu+H]+ |
| 56 | 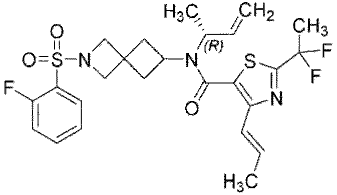 | MS(ESI) m/z;540 [M+H]+ |
| 57 | 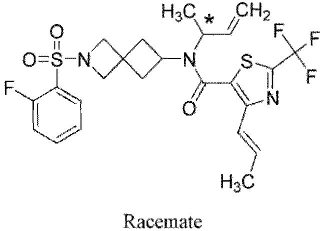 | MS(ESI) m/z;544 [M+H]+ |
| 58 | 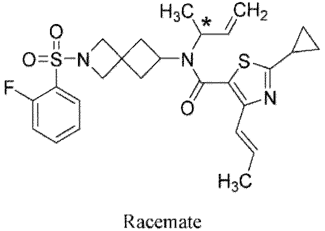 | MS(ESI) m/z;516 [M+H]+ |
| 59 | 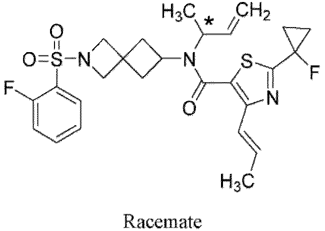 | MS(ESI) m/z;534 [M+H]+ |

**[Table 45-2]**

| | | |
|---|---|---|
| 60 | 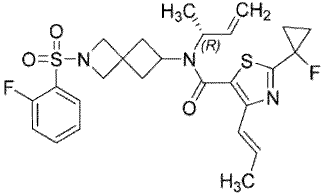 | MS(ESI) m/z;534 [M+H]+ |
| 61 | 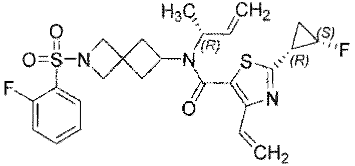 | MS(ESI) m/z;520 [M+H]+ |
| 62 | 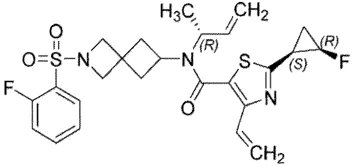 | MS(ESI) m/z;520 [M+H]+ |
| 63 | 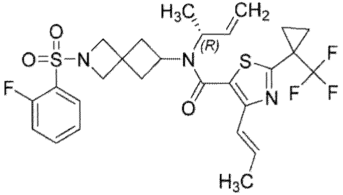 | MS(ESI) m/z;584 [M+H]+ |
| 64 | 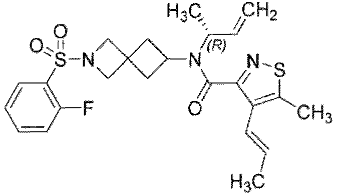 | MS(ESI) m/z;490 [M+H]+ |
| 65 | 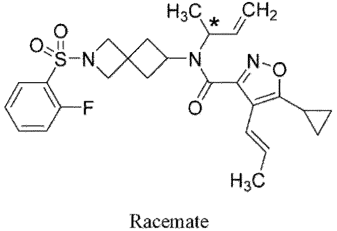 | MS(ESI) m/z;500 [M+H]+ |

**[Table 45-3]**

| | | |
|---|---|---|
| 66 | 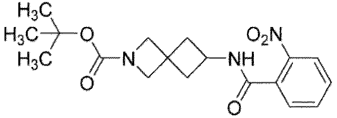 | MS(ESI) m/z;262 [M-Boc+H]+ |
| 67 | 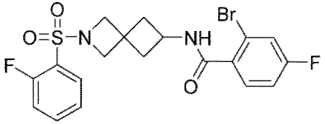 | MS(ESI) m/z;471/473 [M+H]+ |
| 68 | 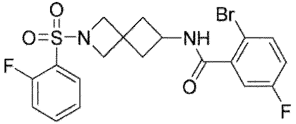 | MS(ESI) m/z;471/473 [M+H]+ |
| 69 | 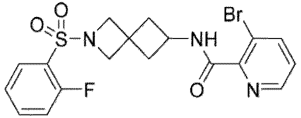 | MS(ESI) m/z;454/456 [M+H]+ |
| 70 | 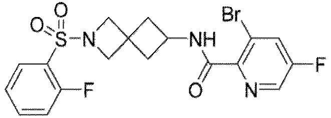 | MS(ESI) m/z;472/474 [M+H]+ |
| 71 | 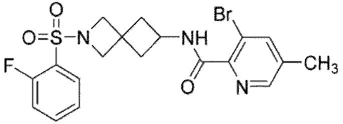 | MS(ESI) m/z;468/470 [M+H]+ |
| 72 | 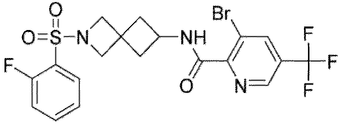 | MS(ESI) m/z;522/524 [M+H]+ |
| 73 | 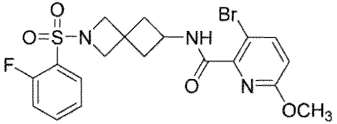 | MS(ESI) m/z;484/486 [M+H]+ |
| 74 | 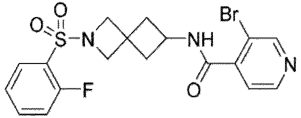 | MS(ESI) m/z;454/456 [M+H]+ |

### Reference Example 75

### Production of N-[2-(2-fluorophenyl)sulfonyl-2-azaspiro [3.3]heptan-6-yl] -5-methyl-N-[(1R)-1-methylallyl]-4-[(E)-propen-1-yl]isoxazole-3-carboxamide

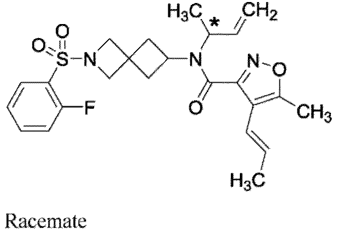

(1) 500 mg of methyl 4-bromo-5-methyl-isoxazole-3-carboxylate, 504 mg of potassium trifluoro-[(E)-propen-1-yl]boronide, 263 mg of tetrakistriphenylphosphine palladium, and 2.27 mL of a 2 mol/L sodium carbonate aqueous solution were suspended in 8 mL of 1,4-dioxane, and the mixture was stirred at 100°C for 3 hours. The reaction mixture was returned to room temperature, a 1 mol/L hydrochloric acid aqueous solution was added, and extraction was performed with ethyl acetate. The organic layer was washed with water and brine and then dried over magnesium sulfate, filtration was performed, and then the filtrate was concentrated under reduced pressure, thereby obtaining a crude product of methyl 5-methyl-4-[(E)-prop-1-enyl]isoxazole-3-carboxylate.
(2) The crude product of methyl 5-methyl-4-[(E)-prop-1-enyl]isoxazole-3-carboxylate obtained in (1) above was dissolved in 3 mL of tetrahydrofuran and 3 mL of methanol, 3 mL of a 1 mol/L sodium hydroxide aqueous solution was added, and the mixture was stirred at room temperature for 4 hours. Ethyl acetate was added to the reaction mixture, and extraction was performed. A 1 mol/L hydrochloric acid aqueous solution was added to the aqueous layer for acidification, and extraction was performed with ethyl acetate. The organic layer was washed with brine, dried over magnesium sulfate, and then filtered. The filtrate was concentrated under reduced pressure to obtain a crude product of 5-methyl-4-[(E)-propen-1-yl]isoxazole-3-carboxylic acid.
(3) The crude product of 5-methyl-4-[(E)-propen-1-yl]isoxazole-3-carboxylic acid obtained in (2) above was dissolved in 5 mL of N,N-dimethylformamide, 600 mg of 2-(2-fluorophenyl)sulfonyl-N-[(1R)-1-methylallyl]-2-azaspiro[3.3]heptan-6-amine, 1.04 g of O-(7-azabenzotriazol-1-yl)-N,N,N',N'-tetramethyluronium hexafluorophosphate (HATU), and 0.472 mL of N,N-diisopropylethylamine were added, and the mixture was stirred at room temperature for 16 hours. Water was added to the reaction mixture, and extraction was performed with ethyl acetate. The organic layer was washed with water and brine, filtration was performed after drying with sodium sulfate, and the filtrate was concentrated under reduced pressure. The resulting residue was purified by silica gel column chromatography (solvent: hexane/ethyl acetate = 75/25 to 50/50) to obtain 346 mg of the title compound (yield 32%) as a colorless powder.
   MS(ESI)m/z:474[M+H]⁺

### Reference Example 76

### Production of ethyl 5-[(2R)-2[[2-(2-fluorophenyl)sulfonyl-2-azaspiro[3.3]heptan-6-yl]amino]propoxy]-1-methyl-pyrazole-4-carboxylate

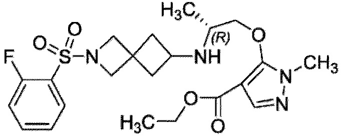

To a mixture of 577 mg of ethyl 5-[(2R)-2-(tert-butoxycarbonylamino)propoxy]-1-methyl-pyrazole-4-carboxylate and 6.00 mL of dichloromethane, 0.410 mL of trimethylsilyl trifluoromethylsulfonate was added at room temperature, the mixture was stirred as it was for 30 minutes, and then the solvent was heated and distilled off under reduced pressure. To the mixture of 6.00 mL of the residue dichloromethane, 0.320 mL of triethylamine, 570 mg of 2-(2-fluorophenyl)sulfonyl-2-azaspiro[3.3]heptan-6-one, 560 mg of sodium triacetoxyborohydride, and 0.110 mL of acetic acid were sequentially added at room temperature, and the mixture was stirred at room temperature overnight. To the reaction mixture, 20 mL of a saturated sodium bicarbonate aqueous solution was added on a water bath, and the mixture was stirred at room temperature for 10 minutes. Chloroform was added, liquid separation was performed, and extraction from the aqueous layer was performed again with chloroform. The organic layers were combined, washed with brine, and dried over magnesium sulfate. The insoluble matter was filtered off, and the filtrate was concentrated under reduced pressure. The solvent was heated and distilled off under reduced pressure, and purification was performed by silica gel column chromatography (solvent: ethyl acetate/methanol = 100/0 to 95/5). Fractions were collected and concentrated to obtain 750 mg of the title compound (yield 89%) as a pale yellow oily product.
MS(ESI)m/z:481[M+H]⁺

### Reference Examples 77 to 138:

Corresponding raw material compounds were treated in the same manner as that of Reference Example 76 to obtain compounds shown in Table 46.

**[Table 46-1]**

| Reference Example | Structural formula | Physical property value and the like |
|---|---|---|
| 77 | 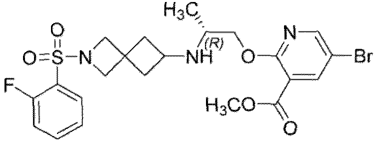 | MS(ESI) m/z;542/544 [M+H]+ |
| 78 | 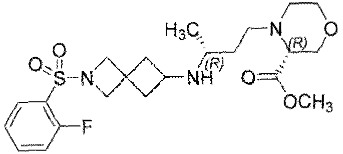 | MS(ESI) m/z;470 [M+H]+ |
| 79 | 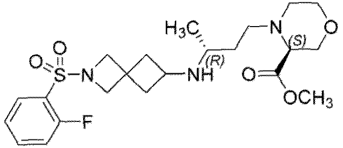 | MS(ESI) m/z;470 [M+H]+ |
| 80 | 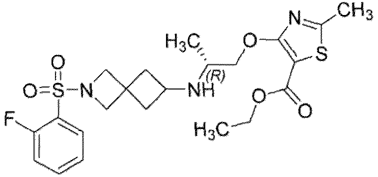 | MS(ESI) m/z;498 [M+H]+ |
| 81 | 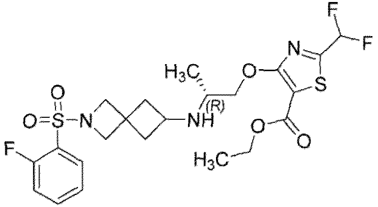 | MS(ESI) m/z;534 [M+H]+ |
| 82 | 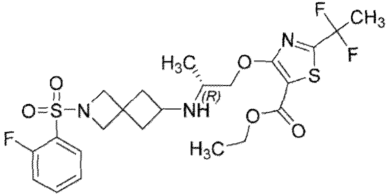 | MS(ESI) m/z; 548 [M+H]⁺ |

**[Table 46-2]**

| | | |
|---|---|---|
| 83 | 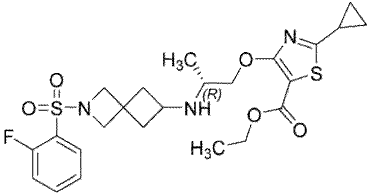 | MS(ESI) m/z;524 [M+H]+ |
| 84 | 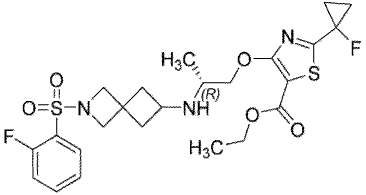 | MS(ESI) m/z;542 [M+H]+ |
| 85 | 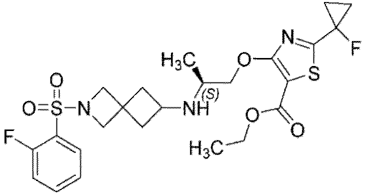 | MS(ESI) m/z;542 [M+H]+ |
| 86 | 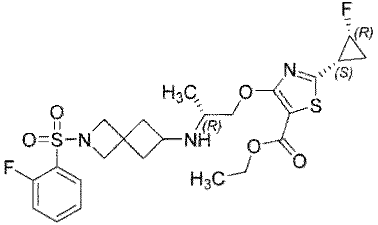 | MS(ESI) m/z;542 [M+H]+ |
| 87 | 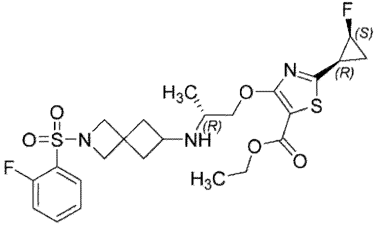 | MS(ESI) m/z;542 [M+H]+ |

**[Table 46-3]**

| | | |
|---|---|---|
| 88 | 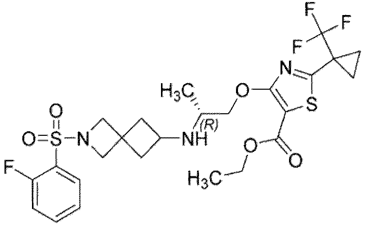 | MS(ESI) m/z; 592 [M+H]+ |
| 89 | 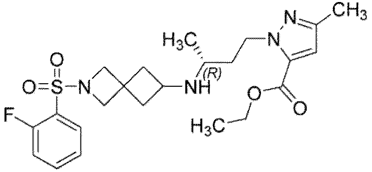 | MS(ESI) m/z;479 [M+H]+ |
| 90 | 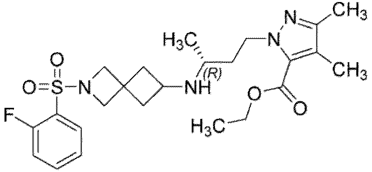 | MS(ESI) m/z;493 [M+H]+ |
| 91 | 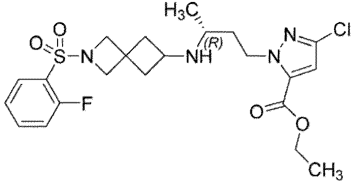 | MS(ESI) m/z;499/501 [M+H ]+ |
| 92 | 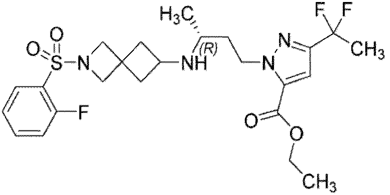 | MS(ESI) m/z;529 [M+H]+ |
| 93 | 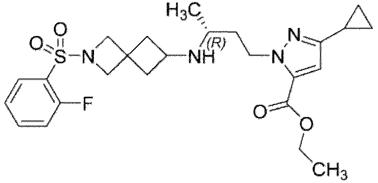 | MS(ESI) m/z;505 [M+H]+ |

**[Table 46-4]**

| | | |
|---|---|---|
| 94 | 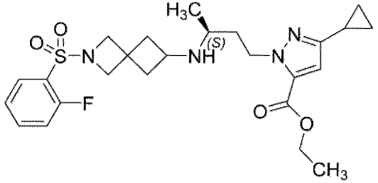 | MS(ESI) m/z; 505 [M+H]+ |
| 95 | 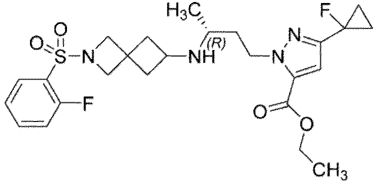 | MS(ESI) m/z;523 [M+H]+ |
| 96 | 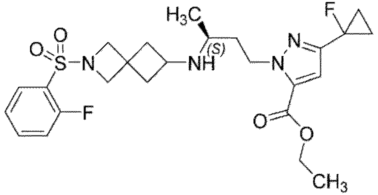 | MS(ESI) m/z;523 [M+H]+ |
| 97 | 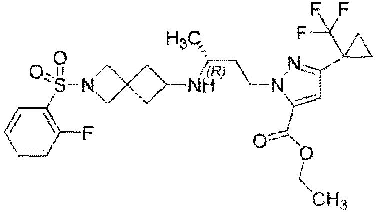 | MS(ESI) m/z;573 [M+H]+ |
| 98 | 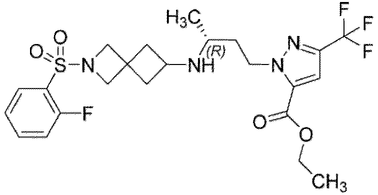 | MS(ESI) m/z;533 [M+H]+ |
| 99 | 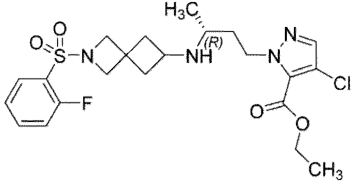 | MS(ESI) m/z;499/501 [M+H ]+ |

**[Table 46-5]**

| | | |
|---|---|---|
| 100 | 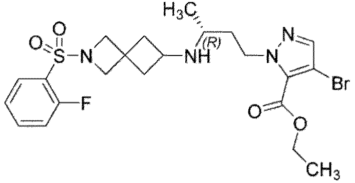 | MS(ESI) m/z;543/545 [M+H]+ |
| 101 | 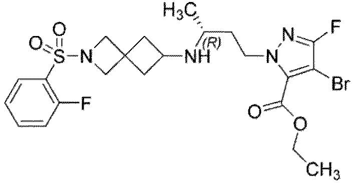 | MS(ESI) m/z;561/563 [M+H]+ |
| 102 | 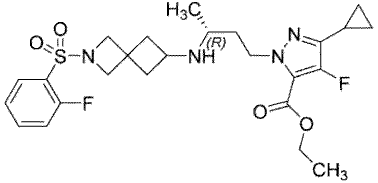 | MS(ESI) m/z;523 [M+H]+ |
| 103 | 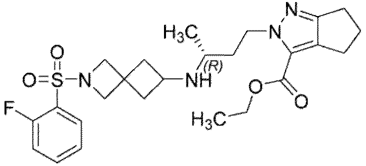 | MS(ESI) m/z;505 [M+H]+ |
| 104 | 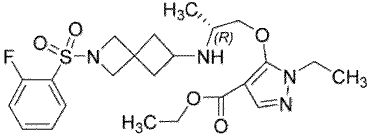 | MS(ESI) m/z;495 [M+H]+ |
| 105 | 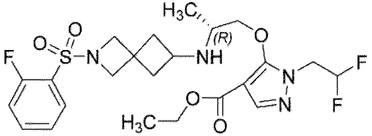 | MS(ESI) m/z;531 [M+H]+ |
| 106 | 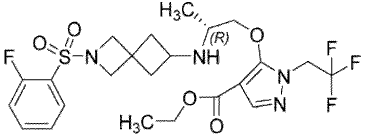 | MS(ESI) m/z;549 [M+H]+ |

**[Table 46-6]**

| | | |
|---|---|---|
| 107 | 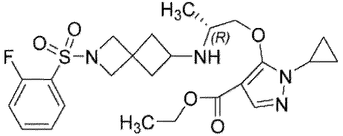 | MS(ESI) m/z;507 [M+H]⁺ |
| 108 | 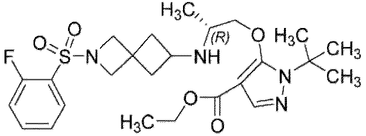 | MS(ESI) m/z;523 [M+H]+ |
| 109 | 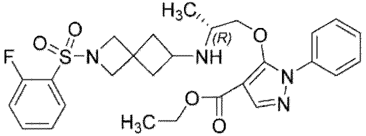 | MS(ESI) m/z;543 [M+H]+ |
| 110 | 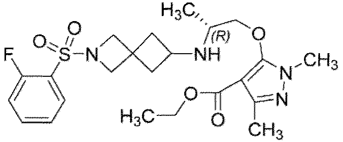 | MS(ESI) m/z;495 [M+H]+ |
| 111 | 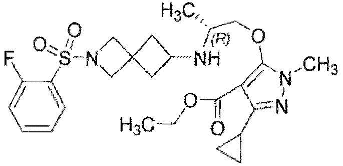 | MS(ESI) m/z;521 [M+H]+ |
| 112 | 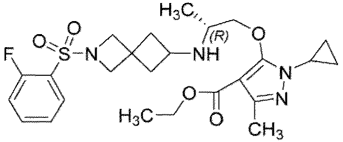 | MS(ESI) m/z;521 [M+H]+ |
| 113 | 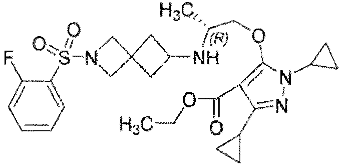 | MS(ESI) m/z;547 [M+H]+ |

**[Table 46-7]**

| | | |
|---|---|---|
| 114 | 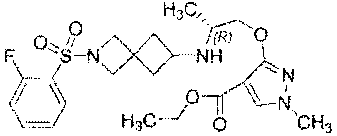 | MS(ESI) m/z;481 [M+H]+ |
| 115 | 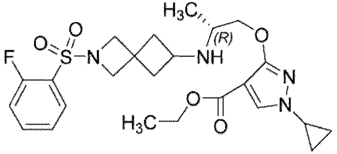 | MS(ESI) m/z;507 [M+H]+ |
| 116 | 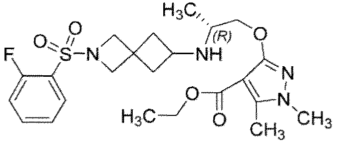 | MS(ESI) m/z;495 [M+H]+ |
| 117 | 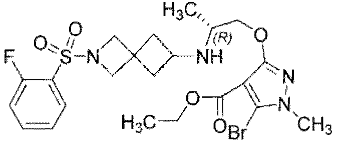 | MS(ESI) m/z;559/561 [M+H]+ |
| 118 | 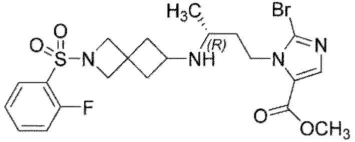 | MS(ESI) m/z;529/531 [M+H]+ |
| 119 | 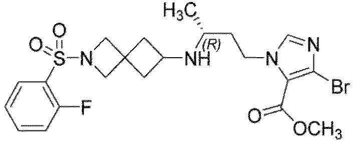 | MS(ESI) m/z;529/531 [M+H]+ |
| 120 | 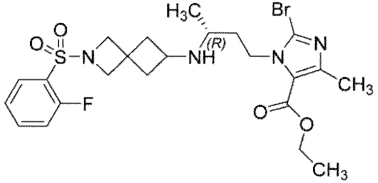 | MS(ESI) m/z;557/559 [M+H]+ |

**[Table 46-8]**

| | | |
|---|---|---|
| 121 | 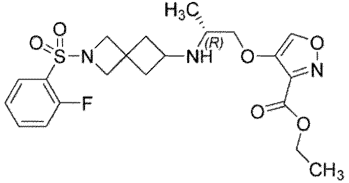 | MS(ESI) m/z;468 [M+H]+ |
| 122 | 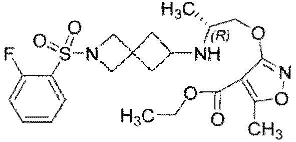 | MS(ESI) m/z;482 [M+H]+ |
| 123 | 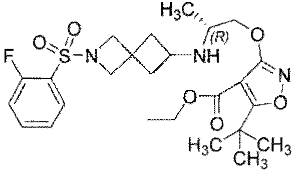 | MS(ESI) m/z;524 [M+H]+ |
| 124 | 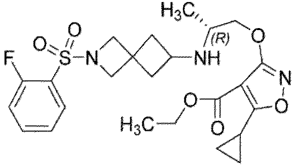 | MS(ESI) m/z;508 [M+H]+ |
| 125 | 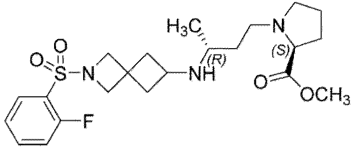 | MS(ESI) m/z;454 [M+H]+ |
| 126 | 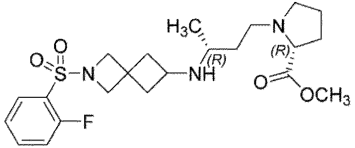 | MS(ESI) m/z;454 [M+H]+ |
| 127 | 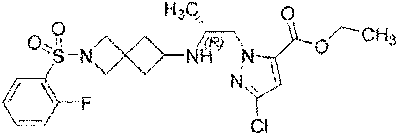 | MS(ESI) m/z;485/487 [M+H]+ |

**[Table 46-9]**

| | | |
|---|---|---|
| 128 | 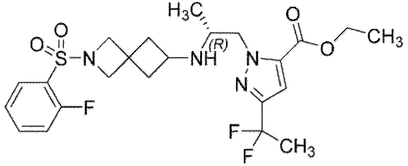 | MS(ESI) m/z;515 [M+H]+ |
| 129 | 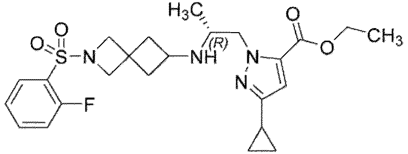 | MS(ESI) m/z;491 [M+H]+ |
| 130 | 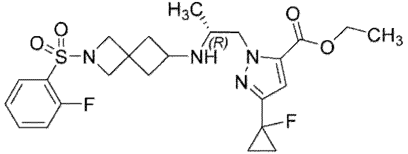 | MS(ESI) m/z;509 [M+H]+ |
| 131 | 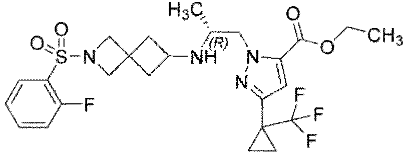 | MS(ESI) m/z;559 [M+H]+ |
| 132 | 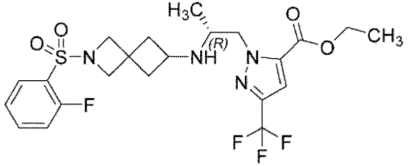 | MS(ESI) m/z;519 [M+H]+ |
| 133 | 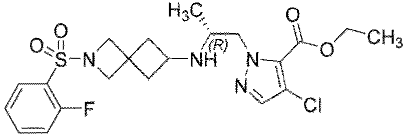 | MS(ESI) m/z;485/487 [M+H]+ |
| 134 | 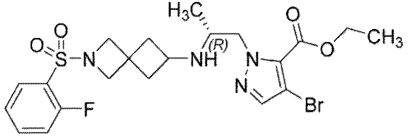 | MS(ESI) m/z;529/531 [M+H]+ |

**[Table 46-10]**

| | | |
|---|---|---|
| 135 | 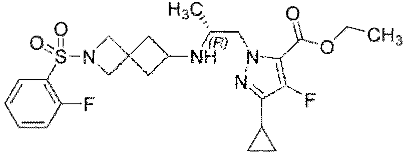 | MS(ESI) m/z;509 [M+H]+ |
| 136 | 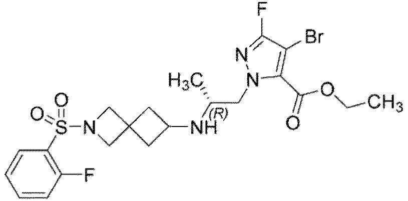 | MS(ESI) m/z;547/549 [M+H]+ |
| 137 | 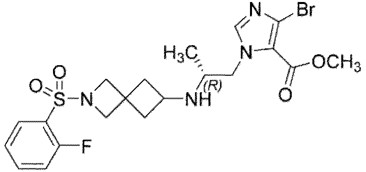 | MS(ESI) m/z;515/517 [M+H]+ |
| 138 | 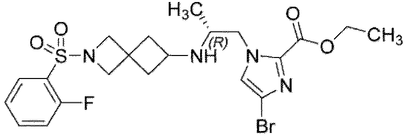 | MS(ESI) m/z;529/531 [M+H]+ |

### Reference Example 139

### Production of 1-fluorocyclopropanecarbothioamide

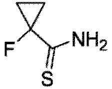

In a 300 mL eggplant flask, 2.33 g of 1-fluorocyclopropanecarboxamide and 6.56 g of Lawesson's reagent were weighed, and 50 mL of tetrahydrofuran was added. The mixture was heated, refluxed, and stirred for 3 hours. The reaction solution was cooled to room temperature, ethyl acetate and a saturated sodium bicarbonate aqueous solution were added, and then the organic layer was extracted. The aqueous layer was extracted again with ethyl acetate. The organic layers were combined, washed with brine, and then dried over magnesium sulfate. The insoluble matter was filtered, and then the solution was concentrated under reduced pressure. 5.84 g of the resulting yellow oily product was purified by NH silica gel column chromatography (solvent: hexane/ethyl acetate = 85/15 to 60/40) to obtain 2.28 g of the title compound (yield 85%) as a colorless powder.
MS(ESI)m/z:120[M+H]⁺

### Reference Examples 140 to 144:

Corresponding raw material compounds were treated in the same manner as that of Reference Example 139 to obtain compounds shown in Table 47.

**[Table 47]**

| Reference Example | Structural formula | Physical property value and the like |
|---|---|---|
| 140 | 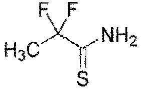 | MS(ESI) m/z;124 [M-H]- |
| 141 | 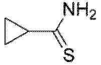 | MS(ESI) m/z;102 [M+H]+ |
| 142 | 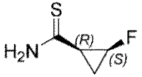 | MS(ESI) m/z;118 [M-H]- |
| 143 | 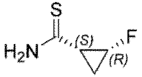 | MS(ESI) m/z;120 [M+H]+ |
| 144 | 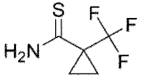 | MS(ESI) m/z;170 [M+H]+ |

### Reference Example 145

### Production of 2-(1-fluorocyclopropyl)-4-hydroxy-thiazole-5-carboxylic acid ethyl ester

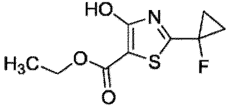

In a 200 mL eggplant flask, 1.25 g of 1-fluorocyclopropanecarbothioamide was weighed, and 20 mL of toluene and 1.84 mL of bromomalonic acid diethyl ester were added. Next, 2.5 mL of pyridine was added, and then the mixture was stirred at room temperature for 5 minutes and then stirred at 100°C for 6 hours and 30 minutes. The reaction solution was cooled to room temperature, a citric acid aqueous solution was added to set the pH to 4, and extraction was performed with ethyl acetate. The organic layer was washed with brine and then dried over magnesium sulfate. The insoluble matter was filtered, and then the solution was concentrated under reduced pressure. 2.40 g of the resulting orange oily product was purified by silica gel column chromatography (solvent: hexane/ethyl acetate = 85/15 to 50/50) to obtain 839 mg of the title compound (yield 35%) as a colorless powder.
MS(ESI)m/z:232[M+H]⁺

### Reference Examples 146 to 152:

Corresponding raw material compounds were treated in the same manner as that of Reference Example 145 to obtain compounds shown in Table 48.

**[Table 48]**

| Reference Example | Structural formula | Physical property value and the like |
|---|---|---|
| 146 | 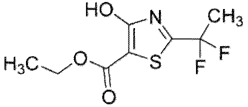 | MS(ESI) m/z;238 [M+H]+ |
| 147 | 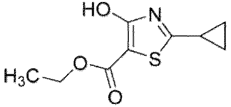 | MS(ESI) m/z;214 [M+H]+ |
| 148 | 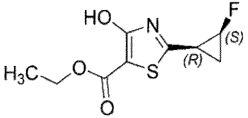 | MS(ESI) m/z;232 [M+H]+ |
| 149 | 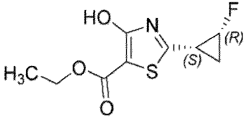 | MS(ESI) m/z;232 [M+H]+ |
| 150 | 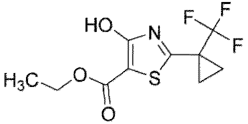 | MS(ESI) m/z;282 [M+H]+ |
| 151 | 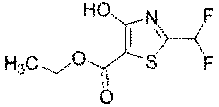 | MS(ESI) m/z;224 [M+H]+ |
| 152 | 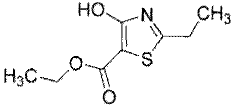 | MS(ESI) m/z;202 [M+H]+ |

### Reference Example 153

### Production of 2-(1-fluorocyclopropyl)-4-(trifluoromethylsulfonyloxy)thiazole-5-carboxylic acid ethyl ester

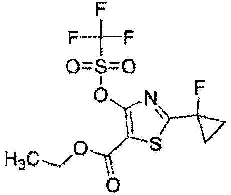

In a 100 mL eggplant flask, 764 mg of 2-(1-fluorocyclopropyl)-4-hydroxy-thiazole-5-carboxylic acid ethyl ester was weighed, 15 mL of dichloromethane and 1.20 mL of pyridine were added, and the mixture was stirred under ice-cooling. Next, 1.00 mL of trifluoromethanesulfonic anhydride was added, and the mixture was stirred at room temperature for 3 hours. The reaction solution was ice-cooled again, and a saturated ammonium chloride aqueous solution was added. Further, water was added for dilution, and extraction was performed with a mixed solvent of ethyl acetate:hexane = 1: 1. The organic layer was washed with brine and then dried over sodium sulfate. The insoluble matter was filtered, and then the solution was concentrated under reduced pressure. 1.31 g of the resulting yellow oily product was purified by silica gel column chromatography (solvent: hexane/ethyl acetate = 95/5 to 80/20) to obtain 1.02 g of the title compound (yield 85%) as a colorless powder.
MS(ESI)m/z:364[M+H]⁺

### Reference Examples 154 to 159:

Corresponding raw material compounds were treated in the same manner as that of Reference Example 153 to obtain compounds shown in Table 49.

**[Table 49]**

| Reference Example | Structural formula | Physical property value and the like |
|---|---|---|
| 154 | 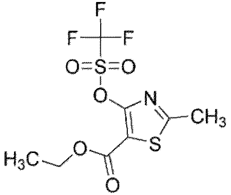 | MS(ESI) m/z;320 [M+H]+ |
| 155 | 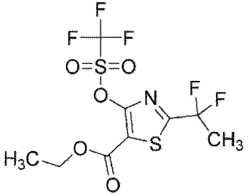 | MS(ESI) m/z;370 [M+H]+ |
| 156 | 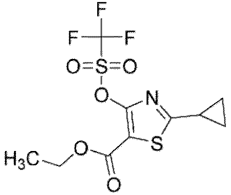 | MS(ESI) m/z;346 [M+H]+ |
| 157 | 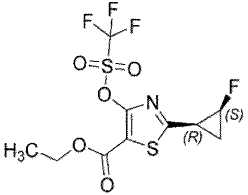 | MS(ESI) m/z;364 [M+H]+ |
| 158 | 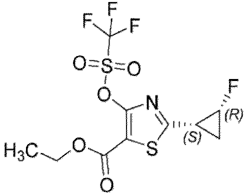 | MS(ESI) m/z;364 [M+H]+ |
| 159 | 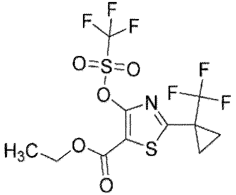 | MS(ESI) m/z;414 [M+H]+ |

### Reference Example 160

### Production of 2-(1-fluorocyclopropyl)-4-[(E)-prop-1-yl]thiazole-5-carboxylic acid ethyl ester

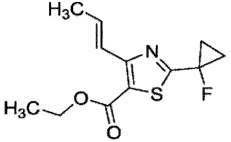

In a 100 mL eggplant flask, 1.01 g of 2-(1-fluorocyclopropyl)-4-(trifluoromethylsulfonyloxy)thiazole-5-carboxylic acid ethyl ester was weighed, and 12 mL of 1,4-dioxane was added. Next, 644 mg of potassium (E)-propenyl-1-trifluoroborate, 378 mg of tetrakis triphenylphosphine palladium, and 3.50 mL of a 2 mol/L sodium carbonate aqueous solution were added, and the mixture was stirred at 100°C for 75 minutes. The reaction solution was cooled to room temperature, ethyl acetate and brine were added, and then the organic layer was extracted. The organic layer was washed with brine and then dried over magnesium sulfate. The insoluble matter was filtered, and then the solution was concentrated under reduced pressure. 1.02 g of the resulting orange oily product was purified by silica gel column chromatography (solvent: hexane/ethyl acetate = 100/0 to 80/20) to obtain 671 mg of the title compound (yield 95%) as a colorless powder.
MS(ESI)m/z:256[M+H]⁺

### Reference Examples 161 to 184:

Corresponding raw material compounds were treated in the same manner as that of Reference Example 160 to obtain compounds shown in Table 50.

**[Table 50-1]**

| Reference Example | Structural formula | Physical property value and the like |
|---|---|---|
| 161 | 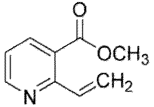 | MS(ESI) m/z;164 [M+H]+ |
| 162 | 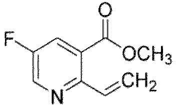 | MS(ESI) m/z;182 [M+H]+ |
| 163 | 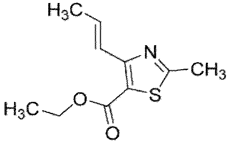 | MS(ESI) m/z;212 [M+H]+ |
| 164 | 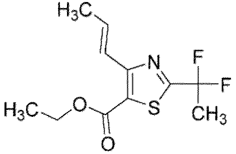 | MS(ESI) m/z;262 [M+H]+ |
| 165 | 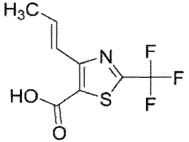 | MS(ESI) m/z;238 [M+H]+ |
| 166 | 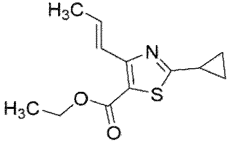 | MS(ESI) m/z;238 [M+H]+ |
| 167 | 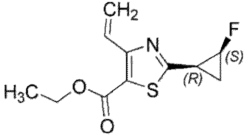 | MS(ESI) m/z;242 [M+H]+ |
| 168 | 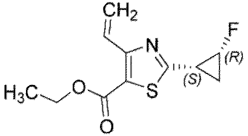 | MS(ESI) m/z;242 [M+H]+ |

**[Table 50-2]**

| | | |
|---|---|---|
| 169 | 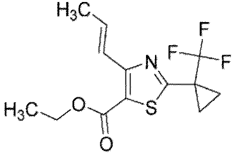 | MS(ESI) m/z;306 [M+H]+ |
| 170 | 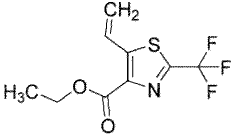 | MS(ESI) m/z;252 [M+H]+ |
| 180 | 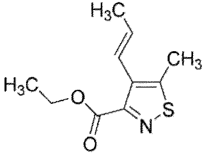 | MS(ESI) m/z;212 [M+H]+ |
| 181 | 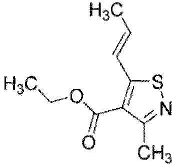 | MS(ESI) m/z;212 [M+H]+ |
| 182 | 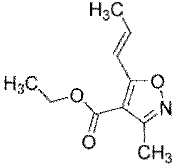 | MS(ESI) m/z;196 [M+H]+ |
| 183 | 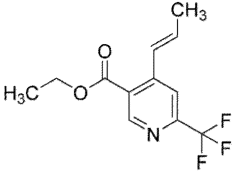 | MS(ESI) m/z;260 [M+H]+ |
| 184 | 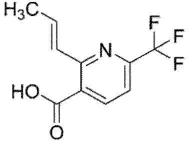 | MS(ESI) m/z;232 [M+H]+ |

### Reference Example 185

### Production of 2-(1-fluorocyclopropyl)-4-[(E)-prop-1-enyl]thiazole-5-carboxylic acid

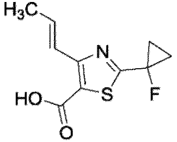

In a 100 mL eggplant flask, 665 mg of 2-(1-fluorocyclopropyl)-4-[(E)-prop-1-enyl]thiazole-5-carboxylic acid ethyl ester was weighed, and 7.0 mL of methanol and 3.0 mL of tetrahydrofuran were added. Next, 6.0 mL of a 1 mol/L sodium hydroxide aqueous solution was added, and the mixture was stirred at room temperature for 150 minutes. 6.0 mL of 1 mol/L hydrochloric acid was added to the reaction solution, about 10 mL of a solvent was distilled off with an evaporator, and then 2.0 mL of 1 mol/L hydrochloric acid and water were added to set the pH to 4. The precipitated solid was collected by filtration, washed with water, and then dried under reduced pressure, thereby obtaining 591 mg of the title compound (yield 100%) as a colorless powder.
MS(ESI)m/z:228[M+H]⁺

### Reference Examples 186 to 198:

Corresponding raw material compounds were treated in the same manner as that of Reference Example 185 to obtain compounds shown in Table 51.

**[Table 51-1]**

| Reference Example | Structural formula | Physical property value and the like |
|---|---|---|
| 186 | 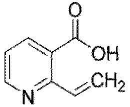 | MS(ESI) m/z;150 [M+H]+ |
| 187 | 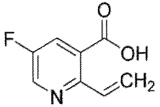 | MS(ESI) m/z;168 [M+H]⁺ |
| 188 | 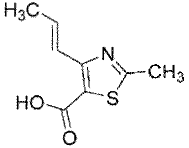 | MS(ESI) m/z;184 [M+H]+ |
| 189 | 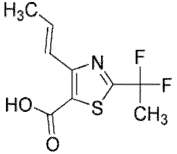 | MS(ESI) m/z;234 [M+H]+ |
| 190 | 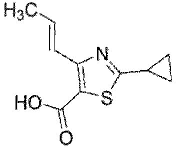 | MS(ESI) m/z;210 [M+H]+ |
| 191 | 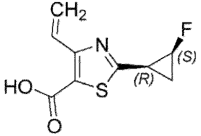 | MS(ESI) m/z;214 [M+H]+ |
| 192 | 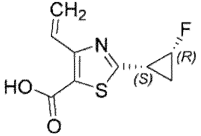 | MS(ESI) m/z;214 [M+H]+ |
| 193 | 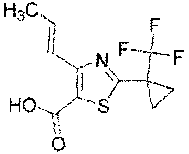 | MS(ESI) m/z;278 [M+H]+ |

**[Table 51-2]**

| | | |
|---|---|---|
| 194 | 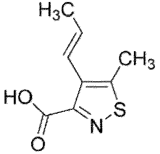 | MS(ESI) m/z;184 [M+H]+ |
| 195 | 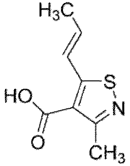 | MS(ESI) m/z;184 [M+H]+ |
| 196 | 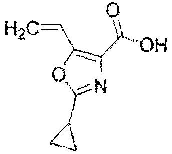 | MS(ESI) m/z;180 [M+H]+ |
| 197 | 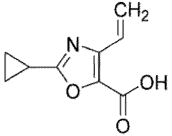 | MS(ESI) m/z;180 [M+H]+ |
| 198 | 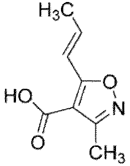 | MS(ESI) m/z;168 [M+H]+ |

### Reference Example 199

### Production of ethyl 3-(1-fluorocyclopropyl)-1H-pyrazole-5-carboxylate

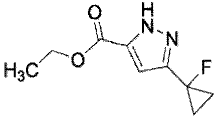

(1) Under ice-cooling, 1.18 g of sodium hydride (60%) was suspended in 20 mL of tetrahydrofuran, and 1,500 mg of 1-(1- fluorocyclopropyl)ethanone was added dropwise thereto. The mixture was stirred under ice-cooling for 0.5 hours. 4.39 g of diethyl oxalate was added to the reaction solution under ice-cooling. The temperature was raised to room temperature, and the mixture was stirred for 1.5 hours. Water was added to the reaction mixture, and extraction was performed with ethyl acetate. A 1 mol/L hydrochloric acid aqueous solution was added to the aqueous layer, and extraction was performed with ethyl acetate. The organic layers were combined and washed with brine, filtration was performed after drying with sodium sulfate, and the filtrate was concentrated under reduced pressure. The resulting residue was purified by silica gel column chromatography (solvent: hexane/ethyl acetate = 85/15 to 60/40) to obtain 2.70 g of ethyl 4-(1-fluorocyclopropyl)-2,4-dioxo-butanoate (yield 91%) as a brown oily product.
   MS(ESI)m/z:203[M+H]⁺
(2) 2.70 g of the ethyl 4-(1-fluorocyclopropyl)-2,4-dioxo-butanoate obtained in (1) above was dissolved in 30 mL of ethanol, and 661 mg of a hydrazine monohydrate was added. The mixture was stirred at 60°C for 1 hour. The reaction solution was concentrated so that a solution amount was about 1/3, water was added, and extraction was performed with ethyl acetate. The organic layer was washed with brine, filtration was performed after drying with sodium sulfate, and the filtrate was concentrated under reduced pressure. The resulting residue was purified by silica gel column chromatography (solvent: hexane/ethyl acetate = 70/30 to 45/55) to obtain 1.99 g of the title compound (yield 76%) as a light yellow powder.
   MS(ESI)m/z: 199[M+H]⁺

### Reference Example 200

### Production of ethyl 3-[1-(trifluoromethyl)cyclopropyl]-1H-pyrazole-5-carboxylate

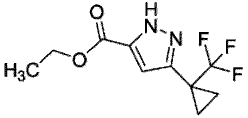

(1) 1,000 mg of N-methoxy-N-methyl-1-(trifluoromethyl)cyclopropanecarboxamide was dissolved in 10 mL of tetrahydrofuran, and 5.07 mL of methylmagnesium bromide (3 mol/L diethyl ether solution) was added dropwise under ice-cooling. The temperature was raised to room temperature, and the mixture was stirred for 1 hour. A saturated ammonium chloride aqueous solution was added to the reaction mixture, and extraction was performed with diethyl ether. The organic layer was washed with brine, filtration was performed after drying with sodium sulfate, and the filtrate was concentrated under reduced pressure at 350 Mbar. When a small amount of solution was remained, concentration was stopped, thereby obtaining a crude product of 1-[1-(trifluoromethyl)cyclopropyl)]ethanone.
(2) 406 mg of sodium hydride (60%) was suspended in 15 mL of tetrahydrofuran, and the crude product of 1-[1-(trifluoromethyl)cyclopropyl)]ethanone produced in (1) above was added under ice-cooling. The mixture was stirred as it was under for 0.5 hours. 1.11 g of diethyl oxalate was added to the reaction solution under ice-cooling. The temperature was raised to room temperature, and the mixture was stirred for 1.5 hours. A saturated ammonium chloride aqueous solution was added to the reaction mixture, and extraction was performed twice with ethyl acetate. The organic layers were combined and brine and then dried over magnesium sulfate, filtration was performed, and then the filtrate was concentrated under reduced pressure, thereby obtaining a crude product of ethyl 2,4-dioxo-4-[1-(trifluoromethyl)cyclopropyl]butanoate.
(3) The crude product of ethyl 2,4-dioxo-4-[1-(trifluoromethyl)cyclopropyl]butanoate obtained in (2) above was dissolved in 10 mL of ethanol, 254 mg of hydrazine monohydrate was added, and the mixture was stirred at 50°C for 0.5 hours. The insoluble matter was filtered off, water was added to the filtrate, and then extraction was performed with ethyl acetate. The organic layer was washed with brine, filtration was performed after drying with magnesium sulfate, and the filtrate was concentrated under reduced pressure. The resulting residue was purified by silica gel column chromatography (solvent: hexane/ethyl acetate = 80/20 to 65/35) to obtain 330 mg of the title compound (three stage yield 26%) as a colorless powder.
   MS(ESI)m/z:249[M+H]⁺

### Reference Example 201

### Production of ethyl 5-hydroxy-1-methyl-pyrazole-4-carboxylate

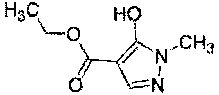

710 mg of methylhydrazine was added at 0°C to a mixture of 3.00 g of diethyl ethoxymethylene malonate and 15.0 mL of ethanol, and the mixture was stirred at room temperature for 2 hours and further heated and refluxed for 3 hours. After cooling to room temperature, the solvent was heated and distilled off under reduced pressure, and purification was performed by silica gel column chromatography (solvent: chloroform/methanol = 100/0 to 95/5). Fractions were collected and concentrated to obtain 364 mg of the title compound (yield 15%) as a colorless powder.
MS(ESI)m/z:171[M+H]⁺

### Reference Example 202

### Production of ethyl 1-ethyl-5-hydroxy-pyrazole-4-carboxylate

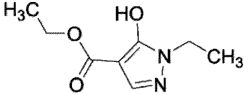

To a mixture of 3.00 g of diethyl ethoxymethylene malonate and 30.0 mL of ethanol, 920 mg of ethyl hydrazine was added at 0°C, and the mixture was stirred as it was for 1 hour. Further, the mixture was heated and refluxed for 1 hour. After cooling to room temperature, 2.30 g of potassium carbonate was added, and the mixture was heated and refluxed for 1 hour. After cooling to room temperature, neutralization was performed with 1 N hydrochloric acid, water and chloroform were added to the reaction mixture, liquid separation was performed, and extraction from the aqueous layer was performed again with chloroform. The organic layers were combined, washed with brine, and dried over magnesium sulfate. The insoluble matter was filtered off, and the filtrate was concentrated under reduced pressure. The residue was suspended and washed with IPE, and a solid was collected by filtration. The resulting solid was dried under reduced pressure to obtain 630 mg of the title compound (yield 25%) as a colorless powder.
MS(ESI)m/z:185[M+H]⁺

### Reference Examples 203 to 206:

Corresponding raw material compounds were treated in the same manner as that of Reference Example 202 to obtain compounds shown in Table 52.

**[Table 52]**

| Reference Example | Structural formula | Physical property value and the like |
|---|---|---|
| 203 | 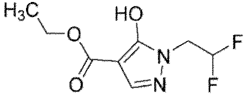 | MS(ESI) m/z;221 [M+H]+ |
| 204 | 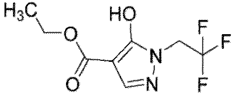 | MS(ESI) m/z;239 [M+H]+ |
| 205 | 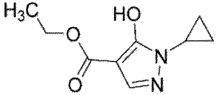 | MS(ESI) m/z;197 [M+H]+ |
| 206 | 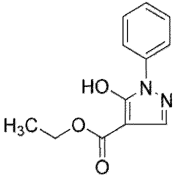 | MS(ESI) m/z;233 [M+H]+ |

### Reference Example 207

### Production of ethyl 5-hydroxy-1,3-dimethyl-pyrazole-4-carboxylate

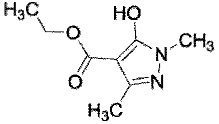

(1) To a mixture of 15.0 g of ethyl malonate and 150 mL of acetonitrile, 9.00 g of magnesium chloride was added at room temperature. After cooling to 0°C, and 19.0 g of triethylamine was added in several times. The mixture was stirred at 0°C for 15 minutes, 7.70 g of acetyl chloride was added thereto, and the mixture was stirred at room temperature overnight. Cooling was performed to 0°C, 6 N hydrochloric acid was added for acidification, water and ethyl acetate were added to the reaction mixture, liquid separation was performed, and then extraction from the aqueous layer was performed again with ethyl acetate. The organic layers were combined, washed with brine, and dried over magnesium sulfate. The insoluble matter was removed by filtration, and the filtrate was concentrated under reduced pressure, thereby obtaining 18.8 g of diethyl 2-acetylpropanedioate (99% yield) as a pale yellow oily product.
(2) To a mixture of 18.8 g of diethyl 2-acetylpropanedioate and 200 mL of DMF, 16.7 g of potassium carbonate was added at room temperature, and the mixture was stirred at room temperature for 30 minutes. 18.6 g of dimethyl sulfate was added at room temperature, and the mixture was stirred for 3 days. To the reaction mixture, a saturated ammonium chloride aqueous solution (100 mL) was added. Ethyl acetate was added, liquid separation was performed, and then extraction from the aqueous layer was performed again with ethyl acetate. The organic layers were combined, washed with brine, and dried over magnesium sulfate. The insoluble matter was filtered off, and the filtrate was concentrated under reduced pressure. The resulting residue was purified by silica gel column chromatography (solvent: hexane/ethyl acetate = 90/10 to 70/30). Fractions were collected and concentrated to obtain 21.4 g of diethyl 2-(1-ethoxyethylidene)propanedioate (66% yield) as a colorless oily product.
(3) To a mixture of 3.00 g of diethyl 2-(1-ethoxyethylidene)propanedioate and 15.0 mL of ethanol, 660 mg of methyl hydrazine was added at 0°C. The mixture was stirred at room temperature for 2 hours and further heated and refluxed for 3 hours. The temperature was cooled to room temperature, and then the mixture was concentrated under reduced pressure. The residue was suspended and washed with ethyl acetate, and a solid was collected by filtration. The resulting solid was dried under reduced pressure to obtain 756 mg of the title compound (yield 32%) as a colorless powder.
   MS(ESI)m/z:185[M+H]⁺

### Reference Example 208:

A corresponding raw material compound was treated in the same manner as that of Reference Example 207 to obtain a compound shown in Table 53.

**[Table 53]**

| Reference Example | Structural formula | Physical property value and the like |
|---|---|---|
| 208 | 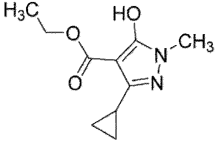 | MS (ESI) m/z;211 [M+H]+ |

### Reference Example 209

### Ethyl 1-cyclopropyl-5-hydroxy-3-methyl-pyrazole-4-carboxylate

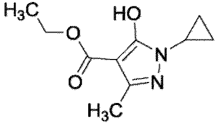

(1) To a mixture of 15.0 g of diethyl malonate and 150 mL of acetonitrile, 9.00 g of magnesium chloride was added at room temperature. After cooling to 0°C, and 19.0 g of triethylamine was added in several times. The mixture was stirred at 0°C for 15 minutes, 7.70 g of acetyl chloride was added thereto, and the mixture was stirred at room temperature overnight. Cooling was performed to 0°C, 6 N hydrochloric acid was added for acidification, water and ethyl acetate were added to the reaction mixture, liquid separation was performed, and then extraction from the aqueous layer was performed again with ethyl acetate. The organic layers were combined, washed with brine, and dried over magnesium sulfate. The insoluble matter was removed by filtration, and the filtrate was concentrated under reduced pressure, thereby obtaining 18.8 g of diethyl 2-acetylpropanedioate (99% yield) as a pale yellow oily product.
(2) To a mixture of 18.8 g of diethyl 2-acetylpropanedioate and 200 mL of DMF, 16.7 g of potassium carbonate was added at room temperature, and the mixture was stirred at room temperature for 30 minutes. 18.6 g of dimethyl sulfate was added at room temperature, and the mixture was stirred for 3 days. To the reaction mixture, a saturated ammonium chloride aqueous solution (100 mL) was added. Ethyl acetate was added, liquid separation was performed, and then extraction from the aqueous layer was performed again with ethyl acetate. The organic layers were combined, washed with brine, and dried over magnesium sulfate. The insoluble matter was filtered off, and the filtrate was concentrated under reduced pressure. The solvent was heated and distilled off under reduced pressure, and purification was performed by silica gel column chromatography (solvent: hexane/ethyl acetate = 90/10 to 70/30). Fractions were collected and concentrated to obtain 21.4 g of diethyl 2-(1-ethoxyethylidene)propanedioate (66% yield) as a colorless oily product.
(3) To a mixture of 2.00 g of diethyl 2-(1-ethoxyethylidene)propanedioate and 20.0 mL of ethanol, 10.6 g of a 20% sodium ethoxide ethanol solution and 1.13 g of cyclopropyl hydrazine hydrochloride were added at room temperature, and the mixture was heated and stirred at 100°C for 1 hour. Cooling was performed to 0°C, 1 N hydrochloric acid was added for hydrochloric acidity, water and ethyl acetate were added to the reaction mixture, liquid separation was performed, and then extraction from the aqueous layer was performed again with ethyl acetate. The organic layers were combined, washed with brine, and dried over magnesium sulfate. The insoluble matter was filtered off, and the filtrate was concentrated under reduced pressure. The residue was suspended and washed with diisopropyl ether, and a solid was collected by filtration. The resulting solid was dried under reduced pressure to obtain 183 mg of the title compound (yield 10%) as a pale yellow powder.
   MS(ESI)m/z:211[M+H]⁺

### Reference Examples 210 and 211:

Corresponding raw material compounds were treated in the same manner as that of Reference Example 209 to obtain compounds shown in Table 54.

**[Table 54]**

| Reference Example | Structural formula | Physical property value and the like |
|---|---|---|
| 210 | 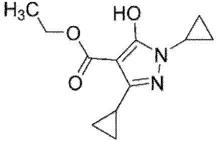 | MS(ESI) m/z;237 [M+H]+ |
| 211 | 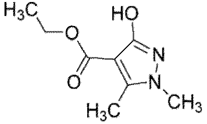 | MS(ESI) m/z;185 [M+H]+ |

### Reference Example 212

### Ethyl 1-cyclopropyl-3-hydroxy-pyrazole-4-carboxylate

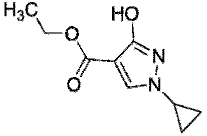

To a mixture of 1.00 g of diethyl ethoxymethylenemalonate and 10.0 mL of ethanol, 4.80 g of a 20% sodium ethoxide ethanol solution and 500 mg of cyclopropyl hydrazine hydrochloride were added at room temperature, and the mixture was heated and stirred at 100°C for 30 minutes. Cooling was performed to 0°C, 1 N hydrochloric acid was added for acidification, water and ethyl acetate were added to the reaction mixture, liquid separation was performed, and then extraction from the aqueous layer was performed again with ethyl acetate. The organic layers were combined, washed with brine, and dried over magnesium sulfate. The insoluble matter was filtered off, and the filtrate was concentrated under reduced pressure. The residue was suspended and washed with diisopropyl ether, and a solid was collected by filtration. The resulting solid was dried under reduced pressure to obtain 312 mg of the title compound (yield 34%) as a colorless powder.
MS(ESI)m/z:197[M+H]⁺

### Reference Example 213

### Ethyl 1-tert-butyl-5-hydroxy-pyrazole-4-carboxylate

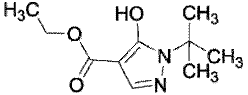

To a mixture of 3.00 g of diethyl ethoxymethylenemalonate and 15.0 mL of ethanol, 1.90 g of t-butylhydrazine hydrochloride and 7.70 g of potassium carbonate were added at 0°C, and the mixture was stirred at room temperature for 1 hour. Further, the mixture was heated and refluxed for 1 hour. After cooling to room temperature, the solvent was heated and distilled off under reduced pressure. 30.0 mL of DMF and 3.90 g of potassium carbonate were added to the residue, and the mixture was stirred at 100°C for 1 hour. Subsequently, the mixture was heated and stirred at 180°C for 1 hour. After cooling to room temperature, neutralization was performed with 1 N hydrochloric acid, water and ethyl acetate were added to the reaction mixture, liquid separation was performed, and extraction from the aqueous layer was performed again with ethyl acetate. The organic layers were combined, washed with brine, and dried over magnesium sulfate. The insoluble matter was filtered off, and the filtrate was concentrated under reduced pressure. The solvent was heated and distilled off under reduced pressure, and purification was performed by silica gel column chromatography (solvent: hexane/ethyl acetate = 90/10 to 70/30). Fractions were collected and concentrated to obtain 1.71 g of the title compound (yield 58%) as a brown oily product.
MS(ESI)m/z:213[M+H]⁺

### Reference Example 214

### Ethyl 3-hydroxy-5-methyl-isoxazole-4-carboxylate

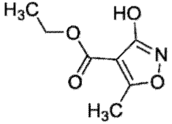

(1) To a mixture of 15.0 g of diethyl malonate and 150 mL of acetonitrile, 9.00 g of magnesium chloride was added at room temperature. After cooling to 0°C, and 19.0 g of triethylamine was added in several times. The mixture was stirred at 0°C for 15 minutes, 7.70 g of acetyl chloride was added thereto, and the mixture was stirred at room temperature overnight. Cooling was performed to 0°C, 6 N hydrochloric acid was added for acidification, water and ethyl acetate were added to the reaction mixture, liquid separation was performed, and then extraction from the aqueous layer was performed again with ethyl acetate. The organic layers were combined, washed with brine, and dried over magnesium sulfate. The insoluble matter was removed by filtration, and the filtrate was concentrated under reduced pressure, thereby obtaining 18.8 g of diethyl 2-acetylpropanedioate (99% yield) as a pale yellow oily product.
(2) To a mixture of 18.8 g of diethyl 2-acetylpropanedioate and 200 mL of DMF, 16.7 g of potassium carbonate was added at room temperature, and the mixture was stirred at room temperature for 30 minutes. 18.6 g of dimethyl sulfate was added at room temperature, and the mixture was stirred for 3 days. To the reaction mixture, a saturated ammonium chloride aqueous solution (100 mL) was added. Ethyl acetate was added, liquid separation was performed, and then extraction from the aqueous layer was performed again with ethyl acetate. The organic layers were combined, washed with brine, and dried over magnesium sulfate. The insoluble matter was filtered off, and the filtrate was concentrated under reduced pressure. The solvent was heated and distilled off under reduced pressure, and purification was performed by silica gel column chromatography (solvent: hexane/ethyl acetate = 90/10 to 70/30). Fractions were collected and concentrated to obtain 21.4 g of diethyl 2-(1-ethoxyethylidene)propanedioate (66% yield) as a colorless oily product.
(3) To a mixture of 2.00 g of diethyl 2-(1-ethoxyethylidene)propanedioate and 20.0 mL of ethanol, 9.20 g of a 20% sodium ethoxide ethanol solution and 850 mg of hydroxylamine hydrochloride were added at room temperature, and the mixture was stirred at room temperature overnight. Cooling was performed to 0°C, 1 N hydrochloric acid was added for acidification, water and ethyl acetate were added to the reaction mixture, liquid separation was performed, and then extraction from the aqueous layer was performed again with ethyl acetate. The organic layers were combined, washed with brine, and dried over magnesium sulfate. The insoluble matter was filtered off, and the filtrate was concentrated under reduced pressure. The solvent was heated and distilled off under reduced pressure, and purification was performed by silica gel column chromatography (solvent: chloroform/methanol = 100/0 to 95/5). Fractions were collected and concentrated to obtain 720 mg of the title compound (yield 48%) as a pale yellow solid.
   MS(ESI)m/z:172[M+H]⁺

### Reference Examples 215 and 216:

Corresponding raw material compounds were treated in the same manner as that of Reference Example 214 to obtain compounds shown in Table 55.

**[Table 55]**

| Reference Example | Structural formula | Physical property value and the like |
|---|---|---|
| 215 | 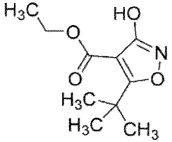 | MS(ESI) m/z;214 [M+H]+ |
| 216 | 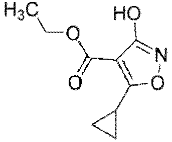 | MS(ESI) m/z;198 [M+H]+ |

### Reference Example 217

### Production of ethyl 5-[(2R)-2-(tert-butoxycarbonylamino)propoxy]-1-methylpyrazole-4-carboxylate

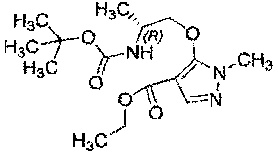

To a mixture of 300 mg of ethyl 5-hydroxy-1-methyl-pyrazole-4-carboxylate and 6.00 mL of tetrahydrofuran, 380 mg of tert-butyl N-[(1R)-2-hydroxy-1-methylethyl]carbamate and 930 mg of triphenylphosphine were added at room temperature. Further, 1.60 mL of a 2.2 mol/L toluene solution of diethyl azodicarboxylate was added at room temperature. The reaction mixture was stirred at room temperature for 1 hour. The filtrate was concentrated under reduced pressure, and the resulting residue was purified by silica gel column chromatography (solvent: hexane/ethyl acetate = 70/30 to 50/50). Fractions were collected and concentrated to obtain 883 mg of the title compound (yield 153%, mixture) as a pale yellow oily product.
MS(ESI)m/z:328[M+H]⁺

### Reference Examples 218 to 286:

Corresponding raw material compounds were treated in the same manner as that of Reference Example 217 to obtain compounds shown in Table 56.

**[Table 56-1]**

| Reference Example | Structural formula | Physical property value and the like |
|---|---|---|
| 218 | 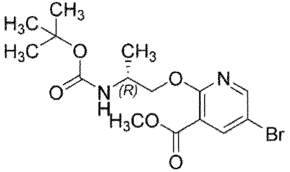 | MS(ESI) m/z;289/291 [M-Boc+H] + |
| 219 | 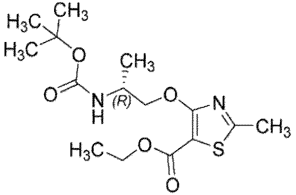 | MS(ESI) m/z;345 [M+H]+ |
| 220 | 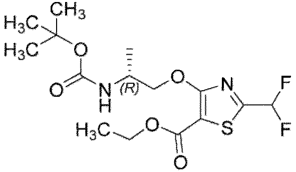 | MS(ESI) m/z;281 [M-Boc+H]+ |
| 221 | 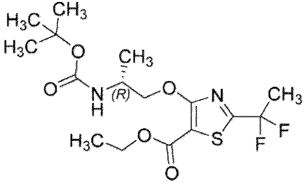 | MS(ESI) m/z;295 [M-Boc+H]+ |
| 222 | 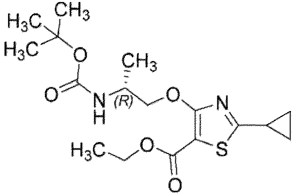 | MS(ESI) m/z;371 [M+H]+ |
| 223 | 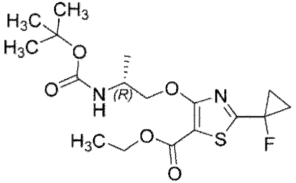 | MS(ESI) m/z;389 [M+H]+ |

**[Table 56-2]**

| | | |
|---|---|---|
| 224 | 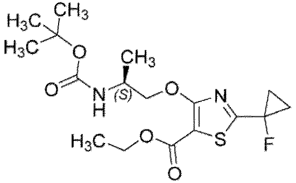 | MS(ESI) m/z;389 [M+H]+ |
| 225 | 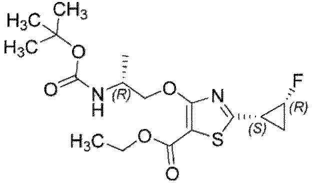 | MS(ESI) m/z;389 [M+H]+ |
| 226 | 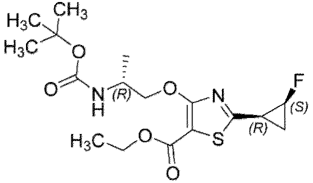 | MS(ESI) m/z;389 [M+H]+ |
| 227 | 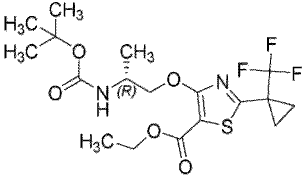 | MS(ESI) m/z;439 [M+H]+ |
| 228 | 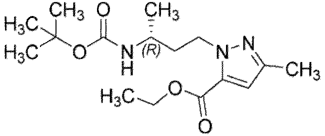 | MS(ESI) m/z;326 [M+H]+ |
| 229 | 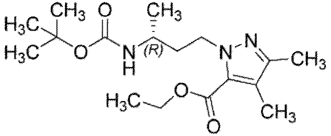 | MS(ESI) m/z;340 [M+H]+ |
| 230 | 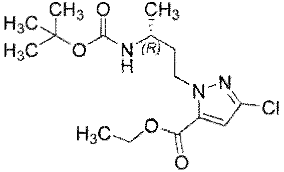 | MS(ESI) m/z;246/248 [M-Boc+H]+ |

**[Table 56-3]**

| | | |
|---|---|---|
| 231 | 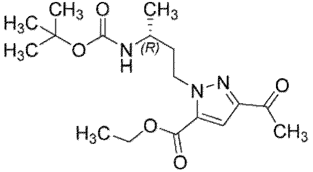 | MS(ESI) m/z;298 [M-tBu+H]+ |
| 232 | 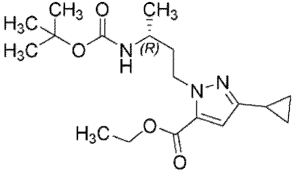 | MS(ESI) m/z;352 [M+H]+ |
| 233 | 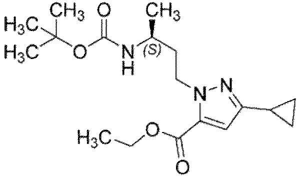 | MS(ESI) m/z;352 [M+H]+ |
| 234 | 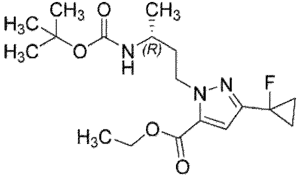 | MS(ESI) m/z;370 [M+H]+ |
| 235 | 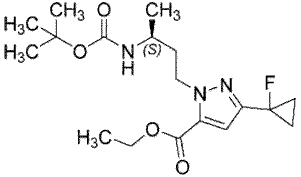 | MS(ESI) m/z;370 [M+H]+ |
| 236 | 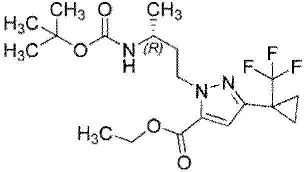 | MS(ESI) m/z;420 [M+H]+ |

**[Table 56-4]**

| | | |
|---|---|---|
| 237 | 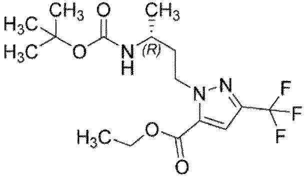 | MS(ESI) m/z;280 [M-Boc+H]+ |
| 238 | 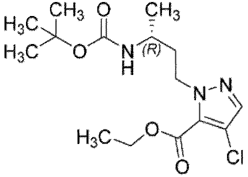 | MS(ESI) m/z;346/348 [M+H]+ |
| 239 | 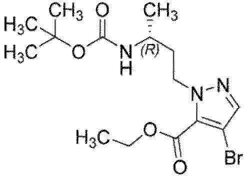 | MS(ESI) m/z;290/292 [M-Boc+H] + |
| 240 | 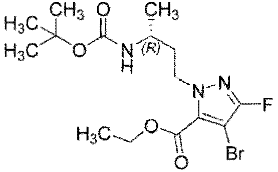 | MS(ESI) m/z;308/310 [M-Boc+H] + |
| 241 | 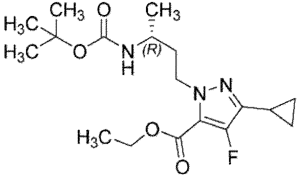 | MS(ESI) m/z;370 [M+H]+ |
| 243 | 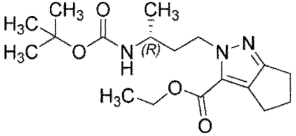 | MS(ESI) m/z;352 [M+H]+ |
| 244 | 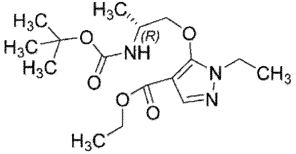 | MS(ESI) m/z; 342 [M+H]+ |

**[Table 56-5]**

| | | |
|---|---|---|
| 245 | 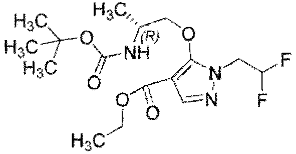 | MS(ESI) m/z;378 [M+H]+ |
| 246 | 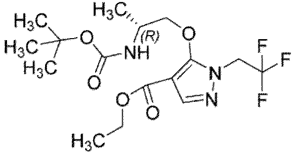 | MS(ESI) m/z;396 [M+H]+ |
| 247 | 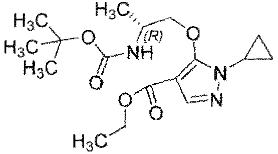 | MS(ESI) m/z;354 [M+H]+ |
| 248 | 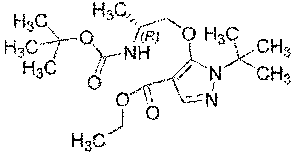 | MS(ESI) m/z;370 [M+H]+ |
| 249 | 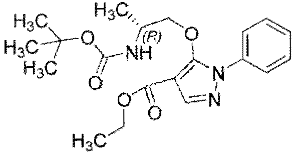 | MS(ESI) m/z;390 [M+H]+ |
| 230 | 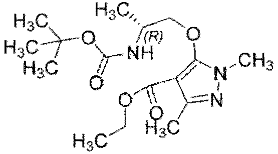 | MS(ESI) m/z;342 [M+H]+ |
| 251 | 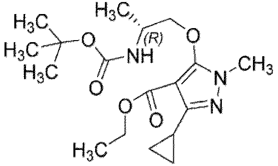 | MS(ESI) m/z;368 [M+H]+ |

**[Table 56-6]**

| | | |
|---|---|---|
| 252 | 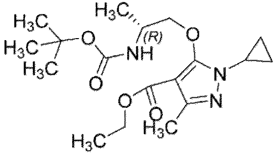 | MS(ESI) m/z;368 [M+H]+ |
| 253 | 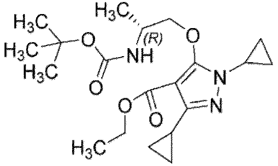 | MS(ESI) m/z;394 [M+H]+ |
| 254 | 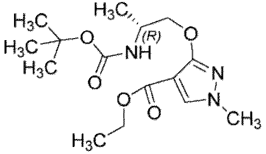 | MS(ESI) m/z;328 [M+H]+ |
| 255 | 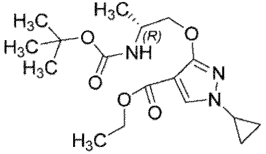 | MS(ESI) m/z;354 [M+H]+ |
| 256 | 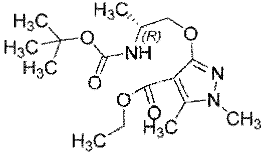 | MS(ESI) m/z;342 [M+H]+ |
| 257 | 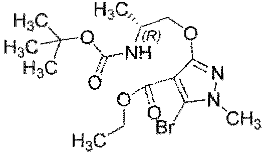 | MS(ESI) m/z;406/408 [M+H]+ |
| 258 | 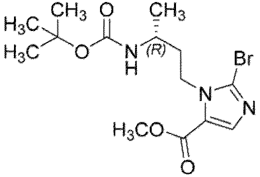 | MS(ESI) m/z;376/378 [M+H]+ |

**[Table 56-7]**

| | | |
|---|---|---|
| 259 | 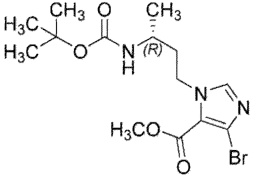 | MS(ESI) m/z;376/378 [M+H]+ |
| 260 | 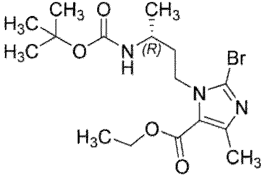 | MS(ESI) m/z;404/406 [M+H]+ |
| 270 | 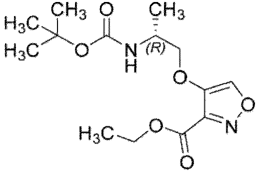 | MS(ESI) m/z;215 [M-Boc+H]+ |
| 271 | 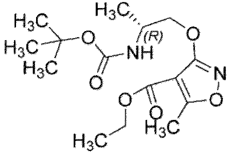 | MS(ESI) m/z; 329 [M+H]+ |
| 272 | 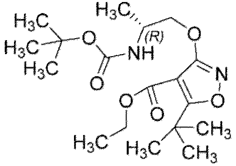 | MS(ESI) m/z;371 [M+H]+ |
| 273 | 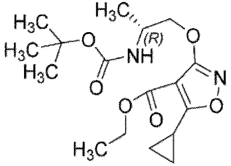 | MS(ESI) m/z; 355 [M+H]+ |

**[Table 56-8]**

| | | |
|---|---|---|
| 274 | 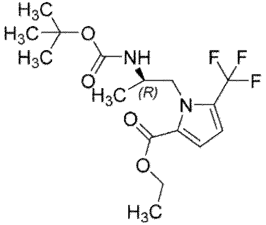 | MS(ESI) m/z;265 [M-Boc+H]+ |
| 275 | 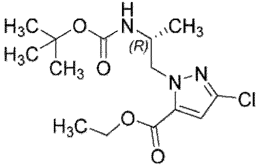 | MS(ESI) m/z;232/234 [M-Boc+H]+ |
| 276 | 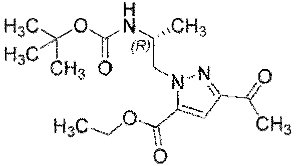 | MS(ESI) m/z;240 [M-Boc+H]+ |
| 277 | 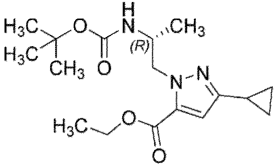 | MS(ESI) m/z;338 [M+H]+ |
| 278 | 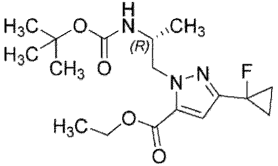 | MS(ESI) m/z;356 [M+H]+ |
| 279 | 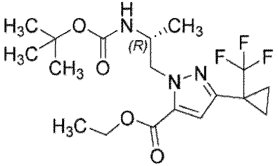 | MS(ESI) m/z;306 [M-Boc+H]+ |

**[Table 56-9]**

| | | |
|---|---|---|
| 280 | 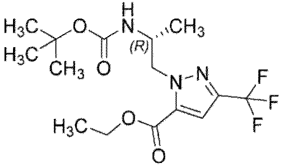 | MS(ESI) m/z;266 [M-Boc+H]+ |
| 281 | 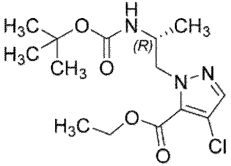 | MS(ESI) m/z;332/334 [M+H]+ |
| 282 | 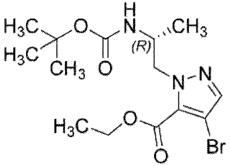 | MS(ESI) m/z;376/378 [M+H]+ |
| 283 | 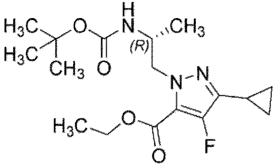 | MS(ESI) m/z;356 [M+H]+ |
| 284 | 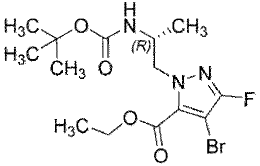 | MS(ESI) m/z;294/296 [M-Boc+H]+ |
| 285 | 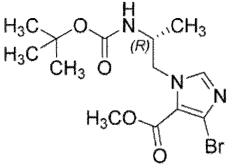 | MS(ESI) m/z;362/364 [M+H]+ |
| 286 | 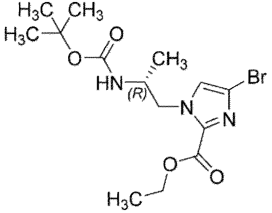 | MS(ESI) m/z;376/378 [M+H]+ |

### Reference Example 287

### Production of ethyl 4-acetonyloxy-2-methyl-thiazole-5-carboxylate

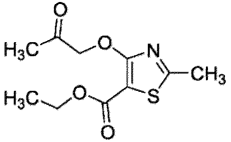

In a 50 mL eggplant flask, 484 mg of ethyl 4-hydroxy-2-methyl-thiazole-5-carboxylate was weighed, and 5.0 mL of N,N-dimethylformamide was added. Next, 109 mg of sodium hydride (60%) was added, and the mixture was stirred at room temperature for 5 minutes. Next, 300 µL of chloroacetone was added, and the mixture was stirred at room temperature for 20 minutes. Thereafter, the mixture was stirred at 50°C for 160 minutes. Ethyl acetate and brine were added to the reaction solution, and the organic layer was extracted. The aqueous layer was extracted again with ethyl acetate, and the organic layer was mixed. The organic layer was washed with brine and then dried over magnesium sulfate. The insoluble matter was filtered, and then the solution was concentrated under reduced pressure. 1.38 g of the resulting brown oily product was purified by silica gel column chromatography (solvent: hexane/ethyl acetate = 70/30 to 30/70) to obtain 225 mg of the title compound (yield 36%) as a yellow solid.
MS(ESI)m/z:244[M+H]⁺

### Reference Examples 288 to 291:

Corresponding raw material compounds were treated in the same manner as that of Reference Example 287 to obtain compounds shown in Table 57.

**[Table 57]**

| Reference Example | Structural formula | Physical property value and the like |
|---|---|---|
| 288 | 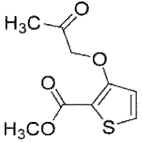 | MS(ESI) m/z;215 [M+H]+ |
| 289 | 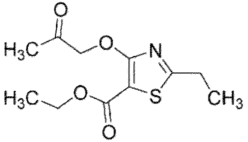 | MS(ESI) m/z;258 [M+H]+ |
| 290 | 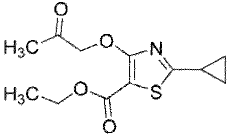 | MS(ESI) m/z;270 [M+H]+ |
| 291 | 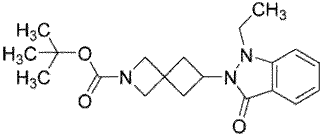 | MS(ESI) m/z;358 [M+H]+ |

### Reference Example 292

### Production of tert-butyl 6-(5-methyl-3-oxo-5H-1,4-benzoxazepin-4-yl)-2-azaspiro[3.3]heptane-2-carboxylate

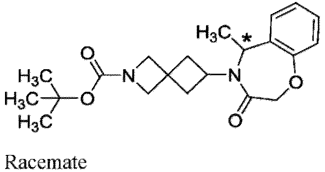

A mixture of 63.9 mg of tert-butyl 6-[(2-bromoacetyl)-[1-(2-hydroxyphenyl)ethyl]amino]-2-azaspiro[3.3]heptane-2-carboxylate, 55 mg of potassium carbonate, and 1.5 mL of acetonitrile was stirred at 95°C for 3 hours. Water was added to the reaction mixture, and extraction was performed with ethyl acetate. The organic layer was dried over sodium sulfate, filtered, and concentrated. The resulting residue was purified by silica gel chromatography (solvent: hexane/ethyl acetate = 80/20 to 0/100) to obtain 38.9 mg of the title compound (yield 78%) as a colorless amorphous solid.
MS(ESI)m/z:373[M+H]⁺

### Reference Example 293

### Production of tert-butyl 6-(5-methyl-3-oxo-2,5-dihydro-1H-1,4-benzodiazepin-4-yl)-2-azaspiro[3.3]-heptane-2-carboxylate

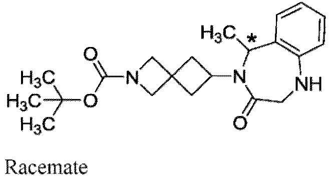

44.6 mg of tert-butyl 6-[(2-bromoacetyl)-[1-[2-[(2-bromoacetyl)amino]phenyl]ethyl]amino]-2-azaspiro[3.3]heptane-2-carboxylate was dissolved in 1 mL of a 4 N sodium hydroxide aqueous solution and 0.5 mL of tetrahydrofuran, and the mixture was stirred at 60°C for 30 minutes and at room temperature for 15 hours. Water was added to the reaction mixture, and extraction was performed with chloroform. The organic layer was dried over sodium sulfate, filtered, and concentrated. The resulting residue was purified by silica gel chromatography (solvent: hexane/ethyl acetate = 70/30 to 0/100) to obtain 2.7 mg of the title compound (yield 8.3%) as a pale yellow viscous oily product.
MS(ESI)m/z:316[M-tBu+H]⁺

### Reference Example 294

### Production of (6R)-2-bromo-7-[2-(2-fluorophenyl)sulfonyl-2-azaspiro[3.3]heptan-6-yl]-6-methyl-5,6-dihydroimidazo[1,2-a]pyrazin-8-one

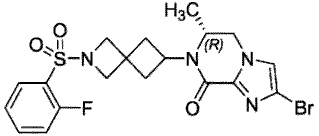

100 mg of ethyl 4-bromo-1-[(2R)-2-[[2-(2-fluorophenyl)sulfonyl-2-azaspiro[3.3]heptan-6-yl]amino]propyl]imidazole-2-carboxylate was dissolved in 2 mL of 1,2-dichloroethane, and 378 µL of trimethylaluminum (1 mol/L hexane solution) was added. The mixture was stirred at 70°C for 4 hours. To the reaction solution, 756 µL of trimethylaluminum (1 mol/L hexane solution) was added, and the mixture was stirred at 70°C for 5 hours. A Rochelle salt aqueous solution was added to the reaction mixture, and extraction was performed with chloroform. The organic layer was washed with water and brine, filtration was performed after drying with magnesium sulfate, and the filtrate was concentrated under reduced pressure. The resulting residue was purified by silica gel column chromatography (solvent: hexane/ethyl acetate = 40/60 to 0/100) to obtain 18 mg of the title compound (yield 20%) as a brown powder.
MS(ESI)m/z:483/485[M+H]⁺

### Reference Example 295

### Production of methyl 2-[[(1R)-3-(tert-butoxycarbonylamino)-1-methylpropyl]-[2-(2-fluorophenyl)sulfonyl-2-azaspiro [3.3]heptan-6-yl]amino]-2-oxo-acetate

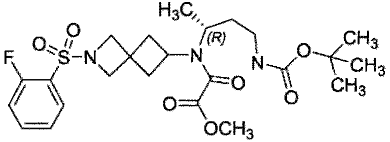

To a mixture of 2.00 g of tert-butyl N-[(3R)-3-[[2-(2-fluorophenyl)sulfonyl-2-azaspiro [3.3]heptan-6-yl]amino]butyl]carbamate and 20.0 mL of dichloromethane, 0.760 mL of triethylamine and 0.46 mL of methyl oxalyl chloride were added at room temperature, and the mixture was stirred as it was for 30 minutes. Water and chloroform were added to the reaction mixture, liquid separation was performed, and extraction from the aqueous layer was performed again with chloroform. The organic layers were combined, washed with brine, and dried over magnesium sulfate. The insoluble matter was filtered off, and the filtrate was concentrated under reduced pressure. The solvent was heated and distilled off under reduced pressure, and purification was performed by silica gel column chromatography (solvent: hexane/ethyl acetate = 70/30 to 50/50). Fractions were collected and concentrated to obtain 2.18 g of the title compound (yield 91%) as colorless amorphous product.
MS(ESI)m/z:528[M+H]⁺

### Reference Example 296:

Corresponding raw material compounds were treated in the same manner as that of Reference Example 295 to obtain compounds shown in Table 58.

**[Table 58]**

| Reference Example | Structural formula | Physical property value and the like |
|---|---|---|
| 296 | 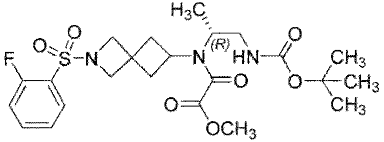 | MS(ESI) m/z;514 [M+H]+ |

### Reference Example 297

### Production of (7R)-1-[2-(2-fluorophenyl)sulfonyl-2-azaspiro[3.3]heptan-6-yl)]-7-methyl-1,4-diazepan-2,3-dione

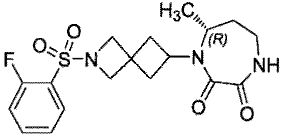

To a mixture of 2.18 g of methyl 2-[[(1R)-3-(tert-butoxycarbonylamino)-1-methyl-propyl]-[2-(2-fluorophenyl)sulfonyl-2-azaspiro[3.3]heptan-6-yl]amino]-2-oxo-acetate and 20.0 mL of dichloromethane, 0.880 mL of trimethylsilyl trifluoromethanesulfonate was added at room temperature, the mixture was stirred as it was for 30 minutes, and then, the solvent was heated and distilled off under reduced pressure. To the resulting residue, 20.0 mL of methylene chloride was added, 2.30 mL of triethylamine was added at room temperature, and then the mixture was heated and refluxed for 1 hour. After cooling to room temperature, 1 N hydrochloric acid was added for neutralization. Chloroform was added, liquid separation was performed, and extraction from the aqueous layer was performed again with chloroform. The organic layers were combined, washed with brine, and dried over magnesium sulfate. The insoluble matter was filtered off, and the filtrate was concentrated under reduced pressure. The solvent was heated and distilled off under reduced pressure, and purification was performed by silica gel column chromatography (solvent: ethyl acetate/methanol = 100/0 to 95/5). Fractions were collected and concentrated to obtain 837 mg of the title compound (yield 51%) as a colorless powder.
MS(ESI)m/z:396[M+H]⁺

### Reference Example 298:

Corresponding raw material compounds were treated in the same manner as that of Reference Example 297 to obtain compounds shown in Table 59.

**[Table 59]**

| Reference Example | Structural formula | Physical property value and the like |
|---|---|---|
| 298 | 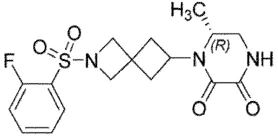 | MS(ESI) m/z;382 [M+H]+ |

### Reference Example 299

### Production of (7R)-1-[2-(2-fluorophenyl)sulfonyl-2-azaspiro[3.3]heptan-6-yl]-7-methyl-3-thioxo-1,4-diazepan-2-one

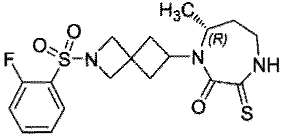

To a mixture of 810 mg of (7R)-1-[2-(2-fluorophenyl)sulfonyl-2-azaspiro[3.3]heptan-6-yl]-7-methyl-1,4-diazepan-2,3-dione and 20.0 mL of tetrahydrofuran, 420 mg of Lawesson's reagent was added at room temperature, and the mixture was heated and stirred at 80°C for 1 hour. After cooling to room temperature, 60.0 mL of tetrahydrofuran and 420 mg of Lawesson's reagent were added, and the mixture was heated and stirred at 80°C for 3 hours. After cooling to room temperature, the residue was purified by silica gel column chromatography (solvent: chloroform/methanol = 100/0 to 95/5). Fractions were collected and concentrated to obtain 413 mg of the title compound (yield 49%) as a yellow oily product.
MS(ESI)m/z:412[M+H]⁺

### Reference Example 300:

Corresponding raw material compounds were treated in the same manner as that of Reference Example 299 to obtain compounds shown in Table 60.

**[Table 60]**

| Reference Example | Structural formula | Physical property value and the like |
|---|---|---|
| 300 | 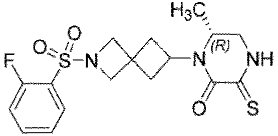 | MS(ESI) m/z;398 [M+H]+ |

### Reference Example 301

### Production of ethyl 2-cyclopropyl-5-methyl-oxazole-4-carboxylate

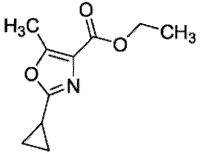

Into a 200 mL eggplant flask, 2.71 g of triphenylphosphine, 2.62 g of iodine, and 2.87 mL of triethylamine were put and dissolved in 25 mL of dichloromethane (solution (1)). Into a 100 mL eggplant flask, 1.10 g of ethyl 2-(cyclopropanecarbonylamino)-3-oxo-butanoate was put and dissolved in 25 mL of dichloromethane, the solution (1) was added dropwise under ice-cooling, the temperature was raised to room temperature, and the mixture was stirred for 0.5 hours. The reaction mixture was concentrated, ethyl acetate was added, and the insoluble matter was filtered through celite. The filtrate was washed with a 10% sodium bisulfite aqueous solution and brine, filtered, and concentrated. The resulting residue was purified by silica gel chromatography (solvent: hexane/ethyl acetate = 90/10 to 55/45) to obtain 592 mg of the title compound (yield 59%) as a pale yellow oily product.
MS(ESI)m/z:196[M+H]⁺

### Reference Example 302

### Production of ethyl 5-(bromomethyl)-2-cyclopropyl-oxazole-4-carboxylate

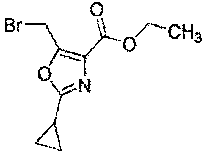

Into a 10 mL eggplant flask, 592 mg of ethyl 2-cyclopropyl-5-methyl-oxazole-4-carboxylate was put and dissolved in 15 mL of carbon tetrachloride. 701 mg of N-bromosuccinimide and 50 mg of 2,2'-azobis(2-methylpropionitrile were put, and the mixture was heated and refluxed at 105°C for 24 hours. After cooling to room temperature, water was added, extraction was performed with chloroform, and concentration was performed. The resulting residue was purified by silica gel chromatography (solvent: hexane/ethyl acetate = 100/0 to 60/40) to obtain 940 mg of the title compound (yield 113%) as a brown viscous product.
MS(ESI)m/z:274/276[M+H]⁺

### Reference Example 303:

A corresponding raw material compound was treated in the same manner as that of Reference Example 302 to obtain a compound shown in Table 61.

**[Table 61]**

| Reference Example | Structural formula | Physical property value and the like |
|---|---|---|
| 303 | 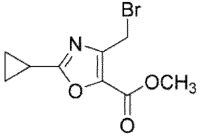 | MS(ESI) m/z;260/262 [M+H]+ |

### Reference Example 304

### Production of ethyl 2-cyclopropyl-5-(hydroxymethyl)oxazole-4-carboxylate

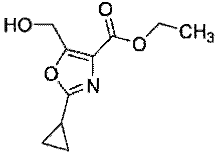

(1) In a 100 mL eggplant flask, 940 mg of ethyl 5-(bromomethyl)-2-cyclopropyl-oxazole-4-carboxylate was put and dissolved in 10 mL of dimethylformamide. 700 mg of sodium formate was added, and the mixture was heated and stirred at 80°C for 15 hours. Cooling was performed to room temperature, water was added, and extraction was performed with ethyl acetate. The organic layer was washed with water and brine, filtered, and concentrated. The resulting residue was purified by silica gel chromatography (solvent: hexane/ethyl acetate = 100/0 to 40/60) to obtain 581 mg of ethyl 2-cyclopropyl-5-(formyloxymethyl)oxazole-4-carboxylate (yield 71%) as a pale yellow oily product.
   MS(ESI)m/z:240[M+H]⁺
(2) In a 50 mL eggplant flask, 581 mg of ethyl 2-cyclopropyl-5-(formyloxymethyl)oxazole-4-carboxylate was put and dissolved in 10 mL of ethanol, and then the mixture was heated and refluxed for 18 hours. The temperature was returned to room temperature, 670 mg of potassium carbonate was added, and the mixture was stirred at room temperature for 120 hours. A saturated ammonium chloride aqueous solution was added, and extraction was performed with chloroform. The organic layer was washed with brine, filtered, and concentrated. The resulting residue was purified by silica gel chromatography (solvent: hexane/ethyl acetate = 95/5 to 0/100) to obtain 504 mg of the title compound (yield 98%) as a colorless oily product.
   MS(ESI)m/z:212[M+H]⁺

### Reference Example 305:

A corresponding raw material compound was treated in the same manner as that of Reference Example 304 to obtain a compound shown in Table 62.

**[Table 62]**

| Reference Example | Structural formula | Physical property value and the like |
|---|---|---|
| 305 | 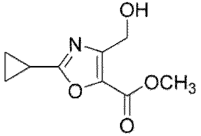 | MS(ESI) m/z;198 [M+H]+ |

### Reference Example 306

### Production of ethyl 2-cyclopropyl-5-formyl-oxazole-4-carboxylate

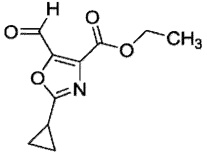

Into a 50 mL eggplant flask, 504 mg of ethyl 2-cyclopropyl-5-(hydroxymethyl)oxazole-4-carboxylate was put and dissolved in 15 mL of dichloromethane. 1,250 mg of Dess-Martin reagent was added under ice-cooling, and the mixture was stirred at room temperature for 1 hour. A saturated sodium bicarbonate aqueous solution was added to the reaction mixture, extraction was performed with chloroform, and the organic layer was concentrated. The resulting residue was purified by silica gel chromatography (solvent: hexane/ethyl acetate = 100/0 to 60/40) to obtain 448 mg of the title compound (yield 90%) as a colorless oily product.
MS(ESI)m/z:210[M+H]⁺

### Reference Example 307:

A corresponding raw material compound was treated in the same manner as that of Reference Example 306 to obtain a compound shown in Table 63.

**[Table 63]**

| Reference Example | Structural formula | Physical property value and the like |
|---|---|---|
| 307 | 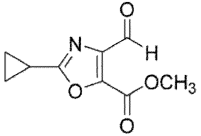 | MS(ESI) m/z;196 [M+H]+ |

### Reference Example 308

### Production of ethyl 2-cyclopropyl-5-vinyl-oxazole-4-carboxylate

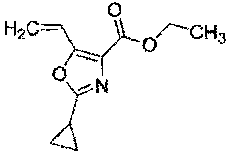

Into a 50 mL eggplant flask, 410 mg of methyltriphenylphosphinium bromide was put and dissolved in 5 mL of tetrahydrofuran. Under ice-cooling, 0.75 mL of a 1.55 M n-butyl lithium hexane solution was added, the temperature was raised to room temperature, and the mixture was stirred for 0.5 hours. After cooling again under ice-cooling, a solution of 156 mg of ethyl 2-cyclopropyl-5-formyl-oxazole-4-carboxylate and 5 mL of tetrahydrofuran was added dropwise, and the mixture was stirred at room temperature for 1 hour. Water was added to the reaction mixture, and extraction was performed with chloroform. The organic layer was washed with brine, dried over sodium sulfate, filtered, and then concentrated. The resulting residue was purified by silica gel chromatography (solvent: hexane/ethyl acetate = 100/0 to 75/25) to obtain 49.9 mg of the title compound (yield 32%) as a colorless oily product.
MS(ESI)m/z:208[M+H]⁺

### Reference Example 309:

A corresponding raw material compound was treated in the same manner as that of Reference Example 308 to obtain a compound shown in Table 64.

**[Table 64]**

| Reference Example | Structural formula | Physical property value and the like |
|---|---|---|
| 309 | 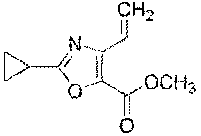 | MS(ESI) m/z;194 [M+H]+ |

### Reference Example 310

### Production of ethyl 5-methylisothiazole-3-carboxylate

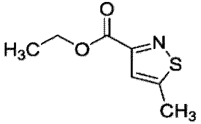

500 mg of 5-methylisothiazole-3-carboxylic acid and 18.7 µL of sulfuric acid were dissolved in 6 mL of ethanol, and the mixture was stirred at 80°C for 4 hours. A saturated sodium bicarbonate aqueous solution was added to the reaction mixture, and extraction was performed with ethyl acetate. The organic layer was dried over magnesium sulfate and then filtered through an NH silica gel pad, and the filtrate was concentrated under reduced pressure, thereby obtaining 516 mg of the title compound (yield 86%) as a brown oily product.
MS(ESI)m/z:172[M+H]⁺

### Reference Example 311

### Production of ethyl 4-iodo-5-methyl-isothiazole-3-carboxylate

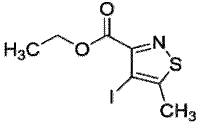

510 mg of ethyl 5-methylisothiazole-3-carboxylate was suspended in 8 mL of trifluoroacetic acid, 1,005 mg of N-iodosuccinimide was added, and the mixture was stirred at room temperature for 23 hours. 1,005 mg of N-iodosuccinimide was added, and the mixture was stirred at room temperature for 22 hours. Further, the temperature was raised to 50°C, and the mixture was stirred for 7 hours. The reaction mixture was concentrated under reduced pressure, a saturated sodium bicarbonate aqueous solution and ethyl acetate were added to the resulting residue, and liquid separation was performed. The organic layer was sequentially washed with a sodium thiosulfate aqueous solution and brine, dried over sodium sulfate, filtered, and concentrated, thereby obtaining 940 mg of the title compound (yield 97%) as a brown powder.
MS(ESI)m/z:298[M+H]⁺

### Reference Example 312:

A corresponding raw material compound was treated in the same manner as that of Reference Example 311 to obtain a compound shown in Table 65.

**[Table 65]**

| Reference Example | Structural formula | Physical property value and the like |
|---|---|---|
| 312 | 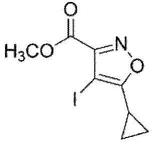 | MS(ESI) m/z;294 [M+H]+ |

### Reference Example 313

### Production of ethyl 2-[(3R)-3-(tert-butoxycarbonylamino)butyl]-5-(1,1-difluoroethyl)pyrazole-3-carboxylate

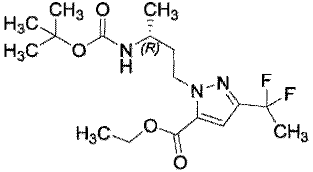

1,002 mg of ethyl 5-acetyl-2-[(3R)-3-(tert-butoxycarbonylamino)butyl]pyrazole-3-carboxylate was dissolved in 7 mL of chloroform, and 7.43 mL of diethylaminosulfur trifluoride was added dropwise under ice-cooling. The temperature was raised to room temperature, and the mixture was stirred for 69 hours. To the reaction mixture, 50 mL of chloroform was added for dilution. The resultant was added dropwise to 100 mL of a saturated sodium bicarbonate aqueous solution under ice-cooling and stirring for quenching. The organic layer was washed with brine, filtration was performed after drying with sodium sulfate, and the filtrate was concentrated under reduced pressure. The resulting residue was purified by silica gel column chromatography (solvent: hexane/ethyl acetate = 85/15 to 70/30) to obtain 343 mg of the title compound (yield 32%) as a brown powder.
MS(ESI)m/z:276[M-Boc+H]⁺

### Reference Example 314:

A corresponding raw material compound was treated in the same manner as that of Reference Example 313 to obtain a compound shown in Table 66.

**[Table 66]**

| Reference Example | Structural formula | Physical property value and the like |
|---|---|---|
| 314 | 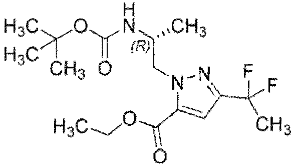 | MS(ESI) m/z;262 [M-Boc+H]+ |

### Reference Example 315

### Production of ethyl 4-bromo-3-fluoro-1H-pyrazole-5-carboxylate

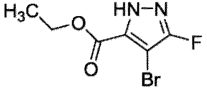

800 mg of ethyl 4-bromo-1H-pyrazole-5-carboxylate was suspended in 15 mL of acetonitrile, 1,533 mg of 1-chloromethyl-4-fluoro-1,4-diazoniabicyclo[2.2.2]octane bis(tetrafluoroborate) was added, and the mixture was stirred at 70°C for 7 hours. To the reaction solution, 1,533 mg of 1-chloromethyl-4-fluoro-1,4-diazoniabicyclo[2.2.2]octane bis(tetrafluoroborate) was added, and the mixture was stirred at 70°C for 14 hours. A saturated ammonium chloride aqueous solution was added to the reaction solution, and extraction was performed with ethyl acetate. The organic layer was washed with water and brine, filtration was performed after drying with magnesium sulfate, and the filtrate was concentrated under reduced pressure. The resulting residue was added to ethyl acetate, suspended and washed, and filtered with Phase separator^{®}. The filtrate was purified by silica gel column chromatography (solvent: hexane/ethyl acetate = 85/15 to 70/30) to obtain 32 mg of the title compound (yield 5%) as a colorless powder.
MS(ESI)m/z:235/237[M-H]⁻

### Reference Example 316:

A corresponding raw material compound was treated in the same manner as that of Reference Example 315 to obtain a compound shown in Table 67.

**[Table 67]**

| Reference Example | Structural formula | Physical property value and the like |
|---|---|---|
| 316 | 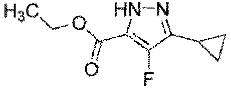 | MS(ESI) m/z;199 [M+H]+ |

### Reference Example 317

### Production of ethyl 4-chloro-1H-pyrazole-5-carboxylate

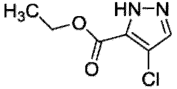

1,000 mg of ethyl 1H-pyrazole-5-carboxylate was suspended in 20 mL of acetonitrile, 953 mg of N-chlorosuccinimide was added, and the mixture was stirred at room temperature for 16 hours. A saturated sodium bicarbonate aqueous solution was added to the reaction mixture, and extraction was performed with ethyl acetate. The organic layer was washed with brine, dried over magnesium sulfate, and then filtered, and the filtrate was concentrated under reduced pressure, thereby obtaining 1,269 mg of the title compound (yield 102%, containing a small amount of impurities) as a colorless powder.
MS(ESI)m/z:175/177[M+H]⁺

### Reference Examples 318 to 320:

Corresponding raw material compounds and reagents were treated in the same manner as that of Reference Example 317 to obtain compounds shown in Table 68.

**[Table 68]**

| Reference Example | Structural formula | Physical property value and the like |
|---|---|---|
| 318 | 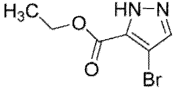 | MS(ESI) m/z;219/221 [M+H]+ |
| 319 | 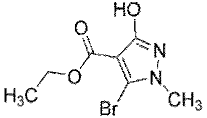 | MS(ESI) m/z;249/251 [M+H]+ |
| 320 | 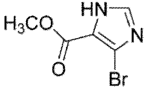 | MS(ESI) m/z;205/207 [M+H]+ |

### Reference Example 321

### Production of tert-butyl 6-[(3R)-8-cyclopropyl-3-methyl-5-oxo-2,3-dihydropyrido[3,2-f] [1,4]oxazepin-4-yl]-2-azaspiro[3.3]heptane-2-carboxylate

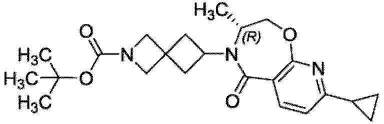

Into a 50 mL eggplant flask, 483.6 mg of tert-butyl 6-[(3R)-8-chloro-3-methyl-5-oxo-2,3-dihydropyrido[3,2-f] [1,4]oxazepin-4-yl]-2-azaspiro[3.3]heptane-2-carboxylate and 153 mg of cyclopropane boronic acid were put and dissolved in 10 mL of toluene and 3 mL of a 1.26 mol/L potassium phosphate aqueous solution. 100 mg of dichloro(tricyclohexylphosphine)palladium was added, and the mixture was heated and refluxed at 130°C for 1.3 hours. To the reaction solution, 153 mg of cyclopropane boronic acid and 100 mg of dichloro(tricyclohexylphosphine)palladium were added, and the mixture was heated and refluxed at 130°C for 1.3 hours. To the reaction solution, 153 mg of cyclopropane boronic acid and 100 mg of dichloro(tricyclohexylphosphine)palladium were added, and the mixture was heated and refluxed at 130°C for 1 hour. 30 mL of water was added to the reaction solution, extraction was performed three times with 30 mL of chloroform, and filtration and concentration were performed. The resulting residue was purified by silica gel column chromatography (solvent: hexane/ethyl acetate = 80/20 to 35/65) to obtain 262 mg of the title compound (yield 54%) as a gray powder.
MS(ESI)m/z:414[M+H]⁺

### Reference Examples 322 and 323:

Corresponding raw material compounds were treated in the same manner as that of Reference Example 321 to obtain compounds shown in Table 69.

**[Table 69]**

| Reference Example | Structural formula | Physical property value and the like |
|---|---|---|
| 322 | 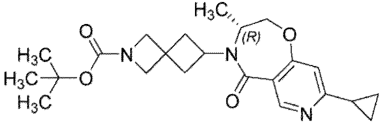 | MS(ESI) m/z;414 [M+H]+ |
| 323 | 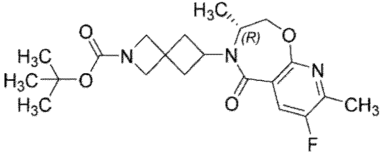 | MS(ESI) m/z;406 [M+H]+ |

### Reference Example 324

### Production of tert-butyl 6-[1-(hydroxymethyl)propylamino]-2-azaspiro[3.3]heptane-2-carboxylate

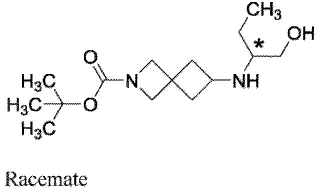

(1) 800 mg of tert-butyl 6-amino-2-azaspiro[3.3]heptane-2-carboxylate and 0.978 mL of N,N-diisopropylethylamine were dissolved in 8 mL of tetrahydrofuran, 1,023 mg of methyl 2-bromobutanoate was added, and the mixture was stirred at room temperature for 2 hours. The temperature was raised to 50°C, and the mixture was stirred for 7 hours. Further, the mixture was heated and refluxed for 8 hours. Water was added to the reaction mixture, and extraction was performed twice with ethyl acetate. The organic layer was washed with brine, filtration was performed after drying with sodium sulfate, and the filtrate was concentrated under reduced pressure. The resulting residue was purified by silica gel column chromatography (solvent: hexane/ethyl acetate = 50/50 to 0/100) to obtain 935 mg of tert-butyl 6-(1-methoxycarbonylpropylamino)-2-azaspiro[3.3]heptane-2-carboxylate as a yellow viscous product.
   MS(ESI)m/z:313[M+H]⁺
(2) 930 mg of tert-butyl 6-(1-methoxycarbonylpropylamino)-2-azaspiro[3.3]heptane-2-carboxylate was dissolved in 10 mL of tetrahydrofuran and 5 mL of ethanol, 338 mg of sodium borohydride and 991 mg of calcium chloride were added, and the mixture was stirred at room temperature for 17 hours. The temperature was raised to 60°C, and the mixture was stirred for 6 hours. To the reaction solution, 225 mg of sodium borohydride and 661 mg of calcium chloride were added, and the mixture was stirred at 60°C for 1 hour. The reaction solution was cooled to room temperature and diluted with chloroform, the insoluble matter was filtered through celite, water was added to the filtrate, and extraction was performed twice with chloroform. The organic layer was washed with brine, dried over sodium sulfate, and then filtered, and the filtrate was concentrated under reduced pressure, thereby obtaining 920 mg of the title compound (yield 109%, containing impurities) as a yellow oily product.
   MS(ESI)m/z:285[M+H]⁺

### Reference Example 325:

A corresponding raw material compound was treated in the same manner as that of Reference Example 324 to obtain a compound shown in Table 70.

**[Table 70]**

| Reference Example | Structural formula | Physical property value and the like |
|---|---|---|
| 325 | 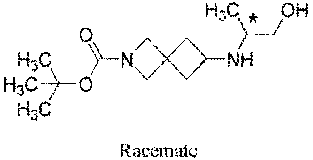 | MS(ESI) m/z;271 [M+H]+ |

### Reference Example 326

### Production of tert-butyl 6-[7-methyl-5-oxo-2-(trifluoromethyl)-7,8-dihydro-1,6-naphthyridin-6-yl]-2-azaspiro[3.3]heptane-2-carboxylate

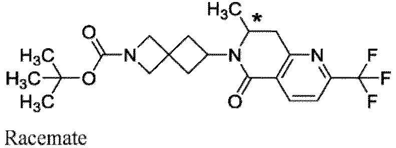

139 mg of tert-butyl 6-amino-2-azaspiro[3.3]heptane-2-carboxylate and 80 mg of methyl 2-[(E)-prop-1-enyl]-6-(trifluoromethyl)pyridine-3-carboxylate were dissolved in 2 mL of methanol, and the mixture was stirred at 50°C for 21 hours. The solvent was substituted with 1 mL of ethanol, and the mixture was stirred at 80°C for 17 hours. The reaction solution was concentrated, and the resulting residue was purified by silica gel column chromatography (solvent: hexane/ethyl acetate = 70/30 to 50/50) to obtain 126 mg of the title compound (yield 91%) as a colorless powder.
MS(ESI)m/z:370[M-tBu+H]⁺

### Reference Example 327

A corresponding raw material compound was treated in the same manner as that of Reference Example 326 to obtain a compound shown in Table 71.

**[Table 71]**

| Reference Example | Structural formula | Physical property value and the like |
|---|---|---|
| 327 | 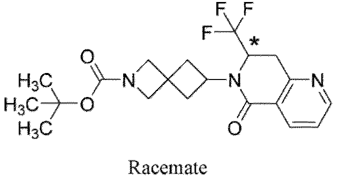 | MS(ESI) m/z;412 [M+H]+ |

### Reference Example 328

### Production of [2-(2-fluorophenyl)sulfonyl-2-azaspiro[3.3]heptan-6-yl]-4-methylbenzenesulfonate

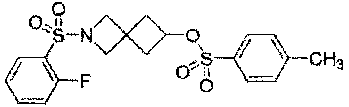

(1) 1,500 mg of 2-azaspiro[3.3]heptan-6-ol hydrochloride was suspended in 15 mL of dichloromethane, and 2.79 mL of triethylamine was added. A solution of 1,951 mg of 2-fluorobenzenesulfonyl chloride and dichloromethane (5 mL) was added under ice-cooling, and the mixture was stirred as it was for 1 hour. Water was added to the reaction mixture, and extraction was performed with chloroform. The organic layer was washed with brine, filtration was performed after drying with magnesium sulfate, and the filtrate was concentrated under reduced pressure. The resulting residue was purified by silica gel column chromatography (solvent: chloroform/methanol = 100/0 to 94/6) to obtain 2,178 mg of 2-(2-fluorophenyl)sulfonyl-2-azaspiro[3.3]heptan-6-ol (yield 80%) as a colorless oily product.
   MS(ESI)m/z:272[M+H]⁺
(2) 2,175 mg of the 2-(2-fluorophenyl)sulfonyl-2-azaspiro[3.3]heptan-6-ol obtained in (1) above, 2.23 mL of triethylamine, and 98 mg of dimethylaminopyridine were dissolved in 25 mL of dichloromethane, 1,681 mg of 4-methylbenzenesulfonyl chloride was added, and the mixture was stirred at room temperature for 24 hours. To the reaction solution, 0.67 mL of triethylamine and 459 mg of 4-methylbenzenesulfonyl chloride were added, and the mixture was stirred at room temperature for 6 hours. Water was added to the reaction mixture, and extraction was performed with ethyl acetate. The organic layer was washed with water and brine, filtration was performed after drying with magnesium sulfate, and the filtrate was concentrated under reduced pressure. The resulting residue was purified by silica gel column chromatography (solvent: hexane/ethyl acetate = 80/20 to 60/40) to obtain 2,984 mg of the title compound (yield 86%) as a colorless powder.
   MS(ESI)m/z:426[M+H]⁺

### Reference Example 329

### Production of [2-(benzenesulfonyl)-2-azaspiro[3.3]heptan-6-yl]benzenesulfonate

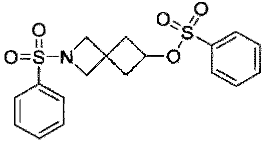

In a 50 mL eggplant flask, 504 mg of 2-azaspiro[3.3]heptan-6-ol hydrochloride was weighed, and 12 mL of dichloromethane and 2.0 mL of triethylamine were added. The reaction solution was ice-cooled, 2.48 g of benzenesulfonic anhydride was added, and the mixture was stirred for 50 minutes. Next, 32.5 mg of 4-dimethylaminopyridine was added, and the mixture was stirred under ice-cooling for 100 minutes. To the reaction solution, 500 µL of triethylamine and 525 mg of benzenesulfonic anhydride were additionally added, and thereafter, the mixture was stirred at room temperature for 14 hours. A saturated sodium bicarbonate aqueous solution was added to the reaction solution, and extraction was performed with ethyl acetate. The organic layer was washed with brine and then dried over magnesium sulfate. The insoluble matter was filtered, and then the filtrate was concentrated under reduced pressure. 1.51 g of the resulting orange oily product was purified by silica gel column chromatography (solvent: hexane/ethyl acetate = 85/15 to 55/45) to obtain 1.24 g of the title compound (yield 94%) as a pale yellow oily product.
MS(ESI)m/z:394[M+H]⁺

### Reference Example 330

### Production of tert-butyl 6-[(2-bromoacetyl)-[1-[2-[(2-bromoacetyl)amino]phenyl]ethyl]amino]-2-azaspiro[3.3]heptane-2-carboxylate

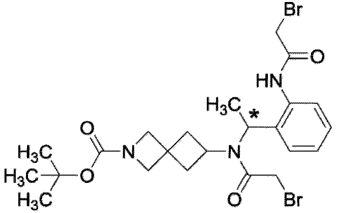

and Reference Example 331

### Production of tert-butyl 6-(5-methyl-2-oxo-3,5-dihydro-1H-1,4-benzodiazepin-4-yl)-2-azaspiro[3.3]heptane-2-carboxylate

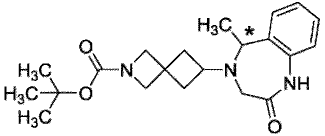

Into a 20 mL eggplant flask, 117.3 mg of tert-butyl 6-[1-(2-aminophenyl)ethylamino]-2-azaspiro[3.3]heptane-2-carboxylate and 0.069 mL triethylamine were put and dissolved in 2 mL of dichloromethane. 0.030 mL of 2-bromoacetyl bromide was added under ice-cooling, and the mixture was stirred at room temperature for 4 hours. To the reaction mixture, a saturated sodium bicarbonate aqueous solution and chloroform were added, and liquid separation was performed. The organic layer was dried over sodium sulfate, filtered, and concentrated. The resulting residue was purified by silica gel chromatography (solvent: hexane/ethyl acetate = 90/10 to 0/100) to obtain 44.6 mg of tert-butyl 6-[(2-bromoacetyl)-[1-[2-[(2-bromoacetyl)amino]phenyl]ethyl]amino]-2-azaspiro[3.3]heptane-2-carboxylate (yield 23.8%) as a pale yellow viscous oily product.
MS(ESI)m/z:516/518/520[M-tBu+H]⁺

In addition, 29.7 mg of tert-butyl 6-(5-methyl-2-oxo-3,5-dihydro-1H-1,4-benzodiazepin-4-yl)-2-azaspiro[3.3]heptane-2-carboxylate (yield 24.4%) to obtain a white powder.
MS(ESI)m/z:372[M+H]⁺

### Reference Example 332

### Production of 2-[2-(2-azaspiro[3.3]heptan-6-ylamino)propyl]phenol

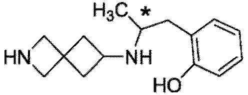

513 mg of tert-butyl 6-[[2-(2-methoxyphenyl)-1-methyl-ethyl]amino]-2-azaspiro[3.3]heptane-2-carboxylic acid was dissolved in 6 mL of dichloromethane, 1.54 mL of a 1 M boron tribromide dichloromethane solution was added at -78°C, the temperature was increased to 0°C, and the mixture was stirred for 1 hour. A saturated sodium bicarbonate aqueous solution and brine were added to the reaction mixture, and extraction was performed with chloroform. The organic layer was dried over sodium sulfate, filtered, and concentrated to obtain 174 mg of the title compound (yield 50%) as a colorless powder.
MS(ESI)m/z:247[M+H]⁺

### Reference Example 333

### Production of 3-(2-azaspiro[3.3]heptan-6-yl)-4-methyl-4,5-dihydro-1,3-benzoxazepin-2-one

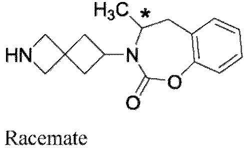

Into a 50 mL eggplant flask, 174 mg of 2-[2-(2-azaspiro[3.3]heptan-6-ylamino)propyl]phenol was put and dissolved in 5 mL of tetrahydrofuran. 343 mg of 1,1'-carbodiimidazole was added, and the mixture was stirred at room temperature for 1 hour. To the reaction mixture, 5 mL of a 1 N sodium hydroxide aqueous solution was added, and the mixture was stirred for a while. Extraction was performed with chloroform, and then the organic layer was dried over sodium sulfate, filtered, and concentrated, thereby obtaining 146 mg of the title compound (yield 76%) as a pale yellow viscous oily product.
MS(ESI)m/z:273[M+H]⁺

### Reference Example 334

### Production of N-[2-(2-fluorophenyl)sulfonyl-2-azaspiro[3.3]heptan-6-yl]-N-(1-methylallyl)-5-(trifluoromethyl)-3-vinyl-pyridine-2-carboxamide

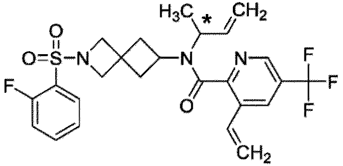

In a 100 mL eggplant flask, 704 mg of 3-bromo-N-[2-(2-fluorophenyl)sulfonyl-2-azaspiro[3.3]heptan-6-yl]-N-(1-methylallyl)-5-(trifluoromethyl)pyridine-2-carboxamide and 352 mg of a 2,4,6-trivinylboroxine-pyridine complex were weighed, and 8.0 mL of 1,4-dioxane was added. Next, 178 mg of tetrakistriphenylphosphine palladium and 1.50 mL of a 2 mol/L sodium carbonate aqueous solution were added, and the mixture was stirred at 100°C for 4 hours. Ethyl acetate and brine were added to the reaction solution, and the organic layer was extracted. The organic layer was washed with brine and then dried over sodium sulfate. The insoluble matter was filtered, and then the solution was concentrated under reduced pressure. 967 mg of the obtained orange oily product was purified by silica gel column chromatography (solvent: hexane/ethyl acetate = 60/40 to 25/75), and a peak of an Rf value of 0.8 was collected by thin-layer chromatography (solvent: hexane/ethyl acetate = 1:2), thereby obtaining 232 mg of the title compound (yield 36%) as a colorless solid.
MS(ESI)m/z:524[M+H]⁺

### Reference Example 335

### Production of 7-[2-(2-fluorophenyl)sulfonyl-2-azaspiro[3.3]heptan-6-yl]-6-methyl-5-methylen-3-(trifluoromethyl)-6H-1,7-naphthyridin-8-one

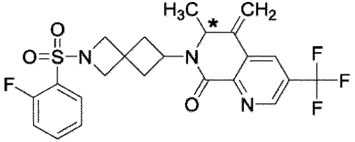

In the silica gel column chromatography (solvent: hexane/ethyl acetate = 60/40 to 25/75) performed in Reference Example 334, a peak of an Rf value of 0.3 was collected by thin-layer chromatography (solvent: hexane/ethyl acetate = 1:2), thereby obtaining 382 mg of the title compound (yield 63%) as a colorless powder.
MS(ESI)m/z:496[M+H]⁺

### Reference Example 336

### Production of 6-[2-(2-fluorophenyl)sulfonyl-2-azaspiro[3.3]heptan-6-yl]-7-methyl-1-oxide-8,9-dihydro-7H-pyrido[3,2-c]azepin-1-ium-5-one

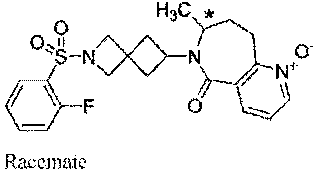

465 mg of 6-[2-(2-fluorophenyl)sulfonyl-2-azaspiro[3.3]heptan-6-yl]-7-methyl-8,9-dihydro-7H-pyrido[3,2-c]azepin-5-one was dissolved in 6 mL of dichloromethane, 280 mg of m-chloroperbenzoic acid (containing about 30% of water) was added, and the mixture was stirred at room temperature for 1 hour. A saturated sodium bicarbonate aqueous solution was added to the reaction mixture, and extraction was performed with chloroform. The organic layer was washed with brine, filtration was performed after drying with magnesium sulfate, and the filtrate was concentrated under reduced pressure. The resulting residue was purified by NH silica gel column chromatography (solvent: ethyl acetate/methanol = 100/0 to 92/8) to obtain 458 mg of the title compound (yield 95%) as a colorless powder.
MS(ESI)m/z:446[M+H]⁺

### Reference Example 337

### Production of 6-[2-(2-fluorophenyl)sulfonyl-2-azaspiro[3.3]heptan-6-yl]-7-methyl-7, 8-dihydropyrido [3,2-c] azepin-5,9-dione

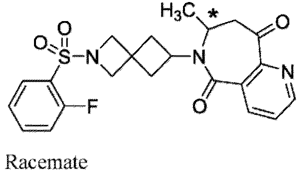

148 mg of 6-[2-(2-fluorophenyl)sulfonyl-2-azaspiro[3.3]heptan-6-yl]-9-hydroxy-7-methyl-8,9-dihydro-7H-pyrido[3,2-c]azepin-5-one was dissolved in 3 mL of dichloromethane, 207 mg of Dess-Martin reagent was added, and the mixture was stirred at room temperature for 1 hour. A saturated sodium bicarbonate aqueous solution was added to the reaction mixture, and extraction was performed with chloroform. The organic layer was washed with water and brine, filtration was performed after drying with magnesium sulfate, and the filtrate was concentrated under reduced pressure. The resulting residue was purified by silica gel column chromatography (solvent: hexane/ethyl acetate = 50/50 to 0/100) and further purified by reverse-phase HPLC (solvent: 10 mM ammonium carbonate aqueous solution/acetonitrile = 80/20 to 50/50), thereby obtaining 117 mg of the title compound (yield 81%) as a colorless powder.
MS(ESI)m/z:444[M+H]⁺

### Reference Example 338

### N-[2-(2-fluorophenyl)sulfonyl-2-azaspiro[3.3]heptan-6-yl]-N-(1-methylallyl)-5 -(trifluoromethyl) -2-vinyl-pyridine- 3 -c arboxamide

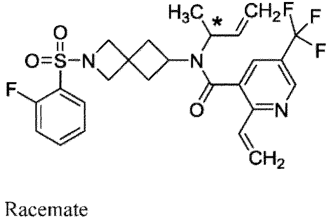

(1) In a 30 mL eggplant flask, 299 mg of 5-(trifluoromethyl)-2-vinyl-pyridine-3-carboxylic acid was weighed, and 6.0 mL of dichloromethane was added. The mixture was stirred under ice-cooling, 180 µL of oxalyl chloride and 10 µL of N,N-dimethylformamide were added, and the mixture was stirred at room temperature for 50 minutes. The reaction solution was concentrated under reduced pressure to obtain a crude product of 2-(2-cloroethyl)-5-(trifluoromethyl)pyridine-3-carboxylic acid chloride as a deep red oily product.
(2) In a 30 mL eggplant flask, 237 mg of 2-(2-fluorophenyl)sulfonyl-N-(1-methylallyl)-2-azaspiro[3.3]heptan-6-amine was weighed, 4.0 mL of dichloromethane and 500 µL of triethylamine were added, and the mixture was stirred under ice-cooling. A solution obtained by dissolving the total amount of the crude product of 2-(2-cloroethyl)-5-(trifluoromethyl)pyridine-3-carboxylic acid chloride obtained in (1) above in 3.0 mL of dichloromethane was added thereto, and the mixture was stirred under ice-cooling for 30 minutes. A saturated sodium bicarbonate aqueous solution was added to the reaction solution, the mixture was stirred at room temperature for 15 minutes, a mixed solvent of ethyl acetate:hexane = 1: 1 was added, and the organic layer was extracted. The organic layer was washed with brine and then dried over magnesium sulfate. The insoluble matter was filtered, and then the solution was concentrated under reduced pressure. 0.46 g of the resulting deep red oily product was purified by silica gel column chromatography (solvent: hexane/ethyl acetate = 85/15 to 55/45) to obtain 258 mg of a mixture of 2-(2-chloroethyl)-N-[2-(2-fluorophenyl)sulfonyl-2-azaspiro[3.3]heptan-6-yl]-N-(1-methylallyl)-5-(trifluoromethyl)pyridine-3-carboxamide and N-[2-(2-fluorophenyl)sulfonyl-2-azaspiro[3.3]heptan-6-yl]-N-(1-methylallyl)-5-(trifluoromethyl)-2-vinyl-pyridine-3-carboxamide as a pale yellow oily product.
   MS(ESI)m/z:560,524[M+H]⁺
(3) The mixture obtained in (2) above was added to a 100 mL eggplant flask and dissolved in 4.0 mL of tetrahydrofuran. Next, 800 µL of N,N-diisopropylethylamine was added, and the mixture was stirred at room temperature for 17 hours. The reaction solution was diluted with a mixed solvent of ethyl acetate:hexane = 1: 1, washing was performed with water, a diluted citric acid aqueous solution, brine, and then drying was performed with magnesium sulfate. The insoluble matter was filtered out, and the solution was concentrated under reduced pressure, thereby obtaining 261 mg of the title compound (yield 3 step 68%) as a pale yellow oily product.
   MS(ESI)m/z:524[M+H]⁺

### Reference Example 339

### Production of 2-chloro-N-[2-[2-[[2-chloro-6-(trifluoromethyl)pyridin-3-carbonyl]-[2-(2-fluorophenyl)sulfonyl-2-azaspiro[3.3]heptan-6-yl]amino]propyldisulfanyl]-1-methyl-ethyl]-N-[2-(2-fluorophenyl)sulfonyl-2-azaspiro[3.3]heptan-6-yl]-6-(trifluoromethyl)pyridine-3-carboxamide

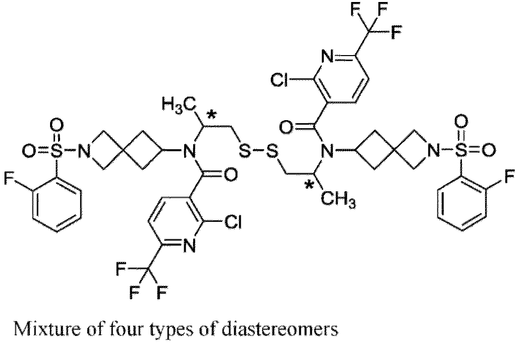

(1) In a 20 mL eggplant flask, 500 mg of 2-chloro-6-(trifluoromethyl)pyridine-3-carboxylic acid was weighed and dissolved in 6 mL of dichloromethane. 0.23 mL of oxalyl chloride and 0.020 mL of dimethylformamide were added, and the mixture was stirred at room temperature for 1 hour. The reaction solution was concentrated to obtain a crude product of 2-chloro-6-(trifluoromethyl)pyridine-3-carbonyl chloride.
(2) The crude product of 2-chloro-6-(trifluoromethyl)pyridine-3-carbonyl chloride obtained in (1) above was dissolved in 2 mL of acetonitrile, and a solution of 335 mg of 2-[[2-(2-fluorophenyl)sulfonyl-2-azaspiro[3.3]heptan-6-yl]amino]propan-1-thiol and 8 mL of acetonitrile, and 0.33 mL of triethylamine were added. The mixture was stirred at room temperature for 2.5 hours. To the reaction mixture, a saturated sodium bicarbonate aqueous solution and ethyl acetate were added, and liquid separation was performed. The organic layer was dried over sodium sulfate, filtered, and concentrated. The resulting residue was purified by silica gel chromatography (solvent: hexane/ethyl acetate = 80/20 to 0/100) to obtain 380 mg of the title compound (yield 37%) as a white powder.
   MS(ESI)m/z: 1101/1103[M+H]⁺

### Reference Example 340

### Production of ethyl 5-bromo-3-methyl-isothiazole-4-carboxylate

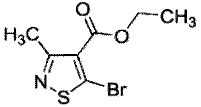

500 mg of ethyl 5-amino-3-methyl-isothiazole-4-carboxylate was suspended in 2 mL of a 48% hydrobromic acid aqueous solution and 3 mL of acetonitrile. 277 mg of sodium nitrite was slowly added under ice-cooling. Subsequently, 2 mL of a 48% hydrobromic acid aqueous solution of 385 mg of copper(I) bromide was added, and the mixture was stirred at room temperature for 2 hours. The temperature was raised to 50°C, the mixture was stirred for 1 hour, the reaction mixture was returned to room temperature, 370 mg of sodium nitrite was slowly added, and the mixture was stirred at room temperature for 0.5 hours. Water was added to the reaction mixture, and extraction was performed with ethyl acetate. The organic layer was washed with brine, dried over magnesium sulfate, filtered, and then concentrated. The resulting residue was purified by silica gel chromatography (solvent: hexane/ethyl acetate = 95/5 to 80/20) to obtain 630 mg of the title compound (yield 94%) as a colorless viscous product.
MS(ESI)m/z:250/252[M+H]⁺

### Reference Example 341

### Production of ethyl 3-acetyl-1H-pyrazole-5-carboxylate

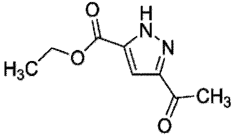

3,000 mg of 3-butyn-2-one was dissolved in 70 mL of diethyl ether, and a solution of 4,600 mg of ethyl 2-diazoacetate and diethyl ether (30 mL) was added dropwise. The mixture was stirred at room temperature for 1 hour. The temperature was raised to an external temperature of 50°C, and the mixture was heated and refluxed for 3 hours. The solvent was volatilized, and the target product was precipitated. The resultant was dried under reduced pressure to obtain 5,361 mg of the title compound (yield 73%) as a light yellow powder.
MS(ESI)m/z:183[M+H]⁺

### Reference Example 342

### Production of 5-cyclopropyl-4-[(E)-prop-1-yl]isoxazole-3-carboxylic acid

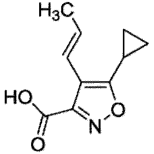

500 mg of ethyl 2-methyl-4-(trifluoromethylsulfonyloxy)thiazole-5-carboxylate, 403 mg of potassium trifluoro-[(E)-prop-1-nyl]borate, 197 mg of tetrakistriphenylphosphine palladium, and 2 mL of a 2 M sodium carbonate aqueous solution were suspended in 8 mL of 1,4-dioxane, and the mixture was stirred at 110°C for 2 hours. Water was added to the reaction mixture, and extraction was performed with ethyl acetate. The aqueous layer was preserved as an aqueous layer (1), and the organic layer was dried over magnesium sulfate, dried, filtered, and then concentrated. The resulting residue was dissolved in 2 mL of tetrahydrofuran and 2 mL of methanol, 2 mL of a 1 N sodium hydroxide aqueous solution was added, and the mixture was stirred at room temperature for 1 hour. Water was added to the reaction solution, and extraction was performed with ethyl acetate. The previous aqueous layer (1) was combined with the aqueous layer and acidified by adding 1 N hydrochloric acid, and extraction was performed with chloroform. The organic layers were combined and dried over magnesium sulfate, and filtration and concentration were performed, thereby obtaining the title compound as a brown viscous product.
MS(ESI)m/z:194[M+H]⁺

### Reference Example 343

### Production of 6-methylpyrano[3,4-b]pyridin-8-one

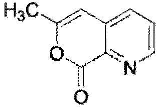

2,000 mg of 3-bromopyridine-2-carboxylic acid, 1,487 mg of pentane-2,4-dione, 189 mg of copper iodide, and 4,203 mg of potassium phosphate were suspended in 15 mL of N,N-dimethylformamide, and the mixture was stirred at 120°C for 6 hours. Water and ethyl acetate were added to the reaction mixture, filtration was performed through celite, the aqueous layer and the organic layer were separated, and then extraction from the aqueous layer was performed three times with chloroform. The organic layers were combined and then dried over sodium sulfate, filtration was performed, and the filtrate was concentrated under reduced pressure. The resulting residue was purified by silica gel column chromatography (solvent: hexane/ethyl acetate = 50/50 to 0/100), and fractions containing only a target product were concentrated. Since N,N-dimethylformamide was contained in a large amount, it was dissolved in ethyl acetate, water was added, and washing was performed. The organic layer was dried over sodium sulfate and then filtered, and the filtrate was concentrated under reduced pressure, thereby obtaining 187 mg of the title compound as a brown product. Other fractions containing the target product were concentrated, and the resulting residue was purified by silica gel column chromatography (solvent: hexane/ethyl acetate = 50/50 to 0/100), thereby obtaining 97 mg of the title compound as a yellow powder. In addition, 284 mg of the title compound (yield 18%) was obtained.
MS(ESI)m/z:162[M+H]⁺

### Reference Example 344

### Production of tert-butyl 6-(6-methyl-8-oxo-5,6-dihydro-1,7-naphthyridin-7-yl)-2-azaspiro [3.3]heptane-2-carboxylate

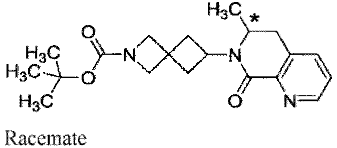

100 mg of 6-methylpyrano[3,4-b]pyridin-8-one was dissolved in 2 mL of methanol, 132 mg of tert-butyl 6-amino-2-azaspiro[3.3]heptane-2-carboxylate was added, and the mixture was stirred room temperature for 16 hours. The temperature was raised to 50°C, and the mixture was stirred for 3 hours. The reaction solution was cooled to room temperature, and 47 mg of sodium borohydride was added. The mixture was stirred at 50°C for 1 hour. The solvent was substituted with 2 mL of ethanol, the temperature was raised to 80°C, and the mixture was stirred for 16 hours. The solvent was substituted with 2 mL of N,N-dimethylformamide, the temperature was raised to 120°C, and the mixture was stirred for 7 hours. Water was added to the reaction mixture, and extraction was performed with chloroform. The organic layer was washed with water and brine, filtration was performed after drying with magnesium sulfate, and the filtrate was concentrated under reduced pressure. The resulting residue was dissolved in dimethyl sulfoxide and purified by reverse-phase HPLC (solvent: 10 mM ammonium carbonate aqueous solution/acetonitrile = 70/30 to 40/60), and then purified by silica gel column chromatography (solvent: ethyl acetate/methanol = 100/0 to 95/5), thereby obtaining 22 mg of the title compound (yield 10%) as a light yellow oily product.
MS(ESI)m/z:358[M+H]⁺

### Reference Example 345

### Production of ethyl 2-acetonylpyridine-3-carboxylate

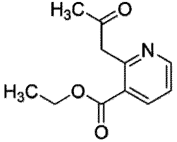

1,000 mg of ethyl 2-chloropyridine-3-carboxylate, 20 mL of acetone, 493 mg of tris(dibenzylideneacetone) dipalladium, 393 mg of 2-(dicyclohexylphosphino)-2'-methylbiphenyl, and 3.43 g of potassium phosphate were suspended in 10 mL of tetrahydrofuran, and the mixture was heated and refluxed under a nitrogen atmosphere for 4 hours. To the reaction solution, 247 mg of tris(dibenzylideneacetone) dipalladium and 196 mg of 2-(dicyclohexylphosphino)-2'-methylbiphenyl were added, and the mixture was heated and refluxed under a nitrogen atmosphere for 16 hours. The insoluble matter in the reaction solution was filtered out with diatomaceous earth, and then filtrate was concentrated under reduced pressure. The resulting residue was purified by silica gel column chromatography (solvent: hexane/ethyl acetate = 60/40 to 40/60) to obtain 163 mg of the title compound (purity 39%, yield 6%) as a brown powder.
MS(ESI)m/z:208[M+H]⁺

### Reference Example 346

### Production of methyl 2-[(E)-prop-1-enyl]-6-(trifluoromethyl)pyridine-3-carboxylate

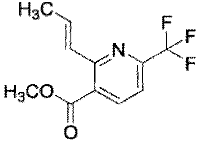

410 mg of 2-[(E)-prop-1-enyl]-6-(trifluoromethyl)pyridine-3-carboxylic acid (purity 56%) was dissolved in 5 mL of acetonitrile, 206 mg of potassium carbonate and 92.7 µL of methyl iodide were added, and the mixture was stirred at room temperature for 15 hours. To the reaction solution, 275 mg of potassium carbonate and 124 µL methyl iodide were added, and the mixture was stirred at room temperature for 7 hours. To the reaction solution, 412 mg of potassium carbonate and 185 µL methyl iodide were added, and the mixture was stirred at room temperature for 17 hours. Water was added to the reaction mixture, and extraction was performed with ethyl acetate. The organic layer was washed with brine, filtration was performed after drying with magnesium sulfate, and the filtrate was concentrated under reduced pressure. The resulting residue was purified by silica gel column chromatography (hexane/ethyl acetate = 95/5 to 85/15) to obtain 187 mg of the title compound (yield 77%) as a colorless viscous product.
MS(ESI)m/z:246[M+H]⁺

### Reference Example 347

### Production of ethyl 2-[(E)-3,3,3-trifluoroprop-1-enyl]pyridine-3-carboxylate

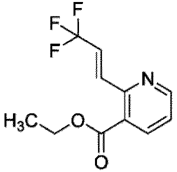

150 mg of ethyl 2-vinylpyridine-3-carboxylate, 559 mg of 3,3-dimethyl-1-(trifluoromethyl)-1,2-benziodoxole, 63 mg of tetrakis(acetonitrile) copper(I) hexafluorophosphate, and 0.255 mL of diazabicycloundecene were dissolved in 3 mL of N,N-dimethylformamide, and the mixture was stirred at 60°C for 3 hours. Water was added to the reaction mixture, and extraction was performed with ethyl acetate. The organic layer was washed with water and brine, filtration was performed after drying with magnesium sulfate, and the filtrate was concentrated under reduced pressure. The resulting residue was purified by silica gel column chromatography (solvent: hexane/ethyl acetate = 98/2 to 88/12) to obtain 70 mg of the title compound (yield 34%) as a colorless powder.
MS(ESI)m/z:246[M+H]⁺

### Reference Example 348

### Production of N- [2-(2-fluorophenyl)sulfonyl-2-azaspiro [3.3]heptan-6-yl] -N-[(1R)-2-hydroxy-1-methyl-ethyl-]-2-(trifluoromethyl)-1H-imidazole-5-carboxamide

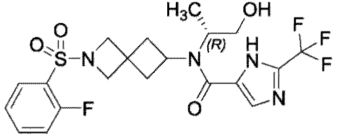

(1) In a 30 mL eggplant flask, 260.2 mg of 2-(trifluoromethyl)-1H-imidazole-5-carboxylic acid was put and dissolved in 0.020 mL of dimethylformamide and 6 mL of dichloromethane. 0.30 mL of oxalyl chloride was added, the mixture was stirred at room temperature for 1 hour, and the reaction solvent was concentrated, thereby obtaining a crude product of 2-(trifluoromethyl)-1H-imidazole-5-carbonyl chloride.
(2) The crude product of 2-(trifluoromethyl)-1H-imidazole-5-carbonyl chloride obtained in (1) above was dissolved in 4 mL of acetonitrile, and a solution of 150 mg of (2R)-2[[2-(2-fluorophenyl)sulfonyl-2-azaspiro[3.3]heptan-6-yl]amino]propan-1-ol and 4 mL of acetonitrile, and 0.60 mL of triethylamine were added. The mixture was stirred at room temperature for 1.5 hours, 6 mL of a 1 N sodium hydroxide aqueous solution was added, and the mixture was further stirred at room temperature for 2 hours. 1 N hydrochloric acid was added, extraction was performed with chloroform at pH = 8, extraction was performed with ethyl acetate at pH = 7, and extraction was performed with ethyl acetate at pH = 6. The organic layer was collected and washed with brine, filtered, and concentrated. The resulting residue was purified by silica gel chromatography (solvent: chloroform/methanol = 100/0 to 92/8) to obtain 57.9 mg of the title compound (yield 8.3%) as a colorless amorphous product.
   MS(ESI)m/z:491[M+H]⁺

### Reference Example 349

### tert-butyl 6-(1,3-dioxoindolin-2-yl)-2-azaspiro[3.3]heptane-2-carboxylate

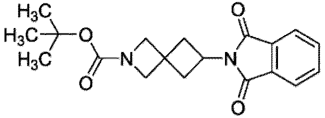

In a 100 mL eggplant flask, 462 mg of tert-butyl 6-amino-2-azaspiro[3.3]heptane-2-carboxylate and 374 mg of phthalic anhydride were weighed, 10 mL of toluene and 100 µL of triethylamine were added, and the mixture was heated and refluxed under installation of a Dean-Stark extraction apparatus for 160 minutes. Thereafter, 127 mg of phthalic anhydride and 100 µL of triethylamine were additionally added, and subsequently, the mixture was heated and refluxed for 150 minutes. The reaction solution was cooled to room temperature, ethyl acetate and brine were added, and then the organic layer was extracted. The organic layer was washed with brine and then dried over magnesium sulfate. The insoluble matter was filtered, and then the solution was concentrated under reduced pressure. 1.14 g of the resulting colorless oily product was purified by silica gel column chromatography (solvent: hexane/ethyl acetate = 100/0 to 75/25) to obtain 673 mg of the title compound (yield 90%) as a colorless powder.
MS(ESI)m/z:287[M-tBu+H]⁺

### Reference Examples 350 to 352:

Corresponding raw material compounds were treated in the same manner as that of Example 45 to obtain compounds shown in Table 72.

**[Table 72]**

| Reference Example | Structural formula | Physical property value and the like |
|---|---|---|
| 350 | 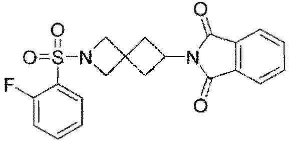 | MS (ESI) m/z;401 [M+H]+ |
| 351 | 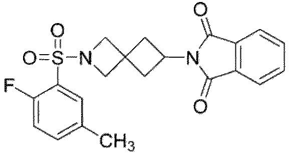 | MS(ESI) m/z;415 [M+H]+ |
| 352 | 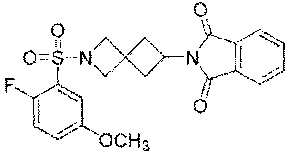 | MS(ESI) m/z;431 [M+H]+ |

### Reference Example 353

### Production of spiro[cyclopropane-1,3'-isoindolin]-1'-one

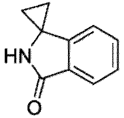

640 mg of methyl 2-cyanobenzoate was dissolved in 20 mL of diethyl ether, and 1.3 mL of tetraisopropyl orthotitanate was added. 7.94 mL of ethylmagnesium bromide (1 mol/L tetrahydrofuran solution) was added dropwise thereto for 5 minutes. The mixture was stirred at room temperature for 4 hours. To the reaction mixture, 1.99 mL of ethylmagnesium bromide (1 mol/L tetrahydrofuran solution) was added dropwise, and the mixture was stirred at room temperature for 0.5 hours. To the reaction solution, 1.99 mL of ethylmagnesium bromide (1 mol/L tetrahydrofuran solution) was added dropwise, and the mixture was stirred at room temperature for 0.5 hours. A 1 mol/L hydrochloric acid aqueous solution was added to the reaction mixture, and extraction was performed with ethyl acetate. The organic layer was washed with brine, filtration was performed after drying with magnesium sulfate, and the filtrate was concentrated under reduced pressure. The resulting residue was purified by silica gel column chromatography (solvent: chloroform/methanol = 100/0 to 92/8) to obtain 213 mg of the title compound (yield 34%) as a brown powder.
MS(ESI)m/z:160[M+H]⁺

### Reference Example 354

### Production of tert-butyl 6-(3-oxo-1H-indazol-2-yl)-2-azaspiro[3.3]heptane-2-carboxylate

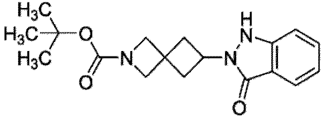

590 mg of tert-butyl 6-[(2-nitrobenzoyl)amino]-2-azaspiro[3.3]heptane-2-carboxylate was suspended in 5 mL of ethanol, 263 mg of zinc and 4.02 mL of a 1 mol/L sodium hydroxide aqueous solution were added, and the mixture was heated and refluxed for 41 hours. The reaction mixture was filtered through celite, water was added to the filtrate, and extraction was performed with ethyl acetate. Re-extraction was performed with tetrahydrofuran because the target product remained in the aqueous layer, the organic layer was washed with brine, filtration was performed after drying with magnesium sulfate, and the filtrate was concentrated under reduced pressure. The resulting residue was purified by silica gel column chromatography (solvent: hexane/ethyl acetate = 50/50 to 0/100) to obtain 253 mg of the title compound (yield 57%) as a brown powder.
MS(ESI)m/z:330[M+H]⁺

### Reference Example 355

### Production of methyl 5-acetyl-2-methyl-thiazole-4-carboxylate

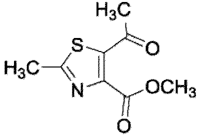

In a 50 mL eggplant flask, 992 mg of methyl 5-bromo-2-methyl-thiazole-4-carboxylate was weighed, and 15 mL of 1,4-dioxane was added. Under a nitrogen atmosphere, 2.20 mL of tributyl(1-ethoxyvinyl)tin and 378 mg of tetrakistriphenylphosphine palladium were added, and the mixture was stirred at 110°C for 7 hours. The reaction solution was cooled to room temperature and diluted with ethyl acetate. The resultant was washed with brine and then dried over magnesium sulfate. The insoluble matter was filtered, and then the solution was concentrated under reduced pressure. 3.66 g of the resulting brown oily product was dissolved in 25 mL of tetrahydrofuran, 10 mL of a 1 mol/L hydrochloric acid was added, and the mixture was stirred at room temperature for 140 minutes. The reaction solution was diluted with ethyl acetate, a saturated sodium bicarbonate aqueous solution was added, and the organic layer was extracted. The organic layer was washed with brine and then dried over magnesium sulfate. The insoluble matter was filtered, and then the solution was concentrated under reduced pressure. 3.59 g of the resulting brown oily product was purified by silica gel column chromatography (solvent: hexane/ethyl acetate = 80/20 to 40/60) to obtain 501 mg of the title compound (yield 60%) as a yellow oily product.
MS(ESI)m/z:200[M+H]⁺

### Reference Example 356:

A corresponding raw material compound was treated in the same manner as that of Reference Example 355 to obtain a compound shown in Table 73.

**[Table 73]**

| Reference Example | Structural formula | Physical property value and the like |
|---|---|---|
| 356 | 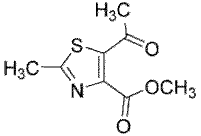 | MS(ESI) m/z;200 [M+H]+ |

### Reference Example 357

### Production of isopropyl 5-[1-[[2-(2-fluorophenyl)sulfonyl-2-azaspiro [3.3]heptan-6-yl] amino] ethyl] -2-methyl-thiazole-4-carboxyllate

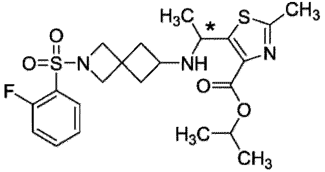

In a 30 mL eggplant flask, 148 mg of 2-(2-fluorophenyl)sulfonyl-2-azaspiro[3.3]heptan-6-amine and 133 mg of 5-acetyl-2-methyl-thiazole-4-carboxylic acid methyl ester were weighed, and 5.0 mL of tetrahydrofuran was added. Next, 480 µL of titanium tetraisopropoxide was added, and the mixture was stirred at room temperature for 4 hours and 30 minutes. Next, 426 mg of sodium triacetoxyborohydride was added to the reaction solution at 60°C for 1 hour and then further stirred at 75°C for 2 hours. Thereafter, the mixture was allowed to stand at room temperature overnight. A saturated sodium bicarbonate aqueous solution was added to the reaction solution, and extraction was performed with ethyl acetate. The organic layer was washed with brine and then dried over sodium sulfate. The insoluble matter was filtered, and then the solution was concentrated under reduced pressure. 302 mg of the resulting pale yellow oily product was purified by NH silica gel column chromatography (solvent: hexane/ethyl acetate = 60/40 to 25/75) to obtain 241 mg of a mixture of the title compound and 5-[1-[[2-(2-fluorophenyl)sulfonyl-2-azaspiro[3.3]heptan-6-yl]amino]ethyl]-2-methyl-thiazole-4-carboxylic acid methyl ester (isopropyl ester:methyl ester = 78:22) as a colorless oily product.
MS(ESI)m/z:482,454[M+H]⁺

### Reference Example 358

### Production of 3-[2-(2-fluorophenyl)sulfonyl-2-azaspiro[3.3]heptan-6-yl]-4-methyl-3-azabicyclo[5.1.0]oct-5-en-2-one

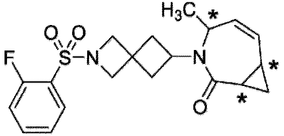

### Cis Configuration of Cyclopropane Moiety and Mixture of Four Types of Diastereomers

65 mg of (1S*,2S*)-N-[2-(2-fluorophenyl)sulfonyl-2-azaspiro[3.3]heptan-6-yl]-N-(1-methylallyl)-2-vinyl-cyclopropanecarboxamide was dissolved in 7.8 mL of dichloroethane, 9.7 mg of a second-generation Hoveyda-Grubbs catalyst was added, and the mixture was stirred at room temperature overnight. To the reaction solution, 19.5 mg of a second-generation Hoveyda-Grubbs catalyst was added, and the mixture was stirred at 70°C for 2 hours. The reaction mixture was concentrated under reduced pressure. The resulting residue was purified by silica gel column chromatography (solvent: hexane/ethyl acetate = 0/100) and then purified again by silica gel column chromatography (solvent: hexane/ethyl acetate = 0/100), thereby obtaining 43.6 mg of the title compound (yield 72%) as a brown oily product.
MS(ESI)m/z:391[M+H]⁺

### Reference Example 359:

A corresponding raw material compound was treated in the same manner as that of Example 1 to obtain a compound shown in Table 74.

**[Table 74]**

| Reference Example | Structural formula | Physical property value and the like |
|---|---|---|
| 359 | 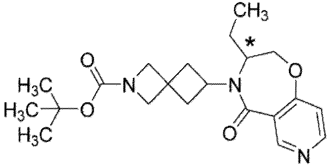 | MS(ESI) m/z;388 [M+H]+ |

### Reference Examples 360 and 361:

Corresponding raw material compounds were treated in the same manner as that of Reference Example 160 to obtain compounds shown in Table 75.

**[Table 75]**

| Reference Example | Structural formula | Physical property value and the like |
|---|---|---|
| 360 | 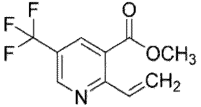 | MS(ESI) m/z;232 [M+H]+ |
| 361 | 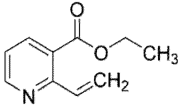 | MS(ESI) m/z;178 [M+H]+ |

### Reference Example 362:

A corresponding raw material compound was treated in the same manner as that of Reference Example 185 to obtain a compound shown in Table 76.

**[Table 76]**

| Reference Example | Structural formula | Physical property value and the like |
|---|---|---|
| 362 | 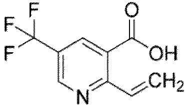 | MS(ESI) m/z;218 [M+H]+ |

### Reference Example 363:

A corresponding raw material compound was treated in the same manner as that of Reference Example 1 to obtain a compound shown in Table 77.

**[Table 77]**

| Reference Example | Structural formula | Physical property value and the like |
|---|---|---|
| 363 | 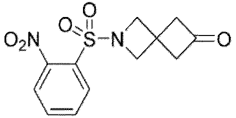 | MS(ESI) m/z;297 [M]+ |

### Reference Examples 364 and 365

Tert-butyl 6-amino-2-azaspiro[3.3]heptan-2-carboxylate and corresponding compounds were used and treated in the same manner as in Example 423 to obtain compounds shown in Table 78.

**[Table 78]**

| Example | Structural formula | Physical property value and the like |
|---|---|---|
| 364 | 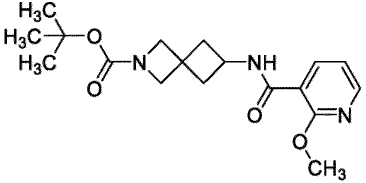 | MS(ESI) m/z; 348 [M+H]+ |
| 365 | 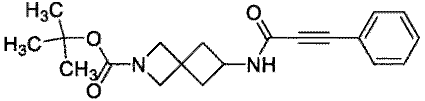 | MS(ESI) m/z; 340 [M+H]+ (APCI) |

### Reference Example 366

### Production of tert-butyl 6-[(2-methoxypyridine-3-carbonyl)-propylamino]-2-azaspiro[3.3]heptane-2-carboxylate

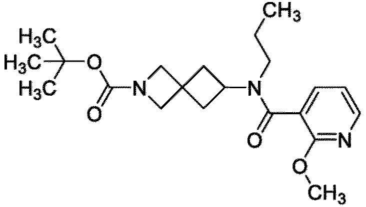

To a mixture of 1.68 g of tert-butyl 6-[(2-methoxypyridine-3-carbonyl)amino]-2-azaspiro[3.3]heptane-2-carboxylate and 15 mL of N,N-dimethylformamide, 240 mg of sodium hydride (60%) was added and the mixture was stirred at room temperature for 10 minutes. To the reaction mixture, 1.0 mL of 1-iodopropane was added and the mixture was stirred at room temperature for 2 hours. To the reaction mixture, 218 mg of sodium hydride (60%) and 1.0 mL of 1-iodopropane were added and the mixture was stirred for 2 hours. The reaction mixture was diluted with ethyl acetate:hexane = 1:1 mixture (80 mL) and water was added thereto. The organic layer was separated, washed with brine, and dried over magnesium sulfate. Insoluble material was removed by filtration, and the filtrate was concentrated under reduced pressure. The resulting residue was purified by silica gel column chromatography (solvent: hexane/ethyl acetate = 75/25 to 25/75) to obtain 1.70 g of the title compound (yield 90%) as a colorless solid.
MS(ESI)m/z:390[M+H]⁺

### Reference Example 367

A corresponding raw material compound was used and treated in the same manner as in Reference Example 366 to obtain a compound shown in Table 79.

**[Table 79]**

| Example | Structural formula | Physical property value and the like |
|---|---|---|
| 367 | 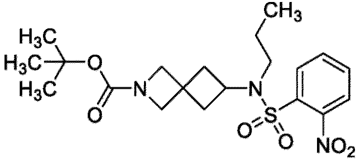 | MS(ESI) m/z; 340 [M-Boc+H]+ |

### Reference Example 368

### Production of N-(2-azaspiro[3.3]heptan-6-yl)-2-methoxy-N-propylpyridine-3-carboxamide

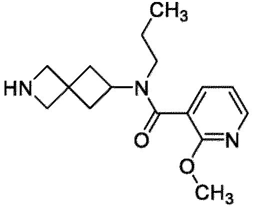

Tert-butyl 6-[(2-methoxypyridine-3-carbonyl)-propylamino]-2-azaspiro[3.3]heptane-2-carboxylate was used and treated as in Example 442 to obtain the title compound.
MS(ESI)m/z:290[M+H]⁺

### Reference Example 369

### Production of 1-[2-[(2-methylpropan-2-yl)oxycarbonyl]-2-azaspiro[3.3]heptan-6-yl]-5-oxo-3-phenyl-2H-pyrrole-2-carboxylic acid

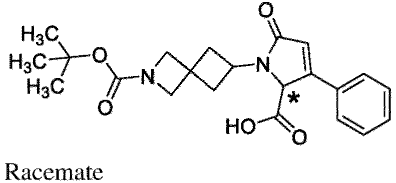

To a mixture of 652 mg of tert-butyl 6-(3-phenylprop-2-inoylamino)-2-azaspiro[3.3]heptane-2-carboxylate and 20 mL of N,N-dimethylformamide, 230 mg sodium hydride (60%) and 0.272 mL of methyl bromoacetate were added in this order under ice-cold conditions, and the mixture was stirred at room temperature all night. Saturated aqueous ammonium chloride solution was added to the reaction mixture and the mixture was extracted twice with ethyl acetate. The aqueous layer was acidified with 1M hydrochloric acid and then extracted twice with ethyl acetate. All organic layers were combined, washed with brine, and dried over magnesium sulfate. Insoluble matter was removed by filtration and the filtrate was concentrated under reduced pressure to obtain 648 mg of the crude product of the title compound (yield 85%) as a pale yellow powder.
MS(APCI)m/z:399[M+H]⁺

### Reference Example 370

### Production of tert-butyl 6-[2-(hydroxymethyl)-5-oxo-3-phenyl-2H-pyrrol-1-yl]-2-azaspiro[3.3]heptane-2-carboxylate

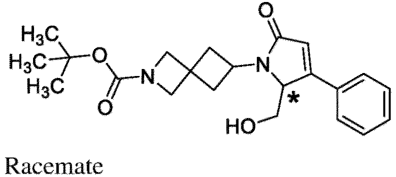

To a mixture of 200 mg of 1-[2-[(2-methylpropan-2-yl)oxycarbonyl]-2-azaspiro[3.3]heptan-6-yl]-5-oxo-3-phenyl-2H-pyrrole-2-carboxylic acid, 0.105 mL of triethylamine and 5 mL of tetrahydrofuran, 0.058 mL of ethyl chloroformate was added, and the mixture was stirred at room temperature for 1 hour and 30 minutes. To the reaction mixture, 50 mg of sodium borohydride and 5 mL of methanol were added, and the mixture was stirred at room temperature for 1 hour and 30 minutes. 1M hydrochloric acid was added to the reaction mixture and the mixture was extracted twice with ethyl acetate. The organic layers were combined, washed with brine, and dried over magnesium sulfate. Insoluble material was removed by filtration, and the filtrate was concentrated under reduced pressure. The resulting residue was purified by silica gel column chromatography (solvent: hexane/ethyl acetate = 40/60 to 0/100) to obtain 112 g of the title compound (yield 58%) as a yellow powder.
MS(ESI)m/z:385[M+H]⁺

### Reference Example 371

### Production of tert-butyl 6-[2-(methoxymethyl)-5-oxo-3-phenyl-2H-pyrrol-1-yl]-2-azaspiro[3.3]heptane-2-carboxylate

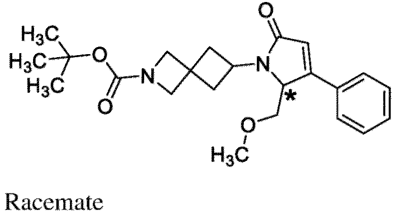

To a mixture of 112 mg of tert-butyl 6-[2-(hydroxymethyl)-5-oxo-3-phenyl-2H-pyrrol-1-yl]-2-azaspiro[3.3]heptane-2-carboxylate, 0.054 mL of methyl iodide and 3 mL of N,N- dimethylformamide, 21 mg of sodium hydride (60%) was added under ice-cold conditions, and the mixture was stirred for 40 minutes while the temperature was raised to room temperature. Water was added to the reaction mixture and the mixture was extracted twice with ethyl acetate. The organic layers were combined, washed with brine, and dried over magnesium sulfate. Insoluble material was removed by filtration, and the filtrate was concentrated under reduced pressure. The resulting residue was purified by silica gel column chromatography (solvent: hexane/ethyl acetate = 70/30 to 30/70) to obtain 103 g of the title compound (yield 78%) as a pale yellow viscous material.
MS(ESI)m/z:399[M+H]⁺

### Reference Examples 372-374

Compounds shown in Table 80 were obtained by treating corresponding compounds in the same manner as in Example 45 or Example 82.

**[Table 80]**

| Example | Structural formula | Physical property value and the like |
|---|---|---|
| 372 | 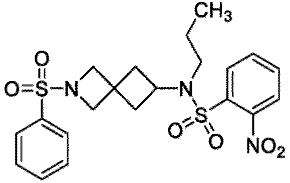 | MS(ESI) m/z; 480 [M+H]+ |
| 373 | 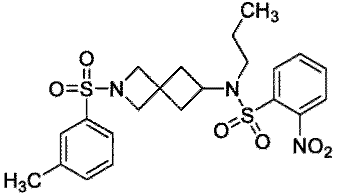 | MS(ESI) m/z; 494 [M+H]+ |
| 374 | 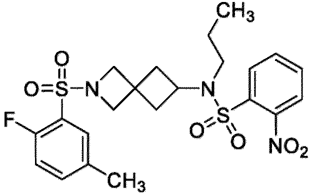 | MS(ESI) m/z; 512 [M+H]+ |

### Reference Example 375

### Production of 2-(2-fluoro-5-methylphenyl)sulfonyl-N-propyl-2-azaspiro[3.3]heptan-6-amine

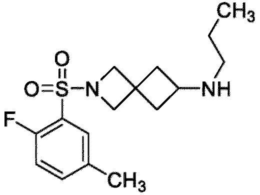

To a mixture of 446mg of N-[2-(2-fluoro-5-methylphenyl)sulfonyl-2-azaspiro[3.3]heptan-6-yl]-2-nitro-N-propylbenzenesulfonamide and 8 mL of N,N-dimethylformamide, 241 mg of mercaptoacetic acid and 220 mg of lithium hydroxide monohydrate were added sequentially, and the mixture was stirred at room temperature for 3 hours. Saturated aqueous sodium bicarbonate solution and ethyl acetate were added to the reaction mixture and the mixture was divided. The organic layer was washed with brine and dried over sodium sulfate. Insoluble material was removed by filtration, and the filtrate was concentrated under reduced pressure. The resulting residue was purified by silica gel column chromatography (solvent: chloroform/methanol = 100/0 to 92/8) to obtain 270 mg of the title compound (yield 95%) as a pale yellow viscous.
MS(ESI)m/z:327[M+H]⁺

### Reference Examples 376 and 377

Corresponding raw material compounds were used and treated in the same manner as in Reference Example 375 to obtain compounds shown in Table 81.

**[Table 81]**

| Example | Structural formula | Physical property value and the like |
|---|---|---|
| 376 | 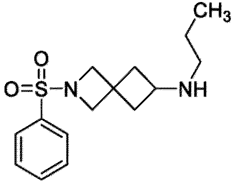 | MS(ESI) m/z; 295 [M+H]+ |
| 377 | 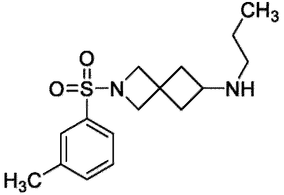 | MS(ESI) m/z; 309 [M+H]+ |

### Reference Example 378

### Production of 2-ethoxypyridine-3-carboxylic acid

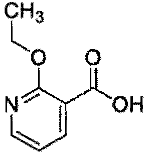

A mixture of 706 mg of 2-chloropyridine-3-carboxylic acid and 20 mL of ethanol was stirred under ice-cold conditions, 291 mg of sodium hydride (60%) was added in portions, and the mixture was heated to reflux for 4 hours. To the reaction mixture, 4 mL of dimethyl sulfoxide was added and the mixture was stirred at 90°C over night. The reaction mixture was brought to room temperature, the pH was adjusted to 4 to 5 by aqueous hydrochloric acid solution and saturated aqueous sodium bicarbonate solution, and then brine was added. The mixture was extracted three times with chloroform. The organic layers were combined and dried over magnesium sulfate. Insoluble material was removed by filtration, and the filtrate was concentrated under reduced pressure. The resulting residue was purified by reversed-phase HPLC (solvent: 0.05% trifluoroacetic acid - water / 0.05% trifluoroacetic acid - acetonitrile = 80/20 to 75/25) to obtain 368 mg of the title compound (yield 49%) as a colorless solid.
MS(ESI)m/z: 168[M+H]⁺

### Reference Examples 379 to 384

Corresponding raw material compounds were treated in the same manner as in Reference Example 3 to obtain compounds shown in Table 82.

**[Table 82]**

| Example | Structural formula | Physical property value and the like |
|---|---|---|
| 379 | 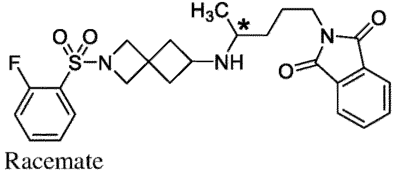 | MS(ESI) m/z; 486 [M+H]+ |
| 380 | 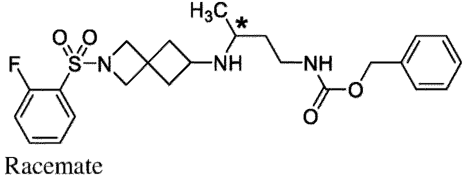 | MS(ESI) m/z; 476 [M+H]+ |
| 381 | 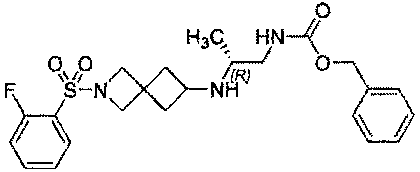 | MS(ESI) m/z; 462 [M+H]+ |
| 382 | 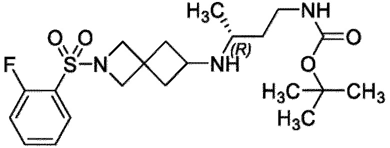 | MS(ESI) m/z; 442 [M+H]+ |
| 383 | 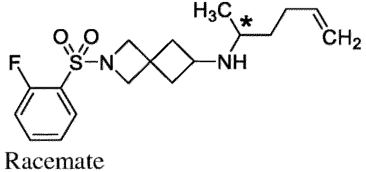 | MS(ESI) m/z; 353 [M+H]+ |
| 384 | 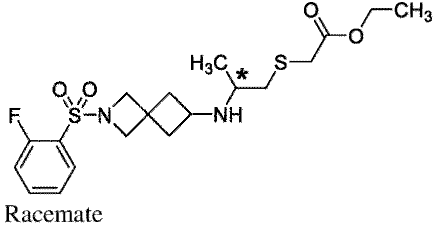 | MS(ESI) m/z; 431 [M+H]+ |

### Reference Example 385

### Production of tert-butyl 3-[(2R)-2-[[2-(2-fluorophenyl)sulfonyl-2-azaspiro[3.3]heptan-6-yl]amino propoxyl prop anoate

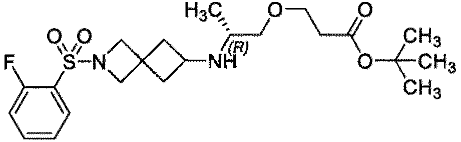

(1) A mixture of 230 mg of tert-butyl 3-[(2R)-2-(benzyloxycarbonylamino)propoxy]propanoate, 80 mg of 10% palladium carbon and 5 mL of tetrahydrofuran was stirred at room temperature under a hydrogen atmosphere for 3 hours. After nitrogen replacement in the system and stirring, insoluble matter was removed by filtration, and the filtrate was concentrated under reduced pressure to obtain a crude product of tert-butyl 3-[(2R)-2-aminopropoxy]propanoate.
(2) The crude product of tert-butyl 3-[(2R)-2-aminopropoxy]propanoate and the corresponding compound were used and treated in the same manner as in Reference Example 3 to obtain the title compound.
   MS(ESI)m/z:457[M+H]⁺

### Reference Example 386

### Production of 4-N-[2-(2-fluorophenyl)sulfonyl-2-azaspiro[3.3]heptan-6-yl]pentane-1,4-diamine

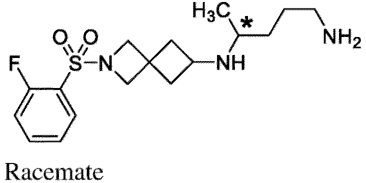

To a mixture of 459 mg of 2-[4-[[2-(2-fluorophenyl)sulfonyl-2-azaspiro[3.3]heptan-6-yl]amino]pentyl]isoindole-1,3-dione and 4.4 mL of methanol, 48.9 mg of hydrazine monohydrate was added and the mixture was stirred at room temperature for 1 hour and 30 minutes. To the reaction mixture, 111 mg of hydrazine monohydrate was added, and the mixture was stirred at 40°C for 3 hours, and then allowed to stand over night. To the reaction mixture, 1M hydrochloric acid and chloroform were added, and after stirring, the insoluble material was removed by filtration, and the filtrate was concentrated under reduced pressure. To the resulting residue, 1M hydrochloric acid was added and the mixture was extracted twice with chloroform. After the aqueous layer was adjusted to pH > 10 by 1M aqueous sodium hydroxide solution, chloroform was added and the mixture was stirred. The organic layer was separated with Phase-separator^{®} and concentrated under reduced pressure to obtain 311 mg of the title compound (yield 98%) as a pale yellow viscous.
MS(ESI)m/z:356[M+H]⁺

### Reference Example 387

### Production of 2-chloro-N-[2-(2-fluorophenyl)sulfonyl-2-azaspiro[3.3]heptan-6-yl]-N-(4-hydroxybutan-2-yl)acetamide

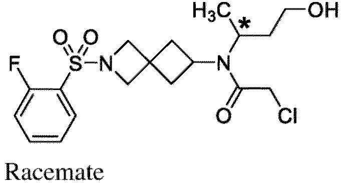

(1) To a mixture of 200 mg of of 2-(2-fluorophenyl)sulfonyl-2-azaspiro[3.3]heptan-6-amine, 84.7 mg of 4-hydroxy-2-butanone and 3.7 mL of methylene chloride, 0.042 mL of acetic acid was added, and the mixture was stirred at room temperature for 10 minutes. Then 235 mg of sodium triacetoxyborohydride was added and the mixture was stirred at room temperature for 1 hour. To the reaction mixture was added a saturated aqueous sodium bicarbonate solution, and after stirring, the organic layer was separated with Phase-separator^{®} and concentrated under reduced pressure to obtain a crude product of 3-[[2-(2-fluorophenyl)sulfonyl-2-azaspiro[3.3]heptan-6-yl]amino]butan-1-ol as a colorless viscous.
(2) To a mixture of the crude product of 3-[[2-(2-fluorophenyl)sulfonyl-2-azaspiro[3.3]heptan-6-yl]amino]butan-1-ol obtained in the above (1) and 3.7 mL of acetonitrile, 0.309 mL of trimethylamine and 0.177 mL of chloroacetyl chloride were added sequentially and the mixture was stirred at room temperature for 1 hour. To the reaction mixture, 3.7 mL of 1M aqueous sodium hydroxide solution was added and the mixture was stirred at room temperature for 5 hours. Water was added to the reaction mixture and the mixture was extracted with ethyl acetate. After washing the organic layer with brine, the organic layer was separated with Phase-separator^{®} and concentrated under reduced pressure. The resulting residue was purified by silica gel column chromatography (solvent: hexane/ethyl acetate = 50/50 to 0/100) to obtain 285 mg of the title compound (yield 92%) as a pale yellow viscous.
   MS(ESI)m/z:419/421[M+H]⁺

Reference Example 388

### Production of N- [2-(2-fluorophenyl)sulfonyl-2-azaspiro [3.3]heptan-6-yl] -N-hexa-5-en-2-ylprop-2-enamide

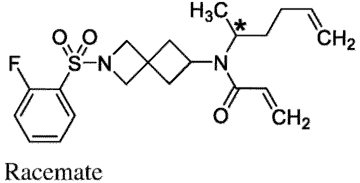

To a mixture of 170 mg of 2-(2-fluorophenyl)sulfonyl-N-hexa-5-en-2-yl-2-azaspiro[3.3]heptan-6-amine and 4.8 mL of chloroform, 0.201 mL of trimethylamine and 87.3 mg of acryloyl chloride were added sequentially, and the mixture was stirred at room temperature for 30 minutes. Saturated aqueous sodium bicarbonate solution and chloroform were added to the reaction mixture and the mixture was stirred, then the organic layer was separated with Phase-separator^{®} and concentrated under reduced pressure. The resulting residue was purified by silica gel column chromatography (solvent: hexane/ethyl acetate = 100/0 to 20/80) to obtain 187 mg of the title compound (yield 95%) as a colorless viscous.
MS(ESI)m/z:407[M+H]⁺

### Reference Example 389

A compound shown in Table 83 was obtained by using and treating a corresponding raw material compound and reagents in the same manner as in Reference Example 388.

**[Table 83]**

| Example | Structural formula | Physical property value and the like |
|---|---|---|
| 389 | 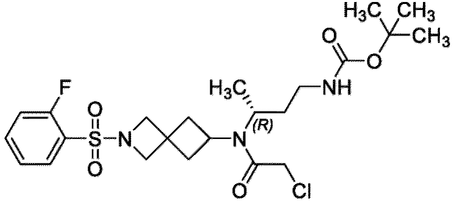 | MS(ESI) m/z; 518/520 [M+H]+ |

### Reference Example 390

### Production of tert-butyl (SR)-4-[2-(2-fluorophenyl)sulfonyl-2-azaspiro[3.3]heptan-6-yl]-5-methyl-3-oxo-1,4-diazepan-1-carboxylate

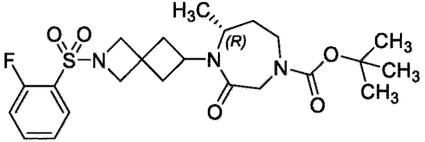

To a mixture of 1.76 g of tert-butyl N-[(3R)-3-[(2-chloroacetyl)-[2-(2-fluorophenyl)sulfonyl-2-azaspiro[3.3]heptan-6-yl]amino]butyl]carbamate and 17 mL of N,N-dimethylformamide, 177 mg of sodium hydride (60%) was added and the mixture was stirred at room temperature for 30 minutes. Water was added to the reaction mixture and the mixture was extracted with ethyl acetate. After washing the organic layer with water and brine in that order, the organic layer was separated with Phase-separator^{®} and concentrated under reduced pressure. The resulting residue was purified by silica gel column chromatography (solvent: ethyl acetate/methanol = 100/0 to 95/5) to obtain 1.39 g of the title compound (yield 79%) as a colorless powder.
MS(ESI)m/z:482[M+H]⁺

### Reference Example 391

### Production of 1-[2-(2-fluorophenyl)sulfonyl-2-azaspiro[3.3]heptan-6-yl]-2-methyl-3,4-dihydro-2H-azepine-7-one

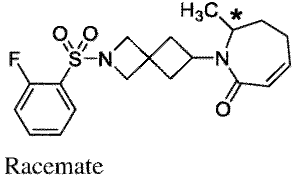

A corresponding raw material compound was treated in the same manner as in Reference Example 358 to obtain the title compound.
MS(ESI)m/z:379[M+H]⁺

### Reference Example 392

### Production of tert-butyl 3-[(2R)-2-(benzyloxycarbonylamino)propoxy]propanoate

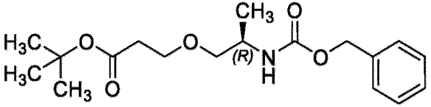

To a mixture of 500 mg of benzyl N-[(1R)-2-hydroxy-1-methylethyl]carbamate, 1.01 g of cesium carbonate and 12 mL of tetrahydrofuran, 0.70 mL of tert-butyl acrylate was added and the mixture was stirred at room temperature for 2 days. Water was added to the reaction mixture and the mixture was extracted with ethyl acetate. The organic layer was washed with water and brine and dried over magnesium sulfate. The insoluble material was removed by filtration, and the filtrate was concentrated under reduced pressure. The resulting residue was purified by silica gel column chromatography (solvent: hexane/ethyl acetate = 100/0 to 50/50) to obtain 239 mg of the title compound (yield 30%) as a colorless oil.
MS(ESI)m/z:338[M+H]⁺

### 2. TRH-DE Inhibition Test

### <Experimental Method>

### Test Compound:

The compounds described in "1. Preparation of Example Compounds" were used in a TRH-DE inhibition test.

### Method:

The enzyme inhibitory activity of each of the compounds was evaluated by measuring the amount of the product Cyclo (His-Pro) of the enzyme reaction using a RapidFire RF 365 HTMS system (manufactured by Agilent Technologies, Inc.) connected to a triple quadrupole mass spectrometer (API 4000, manufactured by SCIEX).

A membrane fraction prepared from Expi 293 cells transiently expressing human TRH-DE was used as an enzyme. The membrane fraction was suspended in 4 µg/ml of a reaction buffer (50 mM sodium phosphate (pH 7.4), 25 mM NaCl, 0.01% Triton X-100, 200 µM N-ehtylmaleimide, and 200µM bacitracin) and mixed with the test compound at each concentration. 10 µM TRH (Glp-His-Pro-NH2), which was a substrate, was added, and the mixture was reacted at room temperature for 2 hours. The reaction was stopped in the same amount of a solution of acetonitrile: methanol (1:1) containing 1 µM of the stable isotope Cyclo (His-Pro) (13C5:15N) as an internal standard, and the supernatant was collected after centrifugation.

In the RapidFire RF 365 HTMS system, a portion of the supernatant was aspirated, loaded onto a Graphic Carbon column (manufactured by Agilent Technologies, Inc.) with a buffer A (water, 0.09% formic acid, 0.01% trifluoroacetic acid) and washed with ultrapure water. Next, washing was performed with acetonitrile. The contents in the column were eluted with a buffer B (80% acetonitrile, 0.09% formic acid, 0.01% trifluoroacetic acid) and injected into a mass spectrometer for analysis. The product Cyclo (His-Pro) and the internal standard Cyclo (His-Pro) (13C5:15N) detected each ion with Q1/Q3 transitions m/z of 234.9 to 109.8 and m/z of 240.9 to 109.6. The ESI voltage was set to 5,500 V and the temperature was set to 650°C.

For the calculation of the inhibition rate, a Ratio value corrected by dividing the product amount by the internal standard amount was used. The inhibition rate (%) was calculated by % Inhibition = {1 - (Ratio value of sample - Ratio value of blank)/(Ratio value of control - Ratio value of blank) } × 100. The IC50 value was calculated from the concentration reactivity test at 16 points. The results are shown in Table 84.

### <Experimental Results>

**[Table 84-1]**

| Example | IC50(*µ*M) | Example | IC50(*µ*M) | Example | IC50(*µ*M) | Example | IC50(*µ*M) | Example | IC50(*µ*M) |
|---|---|---|---|---|---|---|---|---|---|
| 1 | 0.002 | 56 | 0.008 | 109 | 0.003 | 160 | 0.001 | 212 | 0.023 |
| 2 | 1.011 | 57 | 0.253 | 110 | 0.004 | 161 | 0.001 | 214 | 0.011 |
| 6 | 0.013 | 58 | 0.180 | 111 | 0.060 | 162 | 0.002 | 215 | 0.026 |
| 8 | 0.013 | 59 | 0.033 | 112 | 0.032 | 163 | 0.008 | 216 | 0.046 |
| 9 | 0.014 | 60 | 0.049 | 113 | 0.026 | 165 | 0.002 | 217 | 0.035 |
| 10 | 0.012 | 61 | 0.653 | 114 | 0.061 | 166 | 0.005 | 218 | 0.037 |
| 11 | 0.004 | 62 | 0.393 | 115 | 0.083 | 167 | 0.016 | 219 | 0.038 |
| 12 | 0.009 | 63 | 0.022 | 116 | 0.116 | 168 | 0.002 | 220 | 0.012 |
| 13 | 0.002 | 64 | 0.010 | 117 | 0.046 | 169 | 0.001 | 221 | 0.988 |
| 14 | 0.024 | 65 | 0.027 | 118 | 0.025 | 170 | 0.007 | 222 | 0.449 |
| 15 | 0.007 | 66 | 0.031 | 119 | 0.013 | 171 | 0.007 | 223 | 0.018 |
| 16 | 0.020 | 67 | 0.077 | 120 | 0.011 | 172 | 0.110 | 224 | 0.041 |
| 17 | 0.008 | 68 | 0.120 | 121 | 0.021 | 174 | 0.019 | 225 | 0.011 |
| 18 | 0.001 | 69 | 0.066 | 122 | 0.015 | 175 | 0.012 | 226 | 0.016 |
| 19 | 0.002 | 70 | 0.025 | 123 | 0.015 | 176 | 0.013 | 227 | 0.009 |
| 20 | 0.017 | 71 | 0.126 | 124 | 0.017 | 177 | 0.018 | 228 | 0.048 |
| 21 | 0.016 | 72 | 0.841 | 126 | 0.024 | 178 | 0.012 | 229 | 0.033 |
| 22 | 0.002 | 73 | 0.092 | 127 | 0.021 | 179 | 0.007 | 230 | 0.019 |
| 23 | 0.068 | 74 | 0.918 | 128 | 0.224 | 180 | 0.004 | 231 | 0.013 |
| 24 | 0.003 | 75 | 0.120 | 129 | 0.021 | 181 | 0.006 | 232 | 0.021 |
| 25 | 0.054 | 76 | 0.566 | 130 | 0.136 | 182 | 0.154 | 233 | 0.031 |
| 26 | 0.033 | 77 | 0.324 | 131 | 0.032 | 183 | 0.005 | 234 | 0.004 |
| 27 | 0.014 | 78 | 2.331 | 132 | 0.131 | 184 | 0.016 | 235 | 0.003 |
| 28 | 0.023 | 79 | 0.040 | 133 | 0.004 | 185 | 0.024 | 236 | 0.002 |
| 29 | 0.050 | 80 | 0.295 | 134 | 0.002 | 186 | 0.021 | 237 | 0.003 |
| 30 | 0.015 | 81 | 0.670 | 135 | 0.001 | 187 | 0.020 | 238 | 0.357 |
| 31 | 0.009 | 84 | 0.085 | 136 | 0.001 | 188 | 0.008 | 239 | 0.011 |
| 32 | 0.003 | 85 | 0.132 | 137 | 0.001 | 189 | 0.068 | 240 | 0.014 |
| 33 | 0.015 | 86 | 0.060 | 138 | 0.001 | 190 | 0.011 | 241 | 0.616 |
| 34 | 0.008 | 87 | 0.049 | 139 | 0.002 | 191 | 0.086 | 242 | 0.655 |
| 35 | 0.007 | 88 | 0.147 | 140 | 0.001 | 192 | 0.009 | 243 | 0.436 |
| 36 | 0.021 | 89 | 0.286 | 141 | 0.001 | 193 | 0.025 | 244 | 0.042 |
| 37 | 0.006 | 90 | 0.023 | 142 | 0.001 | 194 | 0.036 | 245 | 0.002 |
| 38 | 0.012 | 91 | 0.008 | 143 | 0.001 | 195 | 0.035 | 246 | 0.022 |
| 39 | 0.020 | 92 | 0.012 | 144 | 0.001 | 196 | 0.015 | 247 | 0.002 |
| 40 | 0.005 | 93 | 0.004 | 145 | 0.001 | 197 | 0.013 | 248 | 0.731 |
| 41 | 0.070 | 94 | 0.004 | 146 | 0.001 | 198 | 0.014 | 249 | 0.112 |
| 42 | 0.009 | 95 | 0.004 | 147 | 0.001 | 199 | 0.021 | 250 | 0.214 |
| 43 | 0.032 | 96 | 0.002 | 148 | <0.001 | 200 | 0.019 | 251 | 0.001 |
| 44 | 0.014 | 97 | 0.003 | 149 | 0.001 | 201 | 0.020 | 253 | 0.008 |
| 45 | 0.214 | 98 | 0.021 | 150 | 0.001 | 202 | 0.019 | 254 | 0.002 |
| 46 | 0.129 | 99 | 0.016 | 151 | 0.001 | 203 | 0.034 | 255 | 0.007 |
| 47 | 0.544 | 100 | 0.016 | 152 | 0.001 | 204 | 0.034 | 256 | 0.003 |
| 49 | 0.006 | 101 | 0.014 | 153 | 0.001 | 205 | 0.014 | 257 | 0.061 |
| 50 | 0.019 | 102 | 0.006 | 154 | 0.002 | 206 | 0.005 | 258 | 0.001 |
| 51 | 0.008 | 103 | 0.010 | 155 | 0.001 | 207 | 0.011 | 259 | 0.152 |
| 52 | 0.022 | 104 | 0.014 | 156 | 0.002 | 208 | 0.014 | 260 | 0.097 |
| 53 | 0.042 | 105 | 0.014 | 157 | 0.001 | 209 | 0.022 | 261 | 0.028 |
| 54 | 0.014 | 106 | 0.014 | 158 | 0.006 | 210 | 0.021 | 262 | 0.091 |
| 55 | 0.054 | 107 | 0.018 | 159 | 0.001 | 211 | 0.013 | 263 | 0.032 |

**[Table 84-2]**

| Example | IC50(*µ*M) | Example | IC50(*µ*M) | Example | IC50(*µ*M) | Example | IC50(*µ*M) |
|---|---|---|---|---|---|---|---|
| 264 | 0.459 | 314 | 0.009 | 369 | 0.008 | 419 | 0.685 |
| 265 | 0.287 | 315 | 0.003 | 370 | 0.008 | 420 | 0.701 |
| 266 | 0.092 | 316 | 0.002 | 371 | 0.005 | 421 | 0.480 |
| 267 | 0.664 | 317 | 0.003 | 372 | 0.004 | 422 | 0.218 |
| 268 | 0.108 | 318 | 0.009 | 373 | 0.005 | 423 | 0.974 |
| 269 | 0.134 | 319 | 0.022 | 374 | 0.008 | 424 | 0.795 |
| 270 | 0.189 | 320 | 0.072 | 375 | 0.011 | 425 | 0.860 |
| 271 | 0.024 | 321 | 0.074 | 376 | 0.021 | 426 | 0.095 |
| 272 | 0.101 | 322 | 0.007 | 377 | 0.014 | 427 | 0.052 |
| 273 | 0.063 | 323 | 0.103 | 378 | 0.010 | 428 | 0.014 |
| 274 | 0.151 | 324 | 0.037 | 379 | 0.010 | 429 | 0.014 |
| 275 | 0.042 | 325 | 0.017 | 380 | 0.003 | 430 | 0.048 |
| 276 | 0.074 | 326 | 0.004 | 381 | 0.002 | 431 | 0.347 |
| 277 | 0.102 | 327 | 0.006 | 382 | 0.004 | 432 | 0.344 |
| 278 | 0.146 | 328 | 0.014 | 383 | 0.011 | 433 | 0.868 |
| 279 | 0.112 | 329 | 0.067 | 384 | 0.005 | 434 | 0.279 |
| 280 | 0.089 | 330 | 0.019 | 385 | 0.013 | 435 | 0.492 |
| 281 | 0.120 | 331 | 0.003 | 386 | 0.013 | 436 | 0.033 |
| 282 | 0.124 | 332 | 0.004 | 387 | 0.004 | 437 | 0.292 |
| 283 | 0.067 | 333 | 0.018 | 388 | 0.004 | 438 | 0.126 |
| 284 | 0.537 | 334 | 0.018 | 389 | 0.012 | 439 | 0.124 |
| 285 | 0.955 | 335 | 0.008 | 390 | 0.011 | 440 | 0.199 |
| 286 | 0.213 | 336 | 0.002 | 391 | 0.004 | 441 | 0.247 |
| 287 | 0.090 | 337 | 0.002 | 392 | 0.003 | 442 | 0.551 |
| 288 | 0.336 | 338 | 0.004 | 393 | 0.003 | 443 | 0.157 |
| 289 | 0.338 | 339 | 0.078 | 394 | 0.004 | 444 | 0.789 |
| 290 | 0.104 | 341 | 0.005 | 395 | 0.003 | 445 | 0.216 |
| 291 | 0.215 | 342 | 0.050 | 396 | 0.005 | 446 | 0.171 |
| 292 | 0.154 | 343 | 0.018 | 397 | 0.007 | 447 | 0.403 |
| 293 | 0.121 | 344 | 0.075 | 398 | 0.002 | 448 | 0.474 |
| 294 | 0.118 | 345 | 0.167 | 399 | 0.005 | 449 | 0.065 |
| 295 | 0.355 | 346 | 0.002 | 400 | 0.009 | 450 | 0.081 |
| 296 | 0.419 | 347 | 0.185 | 401 | 0.002 | 451 | 0.076 |
| 297 | 0.018 | 348 | 0.005 | 402 | 0.003 | 452 | 0.088 |
| 298 | 0.025 | 349 | 0.507 | 403 | 0.003 | 453 | 0.348 |
| 299 | 0.033 | 350 | 0.294 | 404 | 0.004 | 454 | 0.070 |
| 300 | 0.168 | 351 | 0.121 | 405 | 0.003 | 455 | 0.291 |
| 301 | 0.091 | 352 | 0.011 | 406 | 0.006 | 456 | 0.090 |
| 302 | 0.047 | 353 | 0.066 | 407 | 0.005 | 457 | 0.171 |
| 303 | 0.082 | 354 | 0.002 | 408 | 0.004 | 458 | 0.280 |
| 304 | 1.070 | 355 | 0.017 | 409 | 0.004 | 459 | 0.176 |
| 305 | 0.062 | 356 | 0.044 | 410 | 0.005 | 460 | 0.424 |
| 306 | 0.056 | 358 | 0.089 | 411 | 0.008 | | |
| 307 | 0.285 | 359 | 0.454 | 412 | 0.016 | | |
| 308 | 0.655 | 360 | 0.086 | 413 | 0.183 | | |
| 309 | 0.032 | 362 | 0.182 | 414 | 0.618 | | |
| 310 | 0.041 | 365 | 0.160 | 415 | 0.847 | | |
| 311 | 0.001 | 366 | 0.008 | 416 | 0.676 | | |
| 312 | 0.002 | 367 | 0.008 | 417 | 0.690 | | |
| 313 | 0.001 | 368 | 0.013 | 418 | 0.075 | | |

### Industrial Applicability

The compound [I], [II], [III] or [IV] of the present invention or the pharmacologically acceptable salt thereof has inhibitory activity on TRH-DE, and is thus useful as a pharmaceutical for preventing or treating various diseases and/or symptoms associated therewith that can be ameliorated by increasing a central TRH concentration, such as spinocerebellar degeneration, pain, a sleep disorder, other nervous system diseases, and/or symptoms associated therewith, or for improving prognosis of these diseases and/or symptoms associated therewith.

The disclosure of Japanese patent application No. 2021-177933 (filing date: Oct. 29, 2021) is incorporated herein by reference in its entirety.

All references, patent applications, and technical standards described herein are incorporated by reference herein to the same extent that individual references, patent applications, and technical standards are specifically and individually noted as being incorporated by reference.

## Claims

1. A compound represented by the following Formula [I] or a pharmacologically acceptable salt thereof: 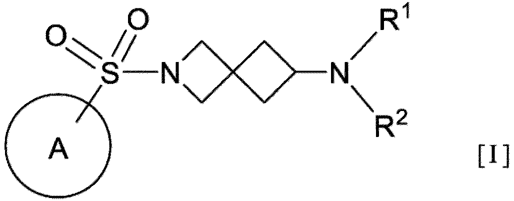 wherein,
a ring A represents an aryl group which may be substituted, a heteroaryl group which may be substituted, or an aliphatic heterocyclyl group which may be substituted, and
R¹ and R² represent, indipendently, an alkyl group which may be substituted, a cycloalkyl group which may be substituted, an alkenyl group which may be substituted, a cycloalkenyl group which may be substituted, an alkynyl group which may be substituted, an aryl group which may be substituted, a heteroaryl group which may be substituted, an aliphatic heterocyclyl group which may be substituted and may have a double bond in a part of a ring, an amino group which may be substituted, an alkoxy group which may be substituted or an alkylthio group which may be substituted, or a group in which R¹ and R² together form a ring.

2. The compound or the pharmacologically acceptable salt thereof according to claim 1, wherein the compound of Formula [I] is a compound represented by the following Formula [II]: 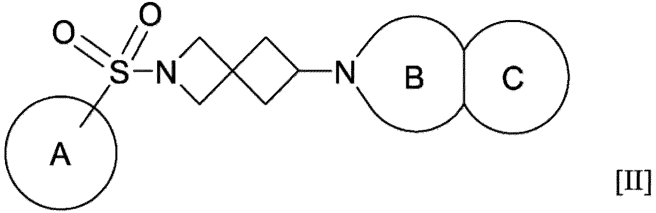 wherein,
a ring A represents an aryl group which may be substituted, a heteroaryl group which may be substituted, or an aliphatic heterocyclyl group which may be substituted,
a ring B represents an aliphatic heterocyclyl group which may be substituted and may have a double bond in a part of a ring, and
a ring C represents an aryl group which may be substituted and may be partially hydrogenated, a heteroaryl group which may be substituted and may be partially hydrogenated, a cycloalkyl group which may be substituted, or an aliphatic heterocyclyl group which may be substituted.

3. The compound or the pharmacologically acceptable salt thereof according to claim 2, wherein in the group represented by Formula [II],
each of substituents of the aryl group which may be substituted, the heteroaryl group which may be substituted, and the aliphatic heterocyclyl group which may be substituted represented by the ring A is one to three groups independently selected from the group consisting of a halogen atom, a cyano group, an alkyl group, an alkoxyalkyl group, a haloalkyl group, an alkoxy group, a haloalkoxy group, an alkanoyl group, an alkoxycarbonyl group, an amino group which may be substituted with one to two alkyl groups, an alkylthio group, a sulfonyl group which may be substituted with an alkyl group, an aminocarbonyl group which may be substituted with one to two alkyl groups, a nitro group, and an aryl group,
an aryl moiety of the aryl group which may be substituted represented by the ring A is a 6- to 10-membered monocyclic or bicyclic aryl,
a heteroaryl moiety of the heteroaryl group which may be substituted represented by the ring A is a 5- to 10-membered monocyclic or bicyclic heteroaryl having 1 to 4 heteroatoms independently selected from the group consisting of oxygen, nitrogen, and sulfur,
an aliphatic heterocyclic moiety of the aliphatic heterocyclyl group which may be substituted represented by the ring A is a 5- to 10-membered monocyclic or bicyclic aliphatic heterocyclic ring having 1 to 3 heteroatoms independently selected from the group consisting of oxygen, nitrogen, and sulfur,
each of substituents of the aliphatic heterocyclyl group which may be substituted and may have a double bond in a part of the ring represented by the ring B; the aryl group which may be substituted and may be partially hydrogenated represented by the ring C; the heteroaryl group which may be substituted and may be partially hydrogenated represented by the ring C; the cycloalkyl group which may be substituted represented by the ring C; and the aliphatic heterocyclyl group which may be substituted represented by the ring C is one to eight groups independently selected from the group consisting of a halogen atom; a hydroxyl group; an oxo group; a cyano group; an alkyl group; a haloalkyl group; an alkoxyalkyl group; an alkoxy group; a cycloalkoxy group which may be substituted with a cyano group; a haloalkoxy group; an alkylene group which may be substituted with one to two groups independently selected from the group consisting of a halogen atom; an amino group which may be substituted with one to two groups independently selected from the group consisting of an alkyl group which may be substituted with a cycloalkyl group or an aryl group, an aryl group which may be substituted with a halogen atom, and a cycloalkyl group; an alkylthio group; an aryl group; a cycloalkyl group which may be substituted with one to two groups independently selected from the group consisting of a halogen atom and a haloalkyl group; an aliphatic heterocyclyl group which may be substituted with one to four groups independently selected from the group consisting of a halogen atom, a cyano group, an alkyl group, a haloalkyl group, an alkylene group, and an alkoxy group; an alkanoyl group which may be substituted with one to two groups independently selected from the group consisting of a cycloalkyl group, an aryl group, and an alkoxy group; an aryloxy group; an aliphatic heterocyclyl oxy group; and an alkoxycarbonyl group which may be substituted with an aryl group,
an aliphatic heterocyclic moiety of the aliphatic heterocyclyl group which may be substituted and may have a double bond in a part of a ring represented by the ring B is a 5- to 7-membered monocyclic aliphatic heterocyclic ring having 1 to 3 heteroatoms independently selected from the group consisting of oxygen, nitrogen, and sulfur,
an aryl moiety of the aryl group which may be substituted and may be partially hydrogenated represented by the ring C is a 6- to 10-membered monocyclic or bicyclic aryl,
a heteroaryl moiety of the heteroaryl group which may be substituted and may be partially hydrogenated represented by the ring C is a 5- to 10-membered monocyclic or bicyclic heteroaryl having 1 to 4 heteroatoms independently selected from the group consisting of oxygen, nitrogen, and sulfur,
an aliphatic heterocyclic moiety of the aliphatic heterocyclyl group which may be substituted represented by the ring C is a 4- to 9-membered monocyclic or bicyclic aliphatic heterocyclic ring having 1 to 3 heteroatoms independently selected from the group consisting of oxygen, nitrogen, and sulfur,
in each of the substituents of the groups represented by the ring B or the ring C, the aryl group in the selected group is a 6- to 10-membered monocyclic or bicyclic aryl, and
in each of the substituents of the groups represented by the ring B or the ring C, the aliphatic heterocyclic moiety of the aliphatic heterocyclyl group which may be substituted in the selected group is a 4- to 9-membered monocyclic or bicyclic aliphatic heterocyclic ring having 1 to 3 heteroatoms independently selected from the group consisting of oxygen, nitrogen, and sulfur.

4. The compound or the pharmacologically acceptable salt thereof according to claim 2 or 3, wherein in the group represented by Formula [II],
the ring A is an aryl group which may be substituted or a heteroaryl group which may be substituted,
an aryl moiety of the aryl group which may be substituted represented by the ring A is a 6- to 10-membered monocyclic or bicyclic aryl,
a heteroaryl moiety of the heteroaryl group which may be substituted represented by the ring A is a 5- to 10-membered monocyclic or bicyclic heteroaryl having 1 to 4 heteroatoms independently selected from the group consisting of oxygen, nitrogen, and sulfur,
an aliphatic heterocyclic moiety of the aliphatic heterocyclyl group which may be substituted and may have a double bond in a part of a ring represented by the ring B, may be substituted is a 5- to 7-membered monocyclic aliphatic heterocyclic ring having 1 to 3 heteroatoms independently selected from the group consisting of oxygen, nitrogen, and sulfur,
the ring C is an aryl group which may be substituted and may be partially hydrogenated, a heteroaryl group which may be substituted and may be partially hydrogenated, a cycloalkyl group which may be substituted, or an aliphatic heterocyclyl group which may be substituted,
an aryl moiety of the aryl group which may be substituted and may be partially hydrogenated represented by the ring C is a 6- to 10-membered monocyclic or bicyclic aryl,
a heteroaryl moiety of the heteroaryl group which may be substituted and may be partially hydrogenated represented by the ring C is a 5- to 10-membered monocyclic or bicyclic heteroaryl having 1 to 4 heteroatoms independently selected from the group consisting of oxygen, nitrogen, and sulfur, and
an aliphatic heterocyclic moiety of the aliphatic heterocyclyl group which may be substituted represented by the ring C is a 4- to 9-membered monocyclic or bicyclic aliphatic heterocyclic ring having 1 to 3 heteroatoms independently selected from the group consisting of oxygen, nitrogen, and sulfur.

5. The compound or the pharmacologically acceptable salt thereof according to any one of claims 2 to 4, wherein in the group represented by Formula [II],
the ring A is a phenyl group which may be substituted or a monocyclic heteroaryl group which may be substituted,
each of substituents of the phenyl group which may be substituted or the monocyclic heteroaryl group which may be substituted represented by the ring A is one to two groups independently selected from the group consisting of a halogen atom, an alkyl group, a haloalkyl group, an alkoxy group, and a nitro group,
a heteroaryl moiety of the monocyclic heteroaryl group which may be substituted represented by the ring A is a 5- or 6-membered monocyclic heteroaryl having 1 to 4 heteroatoms independently selected from the group consisting of oxygen, nitrogen, and sulfur,
a substituent of the aliphatic heterocyclyl group which may be substituted and may have a double bond in a part of a ring is represented by the ring B is one to four groups independently selected from the group consisting of a halogen atom; a hydroxyl group; an oxo group; an alkyl group; a haloalkyl group; an alkoxyalkyl group; an alkoxy group; an alkylene group which may be substituted with one to two groups independently selected from a halogen atom; a cycloalkyl group; an alkanoyl group which may be substituted with one to two groups independently selected from the group consisting of a cycloalkyl group, an aryl group, and an alkoxy group; and an alkoxycarbonyl group which may be substituted with an aryl group,
an aliphatic heterocyclic moiety of the aliphatic heterocyclyl group which may be substituted and may have a double bond in a part of a ring represented by the ring B is a 5- to 7-membered monocyclic aliphatic heterocyclic ring having 1 to 3 heteroatoms independently selected from the group consisting of oxygen, nitrogen, and sulfur,
in the substituents of the groups represented by the ring B, the aryl group in the selected group is phenyl,
the ring C is a phenyl group which may be substituted, a monocyclic heteroaryl group which may be substituted and may be partially hydrogenated, a monocyclic cycloalkyl group which may be substituted, or a monocyclic aliphatic heterocyclyl group which may be substituted,
each of substituents of the phenyl group which may be substituted, the monocyclic heteroaryl group which may be substituted and may be partially hydrogenated, the monocyclic cycloalkyl group which may be substituted, or the monocyclic aliphatic heterocyclyl group which may be substituted represented by the ring C is one to four groups independently selected from the group consisting of a halogen atom; a hydroxyl group; an oxo group; a cyano group; an alkyl group; a haloalkyl group; an alkoxy group; a cycloalkoxy group which may be substituted with a cyano group; a haloalkoxy group; an alkylene group; an amino group which may be substituted with one to two groups independently selected from the group consisting of an alkyl group which may be substituted with a cycloalkyl group or an aryl group, an aryl group which may be substituted with a halogen atom, and a cycloalkyl group; an alkylthio group; an aryl group; a cycloalkyl group which may be substituted with one to two groups independently selected from the group consisting of a halogen atom and a haloalkyl group; an aliphatic heterocyclyl group which may be substituted with one to four groups independently selected from the group consisting of a halogen atom, a cyano group, an alkyl group, a haloalkyl group, an alkylene group, and an alkoxy group; an aryloxy group; an aliphatic heterocyclyl oxy group; and an alkanoyl group,
a heteroaryl moiety of the monocyclic heteroaryl group which may be substituted and may be partially hydrogenated represented by the ring C is a 5- or 6-membered monocyclic heteroaryl having 1 to 4 heteroatoms independently selected from the group consisting of oxygen, nitrogen, and sulfur,
an aliphatic heterocyclic moiety of the monocyclic aliphatic heterocyclyl group which may be substituted represented by the ring C is a 4- to 7-membered monocyclic aliphatic heterocyclic ring having 1 to 3 heteroatoms independently selected from the group consisting of oxygen, nitrogen, and sulfur,
in each of the substituents of the groups represented by the ring C, the aryl group in the selected group is phenyl, and
in each of the substituents of the groups represented by the ring C, an aliphatic heterocyclic moiety of the aliphatic heterocyclyl group which may be substituted in the selected group is a 4- to 9-membered monocyclic or bicyclic aliphatic heterocyclic ring having 1 to 3 heteroatoms independently selected from the group consisting of oxygen, nitrogen, and sulfur.

6. The compound or the pharmacologically acceptable salt thereof according to any one of claims 2 to 5, wherein in the group represented by Formula [II],
the ring A is a phenyl group which may be substituted or a monocyclic heteroaryl group which may be substituted,
each of substituents of the phenyl group which may be substituted or the monocyclic heteroaryl group which may be substituted represented by the ring A is one to two groups independently selected from the group consisting of a halogen atom, an alkyl group, a haloalkyl group, an alkoxy group, and a nitro group,
a heteroaryl moiety of the monocyclic heteroaryl group which may be substituted represented by the ring A is pyrrolyl, furanyl, thienyl, imidazolyl, pyrazolyl, oxazolyl, isoxazolyl, thiazolyl, isothiazolyl, triazolyl, tetrazolyl, pyridyl, pyrazinyl, pyrimidinyl, pyridazinyl, thiazinyl, or triazinyl,
a substituent of the aliphatic heterocyclyl group which may be substituted and may have a double bond in a part of a ring represented by the ring B is one to four groups independently selected from the group consisting of a halogen atom; a hydroxyl group; an oxo group; an alkyl group; a haloalkyl group; an alkoxyalkyl group; an alkoxy group; an alkylene group which may be substituted with one to two groups independently selected from a halogen atom; a cycloalkyl group; an alkanoyl group which may be substituted with one to two groups independently selected from the group consisting of a cycloalkyl group, an aryl group, and an alkoxy group; and an alkoxycarbonyl group which may be substituted with an aryl group,
an aliphatic heterocyclic moiety of the aliphatic heterocyclyl group which may be substituted and may have a double bond in a part of a ring represented by the ring B is azolidinyl, diazolidinyl, azinanyl, oxazinanyl, diazinanyl, thiazinanyl, azepanyl, oxazepanyl, diazepanyl, or thiazepanyl,
in the substituents of the groups represented by the ring B, the aryl group in the selected group is phenyl,
the ring C is a phenyl group which may be substituted, a monocyclic heteroaryl group which may be substituted and may be partially hydrogenated, a monocyclic cycloalkyl group which may be substituted, or a monocyclic aliphatic heterocyclyl group which may be substituted,
each of substituents of the phenyl group which may be substituted; the monocyclic heteroaryl group which may be substituted and may be partially hydrogenated; the monocyclic cycloalkyl group which may be substituted; or the monocyclic aliphatic heterocyclyl group which may be substituted represented by the ring C is one to four groups independently selected from the group consisting of a halogen atom; a hydroxyl group; an oxo group; a cyano group; an alkyl group; a haloalkyl group; an alkoxy group; a cycloalkoxy group which may be substituted with a cyano group; a haloalkoxy group; an alkylene group; an amino group which may be substituted with one to two groups independently selected from the group consisting of an alkyl group which may be substituted with a cycloalkyl group or an aryl group, an aryl group which may be substituted with a halogen atom, and a cycloalkyl group; an alkylthio group; an aryl group; a cycloalkyl group which may be substituted with one to two groups independently selected from the group consisting of a halogen atom and a haloalkyl group; an aliphatic heterocyclyl group which may be substituted with one to four groups independently selected from the group consisting of a halogen atom, a cyano group, an alkyl group, a haloalkyl group, an alkylene group, and an alkoxy group; an aryloxy group; an aliphatic heterocyclyl oxy group; and an alkanoyl group,
a heteroaryl moiety of the monocyclic heteroaryl group which may be substituted and may be partially hydrogenated represented by the ring C, may be substituted is pyrrolyl, furanyl, thienyl, imidazolyl, pyrazolyl, oxazolyl, isoxazolyl, thiazolyl, isothiazolyl, triazolyl, tetrazolyl, pyridyl, pyrazinyl, pyrimidinyl, pyridazinyl, thiazinyl, or triazinyl,
an aliphatic heterocyclic moiety of the monocyclic aliphatic heterocyclyl group which may be substituted represented by the ring C is azetidinyl, oxetanyl, oxolanyl, azolidinyl, thiolanyl, oxazolidinyl, diazolidinyl, thiazolidinyl, oxanyl, azinanyl, dioxanyl, oxazinanyl, diazinanyl, thiazinanyl, thianyl, oxepanyl, azepanyl, thiepanyl, oxazepanyl, diazepanyl, or thiazepanyl,
in each of the substituents of the groups represented by the ring C, the aryl group in the selected group is phenyl, and
in each of the substituents of the groups represented by the ring C, an aliphatic heterocyclic moiety of the aliphatic heterocyclyl group which may be substituted in the selected group is azetidinyl, oxetanyl, oxolanyl, azolidinyl, thiolanyl, oxazolidinyl, diazolidinyl, thiazolidinyl, oxanyl, azinanyl, dioxanyl, oxazinanyl, diazinanyl, thiazinanyl, thianyl, oxepanyl, azepanyl, thiepanyl, oxazepanyl, diazepanyl, thiazepanyl, or a ring in which two aliphatic heterocyclic rings are fused or bonded by a spiro atom.

7. The compound or the pharmacologically acceptable salt thereof according to any one of claims 2 to 6, wherein in the group represented by Formula [II],
the ring B is a 7-membered monocyclic aliphatic heterocyclyl group which may be substituted and may have a double bond in a part of a ring, and
the ring C is a phenyl group which may be substituted, a 6-membered monocyclic heteroaryl group which may be substituted and may be partially hydrogenated, or a 6-membered monocyclic aliphatic heterocyclyl group which may be substituted.

8. The compound or the pharmacologically acceptable salt thereof according to claim 7, wherein in the group represented by Formula [II],
the ring A is a phenyl group which may be substituted or a monocyclic heteroaryl group which may be substituted,
each of substituents of the phenyl group which may be substituted or the monocyclic heteroaryl group which may be substituted represented by the ring A is one to two groups independently selected from the group consisting of a halogen atom, an alkyl group, a haloalkyl group, an alkoxy group, and a nitro group,
a heteroaryl moiety of the monocyclic heteroaryl group which may be substituted represented by the ring A is a 5- or 6-membered monocyclic heteroaryl having 1 to 4 heteroatoms independently selected from the group consisting of oxygen, nitrogen, and sulfur,
the ring B is a 7-membered monocyclic aliphatic heterocyclyl group which may be substituted and may have a double bond in a part of a ring,
a substituent of the 7-membered monocyclic aliphatic heterocyclyl group which may be substituted and may have a double bond in a part of a ring represented by the ring B is one to four groups independently selected from the group consisting of a halogen atom; an oxo group; an alkyl group; an alkoxyalkyl group; an alkylene group; a cycloalkyl group; an alkanoyl group which may be substituted with one to two groups independently selected from the group consisting of a cycloalkyl group, a phenyl group, and an alkoxy group; and an alkoxycarbonyl group which may be substituted with an aryl group,
an aliphatic heterocyclic moiety of the 7-membered monocyclic aliphatic heterocyclyl group which may be substituted and may have a double bond in a part of a ring represented by the ring B is a 7-membered monocyclic aliphatic heterocyclic ring having 1 to 3 heteroatoms independently selected from the group consisting of oxygen, nitrogen, and sulfur,
the ring C is a phenyl group which may be substituted, a 6-membered monocyclic heteroaryl group which may be substituted and may be partially hydrogenated, or a 6-membered monocyclic aliphatic heterocyclyl group which may be substituted,
each of substituents of the phenyl group which may be substituted, the 6-membered monocyclic heteroaryl group which may be substituted and may be partially hydrogenated, or the 6-membered monocyclic aliphatic heterocyclyl group which may be substituted represented by the ring C is one to two groups independently selected from the group consisting of a halogen atom; an oxo group; a cyano group; an alkyl group; a haloalkyl group; an alkoxy group; a cycloalkoxy group which may be substituted with a cyano group; a haloalkoxy group; an amino group which may be substituted with one to two groups independently selected from the group consisting of an alkyl group which may be substituted with a cycloalkyl group or an aryl group, an aryl group which may be substituted with a halogen atom, and a cycloalkyl group; a cycloalkyl group; an aliphatic heterocyclyl group which may be substituted with one to four groups independently selected from the group consisting of a halogen atom, a cyano group, an alkyl group, a haloalkyl group, an alkylene group, and an alkoxy group; an aryloxy group; an aliphatic heterocyclyl oxy group; and an alkanoyl group,
in each of the substituents of the groups represented by the ring C, the aryl group in the selected group is phenyl,
in each of the substituents of the groups represented by the ring C, an aliphatic heterocyclic moiety of the aliphatic heterocyclyl group which may be substituted in the selected group is a 5- to 7-membered monocyclic aliphatic heterocyclic ring having 1 to 3 heteroatoms independently selected from the group consisting of oxygen, nitrogen, and sulfur, or a ring in which two aliphatic heterocyclic rings are fused or bonded by a spiro atom,
a heteroaryl moiety of the 6-membered monocyclic heteroaryl group which may be substituted and may be partially hydrogenated represented by the ring C is a 6-membered monocyclic heteroaryl having 1 to 3 heteroatoms independently selected from the group consisting of oxygen, nitrogen, and sulfur, and
an aliphatic heterocyclic moiety of the 6-membered monocyclic aliphatic heterocyclyl group which may be substituted represented by the ring C is a 6-membered monocyclic aliphatic heterocyclic ring having 1 to 3 heteroatoms independently selected from the group consisting of oxygen, nitrogen, and sulfur.

9. The compound or the pharmacologically acceptable salt thereof according to claim 7 or 8, wherein in the group represented by Formula [II],
a heteroaryl moiety of the heteroaryl group which may be substituted represented by the ring A is thienyl or pyridyl,
an aliphatic heterocyclic moiety of the 7-membered monocyclic aliphatic heterocyclyl group which may be substituted and may have a double bond in a part of a ring represented by the ring B is azepanyl, oxazepanyl, diazepanyl, or thiazepanyl,
an aryl moiety of the aryl group in the substituent of the group represented by the ring B is phenyl,
a heteroaryl moiety of the 6-membered monocyclic heteroaryl group which may be substituted and may be partially hydrogenated represented by the ring C is pyridyl, pyrazinyl, or pyrimidinyl,
an aliphatic heterocyclic moiety of the 6-membered monocyclic aliphatic heterocyclyl group which may be substituted represented by the ring C is oxazinanyl,
in each of the substituents of the groups represented by the ring C, the aryl group in the selected group is phenyl, and
in each of the substituents of the groups represented by the ring C, an aliphatic heterocyclic moiety of the aliphatic heterocyclyl group which may be substituted in the selected group is azolidinyl, azinanyl, oxazinanyl, diazinanyl, azepanyl, or oxazepanyl.

10. The compound or the pharmacologically acceptable salt thereof according to any one of claims 2 to 6, wherein in the group represented by Formula [II],
the ring B is a 7-membered monocyclic aliphatic heterocyclyl group which may be substituted and may have a double bond in a part of a ring, and
the ring C is a 5-membered monocyclic heteroaryl group which may be substituted or a 5-membered monocyclic aliphatic heterocyclyl group which may be substituted.

11. The compound or the pharmacologically acceptable salt thereof according to claim 10, wherein in the group represented by Formula [II],
the ring A is a phenyl group which may be substituted,
a substituent of the phenyl group which may be substituted represented by the ring A is a halogen atom,
the ring B is a 7-membered monocyclic aliphatic heterocyclyl group which may be substituted and may have a double bond in a part of a ring,
a substituent of the 7-membered monocyclic aliphatic heterocyclyl group which may be substituted and may have a double bond in a part of a ring represented by the ring B is one to four groups independently selected from the group consisting of an oxo group; an alkyl group; and an alkylene group which may be substituted with one to two groups independently selected from a halogen atom,
an aliphatic heterocyclic moiety of the 7-membered monocyclic aliphatic heterocyclyl group which may be substituted and may have a double bond in a part of a ring represented by the ring B is a 7-membered monocyclic aliphatic heterocyclic ring having 1 to 3 heteroatoms independently selected from the group consisting of oxygen, nitrogen, and sulfur,
the ring C is a 5-membered monocyclic heteroaryl group which may be substituted or a 5-membered monocyclic aliphatic heterocyclyl group which may be substituted,
each of substituents of the 5-membered monocyclic heteroaryl group which may be substituted or the 5-membered monocyclic aliphatic heterocyclyl group which may be substituted represented by the ring C is one to two groups independently selected from the group consisting of a halogen atom; an alkyl group; a haloalkyl group; an alkylene group; a cycloalkyl group which may be substituted with one to two groups independently selected from the group consisting of a halogen atom and a haloalkyl group; and an aryl group,
in each of the substituents of the groups represented by the ring C, the aryl group in the selected group is phenyl,
a heteroaryl moiety of the 5-membered monocyclic heteroaryl group which may be substituted represented by the ring C is a 5-membered monocyclic heteroaryl having 1 to 4 heteroatoms independently selected from the group consisting of oxygen, nitrogen, and sulfur, and
an aliphatic heterocyclic moiety of the 5-membered monocyclic aliphatic heterocyclyl group which may be substituted represented by the ring C is a 5-membered monocyclic aliphatic heterocyclic ring having 1 to 3 heteroatoms independently selected from the group consisting of oxygen, nitrogen, and sulfur.

12. The compound or the pharmacologically acceptable salt thereof according to claim 10 or 11, wherein in the group represented by Formula [II],
an aliphatic heterocyclic moiety of the 7-membered monocyclic aliphatic heterocyclyl group which may be substituted and may have a double bond in a part of a ring represented by the ring B is azepanyl, oxazepanyl, or diazepanyl,
a heteroaryl moiety of the 5-membered monocyclic heteroaryl group which may be substituted represented by the ring C is imidazolyl, pyrazolyl, oxazolyl, isoxazolyl, thiazolyl, isothiazolyl, or triazolyl,
an aliphatic heterocyclic moiety of the 5-membered monocyclic aliphatic heterocyclyl group which may be substituted represented by the ring C is azolidinyl, and
in each of the substituents of the groups represented by the ring C, the aryl group in the selected group is phenyl.

13. The compound or the pharmacologically acceptable salt thereof according to any one of claims 2 to 6, wherein in the group represented by Formula [II],
the ring B is a 6-membered monocyclic aliphatic heterocyclyl group which may be substituted and may have a double bond in a part of a ring, and
the ring C is a phenyl group which may be substituted or a 6-membered monocyclic heteroaryl group which may be substituted.

14. The compound or the pharmacologically acceptable salt thereof according to claim 13, wherein in the group represented by Formula [II],
the ring A is a phenyl group which may be substituted,
a substituent of the phenyl group which may be substituted represented by the ring A is a halogen atom,
the ring B is a 6-membered monocyclic aliphatic heterocyclyl group which may be substituted and may have a double bond in a part of a ring,
a substituent of the 6-membered monocyclic aliphatic heterocyclyl group which may be substituted and may have a double bond in a part of a ring represented by the ring B is one to three groups independently selected from the group consisting of an oxo group; an alkyl group; and a haloalkyl group,
an aliphatic heterocyclic moiety of the 6-membered monocyclic aliphatic heterocyclyl group which may be substituted and may have a double bond in a part of a ring represented by the ring B is a 6-membered monocyclic aliphatic heterocyclic ring having 1 to 3 heteroatoms independently selected from the group consisting of oxygen, nitrogen, and sulfur,
the ring C is a phenyl group which may be substituted or a 6-membered monocyclic heteroaryl group which may be substituted,
each of substituents of the phenyl group which may be substituted or the 6-membered monocyclic heteroaryl group which may be substituted represented by the ring C is a group independently selected from the group consisting of a halogen atom and a haloalkyl group, and
a heteroaryl moiety of the 6-membered monocyclic heteroaryl group which may be substituted represented by the ring C is a 6-membered monocyclic heteroaryl having 1 to 3 heteroatoms independently selected from the group consisting of oxygen, nitrogen, and sulfur.

15. The compound or the pharmacologically acceptable salt thereof according to claim 13 or 14, wherein in the group represented by Formula [II],
an aliphatic heterocyclic moiety of the 6-membered monocyclic aliphatic heterocyclyl group which may be substituted and may have a double bond in a part of a ring represented by the ring B is azinanyl, oxazinanyl, or diazinanyl, and
a heteroaryl moiety of the 6-membered monocyclic heteroaryl group which may be substituted represented by the ring C is pyridyl.

16. The compound or the pharmacologically acceptable salt thereof according to any one of claims 2 to 6, wherein in the group represented by Formula [II],
the ring B is a 6-membered monocyclic aliphatic heterocyclyl group which may be substituted and may have a double bond in a part of a ring, and
the ring C is a 5-membered monocyclic heteroaryl group which may be substituted.

17. The compound or the pharmacologically acceptable salt thereof according to claim 16, wherein in the group represented by Formula [II],
the ring A is a phenyl group which may be substituted,
a substituent of the phenyl group which may be substituted represented by the ring A is a halogen atom,
the ring B is a 6-membered monocyclic aliphatic heterocyclyl group which may be substituted and may have a double bond in a part of a ring,
a substituent of the 6-membered monocyclic aliphatic heterocyclyl group which may be substituted and may have a double bond in a part of a ring represented by the ring B is one to two groups independently selected from the group consisting of an oxo group and an alkyl group,
an aliphatic heterocyclic moiety of the 6-membered monocyclic aliphatic heterocyclyl group which may be substituted and may have a double bond in a part of a ring represented by the ring B is a 6-membered monocyclic aliphatic heterocyclic ring having 1 to 3 heteroatoms independently selected from the group consisting of oxygen, nitrogen, and sulfur,
the ring C is a 5-membered monocyclic heteroaryl group which may be substituted,
a substituent of the 5-membered monocyclic heteroaryl group which may be substituted represented by the ring C is one to two groups independently selected from the group consisting of a halogen atom; an alkyl group; a haloalkyl group; and a cycloalkyl group which may be substituted with one to two groups independently selected from the group consisting of a halogen atom and a haloalkyl group, and
a heteroaryl moiety of the 5-membered monocyclic heteroaryl group which may be substituted represented by the ring C is a 5-membered monocyclic heteroaryl having 1 to 4 heteroatoms independently selected from the group consisting of oxygen, nitrogen, and sulfur.

18. The compound or the pharmacologically acceptable salt thereof according to claim 16 or 17, wherein in the group represented by Formula [II],
an aliphatic heterocyclic moiety of the 6-membered monocyclic aliphatic heterocyclyl group which may be substituted and may have a double bond in a part of a ring represented by the ring B is diazinanyl, and
a heteroaryl moiety of the 5-membered monocyclic heteroaryl group which may be substituted represented by the ring C is pyrrolyl, imidazolyl, pyrazolyl, or triazolyl.

19. The compound or the pharmacologically acceptable salt thereof according to any one of claims 2 to 6, wherein in the group represented by Formula [I],
the ring B is a 5-membered monocyclic aliphatic heterocyclyl group which may be substituted and may have a double bond in a part of a ring, and
the ring C is a phenyl group which may be substituted or a 6-membered monocyclic heteroaryl group which may be substituted.

20. The compound or the pharmacologically acceptable salt thereof according to claim 19, wherein in the group represented by Formula [II],
the ring A is a phenyl group which may be substituted,
a substituent of the phenyl group which may be substituted represented by the ring A is one to two groups independently selected from the group consisting of a halogen atom, an alkyl group, and an alkoxy group,
the ring B is a 5-membered monocyclic aliphatic heterocyclyl group which may be substituted and may have a double bond in a part of a ring,
a substituent of the 5-membered monocyclic aliphatic heterocyclyl group which may be substituted and may have a double bond in a part of a ring represented by the ring B is one to three groups independently selected from the group consisting of a hydroxyl group; an oxo group; an alkyl group; and an alkylene group,
an aliphatic heterocyclic moiety of the 5-membered monocyclic aliphatic heterocyclyl group which may be substituted and may have a double bond in a part of a ring represented by the ring B is a 5-membered monocyclic aliphatic heterocyclic ring having 1 to 3 heteroatoms independently selected from the group consisting of oxygen, nitrogen, and sulfur,
the ring C is a phenyl group which may be substituted or a 6-membered monocyclic heteroaryl group which may be substituted,
each of substituents of the phenyl group which may be substituted or the 6-membered monocyclic heteroaryl group which may be substituted represented by the ring C is a group independently selected from the group consisting of a halogen atom; an alkyl group; a haloalkyl group; and an alkoxy group, and
a heteroaryl moiety of the 6-membered monocyclic heteroaryl group which may be substituted represented by the ring C is a 6-membered monocyclic heteroaryl having 1 to 3 heteroatoms independently selected from the group consisting of oxygen, nitrogen, and sulfur.

21. The compound or the pharmacologically acceptable salt thereof according to claim 19 or 20, wherein in the group represented by Formula [II],
an aliphatic heterocyclic moiety of the 5-membered monocyclic aliphatic heterocyclyl group which may be substituted and may have a double bond in a part of a ring represented by the ring B is azolidinyl or diazolidinyl, and
a heteroaryl moiety of the 6-membered monocyclic heteroaryl group which may be substituted represented by the ring C is pyridyl.

22. The compound or the pharmacologically acceptable salt thereof according to any one of claims 2 to 6, wherein in the group represented by Formula [II],
the ring B is a 5-membered monocyclic aliphatic heterocyclyl group which may be substituted and may have a double bond in a part of a ring, and
the ring C is a 5-membered monocyclic heteroaryl group which may be substituted.

23. The compound or the pharmacologically acceptable salt thereof according to claim 22, wherein in the group represented by Formula [II],
the ring A is a phenyl group which may be substituted,
a substituent of the phenyl group which may be substituted represented by the ring A is a halogen atom,
the ring B is a 5-membered monocyclic aliphatic heterocyclyl group which may be substituted and may have a double bond in a part of a ring,
a substituent of the 5-membered monocyclic aliphatic heterocyclyl group which may be substituted and may have a double bond in a part of a ring represented by the ring B is one to two groups independently selected from the group consisting of an oxo group and an alkyl group,
an aliphatic heterocyclic moiety of the 5-membered monocyclic aliphatic heterocyclyl group which may be substituted and may have a double bond in a part of a ring represented by the ring B is a 5-membered monocyclic aliphatic heterocyclic ring having 1 to 3 heteroatoms independently selected from the group consisting of oxygen, nitrogen, and sulfur,
the ring C is a 5-membered monocyclic heteroaryl group which may be substituted,
a substituent of the 5-membered monocyclic heteroaryl group which may be substituted represented by the ring C is an alkyl group, and
a heteroaryl moiety of the 5-membered monocyclic heteroaryl group which may be substituted represented by the ring C is a 5-membered monocyclic heteroaryl having 1 to 4 heteroatoms independently selected from the group consisting of oxygen, nitrogen, and sulfur.

24. The compound or the pharmacologically acceptable salt thereof according to claim 20 or 21, wherein in the group represented by Formula [II],
an aliphatic heterocyclic moiety of 5-membered monocyclic aliphatic heterocyclyl group which may be substituted and may have a double bond in a part of a ring represented by the ring B is azolidinyl, and
a heteroaryl moiety of the 5-membered monocyclic heteroaryl group which may be substituted represented by the ring C is thiazolyl.

25. A compound selected from the group consisting of:
(3R)-4-[2-(2-fluorophenyl)sulfonyl-2-azaspiro[3.3]heptan-6-yl]-3-methyl-8-(trifluoromethyl)-2,3-dihydropyrido [3,4-f][1,4]oxazepin-5-one;
(3R)-4-[2-(2-fluorophenyl)sulfonyl-2-azaspiro[3.3]heptan-6-yl]-3-methyl-7-(trifluoromethyl)-2,3-dihydropyrido[3,2-f][1,4]oxazepin-5-one;
(3R)-4-[2-(2-fluorophenyl)sulfonyl-2-azaspiro[3.3]heptan-6-yl]-3-methyl-8-(trifluoromethoxy)-2,3-dihydropyrido[3,2-f][1,4]oxazepin-5-one;
(3R)-4-[2-(2-fluorophenyl)sulfonyl-2-azaspiro[3.3]heptan-6-yl]-3-methyl-8-(trifluoromethyl)-2,3-dihydropyrido[3,2-f][1,4]oxazepin-5-one;
(6R)-2-(1-fluorocyclopropyl)-5-[2-(2-fluorophenyl)sulfonyl-2-azaspiro[3.3]heptan-6-yl]-6-methyl-7,8-dihydro-6H-thiazolo[5,4-c]azepin-4-one;
(7R)-2-[(1S*,SR*)-3-azabicyclo[3.1.0]hexan-3-yl]-6-[2-(2-fluorophenyl)sulfonyl-2-azaspiro[3.3]heptan-6-yl]-7-methyl-7,8-dihydropyrimido[5,4-f] oxazepin- 5 -one;
(6R)-7-[2-(2-fluorophenyl)sulfonyl-2-azaspiro[3.3]heptan-6-yl]-2,6-dimethyl-5,6-dihydrothiazolo[5,4-f][1,4]oxazepin-8-one;
(6R)-2-(1,1-difluoroethyl)-7-[2-(2-fluorophenyl)sulfonyl-2-azaspiro[3.3]heptan-6-yl]-6-methyl-5,6-dihydrothiazolo[5,4-f] [1,4]oxazepin-8-one;
(6R)-2-cyclopropyl-7-[2-(2-fluorophenyl)sulfonyl-2-azaspiro[3.3]heptan-6-yl]-6-methyl-5,6-dihydrothiazolo[5,4-f] [1,4]oxazepin-8-one;
(6R)-2-(1-fluorocyclopropyl)-7-[2-(2-fluorophenyl)sulfonyl-2-azaspiro[3.3]heptan-6-yl]-6-methyl-5,6-dihydrothiazolo[5,4-f][1,4]oxazepin-8-one;
(6R)-2-cyclopropyl-5-[2-(2-fluorophenyl)sulfonyl-2-azaspiro[3.3]heptan-6-yl]-6-methyl-7,8-dihydro-6H-pyrazolo[1,5-a][1,4]diazepin-4-one;
(6R)-2-(1-fluorocyclopropyl)-5-[2-(2-fluorophenyl)sulfonyl-2-azaspiro[3.3]heptan-6-yl]-6-methyl-7,8-dihydro-6H-pyrazolo[1,5-a][1,4]diazepin-4-one;
(6R)-5-[2-(2-fluorophenyl)sulfonyl-2-azaspiro[3.3]heptan-6-yl]-6-methyl-2-(trifluoromethyl)-7,8-dihydro-6H-pyrazolo[1,5-a][1,4]diazepin-4-one;
(3R)-4-[2-(2-fluorophenyl)sulfonyl-2-azaspiro[3.3]heptan-6-yl]-3,8-dimethyl-2,3-dihydropyrido[3,4-f][1,4]oxazepin-5-one;
(3R)-8-cyclopropyl-4-[2-(2-fluorophenyl)sulfonyl-2-azaspiro[3.3]heptan-6-yl]-3-methyl-2,3-dihydropyrido[3,4-f][1,4]oxazepin-5-one;
(3R)-8-cyclopropyl-4-[2-(2-fluorophenyl)sulfonyl-2-azaspiro[3.3]heptan-6-yl]-3-methyl-2,3-dihydropyrido[3,2-f][1,4]oxazepin-5-one;
(7R)-6-[2-(2-fluorophenyl)sulfonyl-2-azaspiro[3.3]heptan-6-yl]-2,7-dimethyl-7,8-dihydropyrimido[5,4-f][1,4]oxazepin-5-one;
(7R)-2-cyclopropyl-6-[2-(2-fluorophenyl)sulfonyl-2-azaspiro[3.3]heptan-6-yl]-7-methyl-7,8-dihydropyrimido[5,4-f][1,4]oxazepin-5-one;
(3R)-4-[2-(2-fluorophenyl)sulfonyl-2-azaspiro[3.3]heptan-6-yl]-3-methyl-8-(trifluoromethyl)-2,3-dihydropyrido[2,3-f][1,4]oxazepin-5-one; and
(3R)-4-[2-(2-fluorophenyl)sulfonyl-2-azaspiro[3.3]heptan-6-yl]-3-methyl-8-(trifluoromethyl)-2,3-dihydro-1H-pyrido[2,3-f][1,4]diazepin-5-one,
or a pharmacologically acceptable salt thereof.

26. The compound or the pharmacologically acceptable salt thereof according to claim 1, wherein the compound of Formula [I] is a compound represented by the following Formula [III]: 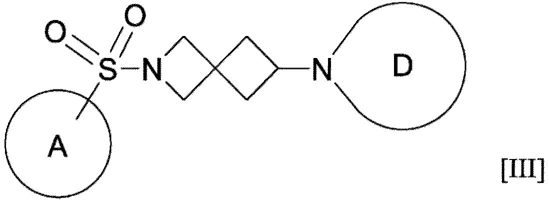 wherein,
a ring A represents an aryl group which may be substituted, a heteroaryl group which may be substituted, or an aliphatic heterocyclyl group which may be substituted, and
a ring D represents an aliphatic heterocyclyl group which may be substituted and may have a double bond in a part of a ring.

27. The pharmacologically acceptable salt thereof according to claim 26, wherein in the group represented by Formula [III],
each of substituents of the aryl group which may be substituted, the heteroaryl group which may be substituted, or the aliphatic heterocyclyl group which may be substituted represented by the ring A is one to three groups independently selected from the group consisting of a halogen atom, a cyano group, an alkyl group, an alkoxyalkyl group, a haloalkyl group, an alkoxy group, a haloalkoxy group, an alkanoyl group, an alkoxycarbonyl group, an amino group which may be substituted with one to two alkyl groups, an alkylthio group, a sulfonyl group which may be substituted with an alkyl group, an aminocarbonyl group which may be substituted with one to two alkyl groups, a nitro group, and aryl group,
an aryl moiety of the aryl group which may be substituted represented by the ring A is a 6- to 10-membered monocyclic or bicyclic aryl,
a heteroaryl moiety of the heteroaryl group which may be substituted represented by the ring A is a 5- to 10-membered monocyclic or bicyclic heteroaryl having 1 to 4 heteroatoms independently selected from the group consisting of oxygen, nitrogen, and sulfur,
an aliphatic heterocyclic moiety of the aliphatic heterocyclyl group which may be substituted represented by the ring A is a 5- to 10-membered monocyclic or bicyclic aliphatic heterocyclic ring having 1 to 3 heteroatoms independently selected from the group consisting of oxygen, nitrogen, and sulfur,
each of substituents of the aliphatic heterocyclyl group which may be substituted and may have a double bond in a part of a ring represented by the ring D is one to eight groups independently selected from the group consisting of an oxo group; a an alkyl group which may be substituted; a cycloalkyl group; an aryl group; a heteroaryl group; an aliphatic heterocyclyl group; an alkoxy group; a cycloalkoxy group; an alkylthio group; an amino group; an alkoxycarbonyl group; an alkanoyl group; an alkylsulfonyl group; a halogen atom; a hydroxyl group; and cyano group, and
an aliphatic heterocyclic moiety of the aliphatic heterocyclyl group which may be substituted and may have a double bond in a part of a ring represented by the ring D is a 5- to 7-membered monocyclic aliphatic heterocyclic ring having 1 to 3 heteroatoms independently selected from the group consisting of oxygen, nitrogen, and sulfur.

28. The compound or the pharmacologically acceptable salt thereof according to claim 26, wherein in the group represented by Formula [III],
the ring A is a phenyl group which may be substituted or a monocyclic heteroaryl group which may be substituted,
each of substituents of the phenyl group which may be substituted or the monocyclic heteroaryl group which may be substituted represented by the ring A is one to two groups independently selected from the group consisting of a halogen atom, an alkyl group, a haloalkyl group, an alkoxy group, and a nitro group,
a heteroaryl moiety of the monocyclic heteroaryl group which may be substituted represented by the ring A is a 5- or 6-membered monocyclic heteroaryl having 1 to 4 heteroatoms independently selected from the group consisting of oxygen, nitrogen, and sulfur,
each of substituents of the aliphatic heterocyclyl group which may be substituted and may have a double bond in a part of a ring represented by the ring D is one to eight groups independently selected from the group consisting of an oxo group; a an alkyl group which may be substituted; a cycloalkyl group; an aryl group; a heteroaryl group; an aliphatic heterocyclyl group; an alkoxy group; a cycloalkoxy group; an alkylthio group; an amino group; an alkoxycarbonyl group; an alkanoyl group; an alkylsulfonyl group; a halogen atom; a hydroxyl group; and cyano group,
in each of the substituents of the groups represented by the ring D, each of substituents of the alkyl group which may be substituted in the selected group is one to five groups independently selected from the group consisting of a cycloalkyl group which may be substituted with an oxo group, a haloalkyl group, or a halogen atom; an aryl group which may be substituted with an alkyl group or a halogen atom; a heteroaryl group which may be substituted with an alkyl group; an aliphatic heterocyclyl group which may be substituted with an oxo group; a halogen atom; a hydroxyl group; a cyano group; an alkoxy group; a cycloalkoxy group; a haloalkoxy group; an amino group; an alkylamino group; a dialkylamino group; a cycloalkylamino group; and alkylthio group, and
an aliphatic heterocyclic moiety of the aliphatic heterocyclyl group which may be substituted and may have a double bond in a part of a ring represented by the ring D is a 5- to 7-membered monocyclic aliphatic heterocyclic ring having 1 to 3 heteroatoms independently selected from the group consisting of oxygen, nitrogen, and sulfur.

29. The compound or the pharmacologically acceptable salt thereof according to claim 26, wherein in the group represented by Formula [III],
the ring A is a phenyl group which may be substituted or a monocyclic heteroaryl group which may be substituted,
each of substituents of the phenyl group which may be substituted or the monocyclic heteroaryl group which may be substituted represented by the ring A is one to two groups independently selected from the group consisting of a halogen atom, an alkyl group, a haloalkyl group, an alkoxy group, and a nitro group,
a heteroaryl moiety of the monocyclic heteroaryl group which may be substituted represented by the ring A is pyrrolyl, furanyl, thienyl, imidazolyl, pyrazolyl, oxazolyl, isoxazolyl, thiazolyl, isothiazolyl, triazolyl, tetrazolyl, pyridyl, pyrazinyl, pyrimidinyl, pyridazinyl, thiazinyl, or triazinyl,
each of substituents of the aliphatic heterocyclyl group which may be substituted and may have a double bond in a part of a ring represented by the ring D is one to eight groups independently selected from the group consisting of an oxo group; a an alkyl group which may be substituted; a cycloalkyl group; an aryl group; a heteroaryl group; an aliphatic heterocyclyl group; an alkoxy group; a cycloalkoxy group; an alkylthio group; an amino group; an alkoxycarbonyl group; an alkanoyl group; an alkylsulfonyl group; a halogen atom; a hydroxyl group; and cyano group,
in each of the substituents of the groups represented by the ring D, each of substituents of the alkyl group which may be substituted in the selected group is one to five groups independently selected from the group consisting of a cycloalkyl group which may be substituted with an oxo group, a haloalkyl group, or a halogen atom; an aryl group which may be substituted with an alkyl group or a halogen atom; a heteroaryl group which may be substituted with an alkyl group; a heterocyclyl group which may be substituted with an oxo group; a halogen atom; a hydroxyl group; a cyano group; an alkoxy group; a cycloalkoxy group; a haloalkoxy group; an amino group; an alkylamino group; a dialkylamino group; a cycloalkylamino group; and alkylthio group, and
an aliphatic heterocyclic moiety of the aliphatic heterocyclyl group which may be substituted and may have a double bond in a part of a ring represented by the ring D is azolidinyl, diazolidinyl, azinanyl, oxazinanyl, diazinanyl, thiazinanyl, azepanil, oxazepanil, diazepanil, or thiazepanil.

30. The compound or the pharmacologically acceptable salt thereof according to claim 1, wherein the compound of Formula [I] is a compound represented by the following Formula [IV]: 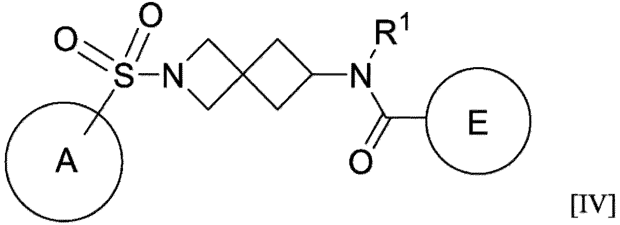 wherein,
a ring A represents an aryl group which may be substituted, a heteroaryl group which may be substituted, or an aliphatic heterocyclyl group which may be substituted,
R¹ represents an alkyl group which may be substituted, a cycloalkyl group which may be substituted, an alkenyl group which may be substituted, a cycloalkenyl group which may be substituted, an alkynyl group which may be substituted, an aryl group which may be substituted, a heteroaryl group which may be substituted, an aliphatic heterocyclyl group which may be substituted, an amino group which may be substituted, an alkoxy group which may be substituted, or an alkylthio group which may be substituted, and
a ring E represents an aryl group which may be substituted, a heteroaryl group which may be substituted, or an aliphatic heterocyclyl group which may be substituted and may have a double bond in a part of a ring.

31. The compound or the pharmacologically acceptable salt thereof according to claim 30, wherein in the group represented by Formula [IV],
each of substituents of the aryl group which may be substituted, the heteroaryl group which may be substituted, or the aliphatic heterocyclyl group which may be substituted represented by the ring A is one to three groups independently selected from the group consisting of a halogen atom, a cyano group, an alkyl group, an alkoxyalkyl group, a haloalkyl group, an alkoxy group, a haloalkoxy group, an alkanoyl group, an alkoxycarbonyl group, an amino group which may be substituted with one to two alkyl groups, an alkylthio group, a sulfonyl group which may be substituted with an alkyl group, an aminocarbonyl group which may be substituted with one to two alkyl groups, a nitro group, and aryl group,
an aryl moiety of the aryl group which may be substituted represented by the ring A is a 6- to 10-membered monocyclic or bicyclic aryl,
a heteroaryl moiety of the heteroaryl group which may be substituted represented by the ring A is a 5- to 10-membered monocyclic or bicyclic heteroaryl having 1 to 4 heteroatoms independently selected from the group consisting of oxygen, nitrogen, and sulfur,
an aliphatic heterocyclic moiety of the aliphatic heterocyclyl group which may be substituted represented by the ring A is a 5- to 10-membered monocyclic or bicyclic aliphatic heterocyclic ring having 1 to 3 heteroatoms independently selected from the group consisting of oxygen, nitrogen, and sulfur,
R¹ is an alkyl group which may be substituted, a cycloalkyl group which may be substituted, an alkenyl group which may be substituted, a cycloalkenyl group which may be substituted, or an alkynyl group which may be substituted,
each of substituents of the alkyl group which may be substituted, the alkenyl group which may be substituted, and the alkynyl group which may be substituted in the group represented by R¹ is a cycloalkyl group or a cycloalkenyl group,
each of substituents of the cycloalkyl group which may be substituted, and the cycloalkenyl group which may be substituted is an alkyl group, an alkenyl group or an alkynyl group,
an aryl moiety of the aryl group which may be substituted represented by the ring E is a 6- to 10-membered monocyclic or bicyclic aryl,
a heteroaryl moiety of the heteroaryl group which may be substituted represented by the ring E is a 5- to 10-membered monocyclic or bicyclic heteroaryl having 1 to 4 heteroatoms independently selected from the group consisting of oxygen, nitrogen, and sulfur,
an aliphatic heterocyclic moiety of the aliphatic heterocyclyl group which may be substituted and may have a double bond in a part of a ring represented by the ring E is a 5- to 10-membered monocyclic or bicyclic aliphatic heterocyclic ring having 1 to 3 heteroatoms independently selected from the group consisting of oxygen, nitrogen, and sulfur, and
each of substituents of the aryl group which may be substituted, the heteroaryl group which may be substituted, or the aliphatic heterocyclyl group which may be substituted and may have a double bond in a part of a ring represented by the ring E is one to three groups independently selected from the group consisting of an alkyl group; a cycloalkyl group; an aryl group; a heteroaryl group; a halogen atom; a haloalkyl group; a hydroxyl group; a cyano group; an alkoxy group; a cycloalkoxy group; an aryloxy group; a haloalkoxy group; an amino group; an alkylamino group; a dialkylamino group; a cycloalkylamino group; and an alkylthio group.

32. The compound or the pharmacologically acceptable salt thereof according to claim 30, wherein in the group represented by Formula [IV],
the ring A is a phenyl group which may be substituted or a monocyclic heteroaryl group which may be substituted,
each of substituents of the phenyl group which may be substituted or the monocyclic heteroaryl group which may be substituted represented by the ring A is one to two groups independently selected from the group consisting of a halogen atom, an alkyl group, a haloalkyl group, an alkoxy group, a nitro group, and aryl group,
a heteroaryl moiety of the monocyclic heteroaryl group which may be substituted represented by the ring A is a 5- to 6-membered monocyclic heteroaryl having 1 to 4 heteroatoms independently selected from the group consisting of oxygen, nitrogen, and sulfur,
R¹ is an alkyl group which may be substituted, a cycloalkyl group which may be substituted, an alkenyl group which may be substituted, a cycloalkenyl group which may be substituted, or an alkynyl group which may be substituted,
each of substituents of the alkyl group which may be substituted, the alkenyl group which may be substituted, and the alkynyl group which may be substituted in the group represented by R¹ is a cycloalkyl group or a cycloalkenyl group,
each of substituents of the cycloalkyl group which may be substituted, and the cycloalkenyl group which may be substituted in the group represented by R¹ is an alkyl group, an alkenyl group or an alkynyl group,
the ring E is an aryl group which may be substituted, or a heteroaryl group which may be substituted,
an aryl moiety of the aryl group which may be substituted represented by the ring E is a 6- to 10-membered monocyclic or bicyclic aryl,
a heteroaryl moiety of the heteroaryl group which may be substituted represented by the ring E is a 5- to 10-membered monocyclic or bicyclic heteroaryl having 1 to 4 heteroatoms independently selected from the group consisting of oxygen, nitrogen, and sulfur, and
each of substituents of the aryl group which may be substituted, and the heteroaryl group which may be substituted represented by the ring E is one to three groups independently selected from the group consisting of an alkyl group; a cycloalkyl group; an aryl group; a heteroaryl group; a halogen atom; a haloalkyl group; a hydroxyl group; a cyano group; an alkoxy group; a cycloalkoxy group; an aryloxy group; a haloalkoxy group; an amino group; an alkylamino group; a dialkylamino group; a cycloalkylamino group; and an alkylthio group.

33. The compound or the pharmacologically acceptable salt thereof according to claim 30, wherein in the group represented by Formula [IV],
the ring A is a phenyl group which may be substituted or a monocyclic heteroaryl group which may be substituted,
each of substituents of the phenyl group which may be substituted or the monocyclic heteroaryl group which may be substituted represented by the ring A is one to two groups independently selected from the group consisting of a halogen atom, an alkyl group, a haloalkyl group, an alkoxy group, a nitro group, and an aryl group,
a heteroaryl moiety of the monocyclic heteroaryl group which may be substituted represented by the ring A is pyrrolyl, furanyl, thienyl, imidazolyl, pyrazolyl, oxazolyl, isoxazolyl, thiazolyl, isothiazolyl, triazolyl, tetrazolyl, pyridyl, pyrazinyl, pyrimidinyl, pyridazinyl, thiazinyl, or triazinyl,
R¹ is an alkyl group,
the ring E is an aryl group which may be substituted, or a heteroaryl group which may be substituted,
an aryl moiety of the aryl group which may be substituted represented by the ring E is phenyl or naphthyl,
a heteroaryl moiety of the heteroaryl group which may be substituted represented by the ring E is pyridyl, azaindolyl, imidazopyridyl or benzoimidazolyl, and
each of substituents of the aryl group which may be substituted, and the heteroaryl group which may be substituted represented by the ring E is a group independently selected from the group consisting of an alkyl group; a cycloalkyl group; an aryl group; a heteroaryl group; a halogen atom; a haloalkyl group; a hydroxyl group; a cyano group; an alkoxy group; a cycloalkoxy group; an aryloxy group; a haloalkoxy group; an amino group; an alkylamino group; a dialkylamino group; a cycloalkylamino group; and an alkylthio group.

34. A pharmaceutical composition comprising the compound or the pharmacologically acceptable salt thereof according to any one of claims 1 to 33 as an active ingredient.

35. The pharmaceutical composition according to claim 34, wherein the pharmaceutical composition is used for preventing or treating various diseases and/or symptoms associated therewith that are ameliorated by increasing a central TRH concentration, or for improving prognosis of these diseases and/or symptoms associated therewith.

36. The pharmaceutical composition according to claim 35, wherein the pharmaceutical composition is an agent for preventing or treating spinocerebellar degeneration, chronic pain, a sleep disorder, or other psychoneurotic diseases and/or symptoms associated therewith.

37. The pharmaceutical composition according to claim 36, wherein the chronic pain is neuropathic pain or nociceptive pain.

38. The pharmaceutical composition according to claim 37, wherein the neuropathic pain is pain associated with diabetic neuropathy, postherpetic neuralgia, pain associated with peripheral neuropathy due to chemotherapy, trigeminal neuralgia, complex regional pain syndrome, post-stroke pain, spinal cord injury pain, neuralgia, or pain associated with nerve damage.

39. The pharmaceutical composition according to claim 37, wherein the nociceptive pain is pain associated with rheumatoid arthritis, pain associated with osteoarthritis deformans, postoperative pain, or myofascial pain.

40. The pharmaceutical composition according to claim 36, wherein the sleep disorder is hypersomnia, a circadian rhythm disorder, sleep-disordered breathing, or other sleep disorders.

41. The pharmaceutical composition according to claim 40, wherein the hypersomnia is narcolepsy, idiopathic hypersomnia, or repetitive hypersomnia.

42. The pharmaceutical composition according to claim 40, wherein the circadian rhythm disorder is a sleep disorder due to shift work, delayed sleep phase syndrome, advanced sleep phase syndrome, non-24-hour sleep-wake syndrome, or an irregular sleep-wake pattern.

43. The pharmaceutical composition according to claim 40, wherein the sleep-disordered breathing is sleep apnea syndrome.

44. The pharmaceutical composition according to claim 40, wherein the other sleep disorders are jet lag syndrome, desynchronosis syndrome, restless legs syndrome, a periodic limb movement disorder, hypersomnia associated with insomnia associated with a neurological disorder or a mental disorder, or a rapid eye movement (REM) sleep behavior disorder.

45. The pharmaceutical composition according to claim 36, wherein the other psychoneurotic diseases and/or the symptoms associated therewith are spinal muscular atrophy, amyotrophic lateral sclerosis, Parkinson's disease, Alzheimer's disease, chronic fatigue syndrome, vascular dementia, a bipolar disorder, depression, a spinal cord injury, cerebral infarction, head trauma, a pervasive consciousness disorder, a language disorder, fatigability, dysfunction due to nerve damage, or dysfunction due to surgery.

46. A treatment method for various diseases and/or symptoms associated therewith that are ameliorated by increasing a central TRH concentration, the treatment method comprising administering a therapeutically effective amount of the compound or the pharmacologically acceptable salt thereof according to any one of claims 1 to 33 to a patient.

47. The treatment method according to claim 46, wherein the various diseases and/or the symptoms associated therewith that are ameliorated by increasing a central TRH concentration are spinocerebellar degeneration, pain, a sleep disorder, or other psychoneurotic diseases and/or symptoms associated therewith.
